(19) Europäisches Patentamt
European Patent Office
Office européen des brevets

(11) Publication number: **0 393 502 B1**

(12) # EUROPEAN PATENT SPECIFICATION

(45) Date of publication of patent specification: **27.12.95**

(51) Int. Cl.[6]: **C12N 15/12**, C07K 14/705, C12N 1/21, A61K 39/395

(21) Application number: **90106992.2**

(22) Date of filing: **11.04.90**

(54) **Soluble interferon-gamma receptors and methods for their production**

(30) Priority: **19.04.89 EP 89810295**

(43) Date of publication of application:
**24.10.90 Bulletin 90/43**

(45) Publication of the grant of the patent:
**27.12.95 Bulletin 95/52**

(84) Designated Contracting States:
**AT BE CH DE DK FR GB IT LI LU NL SE**

(56) References cited:
**EP-A- 0 240 975**

**PROCEEDINGS OF THE NATIONAL ACADEMY OF SCIENCES OF THE USA, vol. 85, April 1988, pages 2357-2361; E.A. BERGER et al.: "A soluble recombinant polypeptide comprising the amino-terminal half of the extracellular region of the CD4 molecule contains an active binding site for human immunodeficiency virus"**

**CELL, vol. 55, 21st October 1988, pp. 273-280, Cell Press, Cambridge, Massachusetts, US; M. AGUET et al.: "Molecular cloning and expression of the human interferon-gamma receptor"**

(73) Proprietor: **F. HOFFMANN-LA ROCHE AG**
**Postfach 3255**
**CH-4002 Basel (CH)**

(72) Inventor: **Fountoulakis, Michael**
**4 Melchior Berri-Strasse**
**CH-4142 Münchenstein (CH)**
Inventor: **Garotta, Gianni**
**121 Stockackerstrasse**
**CH-4153 Reinach (CH)**
Inventor: **Stüber, Dietrich**
**9 Bandweg**
**D-7889 Grenzach-Wyhlen (DE)**

(74) Representative: **Mezger, Wolfgang, Dr. et al**
**Grenzacherstrasse 124**
**Postfach 3255**
**CH-4002 Basel (CH)**

EP 0 393 502 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

The present invention relates to soluble interferon-gamma receptors comprising the extracellular domain of the natural interferon-gamma receptor capable of specifically binding interferon-gamma (IFN-γ) and missing the cytoplasmic and transmembrane domains of the natural IFN-γ receptor, to DNA sequences coding for these soluble IFN-γ receptors, to expression vectors and transformants expressing these soluble IFN-γ receptors and methods for making these using recombinant DNA technology. In addition, the present invention relates to the use of these soluble IFN-γ receptors as therapeutically active agents for the treatment of diseases, especially for the treatment of autoimmune diseases, chronic inflammations, delayed hypersensitivities and allotransplant rejections and multiple sclerosis.

As has been shown with other polypeptide hormones and lymphokines, interferons (IFNs) act on various cells via specific cell surface receptors. IFN-α and IFN-β share a common receptor which is distinct from the IFN-γ receptor (Branca and Baglioni, Nature 294, 768-770 [1981]). The existence of a specific receptor for IFN-γ on various cells was demonstrated by cross-linking experiments, by binding of radiolabeled IFN-γ and by specific competition with unlabeled IFN-γ. Cross-linked complexes between IFN-γ and its receptor with a $M_r$ ranging from 70,000 to 165,000 have been described (Rubinstein et al., Immunol. Rev. 97, 29-50 [1987]). Despite this apparent heterogeneity, most binding studies revealed only one class of IFN-γ binding sites with a dissociation constant of about $10^{-11}$ to $10^{-10}$M (Sarkar and Gupta, Proc. Natl. Acad. Sci. USA 81, 5160-5164 [1984], Uecer et al., Cancer Res. 46, 5339-5343 [1986], Novick et al., J. Biol. Chem. 262, 8483-8487 [1987]). Human IFN-γ receptors have been purified from Raji cells by affinity chromatography on immobilized recombinant human IFN-γ (Aguet and Merlin, J. Exp. Med. 165, 988-999 [1987], Calderon et al., Proc. Natl. Acad. Sci. USA 85, 4837-4841 [1988]). A major protein species of apparent $M_r$ of 90 Kd (p90) was highly enriched and shown to bind human IFN-γ specifically. However, an additional 50 Kd (p50) component of human IFN-γ receptor might be detected. Some evidence suggested that the intact IFN-γ receptor is indeed the p90 protein and that p50 may be a proteolytic degradation product of p90 (Aguet and Merlin, supra, Sheehan et al., J. Immunol. 140, 4231-4237 [1988]).

In general, IFN-γ receptors seem to be expressed to a lesser extend on normal cells (up to $10^3$ sites per cell) than on tumor cells. Thus, some human carcinoma and B-cell lines were reported to express on the order of $10^4$ binding sites per cell (Uecer et al., supra, Aguet and Merlin, supra).

In conclusion the human IFN-γ receptor is an ubiquitous membrane anchored protein of about 90 Kd molecular mass.

EP-A-240 975 discloses the isolation of natural human interferon gamma receptor protein from various cell types by ligand affinity chromatography. The receptors were identified as proteins of 90-140 Kd molecular mass, i.e. comprising the extracellular, cytoplasmic and transmembrane domains (full length receptor proteins).

Molecular cloning and expression of the human IFN-γ receptor has been described (Aguet et al., Cell 55, 273-280 [1988]).

However, because the natural IFN-γ receptor as well as the recombinant IFN-γ receptor are membrane anchored proteins, they have the disadvantage that they are insoluble in physiological solution. Consequently, search or design of IFN-γ agonists and antagonists, and administration of purified IFN-γ receptor to mammals to inhibit IFN-γ binding to its specific receptor thereby preventing, suppressing and/or modulating the course of autoimmune disorders, chronic inflammations, delayed hypersensitivities and allotransplant rejections was difficult to accomplish.

To solve the foregoing problem, the soluble IFN-γ receptors of the present invention have been produced by culturing transformants carrying an expression vector containing a DNA sequence coding for a soluble IFN-γ receptor and isolating said soluble IFN-γ receptor.

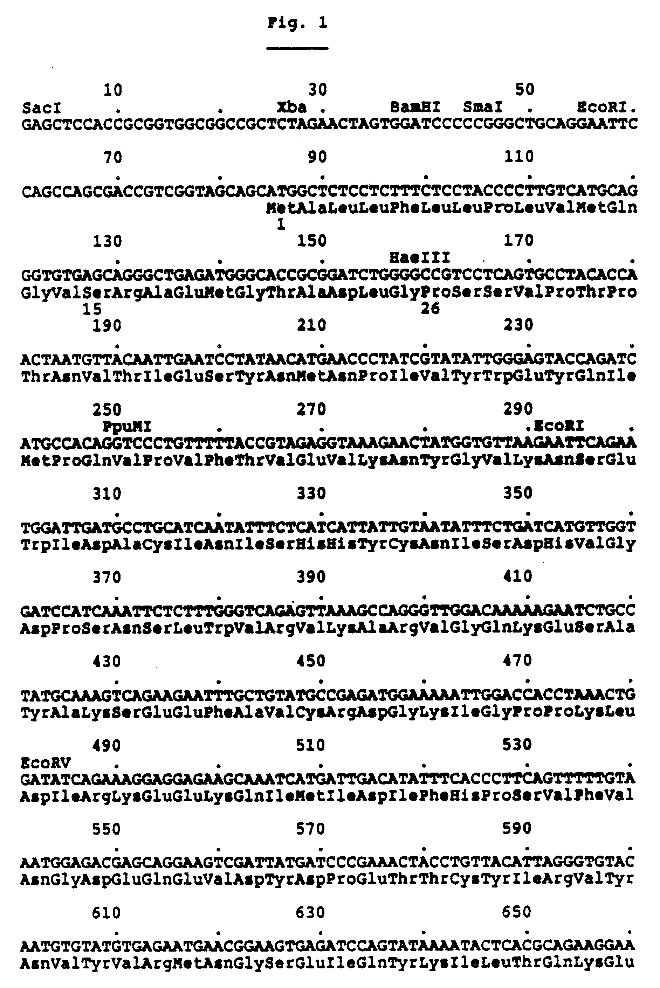

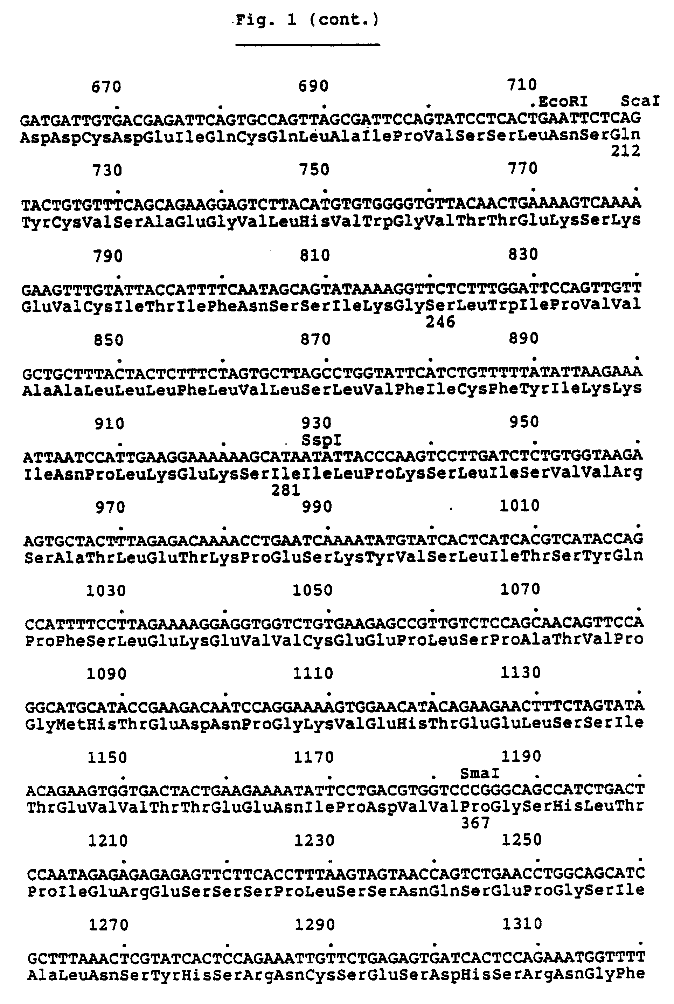

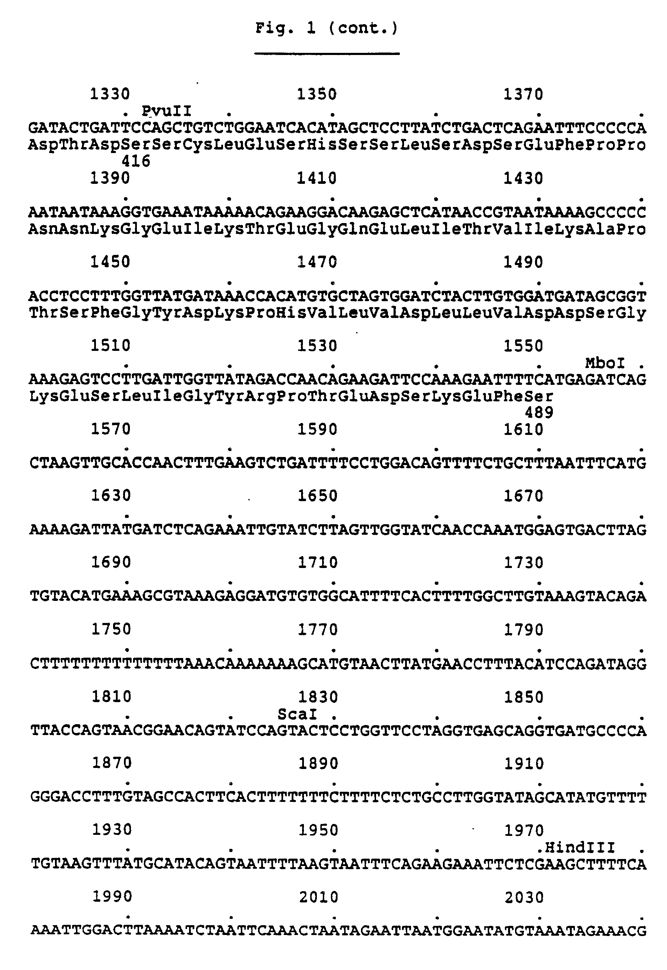

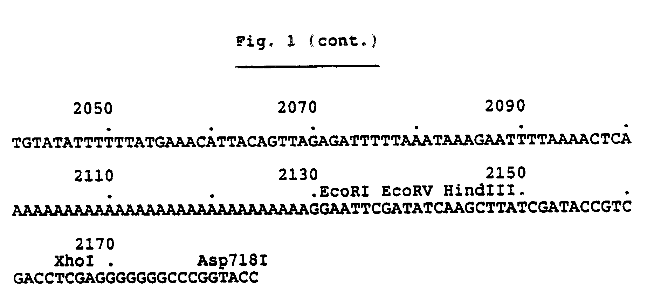

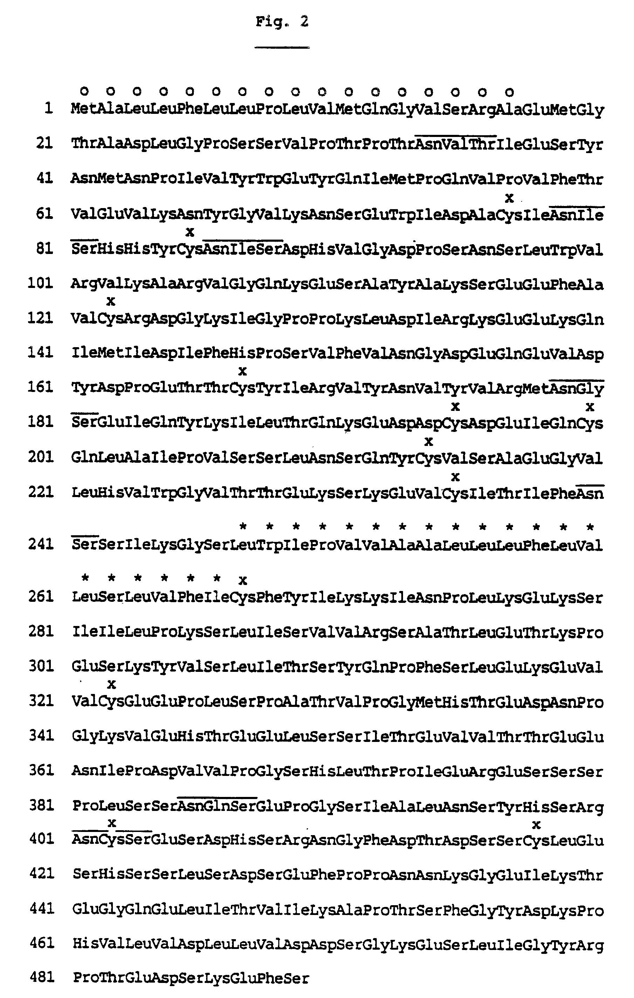

More precisely, the present invention provides soluble IFN-γ receptors capable of specifically binding IFN-γ comprising the N-terminal portion of the natural IFN-γ receptor containing at least amino acids 26-246 of the natural IFN-γ receptor sequence shown in Figure 1 or 2 and missing the cytoplasmic and transmembrane domains of the natural IFN-γ receptor.

The term "Soluble IFN-γ receptors" as used herein refers to IFN-γ receptors which are soluble in physiological solutions without addition of detergents.

The term "cytoplasmic and transmembrane domains of the natural IFN-γ receptor" as used in describing the soluble IFN-γ receptors of the present invention refers to amino acids 247-489 of the natural IFN-γ receptor sequence shown in Fig. 1 or 2.

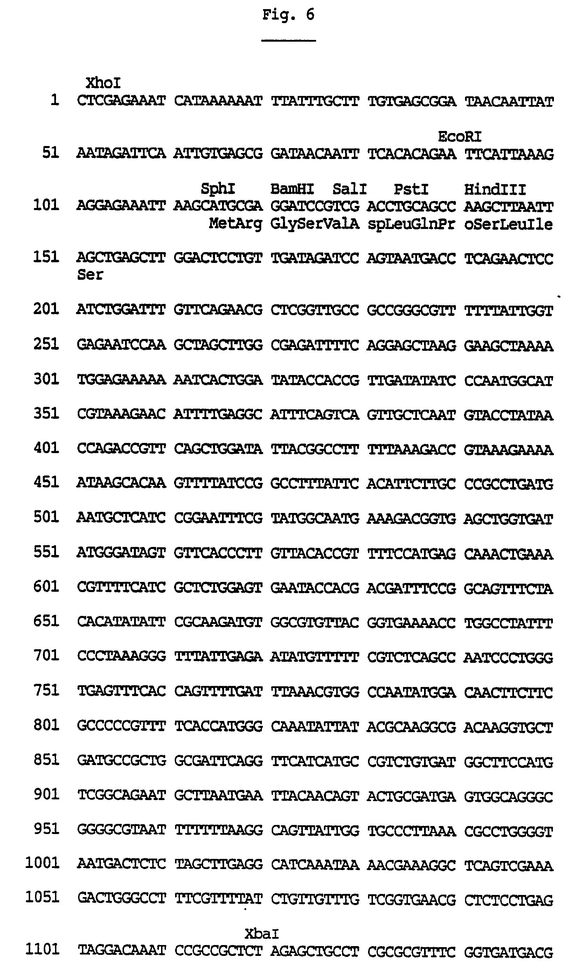

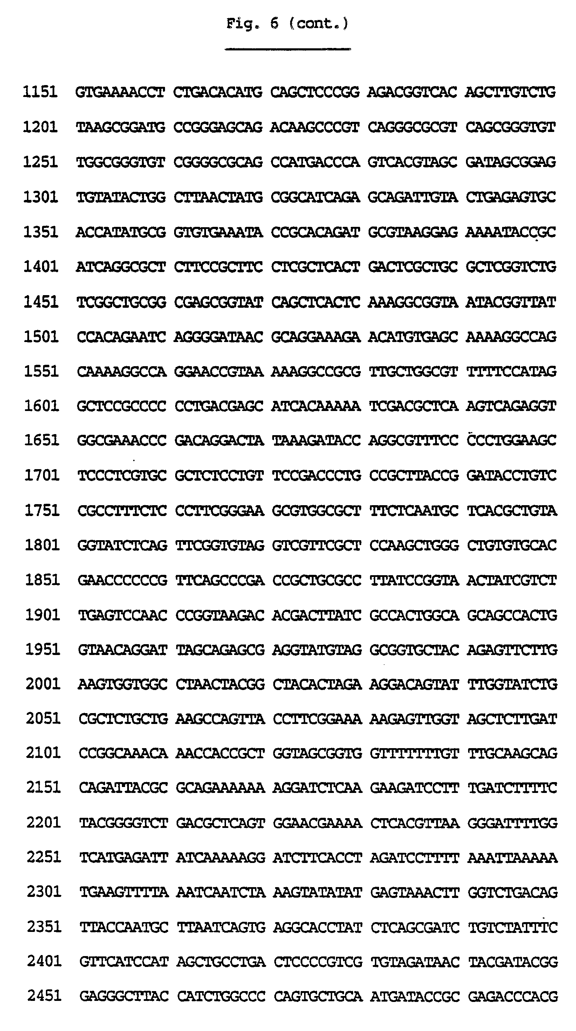

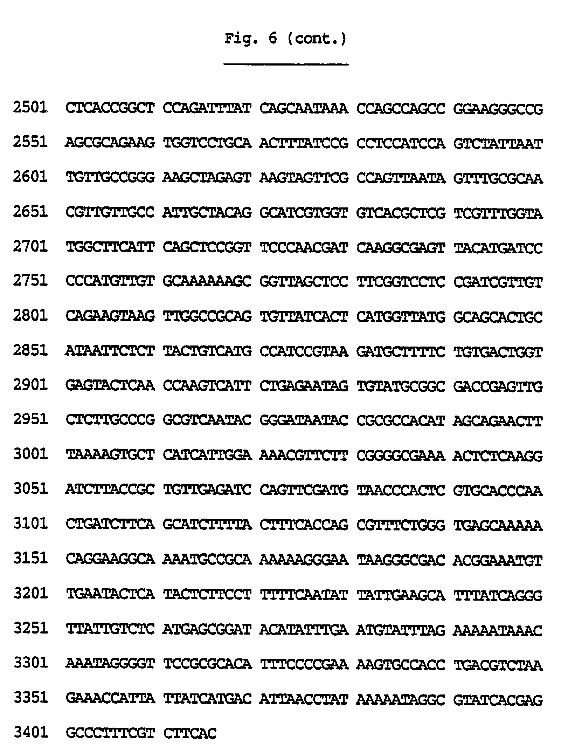

The soluble IFN-γ receptors of the present invention may contain amino acid substitutions if such substitutions do not generally alter the IFN-γ binding activity and solubility. Amino acid substitutions in proteins and polypeptides which do not essentially alter biological activity are known in the art and described, e.g. by H. Neurath and R.L. Hill in "The Proteins", Academic Press, New York (1979), in particular in Fig. 6 of page 14. The most frequently observed amino acid substitutions are Ala/Ser, Val/Ile, Asp/Glu, Thr/Ser, Ala/Gly, Ala/Thr, Ser/Asn, Ala/Val, Ser/Gly, Tyr/Phe, Ala/Pro, Lys/Arg, Asp/Asn, Leu/Ile, Leu/Val, Ala/Glu, Asp/Gly and vice versa. The soluble IFN-γ receptors of the present invention can additionally contain sequences of several amino acids which are coded for by "linker" sequences. These sequences arise as a result from the expression vectors used for expression of the soluble IFN-γ receptors as defined above.

The soluble IFN-γ receptors of the present invention can also contain specific sequences that preferably bind to an affinity carrier material. Examples of such sequences are sequences containing at least two adjacent histidine residues (see in this respect European Patent Application Publication No. 282 042). Such sequences bind selectively to nitrilotriacetic acid nickel chelate resins (Hochuli and Döbeli, Biol. Chem. Hoppe-Seyler 368, 748 (1987); European Patent Application Publication No. 253 303). Soluble IFN-γ receptors which contain such a specific sequence can, therefore, be separated selectively from the remaining polypeptides. The specific sequence can be linked either to the C-terminus or the N-terminus of the soluble IFN-γ receptor amino acid sequences.

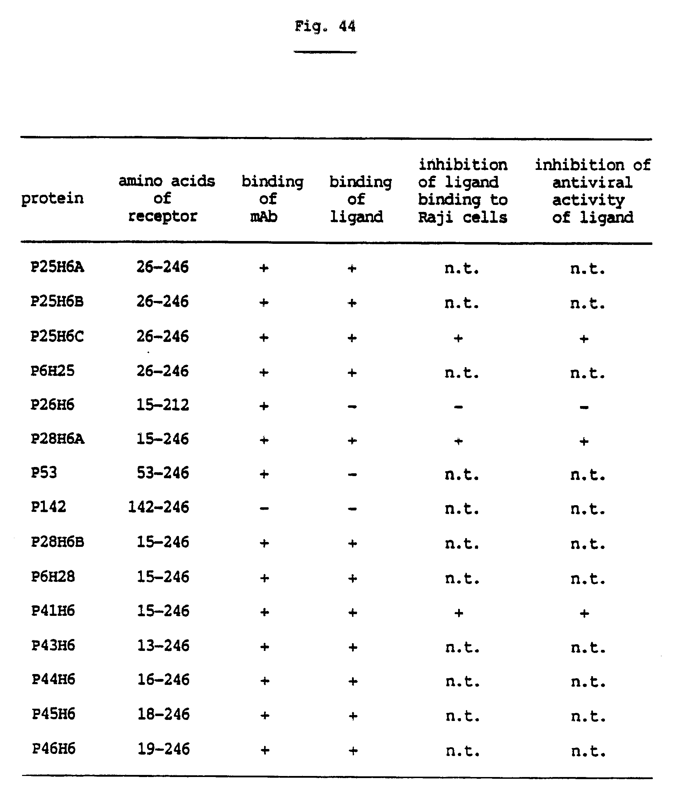

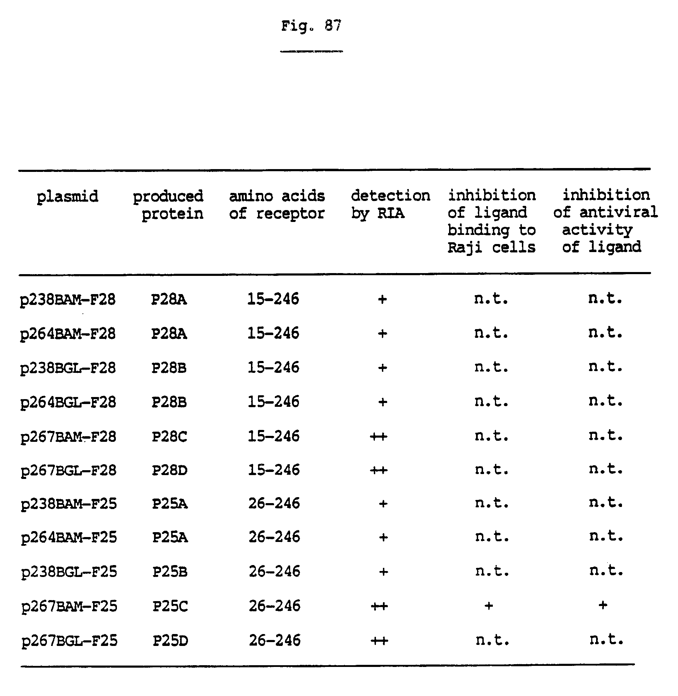

Preferred soluble IFN-γ receptors of the present invention are those with the designations P25H6C, P28H6A, P41H6, P25C and P57 (see e.g. Figs. 44 and 87 and Table 1).

The present invention also provides DNA sequences which code for the soluble IFN-γ receptors of the present invention, expression vectors which contain these DNA sequences, host cells containing such vectors for the production of the soluble IFN-γ receptors and processes for the production of such DNA sequences, recombinant vectors and host cells. Methods for the expression, isolation and purification of the soluble IFN-γ receptors are also described.

Because the complete DNA sequence of the gene coding for the natural IFN-γ receptor is known (Aguet et al., supra), amino acid sequences coding for the soluble IFN-γ receptors of the present invention can be chemically synthesized using standard methods known in the art, preferably solid state methods, such as the methods of Merrifield (J. Am. Chem. Soc. 85, 2149-2154 [1963]). Alternatively, the soluble IFN-γ receptors of the present invention can be produced using methods of DNA recombinant technology (Maniatis et al. in "Molecular Cloning - A Laboratory Manual", Cold Spring Harbor Laboratory [1982]). Preferably, a DNA sequence coding for a soluble IFN-γ receptor comprising the extracellular domain of the natural IFN-γ receptor and missing the cytoplasmic and transmembrane domains of the natural IFN-γ receptor is prepared from DNA coding for the natural IFN-γ receptor and subsequently incorporated into a suitable expression vector which produces the requisite expression signals.

Expression vectors suitable for use in prokaryotic host cells are mentioned, for example, in the aforementioned textbook of Maniatis et al. Especially suitable vectors are plasmids of the pDS family [Bujard et al., Methods in Enzymology, eds. Wu and Grossmann, Academic Press, Inc., Vol. 155, 416-433 (1987)].

Such prokaryotic expression vectors which contain the DNA sequences coding for the soluble IFN-γ receptors of the present invention operatively linked with an expression control sequence can be incorporated using conventional methods into any suitable prokaryotic host cell. The selection of a suitable prokaryotic host cell is determined by different factors which are well-known in the art. Thus, for example, compatibility with the chosen vector, toxicity of the expression product, expression characteristics, necessary biological safety precautions and costs play a role and a compromise between all of these factors must be found.

Suitable prokaryotic host organisms include gram-negative and gram-positive bacteria, for example E.coli and B.subtilis strains. Especially preferred prokaryotic host organisms of the present invention are E.coli strain M15 (described as strain OZ 291 by Villarejo et al. in J. Bacteriol. 120, 466-474 [1974] and E.coli W3110 [ATCC No. 27325]). In addition to the aforementioned E.coli strain, however, other generally accessible E.coli strains such as E.coli 294 (ATCC No. 31446) and E.coli RR1 (ATCC No. 31343) can also be used.

Expression vectors suitable for use in mammalian host cells include but are not limited to pBC12MI, pBC12BI, pSV2dhFr, p91023(B), pcDV1, pRSVcat, pGA291, pGA293, pGA296, pBC12/HIV/IL-2 and pGA300. Vectors pBC12BI and pBC12/HIV/IL-2 are preferred. Such vectors are preferably introduced into suitable mammalian host cells by transfection.

Mammalian host cells that could be used include e.g. human Hela, H9 and Jurkat cells, mouse NIH3T3 and C127 cells, CV1 African green monkey kidney cells, quail QC1-3 cells, Chinese hamster ovary (CHO) cells, mouse L cells and the COS cell lines. The COS-1 cell line is preferred.

The manner in which the expression of the soluble IFN-γ receptors of the present invention is carried out depends on the chosen expression vector/host cell system.

Usually, the prokaryotic host organisms which contain a desired expression vector are grown under conditions which are optimal for the growth of the prokaryotic host organisms. At the end of the exponential growth, when the increase in cell number per unit time decreases, the expression of the desired soluble IFN-$\gamma$ receptor is induced, i.e. the DNA coding for the desired soluble IFN-$\gamma$ receptor is transcribed and the transcribed mRNA is translated. The induction can be carried out by adding an inducer or a derepressor to the growth medium or by altering a physical paramter, e.g. a change in temperature. In the expression vectors used in the preferred embodiments of the present invention, the expression is controlled by the lac repressor. By adding isopropyl-$\beta$-D-thiogalactopyranoside (IPTG), the expression control sequence is derepressed and the synthesis of the desired soluble IFN-$\gamma$ receptor is thereby induced.

The mammalian host cells which contain a desired expression vector are grown under conditions which are optimal for the growth of the mammalian host cells. A typical expression vector contains the promoter element, which mediates the transcription of mRNA, the protein coding sequence, and the signals required for efficient termination and polyadenylation of the transcript. Additional elements may include enhancers and intervening sequences bounded by spliced donor and acceptor sites.

Most of the vectors used for the transient expression of a given coding sequence carry the SV40 origin of replication, which allows them to replicate to high copy numbers in cells (e.g. COS cells) that constitutively express the T antigen required to initiate viral DNA synthesis. Transient expression is not limited to COS cells. Any mammalian cell line that can be transfected can be utilized for this purpose. Elements that control a high efficient transcription include the early or the late promoters from SV40 and the long terminal repeats (LTRs) from retroviruses, e.g. RSV, HIV, HTLVI. However, also cellular signals can be used (e.g. human-$\beta$-actin-promoter).

Alternatively stable cell lines carrying a gene of interest integrated into the chromosome can be selected upon co-transfection with a selectable marker such as gpt, dhfr, neomycin or hygromycin.

Now, the transfected gene can be amplified to express large quantities of a foreign protein. The dihydrofolate reductase (DHFR) is a useful marker to develop lines of cells carrying more than 1000 copies of the gene of interest. The mammalian cells are grown in increasing amounts of methotrexate. Subsequently, when the methotrexate is withdrawn, cell lines contain the amplified gene integrated into the chromosome. In the expression vectors used in the preferred embodiments of the present invention, the expression is controlled by the Rous sarcoma virus LTR.

The baculovirus-insect cell vector system can also be used for the production of the soluble IFN-$\gamma$ receptors of the present invention (for review see Luclow and Summers, Bio/Technology 6, 47-55 [1988]). The soluble IFN-$\gamma$ receptors produced in insect cells infected with recombinant baculovirus can undergo post-translational processing including N-glycosylation (Smith et al., Proc. Nat. Acad. Sci. USA 82, 8404-8408) and O-glycosylation (Thomsen et al., 12. International Herpesvirus Workshop, University of Philadelphia, Pennsylvania).

For the isolation of small amounts of soluble IFN-$\gamma$ receptors expressed in prokaryotic host cells for analytical purposes, e.g. for polyacrylamide gel electrophoresis, the host cells can be disrupted by treatment with a detergent, e.g. sodium dodecyl sulphate (SDS). Larger quantities of these soluble IFN-$\gamma$ receptors can be obtained by mechanical [Charm et al., Meth. Enzymol. 22, 476-556 (1971)], enzymatic (lysozyme treatment) or chemical (detergent treatment, urea or guanidinium hydrochloride treatment, etc.) treatments followed by use of known methods, e.g. by centrifugation at different gravities, precipitation with ammonium sulphate, dialysis (at normal pressure or at reduced pressure), preparative isoelectric focusing, preparative gel electrophoresis or by various chromatographic methods such as gel filtration, high performance liquid chromatography (HPLC), ion exchange chromatography, reverse phase chromatography and affinity chromatography (e.g. on Sepharose$^R$ Blue C1-6B or on carrier-bound monoconal antibodies which are directed against the native IFN-$\gamma$ receptor and the soluble IFN-$\gamma$ receptors of the present invention).

Preferably, the IFN-$\gamma$ receptors expressed in prokaryotic host cells are obtained after the following steps:

(I) Disruption of the cells and extraction of the proteins with guanidine-hydrochhloride;

(II) Ni-chelate affinity chromatography using a nitrolotriacetic acid (NTA) resin as described in European Patent Applications Publication Nos. 282 042 and 253 303;

(III) Chromatography on a sizing column in the presence of urea;

(IV) Dialysis (refolding); and

(V) Chromatography on an anion exchanger column.

The soluble IFN-$\gamma$ receptors expressed in mammalian host cells or in the baculovirus-insect cell vector system can be isolated from the host cell medium using standard protein purification methods.

The soluble IFN-$\gamma$ receptors can be used for the preparations of pharmaceutical compositions and for the treatment of autoimmune diseases, e.g. type 1 diabetes or lupus erythematosus or rheumatoid arthritis, chronic inflammations, e.g. Schwartzman Reaction, delayed hypersensitivity allotransplant rejections and multiple sclerosis.

They may be administered in pharmaceutically acceptable oral, injectable or topical compositions and modes. Dosage and dose rate may parallel that currently being used in clinical applications of the known IFNs, lymphokines like IFN-$\alpha$ typically about 8-53 $\mu g/m^2$ (1.8-10x$10^6$ U/$m^2$) daily or 3 days per week. The pharmaceutical compositions of the present invention contain soluble IFN-$\gamma$ receptors in association with a compatible pharmaceutically acceptable carrier material. Any conventional carrier material can be utilized. The carrier material can be an organic or inorganic one suitable for enteral, percutaneous or parenteral administration. Suitable carriers include water, gelatin, gum arabic, lactose, starch, magnesium stearate, talc, vegetable oils, polyalkylene-glycols, petroleum jelly and the like. Furthermore, the pharmaceutical preparations may contain other pharmaceutically active agents. Additional additives such as flavouring agents, preservatives, stabilizers, emulsifying agents, buffers and the like may be added in accordance with accepted practices of pharmaceutical compounding.

The pharmaceutical preparations can be made up in any conventional form including: a) a solid form for oral administration such as tablets, capsules, pills, powders, granules and the like; b) a liquid form for oral administration such as solutions, syrups, suspensions, elixirs and the like; c) preparations for parenteral administration such as sterile solutions, suspensions or emulsions; and d) preparations for topical administrations such as solutions, suspensions, ointments, creams, gels, micronized powders, aerosols and the like. The pharmaceutical preparations may be sterilized and/or may contain adjuvants such as preservatives, stabilizers, wetting agents, emulsifiers, salts for varying the osmotic pressure and/or buffers.

Parenteral dosage forms may be infusions or injectable solutions which can be injected intravenously or intramuscularly. These preparations can also contain other medicinally active substances. Additional additives such as preservatives, stabilizers, emulsifying agents, buffers and the like may be added in accordance with accepted practices of pharmaceutical compounding.

Furthermore, antibodies can be raised against the soluble IFN-$\gamma$ receptors of the present invention. These antibodies can be used in a well-known manner for diagnostic or therapeutic purposes as well as for purification purposes. Such antibodies can be produced by injecting a mammalian or avian animal with a sufficient amount of a vaccine formulation comprising a soluble IFN-$\gamma$ receptor of the present invention and a compatible pharmaceutical carrier to elicit the production of antibodies against said receptor. The appropriate amount of the soluble IFN-$\gamma$ receptor which would be required would be known to one of skill in the art or could be determined by routine experimentation. As used in connection with this invention, the term "pharmaceutical carrier" can mean either the standard compositions which are suitable for human administration or the typical adjuvants employed in animal vaccinations.

Suitable adjuvants for the vaccination of animals include but are not limited to Freund's complete or incomplete adjuvant (not suitable for human or livestock use). Adjuvant 65 (containing peanut oil, mannide monooleate and aluminum phosphate and alum; surfactants such as hexadecylamine, octadecylamine, lysolecithin, dimethyldioctadecylammonium bromide, $N_1$-N-dioctadecyl-N'-N-bis(2-hydroxyethylpropanediamine), methoxyhexadecylglycerol, and pluronic polyols; polyanions such as pyran, dextran sulfate, poly IC, polyacrylic acid, carbopol; peptides such as muramyl dipeptide, dimentylglycine, tuftsin; and oil emulsions. The soluble IFN-$\gamma$ receptors could also be administered following incorporation into liposomes or other microcarriers, or after conjugation to polysaccharides, other proteins or other polymers or in combination with Quil-A to form "Iscoms" (immunostimulating complexes) (Morein et al., Nature 308, 457 [1984]).

Typically, the initial vaccination is followed some weeks later by one or more "booster" vaccinations, the net effect of which is the production of high titers of antibodies against the soluble IFN-$\gamma$ receptors which can be harvested in the usual way.

Another method consists in using the well-known Koehler and Milstein technique for producing monoclonal antibodies. In order to find out different monoclonal antibodies which are directed against the same antigen but against different epitopes, the method of Stähli et al. [J. of Immunological Methods 32, 297-304 (1980)] can be used. Finally, the soluble IFN-$\gamma$ receptors of the present invention are useful in screening methods for identifying IFN-$\gamma$ agonists or antagonists.

Having now generally described this invention, the same will become better understood by reference to the specific examples, which are included herein for purpose of illustration only and are not intended to be limiting unless otherwise specified, in connection with the following figures:

The following abbreviations and symbols used are: B, Bg, Bs, E, H, N, Nh, P, Pv, S, Sa, Sm, Sp, X and Xb which indicate cleavage sites for restriction enzymes BamHI, BglII, BstEII, EcoRI, HindIII, NcoI, NheI, PstI, PvuI, SalI, SacI, SmaI, SphI, XhoI and XbaI, respectively.

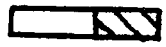

represents the regulatable promoter/operator element N25OPSN25OP29;

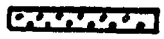

represents ribosomal binding sites RBSII, RBSII,NcoI and RBSII,SphI; → represents coding regions under control of these ribosomal binding sites and open reading frames in plasmids replicating in COS-cells;

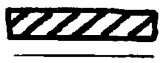

represents a region encoding six histidine residues;

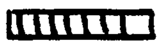

represents terminators $t_o$ and T1; ⇆ represents the region required for DNA replication in E.coli (repl.);

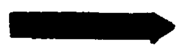

represents coding regions for dihydrofolate reductase (dhfr), chloramphenicol acetyltransferase (cat), $\beta$-lactamase (bla), lac repressor (lac1), neomycin phosphotransferase (neo); the tetracyclin resistance protein (tet', interrupted) and rat preproinsulin;

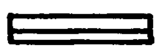

represents the long terminal repeat (LTR) of Rous sarcoma virus;

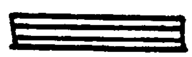

represents a region of the SV40 virus required for replication in COS-cells (ori); and ▭ represents signal peptides S.P.1 and S.P.2, the 5'-intron (5') and 3'-intron (3') of the rat preproinsulin gene, the polyadenylation signal of the same gene (polyA) and a region encoding three translational stop codons (stop).

Figure 1 displays the nucleotide sequence of the SacI/Asp718I fragment of plasmid pBABLUE with the coding region for the human IFN-$\gamma$ receptor and the amino acid sequence of the human IFN-$\gamma$ receptor predicted therefrom. In the nucleotide sequence, the recognition sites for several restriction enzymes are indicated. In the amino acid sequence, the numbers refer to the position of the amino acid with respect to the methionine at position 1.

Figure 2 displays the amino acid sequence of the human IFN-$\gamma$ receptor as predicted from the nucleotide sequence displayed in Fig. 1. In this sequence the putative signal peptide is indicated by a series of circles, whereas the transmembrane region is indicated by a series of stars. Potential N-linked glycosylation sites are overlined and cysteine residues are marked by a cross.

Figure 3 is a schematic drawing of the plasmid pDS56/RBSII,NcoI.

Figure 4 displays the complete nucleotide sequence of plasmid pDS56/RBSII,NcoI. In this sequence, the recognition sequences of the restriction enzymes depicted in Fig. 3 are indicated. The amino acid sequence shown represents the open reading frame under control of ribosomal binding site RBSII,NcoI.

Figure 5 is a schematic drawing of the plasmid pDS56/RBSII,SphI.

Figure 6 displays the complete nucleotide sequence of plasmid pDS56/RBSII,SphI. In this sequence, the recognition sequences of the restriction enzymes depicted in Fig. 5 are indicated. The amino acid sequence shown represents the open reading frame under control of ribosomal binding site RBSII,SphI.

Figure 7 is a schematic drawing of the plasmid pDS56/RBSII,6xHis.

Figure 8 displays the complete nucleotide sequence of plasmid pDS56/RBSII,6xHis. In this sequence, the recognition sequences of the restriction enzymes depicted in Fig. 7 are indicated. The amino acid sequence shown represents the open reading frame under control of ribosomal binding site RBSII.

Figure 9 is a schematic drawing of the plasmid pDS56/RBSII-6xHis.

Figure 10 displays the complete nucleotide sequence of plasmid pDS56/RBSII-6xHis. In this sequence, the recognition sequences of the restriction enzymes depicted in Fig. 9 are indicated. The amino acid sequence shown represents the open reading frame under control of ribosomal binding site RBSII.

Figure 11 is a schematic drawing of the plasmid pDS781/RBSII,6xHis.

Figure 12 displays the complete nucleotide sequence of plasmid pDS781/RBSII,6xHis. In this sequence, the recognition sequences of the restriction enzymes depicted in Fig. 11 are indicated. The amino acid sequence shown represents the open reading frame under control of ribosomal binding site RBSII.

Figure 13 is a schematic drawing of the plasmid pDHFR-(0/-1)-6xHis.

Figure 14 displays the complete nucleotide sequence of plasmid pDHFR-(0/-1)-6xHis. In this sequence, the recognition sequences of the restriction enzymes depicted in Fig. 13 are indicated. The amino acid sequence shown represents the open reading frame under control of ribosomal binding site RBSII.

Figure 15 is a schematic drawing of the plasmid pDMI,1.

Figure 16 displays the complete nucleotide sequence of plasmid pDMI,1. In this sequence, the recognition sequences of the restriction enzymes depicted in Fig. 15 are indicated. The amino acids shown enclose the open reading frames encoding the neomycin phosphotransferase (Met,Phe) and the lac repressor (Met,Gln; in brackets to indicate the reverse orientation of the gene).

Figure 17 displays the nucleotide sequences of the linkers, primers and adaptors used in the construction of plasmids. Recognition sequences for BamHI and BglII are indicated, cohesive ends produced upon cleavage with certain restriction enzymes are labeled.

Figure 18 displays the nucleotide sequence of DNA fragment F7 and the amino acid sequence predicted therefrom. In the nucleotide sequence, the recognition sites for several restriction enzymes are indicated. The amino acid sequence comprises as indicated amino acids 1 to 212 of the human IFN-$\gamma$ receptor.

Figure 19 displays the nucleotide sequence of DNA fragment F26 and the amino acid sequence predicted therefrom. In the nucleotide sequence, the recognition sites for several restriction enzymes are indicated. The amino acid sequence comprises as indicated amino acids 15 to 212 of the human IFN-$\gamma$ receptor.

Figure 20 displays the amino acid sequence of protein P26H6 comprising as indicated amino acids 15 to 212 of the 17man IFN-$\gamma$ receptor.

Figure 21 displays the nucleotide sequence of DNA fragment F12 and the amino acid sequence predicted therefrom. In the nucleotide sequence, the recognition sites for several restriction enzymes are indicated. The amino acid sequence comprises as indicated amino acids 26 to 416 of the human IFN-$\gamma$ receptor.

Figure 22 displays the nucleotide sequence of DNA fragment F27 and the amino acid sequence predicted therefrom. In the nucleotide sequence, the recognition sites for several restriction enzymes are indicated. The amino acid sequence comprises as indicated amino acids 26 to 416 of the human IFN-$\gamma$ receptor.

Figure 23 displays the nucleotide sequence of DNA fragment F25 and the amino acid sequence predicted therefrom. In the nucleotide sequence, the recognition sites for several restriction enzymes are indicated. The amino acid sequence comprises as indicated amino acids 26 to 246 of the human IFN-$\gamma$ receptor.

Figure 24 displays the amino acid sequence of protein P25H6A comprising as indicated amino acids 26 to 246 of the human IFN-$\gamma$ receptor.

Figure 25 displays the amino acid sequence of protein P25H6B comprising as indicated amino acids 26 to 246 of the human IFN-$\gamma$ receptor.

Figure 26 displays the amino acid sequence of protein P25H6C comprising as indicated amino acids 26 to 246 of the human IFN-$\gamma$ receptor.

Figure 27 displays the amino acid sequence of protein P6H25 comprising as indicated amino acids 26 to 246 of the human IFN-γ receptor.

Figure 28 displays the amino acid sequence of protein P28H6A comprising as indicated amino acids 15 to 246 of the human IFN-γ receptor.

Figure 29 displays the nucleotide sequence of DNA fragment F28 and the amino acid sequence predicted therefrom. In the nucleotide sequence, the recognition sites for several restriction enzymes are indicated. The amino acid sequence comprises as indicated amino acids 15 to 246 of the human IFN-γ receptor.

Figure 30 displays the amino acid sequence of protein P28H6B comprising as indicated amino acids 15 to 246 of the human IFN-γ receptor.

Figure 31 displays the amino acid sequence of protein P6H28 comprising as indicated amino acids 15 to 246 of the human IFN-γ receptor.

Figure 32 displays the nucleotide sequence of DNA fragment F41 and the amino acid sequence predicted therefrom. In the nucleotide sequence, the recognition sites for several restriction enzymes are indicated and the nucleotides are given in which fragment F28 differs from fragment F41. The amino acid sequence comprises as indicated amino acids 15 to 246 of the human IFN-γ receptor.

Figure 33 displays the amino acid sequence of protein P41H6 comprising as indicated amino acids 15 to 246 of the human IFN-γ receptor.

Figure 34 displays the nucleotide sequence of DNA fragment F43 and the amino acid sequence predicted therefrom. In the nucleotide sequence, the recognition sites for several restriction enzymes are indicated. The amino acid sequence comprises as indicated amino acids 13 to 246 of the human IFN-γ receptor.

Figure 35 displays the amino acid sequence of protein P43H6 comprising as indicated amino acids 13 to 246 of the human IFN-γ receptor.

Figure 36 displays the nucleotide sequence of DNA fragment F44 and the amino acid sequence predicted therefrom. In the nucleotide sequence, the recognition sites for several restriction enzymes are indicated. The amino acid sequence comprises as indicated amino acids 16 to 246 of the human IFN-γ receptor.

Figure 37 displays the amino acid sequence of protein P44H6 comprising as indicated amino acids 16 to 246 of the human IFN-γ receptor.

Figure 38 displays the nucleotide sequence of DNA fragment F45 and the amino acid sequence predicted therefrom. In the nucleotide sequence, the recognition sites for several restriction enzymes are indicated. The amino acid sequence comprises as indicated amino acids 18 to 246 of the human IFN-γ receptor.

Figure 39 displays the amino acid sequence of protein P45H6 comprising as indicated amino acids 18 to 246 of the human IFN-γ receptor.

Figure 40 displays the nucleotide sequence of DNA fragment F46 and the amino acid sequence predicted therefrom. In the nucleotide sequence, the recognition sites for several restriction enzymes are indicated. The amino acid sequence comprises as indicated amino acids 19 to 246 of the human IFN-γ receptor.

Figure 41 displays the amino acid sequence of protein P46H6 comprising as indicated amino acids 18 to 246 of the human IFN-γ receptor.

Figure 42 is a summary of the plasmids used for production of receptor derivatives in E.coli, of the proteins encoded by these plasmids and of the regions of the human IFN-γ receptor contained in these proteins.

Figure 43 is a schematic summary of the amino acid sequences of the protease expressed in E.coli. Stretches of amino acids of the human IFN-γ receptor (Receptor) present in these proteins are represented by stars.

Figure 44 is a summary of activities of the receptor derivatives expressed in E.coli. Monoclonal antibody and human IFN-γ receptor are abbreviated by mAb and ligand, respectively, whereas n.t. indicates, that this activity of the very protein has not been tested for.

Figure 45 is a summary of the purification of protein P41H6.

Figure 46 is a schematic drawing of the plasmid p79BGL.

Figure 47 displays the complete nucleotide sequence of plasmid p79BGL. In the sequence, the recognition sequences of the restriction enzymes depicted in Fig. 46 are indicated. The star at position 279 marks the first nucleotide of the transcripts initiated within the LTR, a polyadenylation signal is overlined. A short open reading frame starting at position 505 of the sequence is represented by the encoded amino acids. Three translational stop codons to the right of the unique cleavage site for restriction enzyme BamHI are overlined.

Figure 48 is a schematic drawing of the plasmid p79DBAM.

Figure 49 displays the complete nucleotide sequence of plasmid p79DBAM. In this sequence, the recognition sequences of the restriction enzymes depicted in Fig. 48 are indicated. The star at position 279 marks the first nucleotide of the transcripts initiated within the LTR. Three translation stop codons to the right of the unique cleavage site for restriction enzyme BamHI and a polyadenylation signal are overlined.

Figure 50 is a schematic drawing of the plasmid p238BGL.

Figure 51 displays the complete nucleotide sequence of plasmid p238BGL. In this sequence, the recognition sequences of the restriction enzymes depicted in Fig. 50 are indicated. The star at position 279 marks the first nucleotide of the transcripts initiated within the LTR. Three translational stop codons to the right of the cleavage site for restriction enzyme BglII and a polyadenylation signal are over lined. In the amino acid sequence shown, signal peptide S.P.1 is indicated by circles.

Figure 52 is a schematic drawing of the plasmid p238BAM.

Figure 53 displays the complete nucleotide sequence of plasmid p238BAM. In this sequence, the recognition sequences of the restriction enzymes depicted in Fig. 52 are indicated. The star at position 279 marks the first nucleotide of the transcripts initiated within the LTR. Three translational stop codons to the right of the unique cleavage site for restriction enzyme BamHI and a polyadenylation signal are overlined. In the amino acid sequence shown, signal peptide S.P.1 is indicated by circles.

Figure 54 is a schematic drawing of the plasmid p264BGL.

Figure 55 displays the complete nucleotide sequence of plasmid p264BGL. In this sequence, the recognition sequences of the restriction enzymes depicted in Fig. 54 are indicated. The star at position 279 marks the first nucleotide of the transcripts initiated within the LTR. Three translational stop codons to the right of the unique cleavage site for restriction enzyme BglII and a polyadenylation signal are overlined. In the amino acid sequence shown, signal peptide S.P.1 is indicated by circles.

Figure 56 is a schematic drawing of the plasmid p264BAM.

Figure 57 displays the complete nucleotide sequence of plasmid p264BAM. In this sequence, the recognition sequences of the restriction enzymes depicted in Fig. 56 are indicated. The star at position 279 marks the first nucleotide of the transcripts initiated within the LTR. Three translational stop codons to the right of the unique cleavage site for restriction enzyme BamHI and a polyadenylation signal are overlined. In the amino acid sequence shown, signal peptide S.P.1 is indicated by circles.

Figure 58 is a schematic drawing of the plasmid p267BGL.

Figure 59 displays the complete nucleotide sequence of plasmid p267BGL. In this sequence, the recognition sequences of the restriction enzymes depicted in Fig. 58 are indicated. The star at position 279 marks the first nucleotide of the transcripts initiated within the LTR. Three translational stop codons to the right of the unique cleavage site for restriction enzyme BglII and a polyadenylation signal are overlined. In the amino acid sequence shown, signal peptide S.P.2 is indicated by circles.

Figure 60 is a schematic drawing of the plasmid p267BAM.

Figure 61 displays the complete nucleotide sequence of plasmid p267BAM. In the sequence, the recognition sequences of the restriction enzymes depicted in Fig. 60 are indicated. The star at position 279 marks the first nucleotide of the transcripts initiated within the LTR. Three tranlational stop codons to the right of the unique cleavage site for restriction enzyme BamHI and a polyadenylation signal are overlined. In the amino acid sequence shown, signal peptide S.P.2 is indicated by circles.

Figure 62 displays the nucleotide sequence of DNA fragment F1 and the amino acid sequence predicted therefrom. In the nucleotide sequence, the recognition sites for several restriction enzymes are indicated. The amino acid sequence comprises as indicated amino acids 1 to 489 of the human IFN-$\gamma$ receptor.

Figure 63 displays the nucleotide sequence of DNA fragment F3 and the amino acid sequence predicted therefrom. In the nucleotide sequence, the recognition sites for several restriction enzymes are indicated. The amino acid sequence comprises as indicated amino acids 1 to 367 of the human IFN-$\gamma$ receptor.

Figure 64 displays the nucleotide sequence of DNA fragment F30 and the amino acid sequence predicted therefrom. In the nucleotide sequence, the recognition sites for several restriction enzymes are indicated. The amino acid sequence comprises as indicated amino acids 1 to 281 of the human IFN-$\gamma$ receptor.

Figure 65 displays the nucleotide sequence of DNA fragment F31 and the amino acid sequence predicted therefrom. In the nuecleotide sequence, the recognition sites for several restriction enzymes are indicated. The amino acid sequence comprises as indicated amino acids 1 to 489 of the human IFN-$\gamma$ receptor.

Figure 66 displays the amino acid sequence of protein PF1 comprising the entire human IFN-γ receptor.

Figure 67 displays the amino acid sequence of protein PF3 comprising amino acids 1 to 367 of the human IFN-γ receptor.

Figure 68 displays the amino acid sequence of protein PBGLF12 comprising as indicated amino acids 26 to 416 of the human IFN-γ receptor and signal peptide S.P.2, which is indicated by circles.

Figure 69 displays the amino acid sequence of protein PF30 comprising as indicated amino acids 1 to 281 of the human IFN-γ receptor.

Figure 70 displays the amino acid sequence of protein PF12F1 comprising as indicated amino acids 26 to 489 of the human IFN-γ receptor and signal peptide S.P.2, which is indicated by circles.

Figure 71 displays the amino acid sequence of protein PF12F12 comprising as indicated amino acids 26 to 416 of the human IFN-γ receptor and signal peptide S.P.2, which is indicated by circles.

Figure 72 displays the amino acid sequence of protein PF12F3 comprising as indicated amino acids 26 to 367 of the human IFN-γ receptor and signal peptide S.P.2, which is indicated by circles.

Figure 73 displays the amino acid sequence of protein PF12F30 comprising as indicated amino acids 26 to 281 of the human IFN-γ receptor and signal peptide S.P.2 which is indicated by circles.

Figure 74 displays the amino acid sequence of protein P28A comprising as indicated amino acids 15 to 246 of the human IFN-γ receptor and signal peptide S.P.1, which is indicated by circles.

Figure 75 displays the amino acid sequence of protein P28B comprising as indicated amino acids 15 to 246 of the human IFN-γ receptor and signal peptide S.P.1, which is indicated by circles.

Figure 76 displays the amino acid sequence of protein P28C comprising as indicated amino acids 15 to 246 of the human IFN-γ receptor and signal peptide S.P.2, which is indicated by circles.

Figure 77 displays the amino acid sequence of protein P28D comprising as indicated amino acids 15 to 246 of the human IFN-γ receptor and signal peptide S.P.2, which is indicated by circles.

Figure 78 displays the amino acid sequence of protein P25A comprising as indicated amino acids 26 to 246 of the human IFN-γ receptor and signal peptide S.P.1, which is indicated by circles.

Figure 79 displays the amino acid sequence of protein P25B comprising as indicated amino acids 26 to 246 of the human IFN-γ receptor and signal peptide S.P.1, which is indicated by circles.

Figure 80 displays the amino acid sequence of protein P25C comprising as indicated amino acids 26 to 246 of the human IFN-γ receptor and signal peptide S.P.2, which is indicated by circles.

Figure 81 displays the amino acid sequence of protein P25D comprising as indicated amino acids 26 to 246 of the human IFN-γ receptor and signal peptide S.P.2, which is indicated by circles.

Figure 82 is a summary of the plasmids used for production of cell-bound receptor derivatives in COS-1 cells, of the proteins encoded by these plasmids, of the regions of the human IFN-γ receptor contained in these proteins and of the signal peptides directing these proteins to the surface of the cells. Human IFN-γ receptor is abbreviated by receptor.

Figure 83 is a schematic summary of the amino acid sequences of the cell-bound proteins expressed in COS-1 cells. Stretches of amino acids of the human IFN-γ receptor (Receptor) present in these proteins are represented by stars, signal peptides S.P.2 is indicated by circles.

Figure 84 is a summary of the capacity to bind IFN-γ (ligand) shown by the cell-bound Receptors produced in COS-1 cells. The symbol + + indicates that transfected cells bind 3-5 fold more IFN-γ than mock transfected cells.

Figure 85 is a summary of the plasmids used for the production in COS-1 cells of secreted proteins, of the proteins encoded by these plasmids, of the regions of the human IFN-γ receptor contained in these proteins and of the nature of the signal peptides directing the secretion of these proteins.

Figure 86 is a schematic summary of the amino acid sequences of the proteins secreted upon production in COS-1 cells. Strechtes of amino acids of the human IFN-γ receptor present in these proteins are represented by stars, signal peptide S.P.1 is indicated by crosses and signal peptide S.P.2 is indicated by circles.

Figure 87 is a summary of activities of the proteins secreted upon production in COS-1 cells. Radioimmune assay and human IFN-γ are abbreviated by RIA and ligand, respectively, whereas n.t. indicates, that this activity of the very protein has not been tested for. The symbol + + indicates that about 50 fold more of the proteins secreted were bound by both cold and radiolabelled mAb.

Unless otherwise specified, percentages given below for solids in solid mixtures, liquids in liquids and solids in liquids are on a wt/wt, vol/vol and wt/vol basis, respectively.

Example 1

Description of the source of the DNA used for the expression of the soluble human IFN-γ receptors

Several cDNAs encoding the human IFN-γ receptor have been described (Aguet et al., supra). One of these cDNAs was cloned into the EcoRI restriction endonuclease cleavage site of the λZAP vector (Strategene). Following the circularization procedure provided by Stratagene, plasmid pBABLUE was obtained, in which the gene for the human IFN-γ receptor is flanked by several restriction endonuclease cleavage sites. Plasmid pBABLUE was the source of the DNA for the expression of the soluble human IFN-γ receptors. The nucleotide sequence of the relevant part of this plasmid is shown together with the deduced amino acid sequence of the human IFN-γ receptor in Fig. 1. For convenience, the amino acid sequence of the human IFN-γ receptor with its relevant characteristics is also shown in Fig. 2.

Example 2

Description of plasmids used for the cloning of human IFN-γ receptor encoding DNA fragments and expression in E.coli of the soluble human IFN-γ receptors

A. Principles

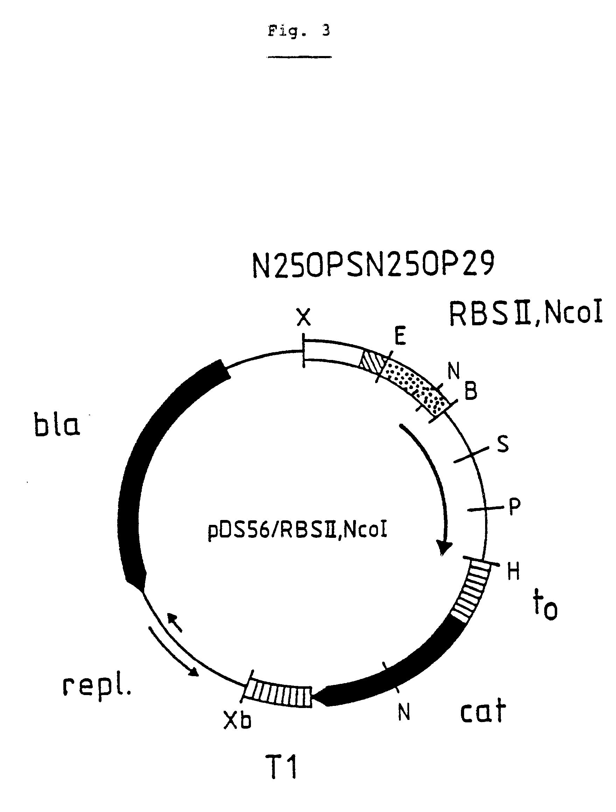

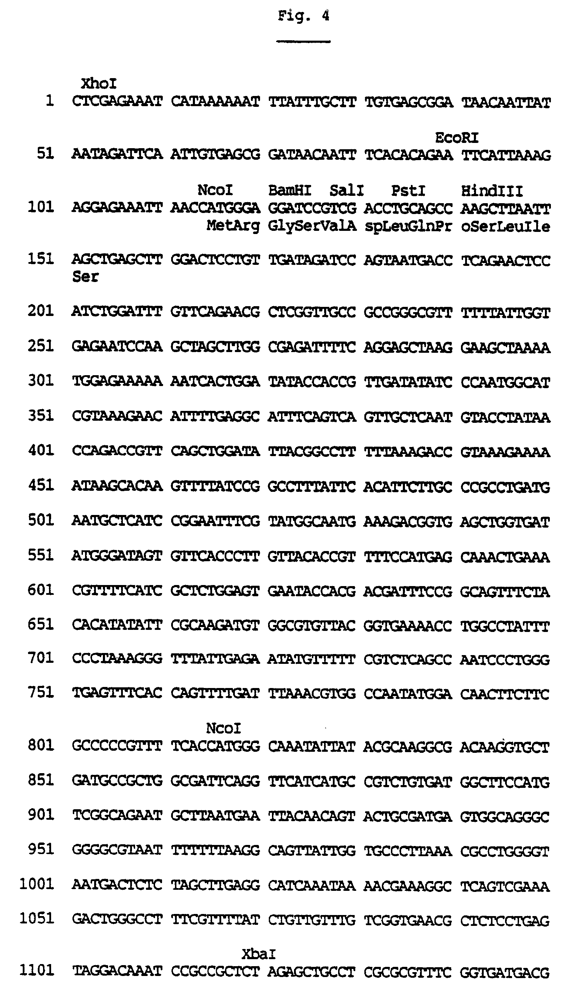

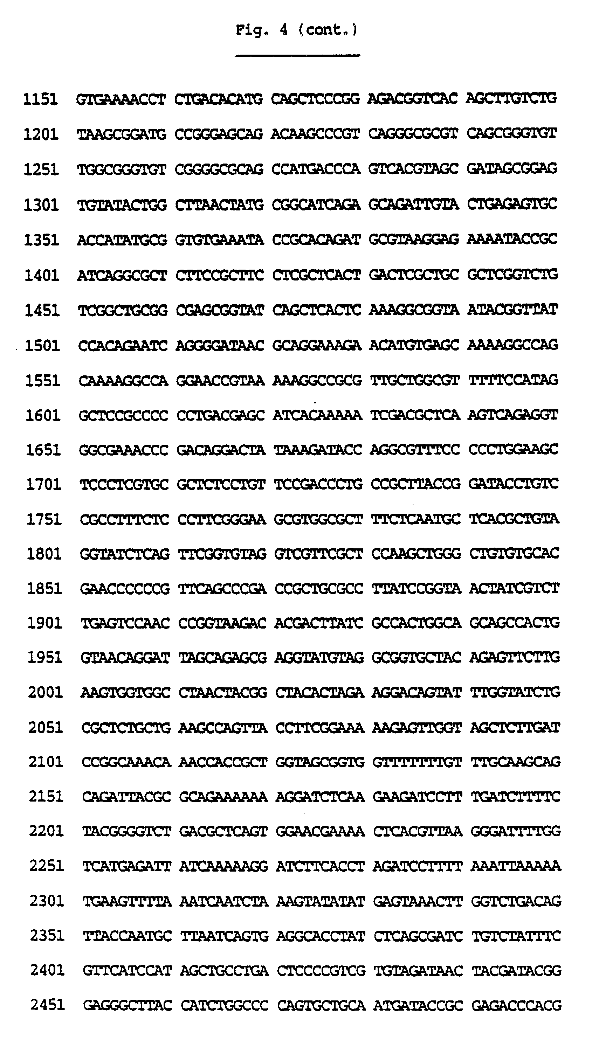

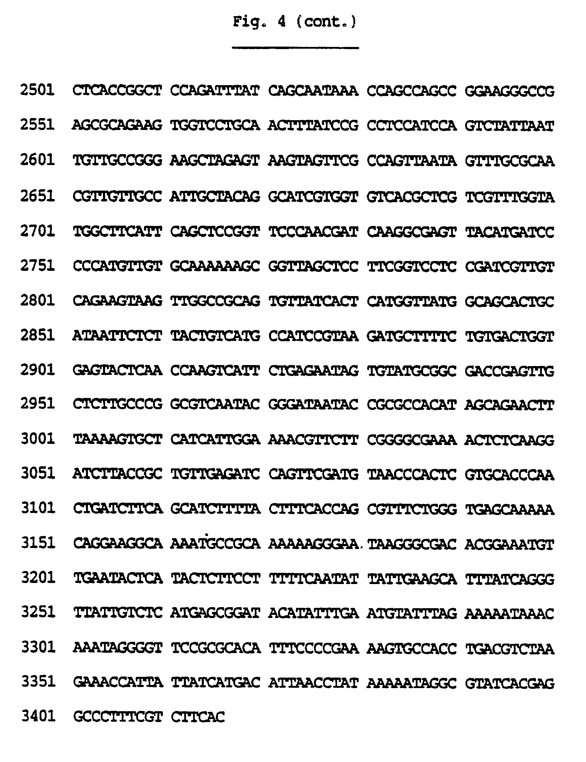

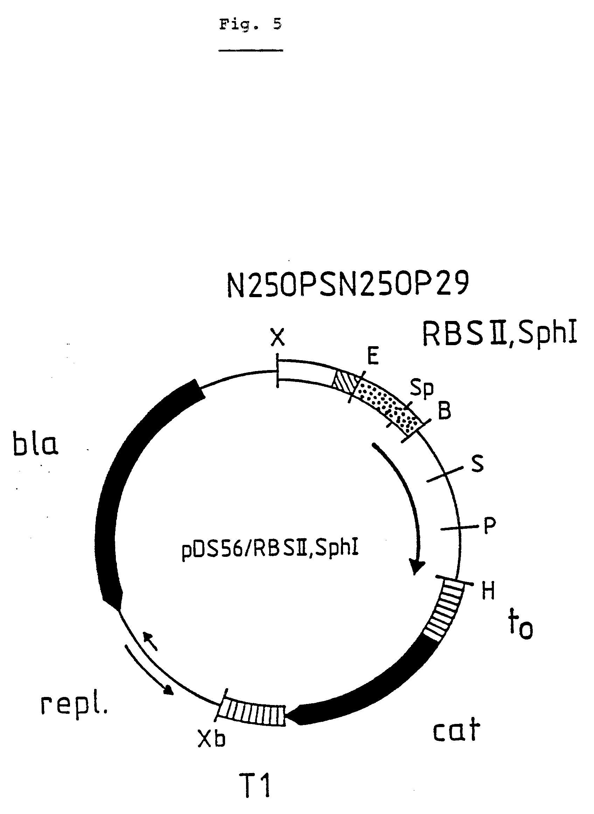

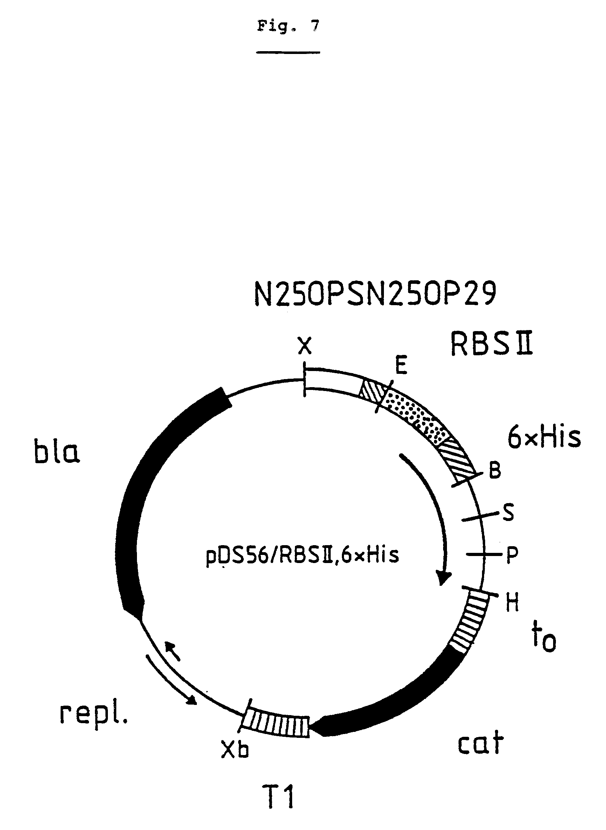

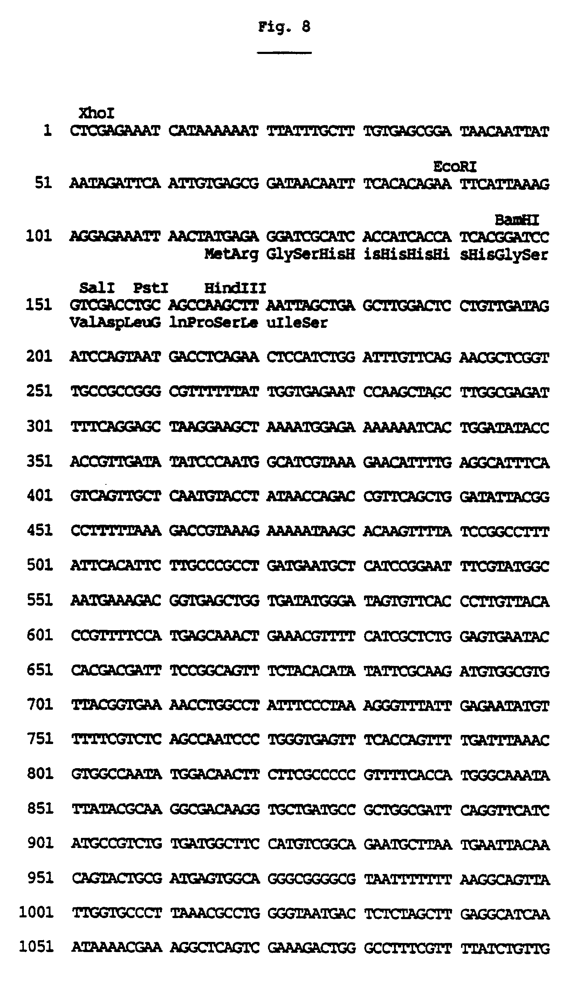

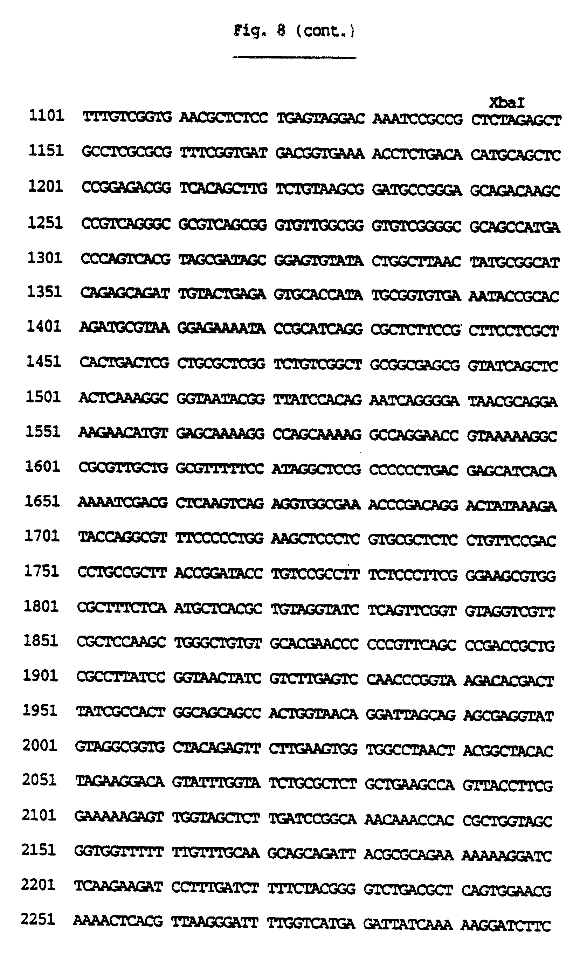

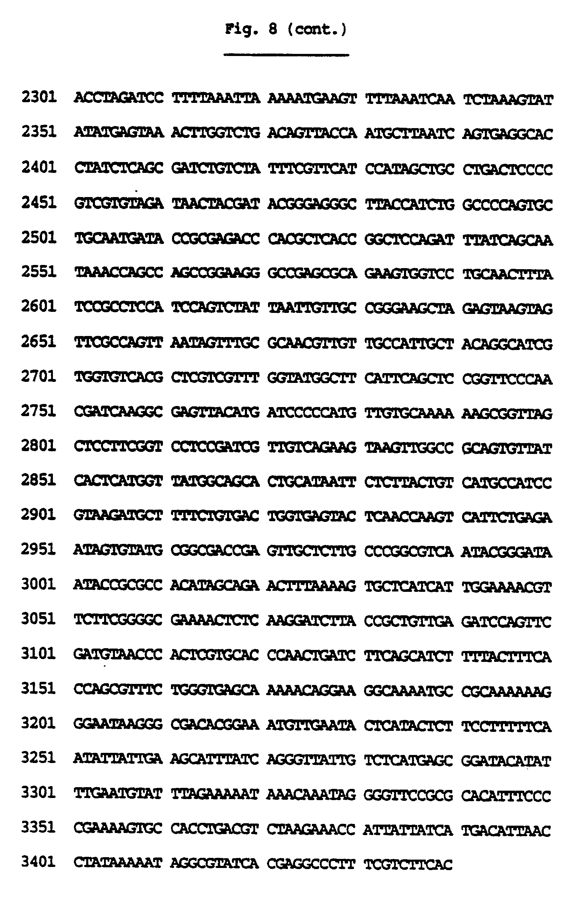

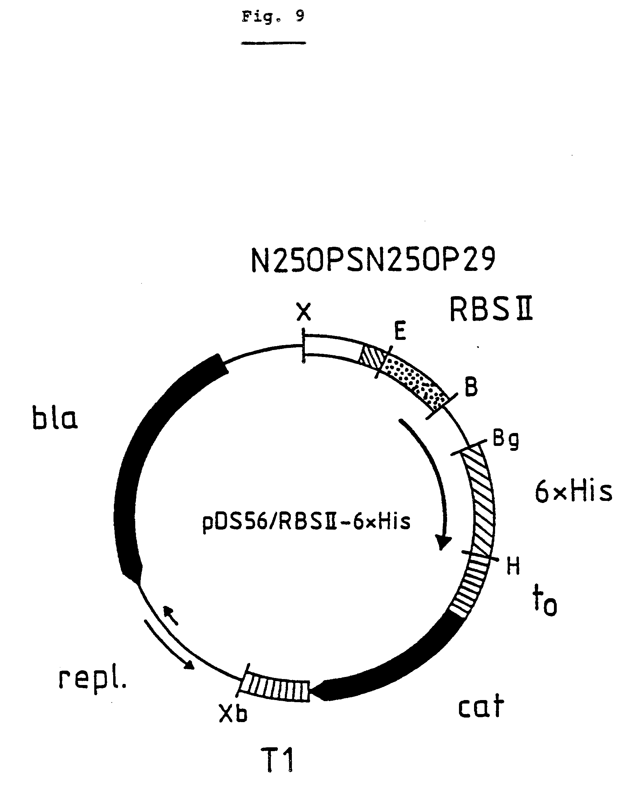

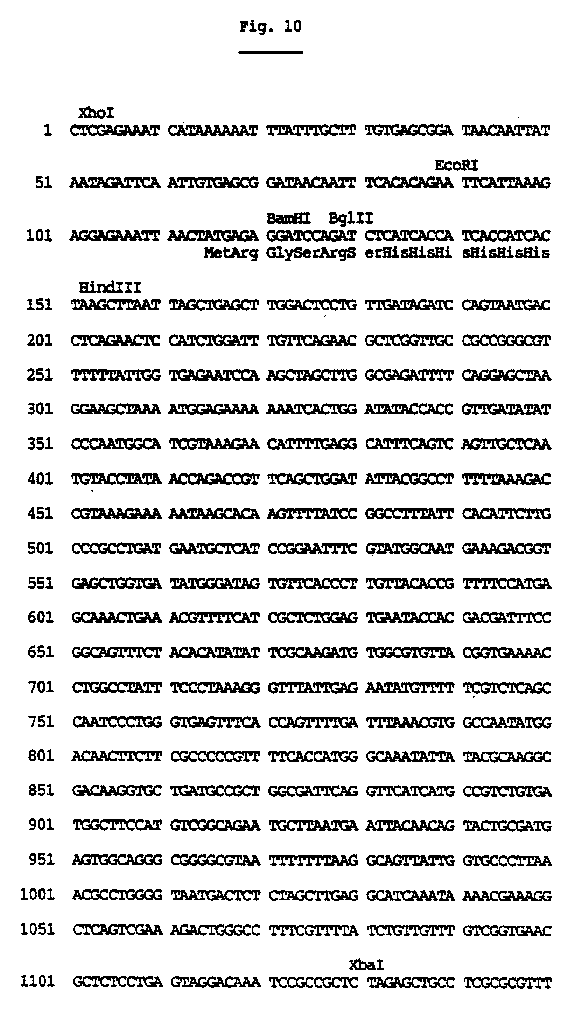

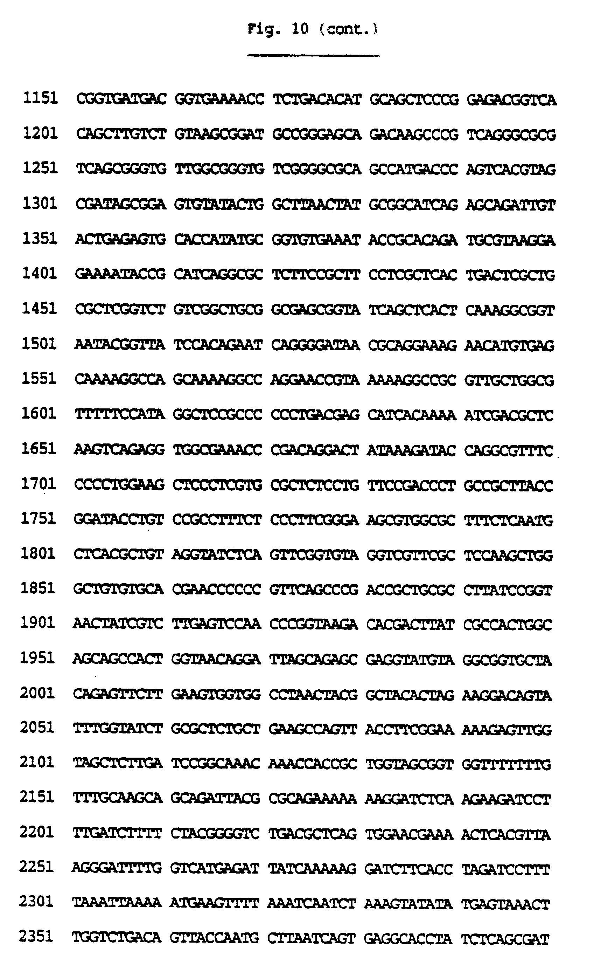

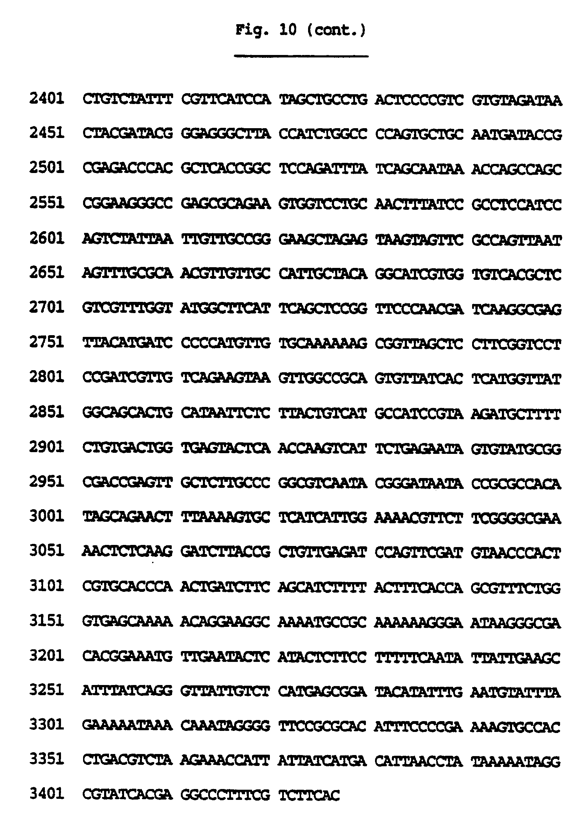

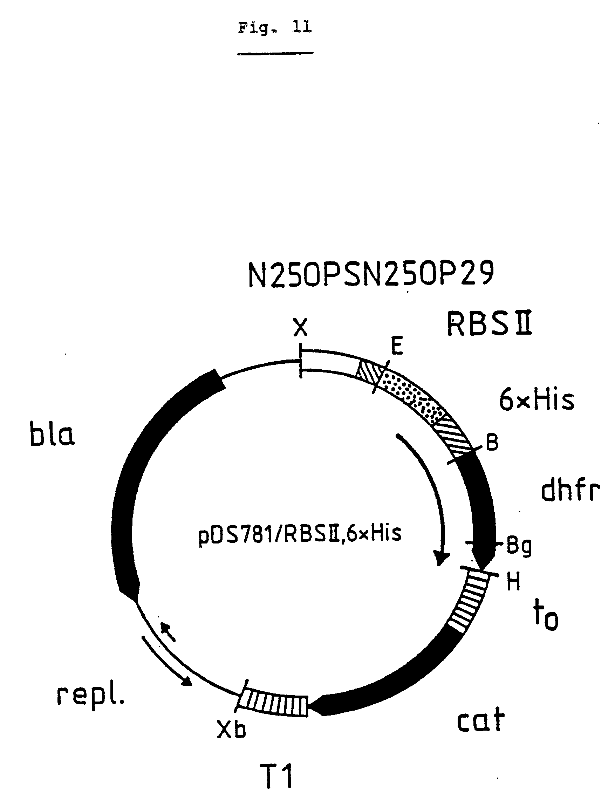

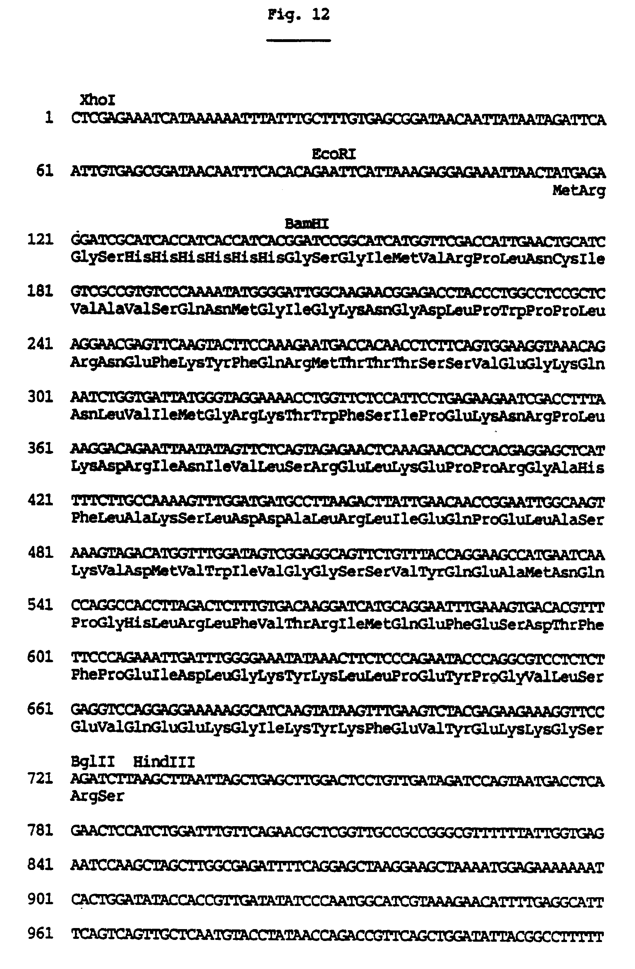

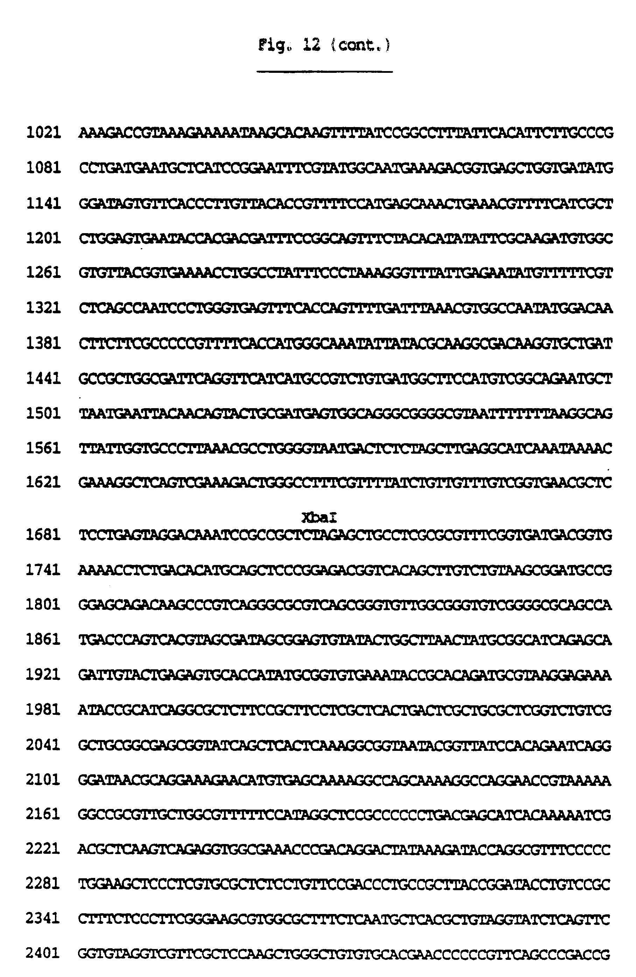

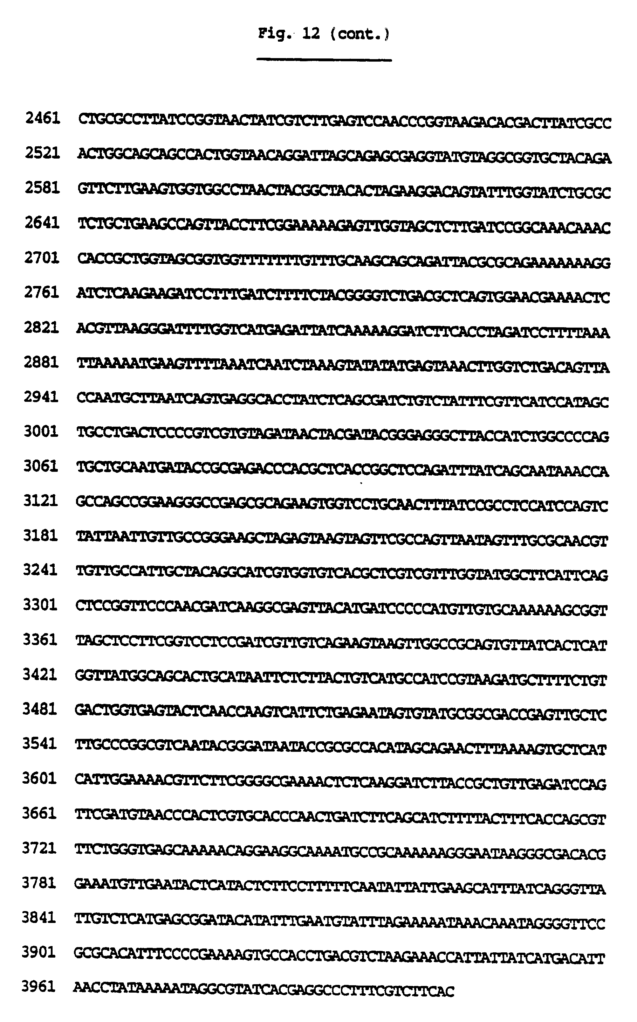

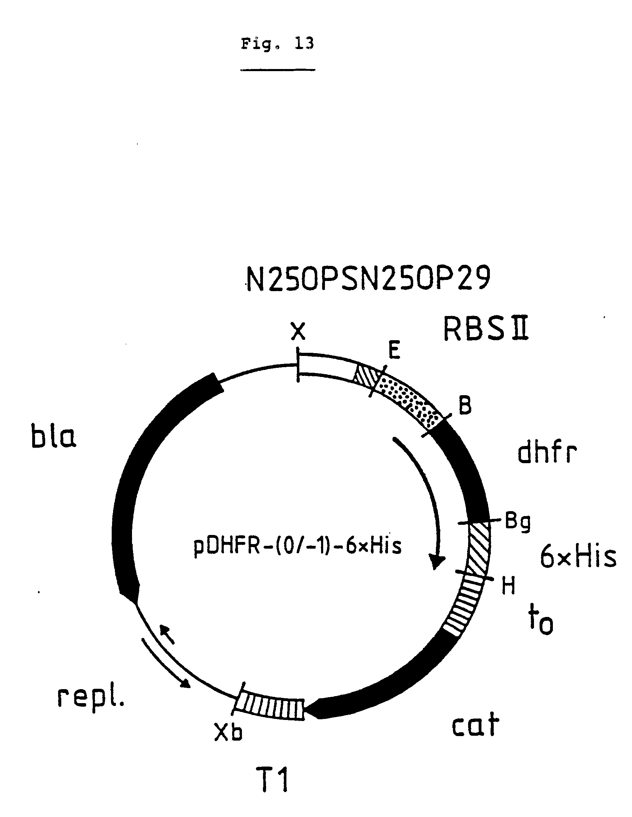

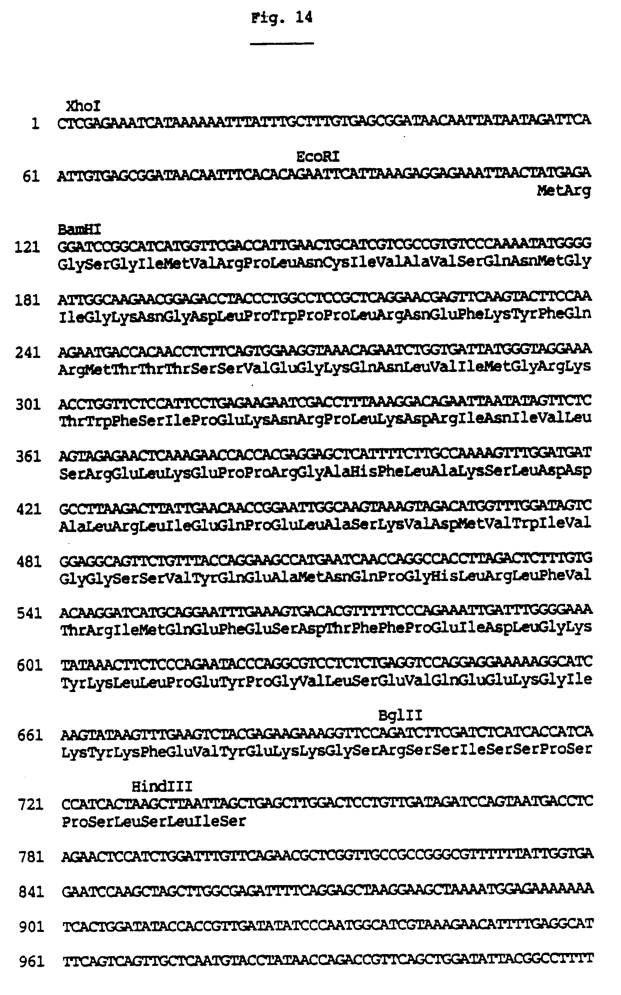

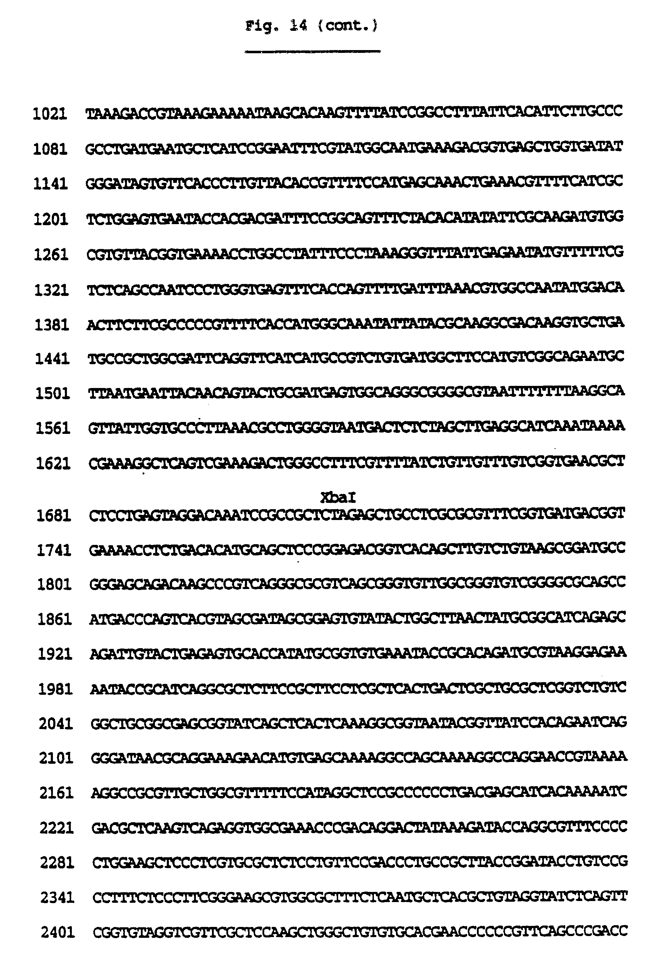

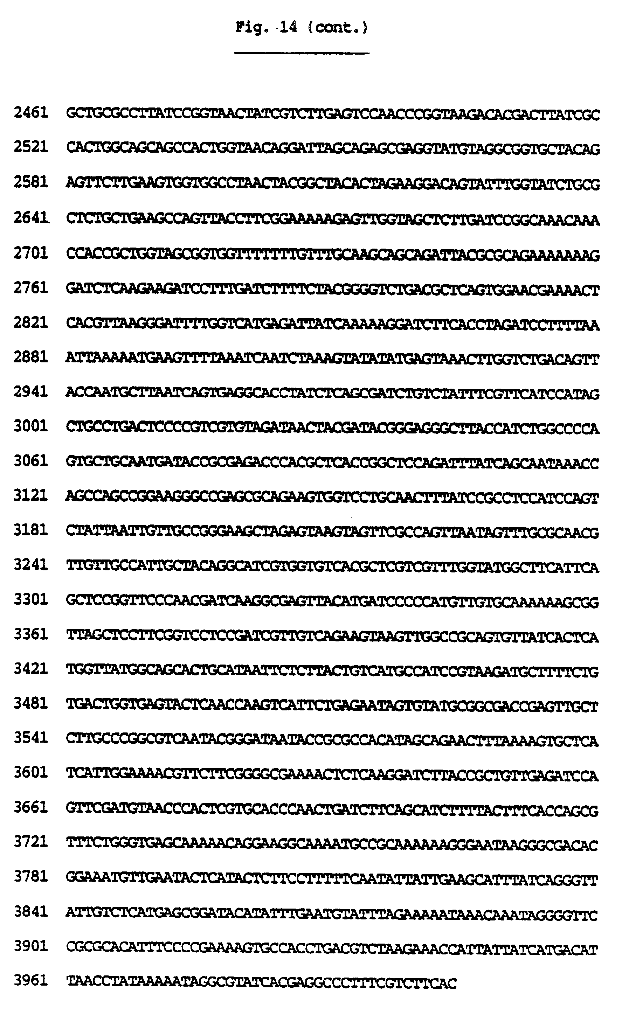

Plasmids pDS56/RBSII,NcoI (Figs. 3 and 4), pDS56/RBSII,SphI (Figs. 5 and 6), pDS56/RBSII,6xHis (Figs. 7 and 8), pDS56/RBSII-6xHis (Figs. 9 and 10), pDS781/RBSII,6xHis (Figs. 11 and 12) and pDHFR-(0/-1)-6xHis (Figs. 13 and 14) were used for the cloning of relevant DNA fragments and the expression in E.coli of the soluble human IFN-γ receptor of the present invention.

The following E.coli cells transformed with these plasmids were deposited at the Deutsche Sammlung von Microorganismen in Braunschweig on April 6, 1989, in accordance with the Budapest Treaty:
E.coli M15 (pDS56/RBSII,NcoI ; pDMI,1), DSM No.: 5302,
E.coli M15 (pDS56/RBSII,SphI ; pDMI,1), DSM No.: 5300,
E.coli M15 (pDS56/RBSII,6xHis ; pDMI,1), DSM No.: 5298,
E.coli M15 (pDS56/RBSII-6xHis ; pDMI,1), DSM No.: 5299,
E.coli M15 (pDS781/RBSII,6xHis ; pDMI,1), DSM No.: 5301 and
E.coli M15 (pDHFR-(0/-1)-6xHis ; pDMI,1), DSM No.: 5297.

The above-mentioned vectors contain the regulatable promoter/operator element N250PSN250P29 and the ribosomal binding sites RBSII,SphI, RBSII,NcoI or RBSII. These ribosomal binding sites were derived from the ribosomal binding site of the promoter $P_{G25}$ of the E.coli phage T5 (R. Gentz, Dissertation, Universität Heidelberg, BRD [1984]) and were obtained via DNA synthesis.

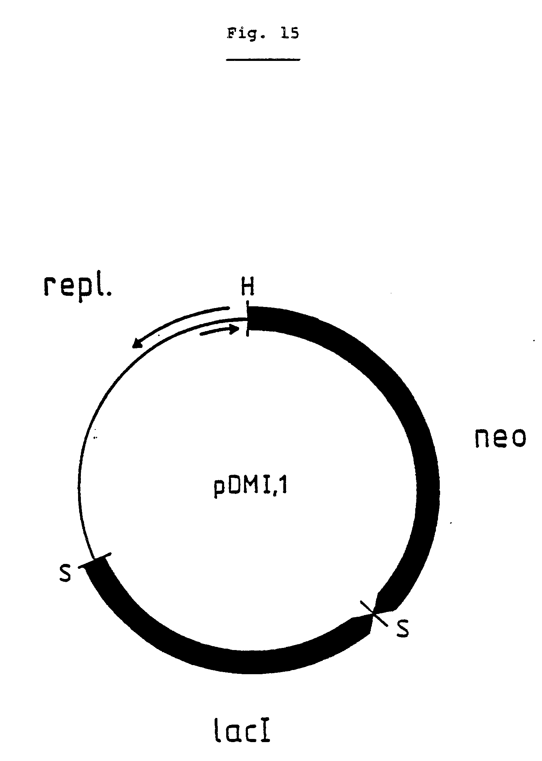

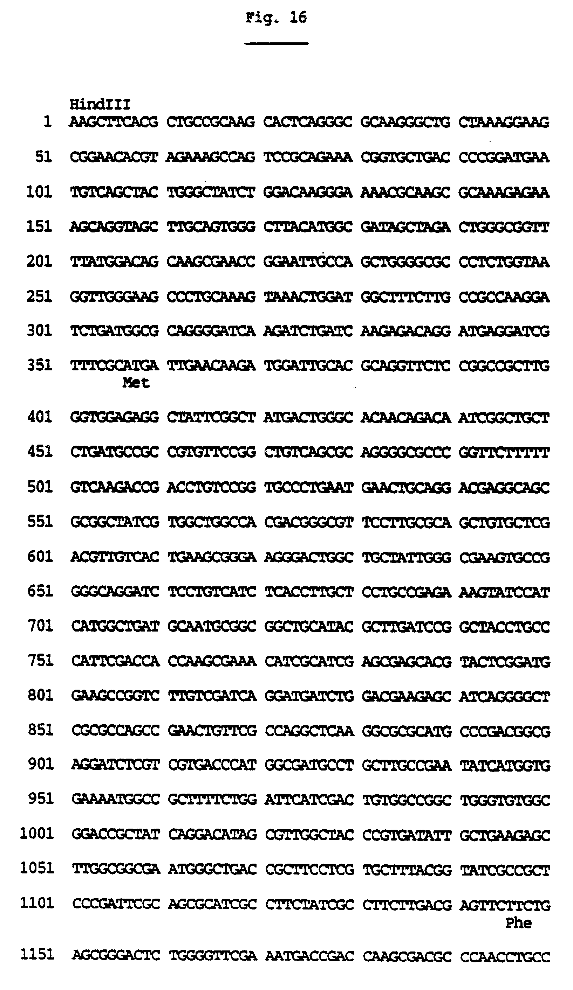

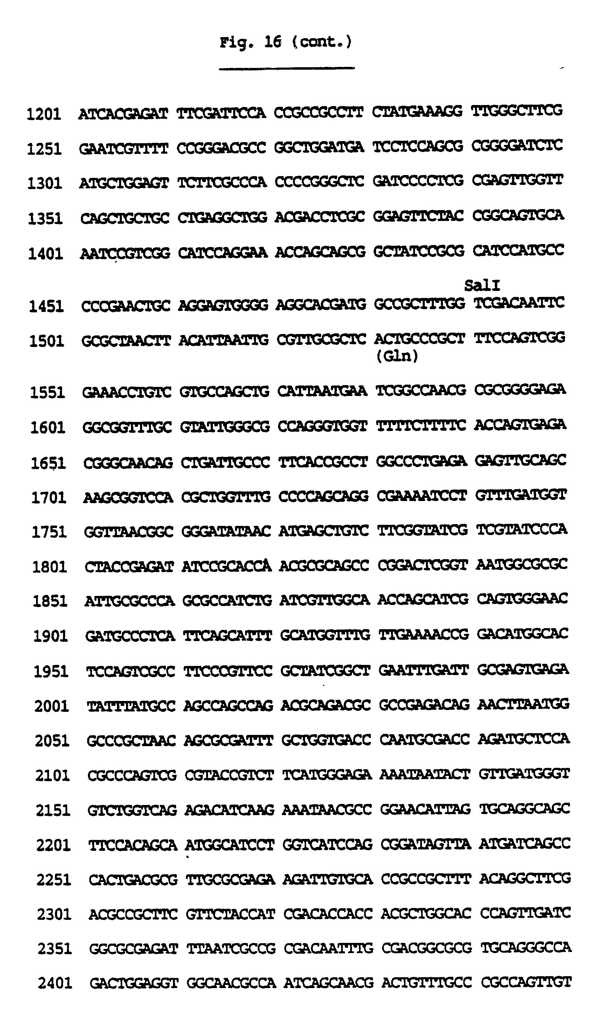

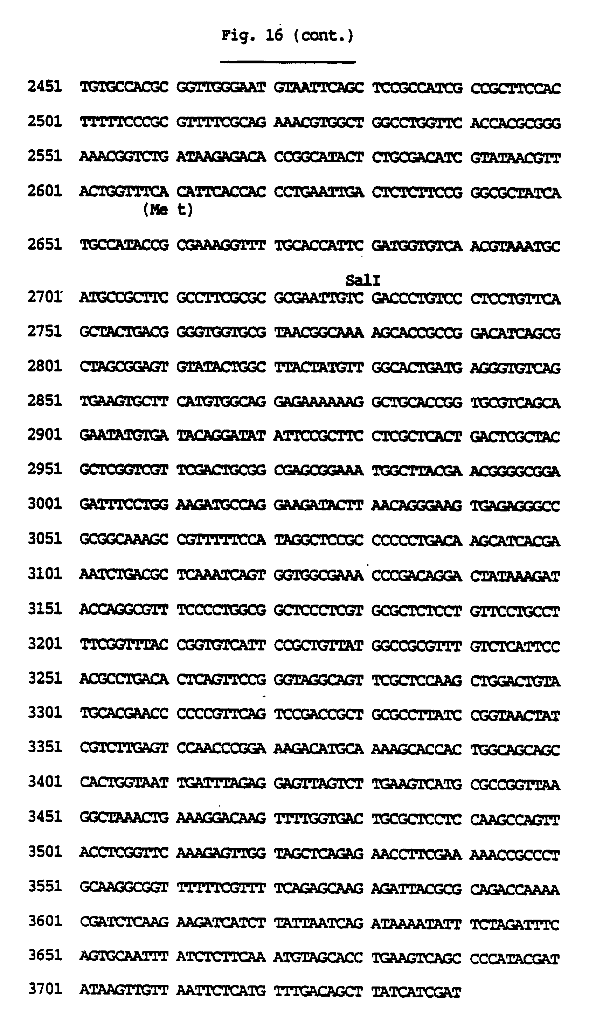

Due to the high efficiency of expression, the above-mentioned plasmids can be maintained in E.coli cells only if the promoter/operator element is repressed by the binding of a lac repressor to the operator. The lac repressor is coded in the lacI gene. N250PSN250P29 can be repressed efficiently only when a sufficient number of repressor molecules is present in the cells. Therefore, the $lacI^q$ allel, which contains a promoter mutant which leads to an increased expression of the repressor gene, was used. This $lacI^q$ allel is present in the plasmid pDMI,1 (Figs. 15 and 16).

This plasmid carries, in addition to the lac-I gene, the neo gene, which confers kanamycin resistance to the bacteria and which is used as the selection marker. pDMI,1 is compatible with the aforementioned plasmids. E.coli cells which are transformed with such expression vectors must contain pDMI,1 to guarantee that the expression vector is held stable in the cells. An induction of this system is achieved by adding IPTG to the medium at the desired cell density.

B. Plasmid pDS56/RBSII,NcoI

The part of pDS56/RBSII,NcoI (Figs. 3 and 4) which lies between the restriction cleavage sites for XbaI and XhoI and which contains the replication region and the gene for β-lactamase (which confers ampicillin resistance to the cells) was derived originally from the plasmid pBR322 (Bolivar et al., Gene 2, 95-113 [1977]; Sutcliffe, Cold Spring Harbor Symp. Quant. Biol. 43, 77-90 [1979]). However, the gene for β-lactamase is modified by elimination of the cleavage sites for the restriction enzymes HincII and PstI. These alterations in the DNA sequence have no effect on the amino acid sequence of the β-lactamase. The remaining part of the plasmid carries the regulatable promoter/operator element N250PSOP29 followed by the ribosomal binding site RBSII,NcoI, which is part of an EcoRI/BamHI fragment, cleavage sites for the

restriction enzymes SalI,PstI and HindIII, the terminator $t_o$ of E.coli phage lambda (Schwarz et al., Nature 272, 410-414 [1978]), the promoter-free gene of chloramphenicol acetyltransferase (Marcoli et al., FEBS Letters, 110, 11-14 [1980]) and the terminator T1 of the E.coli rrnB operon (Brosius et al., J. Mol. Biol. 148, 107-127 [1981]).

C. Plasmid pDS56/RBSII,SpHI

Plasmid pDS56/RBSII,SphI (Figs. 5 and 6) is similar to plasmid pDS56/RBSII,NcoI but contains the ribosomal binding site RBSII,SphI.

D. Plasmid pDS56/RBSII,6xHis

Plasmid pDS56/RBSII,6xHis (Figs. 7 and 8) is similar to plasmid pDS56/RBSII,NcoI but contains the ribosomal binding site RBSII followed by a region encoding six histidine residues.

E. Plasmid pDS56/RBSII-6xHis

Plasmid pDS56/RBSII-6xHis (Figs. 9 and 10) is similar to plasmid pDS56/RBSII,NcoI but contains the ribosomal binding site RBSII followed by cleavage sites for the restriction enzymes BamHI and BglII, a region encoding six histidine residues, and a translational stop codon preceeding the cleavage site for restriction enzyme HindIII.

F. Plasmid pDS781/RBSII,6xHis

The part of plasmid pDS781/RBSII,6xHis (Figs. 11 and 12) which lies between the restriction cleavage sites for XbaI and XhoI and which contains the replication region and the gene for $\beta$-lactamase (which confers ampicillin resistance to the cells) was derived originally from the plasmid pBR322 (Bolivar et al., supra; Sutcliffe, supra). However, the gene for $\beta$-lactamase is modified in the manner described for the plasmid pDS5/RBSII,NcoI. The remaining part of the plasmid carries the regulatable promoter/operator element N250PSN250P29 followed by the ribosomal binding site RBSII; a region encoding six histidine residues; a BamHI restriction endonuclease cleavage site, the dihydrofolate reductase (DHFR) gene of mouse cell line AT-3000 (Chang et al., Nature 275, 617-624 [1978]; Masters et al., Gene 21, 59-63 [1983]), which has been altered by introducing a cleavage site for the restriction enzyme BglII immediately prior to the end of the structural gene; a translational stop codon; a cleavage site for the restriction enzyme HindIII; the terminator $t_o$ (Schwarz et al., supra); the promoter-free gene for chloramphenicol acetyltransferase (Marcoli et al., supra) and the terminator T1 (Brosius et al., supra).

G. Plasmid pDHFR-(0/-1)-6xHis

Plasmid pDHFR-(0/-1)-6xHis (Figs. 13 and 14) is similar to plasmid pDS781/RBSII,6xHis but contains the region encoding six histidine residues between the cleavage site for the restriction enzyme BglII and the translational stop codon preceeding the cleavage site for the restriction enzyme HindIII.

H. Plasmid pDMI,1

Plasmid pDMI,1 (Figs. 15 and 16) carries the gene for neomycin phosphotransferase from the transposon Tn5 (Beck et al., Gene 19, 327-336 [1982]), which confers kanamycin resistance to E.coli cells, and the lacI gene (Farabough, Nature 274, 765-769 [1978]) with the promoter mutation $I^q$ (Calos, Nature 274, 762-765 [1978]), which codes for the lac repressor. Moreover, plasmid pDMI,1 contains a region of the plasmid pACYC184 (Chang and Cohen, J. Bacteriol. 134, 1141-1156 [1978]), which contains all information required for the replication and stable transmission to the daughter cells.

Example 3

Construction of plasmids used for the expression of the soluble human IFN-γ receptors in E.coli

A. Principles

The methods used for the construction of plasmids are well known for those that are skilled in the art, e.g. from the laboratory manual "Molecular Cloning" by Maniatis et al. Cold Spring Harbor Laboratory, 1982. These methods include the use of restriction enzymes to selectively cut DNA, the use of Klenow enzyme to fill-in protruding ends generated by action of restriction enzymes, the analysis of intact plasmids and of DNA fragments by electrophoresis in agarose or polyacrylamide gels, the isolation of DNA out of those gels, the ligation of DNA fragments, the transformation of DNA into competent E.coli cells and the preparation of such cells, and the growth of transformed E.coli cells. Plasmid DNA was extracted from those cells according to the method of Birnboim and Doly (Nucleic Acids Res. 7, 1513-1523 [1979]. Plasmid DNA used for the transfection of COS-1 cells was prepared by CsCl/Ethidium bromide centrifugation as described (Vinograd et al., Proc. Natl. Acad. Sci. USA 71, 1790-1794 [1967]).

Oligonucleotides (linkers, adaptors, primers) were either purchased or synthesized using controlled pore glass (CPG) as support material (Kiefer et al., Immunol. Methods 3, 69-83 [1985]; Adams et al., J. Amer. Chem. Soc. 105, 661-663 [1983]). Oligonucleotide-directed in vitro mutagenesis was performed using the 'Oligonucleotide-directed in vitro mutagenesis system' of Amersham. DNA sequences were verified by sequencing single-stranded or double-stranded DNA using the Sequenase$^{R}$ system of United States Biochemical Corporation.

B. Construction of plasmid pDS56/RBSII-F26-6xHis encoding protein P26H6

Plasmid pDS56/RBSII-F26-6xHis was constructed in two steps; 1) a DNA fragment, termed F7, was prepared from plasmid pBABLUE and integrated into plasmid pDHFR-(0/-1)-6xHis resulting in plasmid pDHFR-F7-6xHis and 2) a DNA fragment, termed F26, was prepared from this plasmid and integrated into plasmid pDS56/RBSII-6xHis resulting in plasmid pDS56/RBSII-F26-6xHis.

1. Construction of plasmid pDHFR-F7-6xHis

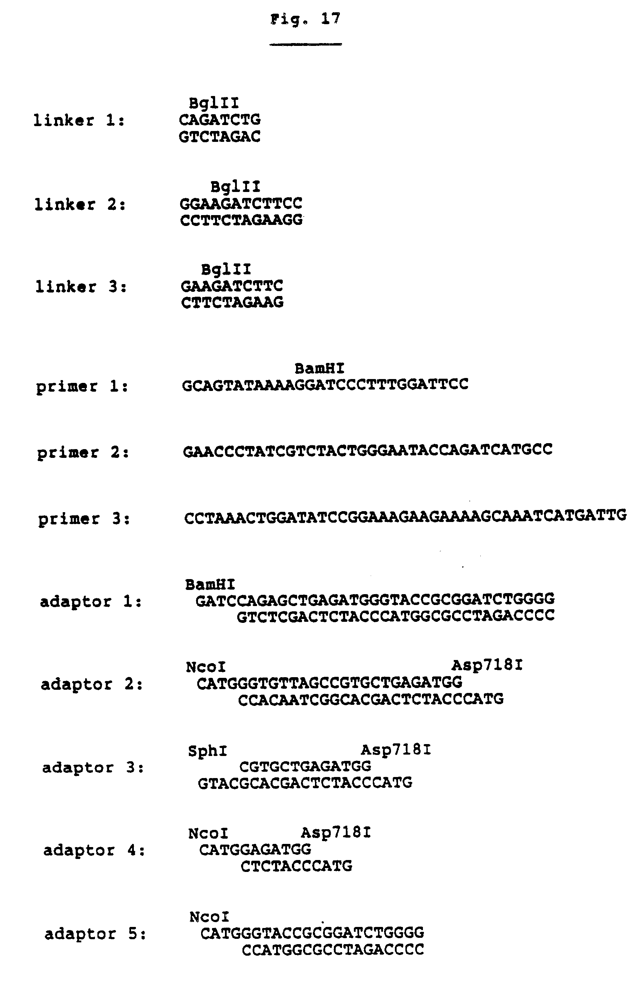

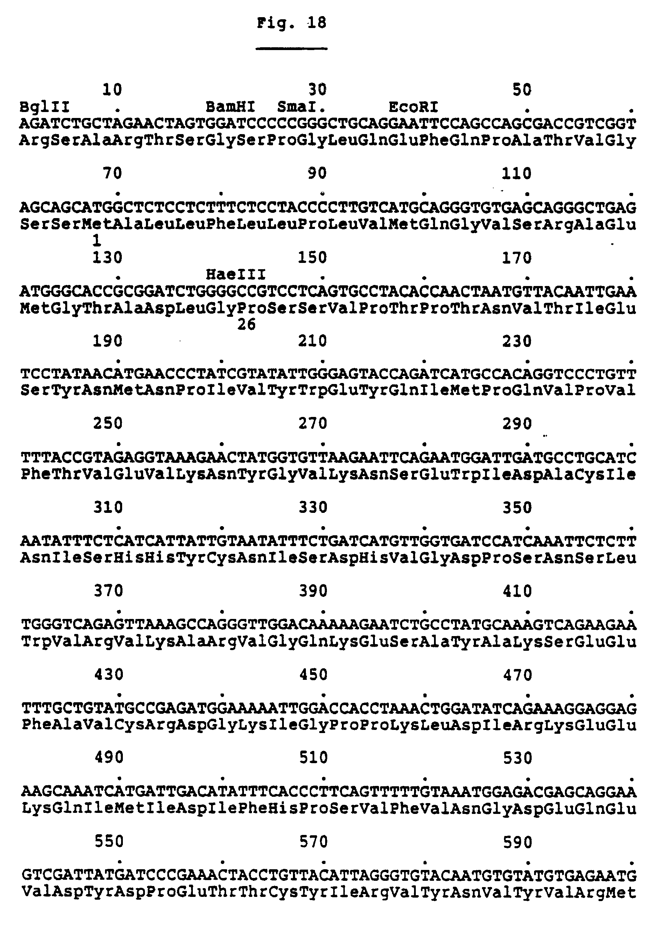

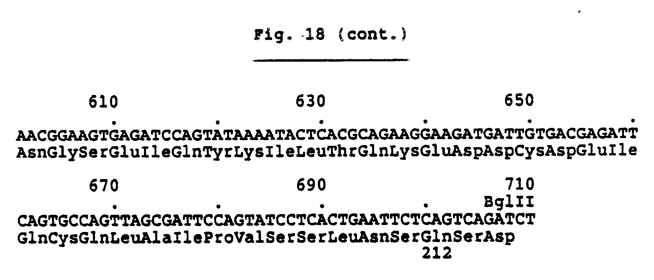

Plasmid pBABLUE was digested with restriction endonucleases XbaI and ScaI (see Fig. 1). The protruding ends of the resulting DNA fragments were filled-in using Klenow enzyme before phosphorylated BglII-linkers (linker 1, Fig. 17) were added. After cleavage with restriction enzyme BglII, fragment F7 was isolated from the resulting mixture of DNA fragments. The nucleotide sequence and the deduced amino acid sequence of F7 are shown in Fig. 18. Fragment F7 encodes amino acids 1 to 212 of the human IFN-γ receptor and some additional amino acids. Finally, fragment F7 was integrated into the unique cleavage site for restriction enzyme BglII of plasmid pDHFR-(0/-1)-6xHis (see Figs. 13 and 14) resulting in plasmid pDHFR-F7-6xHis.

2. Construction of plasmid pDS56/RBSII-F26-6xHis

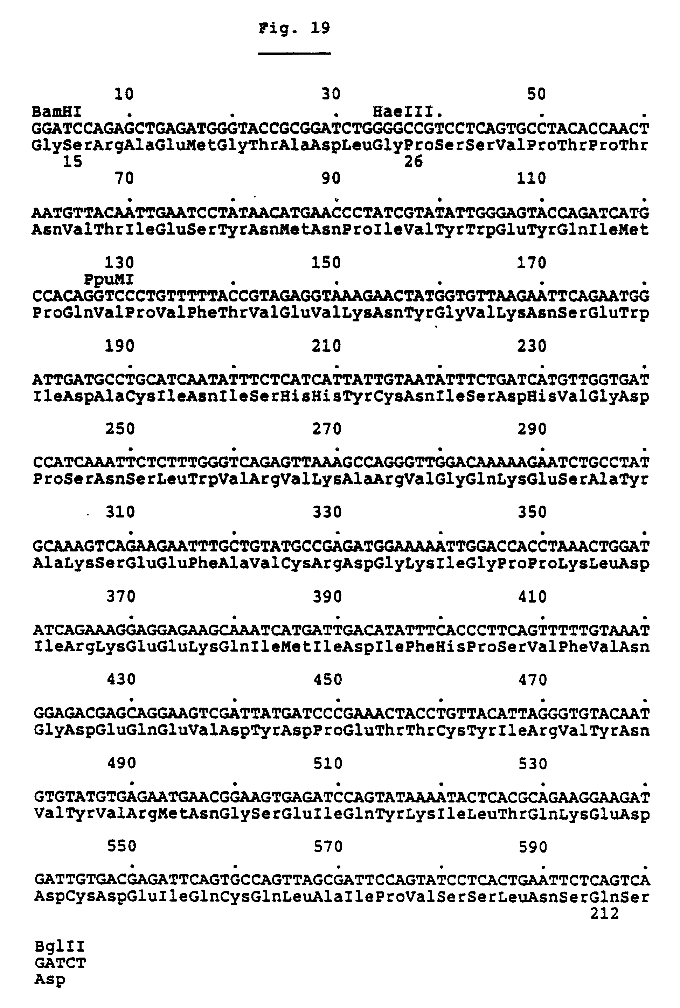

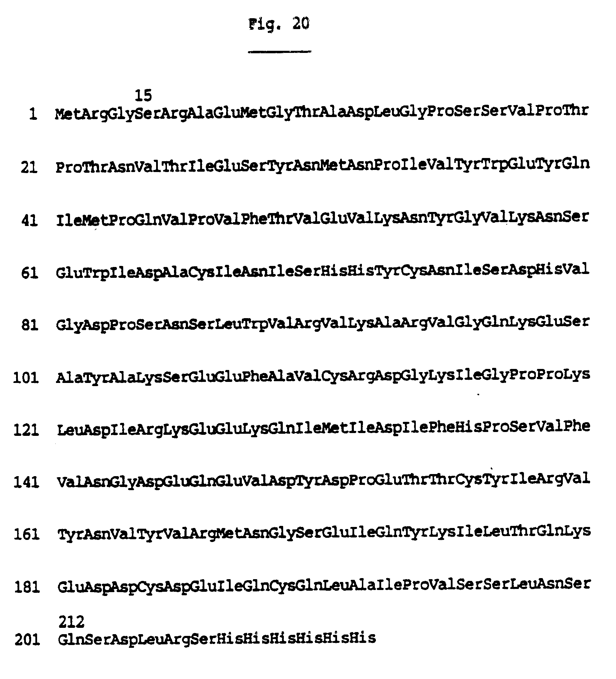

Plasmid pDHFR-F7-6xHis was digested with restriction enzyme HaeIII. To the ends of the resulting DNA fragments a phosphorylated DNA adaptor (adaptor 1, Fig. 17) was ligated before the DNA was digested with restriction enzymes BamHI and BglII. Subsequently, fragment F26 was isolated from the mixture of DNA fragments. The nucleotide sequence and the deduced amino acid sequence of F26 are shown in Fig. 19. Fragment F26 encodes amino acids 15 to 212 of the human IFN-γ receptor and some additional amino acids. Finally, fragment F26 was integrated in the proper orientation into the unique cleavage sites for restriction enzymes BamHI and BglII of plasmid PDHFR-(0/-1)-6xHis (see Figs. 13 and 14), thereby replacing the gene for DHFR. In the resulting plasmid pDS56/RBSII-F26-6xHis the coding region for the receptor is in frame with the region encoding six histidines. Plasmid pDS56/RBSII-F26-6xHis encodes protein P26H6 (see Fig. 20) and was used for the production of this protein.

C. Construction of plasmids pDS56/RBSII-F25-6xHis(A), pDS56/RBSII-F25-6xHis(B) and pDS56/RBSII-F25-6xHis(C) encoding proteins P25H6A, P25H6B and P25H6C, respectively

Plasmids pDS56/RBSII-F25-6xHis(A), pDS56/RBSII-F25-6xHis(B) and pDS56/RBSII-F25-6xHis(C) were constructed in three steps: 1) a DNA fragment, termed F12, was prepared from plasmid pBABLUE and integrated into plasmid pDHFR-(0/-1)-6xHis resulting in plasmid pDHFR-F12-6xHis; 2) a DNA fragment of this plasmid was integrated into plasmid M13mp19 and a cleavage site for the restriction enzyme BamHI was introduced into the gene for the human IFN-$\gamma$ receptor by in-vitro mutagenesis resulting in fragment F27; and 3) a DNA fragment, termed F25, was prepared from the mutagenized DNA and integrated in three different ways into plasmid pDS56/RBSII-6xHis resulting in plasmids pDS56/RBSII-F25-6xHis(A), pDS56/RBSII-F25-6xHis(B) and pDS56/RBSII-F25-6xHis(C).

1. Construction of plasmid pDHFR-F12-6xHis

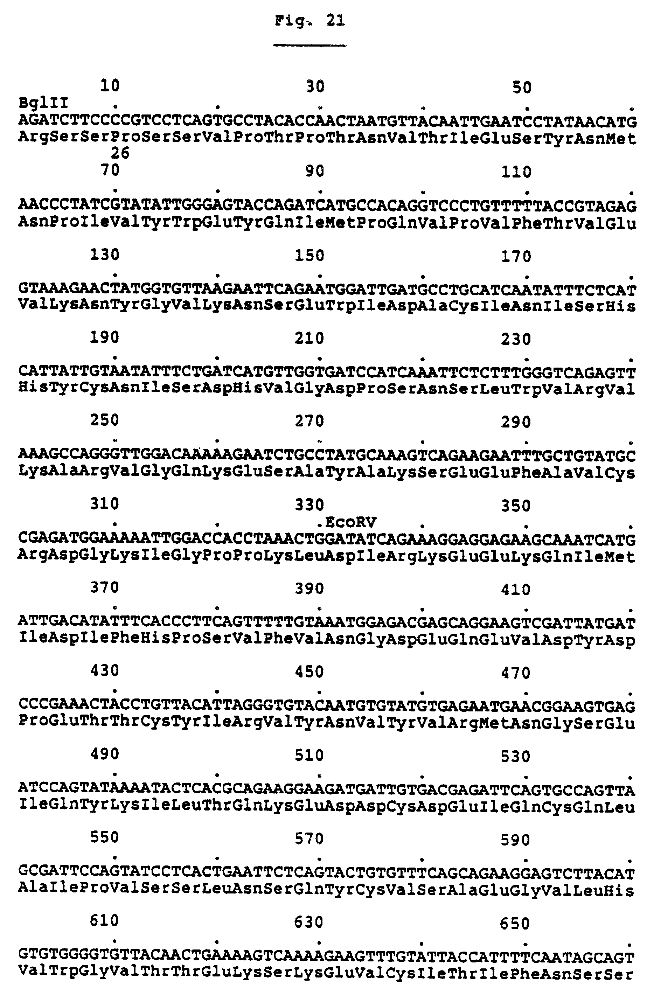

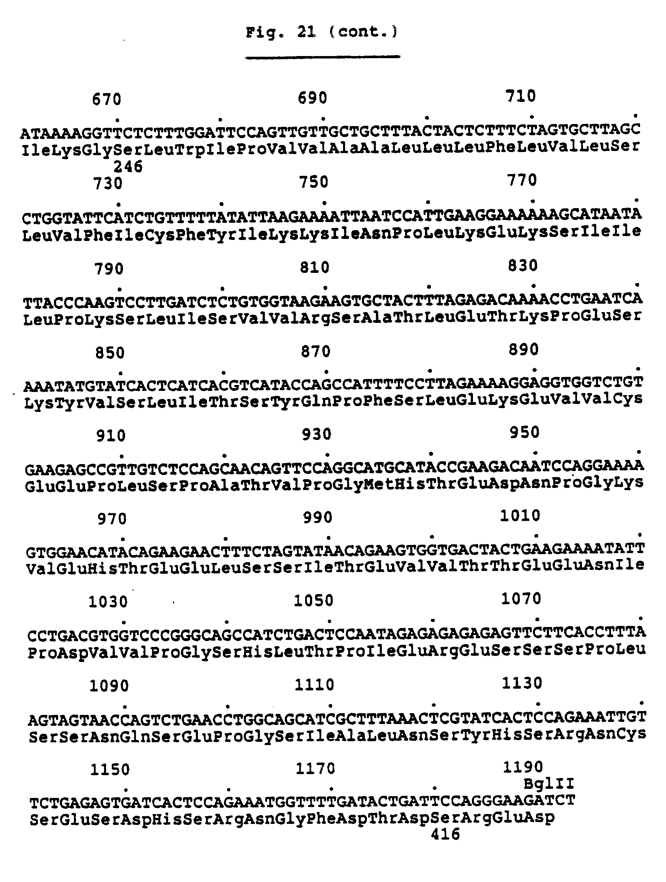

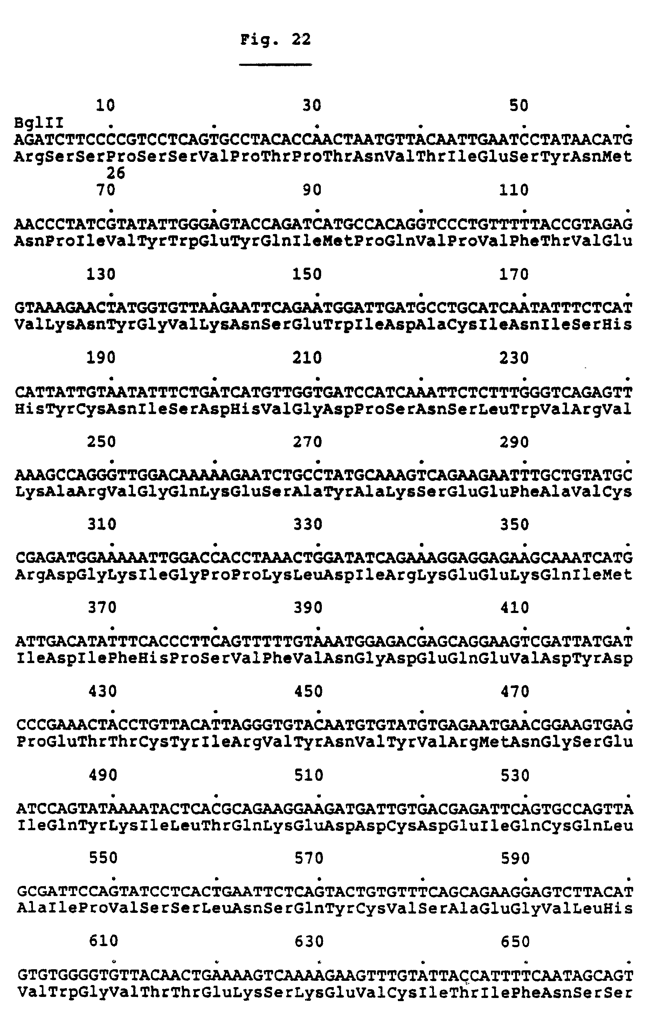

Plasmid pBABLUE was digested with restriction enzymes HaeIII and PvuII (see Fig. 1). To the ends of the resultung DNA fragments BglII-linkers (linker 2, Fig. 17) were added. After cleavage with restriction enzyme BglII, fragment F12 was isolated from the mixture of DNA fragments. The nucleotide sequence and the deduced amino acid sequence of F12 are shown in Fig. 21. Fragment F12 encodes amino acids 26 to 416 of the human IFN-$\gamma$ receptor and some additional amino acids. Finally, fragment F12 was integrated into the unique cleavage site for restriction enzyme BglII of plasmid pDHFR-(0/-1)-6xHis (see Figs. 13 and 14) resulting in plasmid pDHFR-F12-6xHis.

2. Preparation of fragment F27

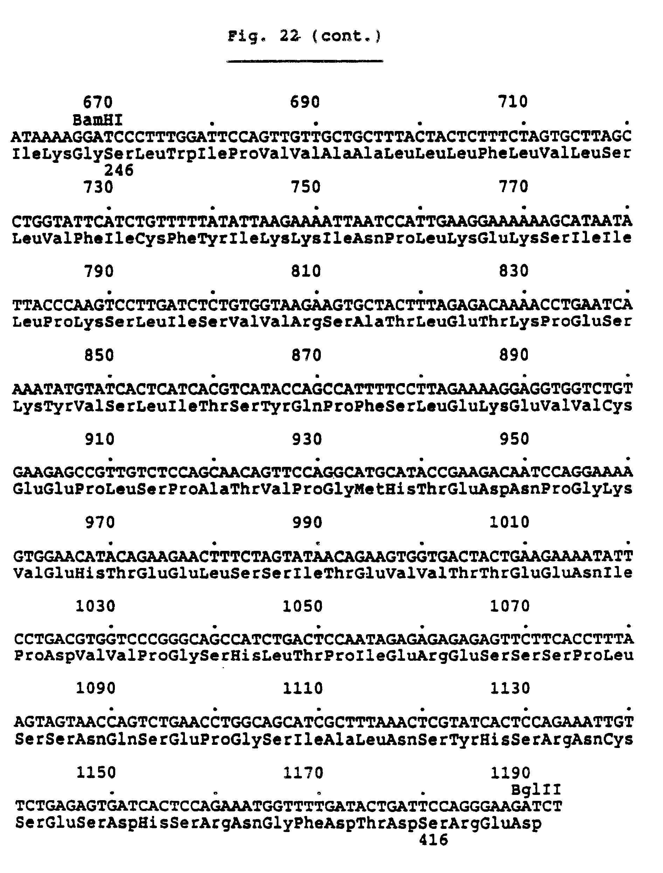

The EcoRI/HindIII fragment of plasmid pDHFR-F12-6xHis comprising the ribosomal binding site RBSII, the gene for DHFR, fragment F12 and the region encoding six histidines was integrated into the unique cleavage sites for EcoRI and HindIII of plasmid M13mp19 (Pharmacia). In the resulting plasmid, the gene for the receptor was mutagenized using primer 1 (Fig. 17) to introduce a cleavage site for the restriction enzyme BamHI just in front of the DNA encoding the transmembrane region of the receptor (see Figs. 1,2 and 22). The resulting plasmid carries fragment F27, whose nucleotide sequence and deduced amino acid sequence are shown in Fig. 22. Since the DNA sequence was mutagenized without affecting the encoded amino acid sequence of the receptor, fragment F27 differs from fragment F12 only by the presence of the cleavage site for restriction enzyme BamHI (see Figs. 21 and 22).

3. Construction of plasmids pDS56/RBSII-F25-6xHis(A), pDS56/RBSII-F25-6xHis(B) and pDS56/RBSII-F25-6xHis(C)

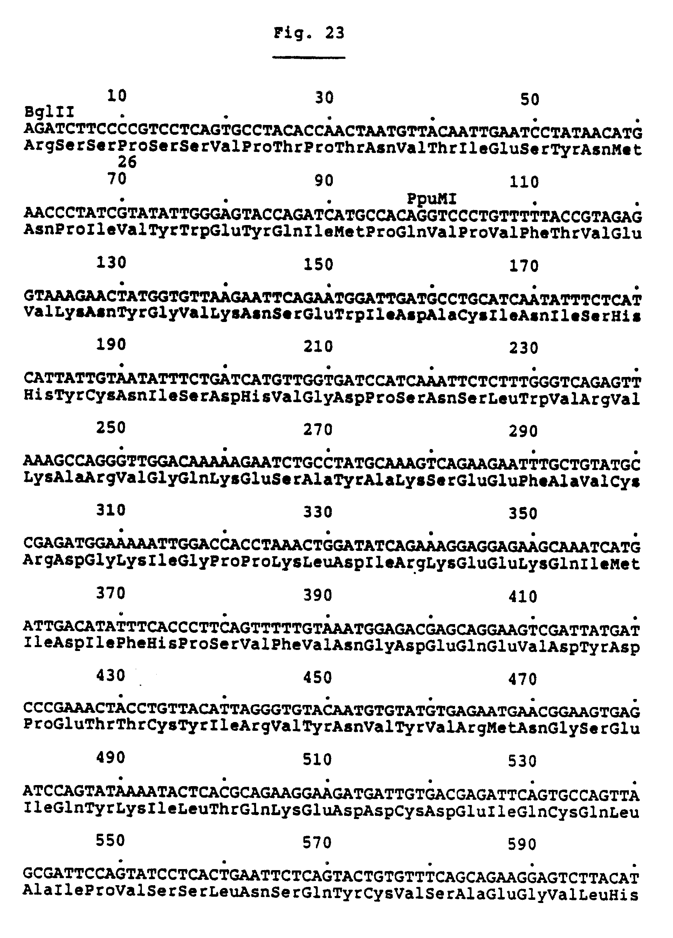

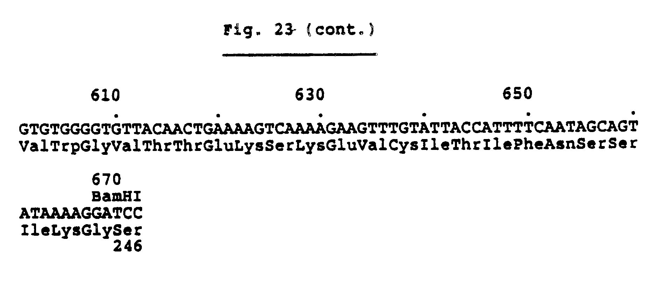

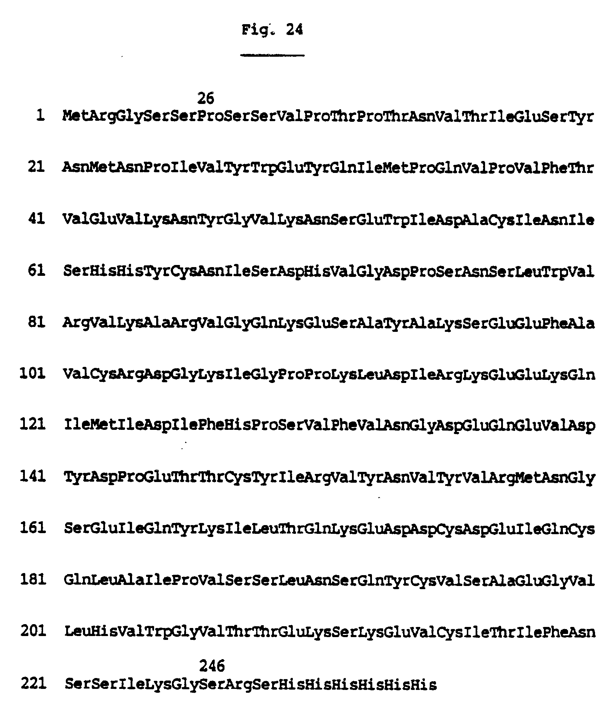

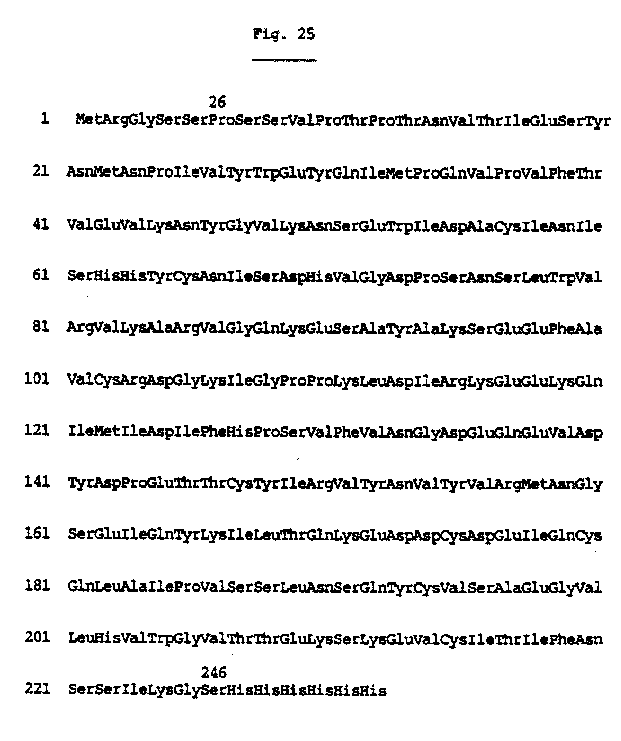

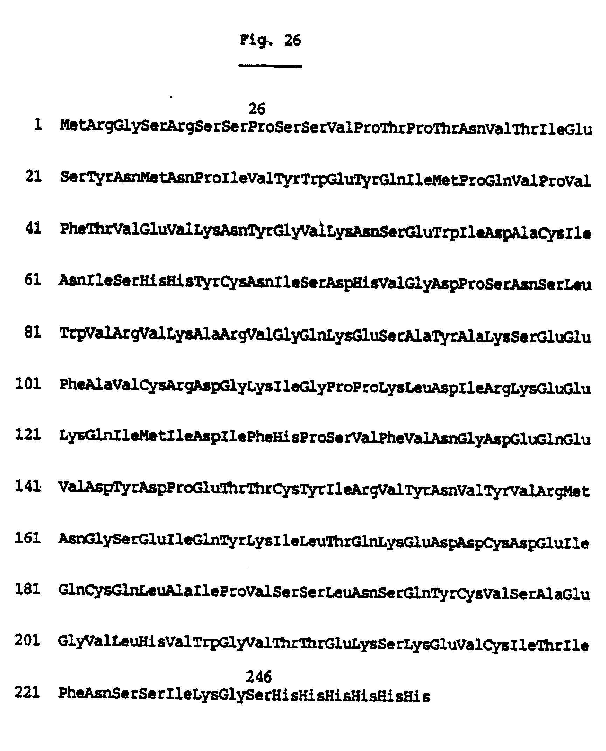

The mutagenized DNA was digested with restriction enzymes BglII and BamHI and fragment F25 was isolated from the resulting mixture of DNA fragments. The nucleotide sequence and deduced amino acid sequence of F25 are shown in Fig. 23. Fragment F25 encodes amino acids 26 to 246 of the human IFN-$\gamma$ receptor (amino acid 246 preceedes the transmembrane region of the receptor) and some additional amino acids. Finally, fragment F25 was integrated in the proper orientation in three different ways into the unique cleavage sites for BamHI and BglII of plasmid pDS56/RBSII-6xHis (see Figs. 9 and 10): 1) into the BamHI site of the plasmid resulting in plasmid pDS56/RBSII-F25-6xHis(A), 2) between the BamHI and BglII sites of the plasmid, thereby replacing some nucleotides of the plasmid, resulting in plasmid pDS56/RBSII-F25-6xHis(B), and 3) into the BglII site of the plasmid resulting in plasmid pDS56/RBSII-F25-6xHis(C). Plasmids pDS56/RBSII-F25-6xHis(A), pDS56/RBSII-F25-6xHis(B) and pDS56/RBSII-F25-6xHis(C) encode proteins P25H6A (Fig. 24), P25H6B (Fig. 25) and P25H6C (Fig. 26), respectively, and were used for the production of these proteins.

D. Construction of plasmid pDS56/RBSII,6xHis-F25 encoding protein P6H25

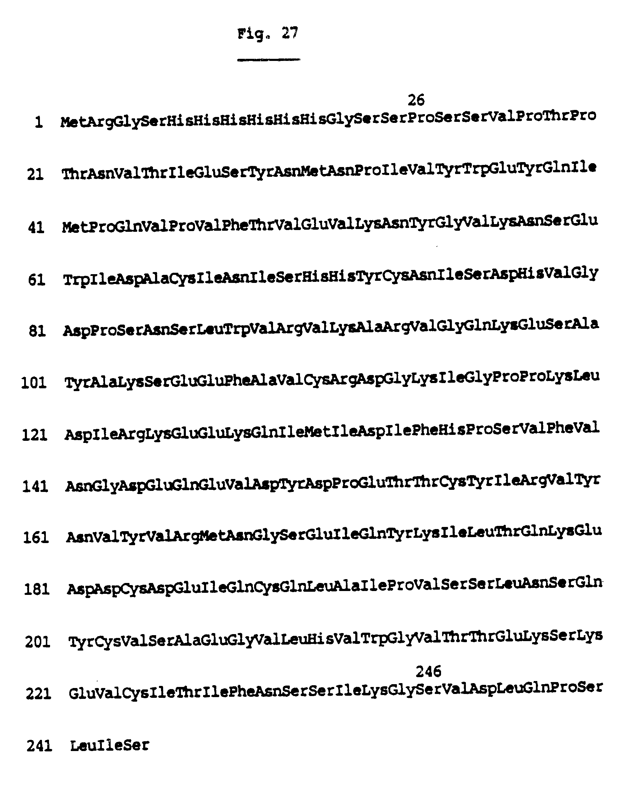

Plasmid pDS56/RBSII,6xHis-F25 was constructed by integrating fragment F25 (Fig. 23) into the unique cleavage site for restriction enzyme BamHI of plasmid pDS56/RBSII,6xHis (Figs. 7 and 8). Plasmid pDS56/RBSII,6xHis-F25 encodes protein P6H25 (Fig. 27) and was used for the production of this protein.

E. Construction of plasmid pDS56/RBSII-F28-6xHis(A) encoding protein P28H6A

For construction of plasmid pDS56/RBSII-F28-6xHis(A) first fragments BamHI-F26'-PpuMI and PpuMI-F25'-BamHI were isolated from plasmids pDS56/RBSII-F26-6xHis and pDS56/RBSII-F25-6xHis(A), respectively. Subsequently, these DNA fragments were together integrated into plasmid pDS56/RBSII-6xHis resulting in plasmid pDS56/RBSII-F28-6xHis(A).

1. Preparation of fragment BamHI-F26'-PpuMI

Plasmid pDS56/RBSII-F26-6xHis (see Example 3.B) was digested with restriction enzymes BamHI and PpuMI. From the resulting mixture of DNA fragments, fragment BamHI-F26'-PpuMI (see Fig. 19) was isolated.

2. Preparation of fragment PpuMI-F25'-BamHI

Plasmid pDS56/RBSII-F25-6xHis(A) (see Example 3.C) was digested with restriction enzymes PpuMI and BamHI. From the resulting mixture of DNA fragment, fragment PpuMI-F25'-BamHI (see Fig. 23) was isolated.

3. Assembly of plasmid pDS56/RBSII-F28-6xHis(A)

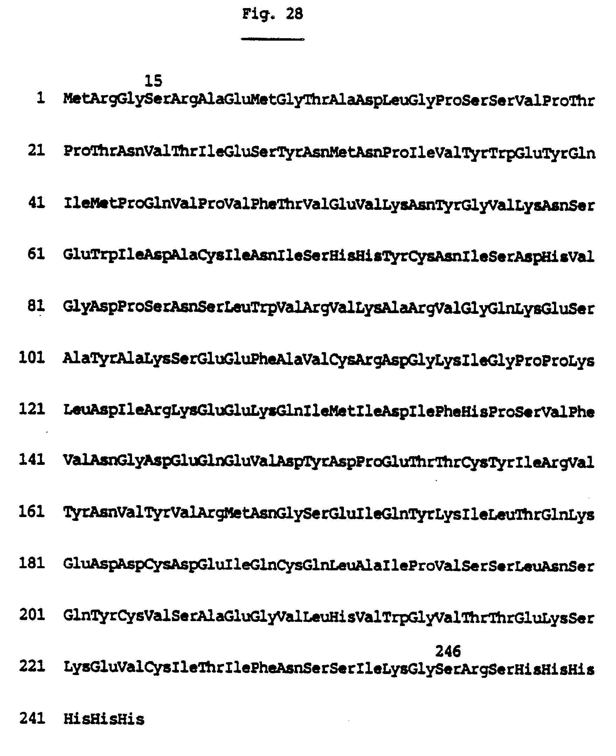

Together, fragments BamHI-F26'-PpuMI and PpuMI-F25'-BamHI were integrated into the unique cleavage site for restriction enzyme BamHI of plasmid pDS56/RBSII-6xHis (see Figs. 9 and 10). The resulting plasmid pDS56/RBSII-F28-6xHis(A) encodes protein P28H6A (Fig. 28) and was used for the production of this protein, which comprises amino acids 15 to 246 of the human IFN-$\gamma$ receptor.

F. Construction of plasmid pDS56/RBSII-F28-6xHis(B) encoding protein P28H6B

For construction of plasmid pDS56/RBSII-F28-6xHis(B) first fragment F28 was isolated from plasmid pDS56/RBSII-F28-6xHis(A). Subsequently, this DNA fragment was integrated into plasmid pDS56/RBSII-6xHis resulting in plasmid pDS56/RBSII-F28-6xHis(B).

1. Preparation of fragment F28

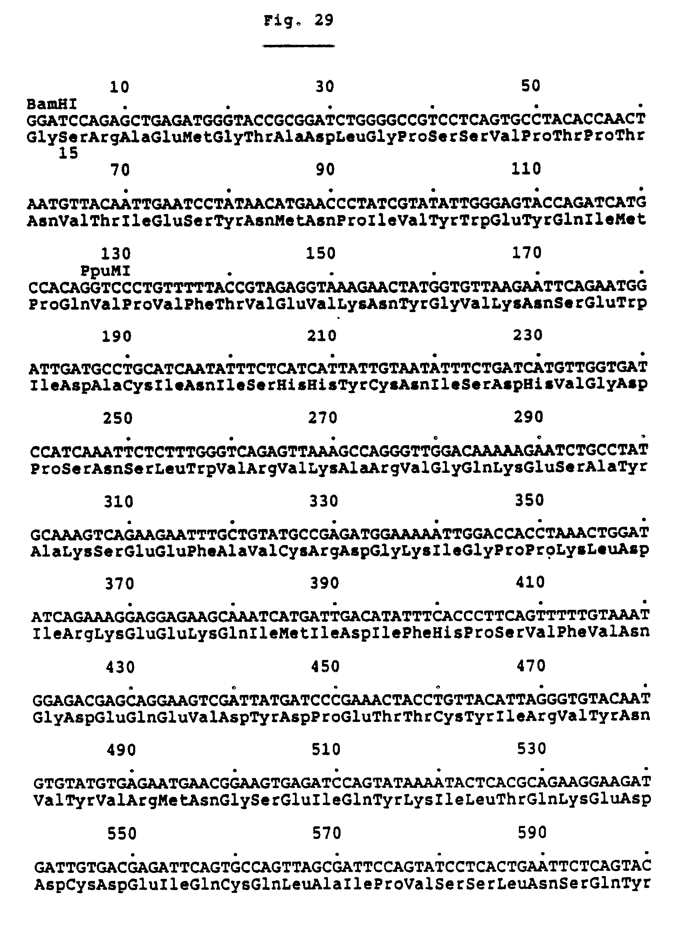

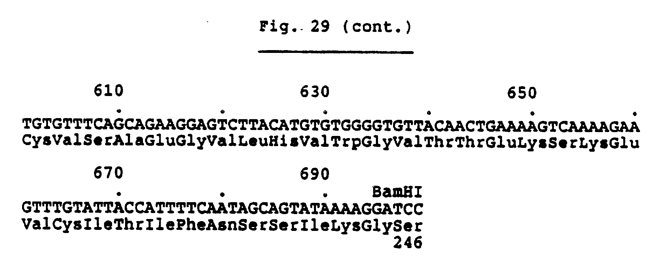

Plasmid pDS56/RBSII-F28-6xHis(A) was digested with restriction enzyme BamHI. From the resulting mixture of DNA fragments, fragment F28 (Fig. 29) encoding amino acids 15 to 246 of the human IFN-$\gamma$ receptor and one additional amino acid was isolated.

2. Assembly of plasmid pDS56/RBSII-F28-6xHis(B)

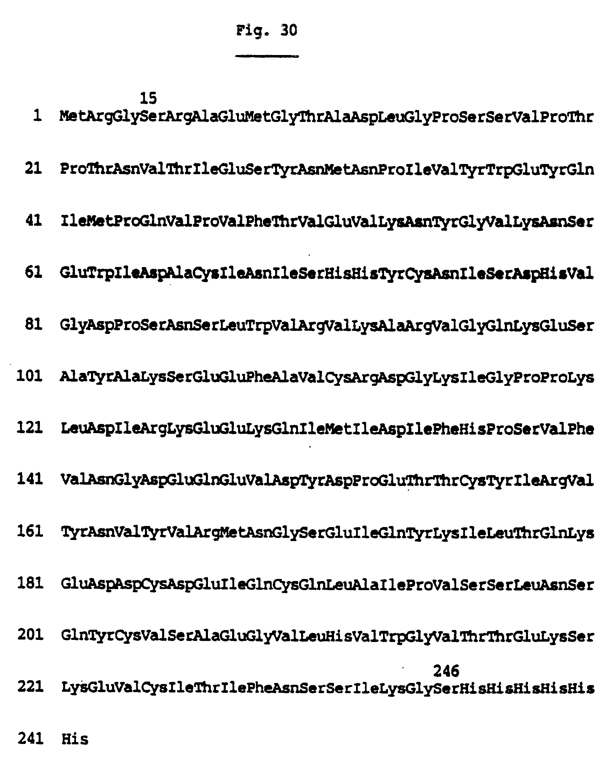

Fragment F28 was integrated between the unique cleavage sites for restriction enzymes BamHI and BglII of plasmid pDS56/RBSII-6xHis. The resulting plasmid pDS56/RBSII-F28-6xHis(B) encodes protein P28H6B (Fig. 30) and was used for the production of this protein, which comprises amino acids 15 to 246 of the human IFN-$\gamma$ receptor.

G. Construction of plasmid pDS56/RBSII,6xHis-F28 encoding protein P6H28

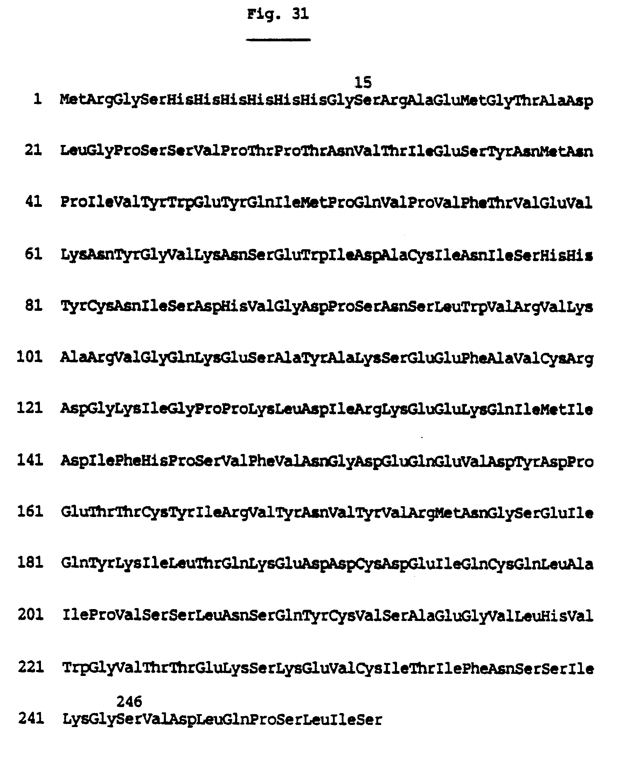

Plasmid pDS56/RBSII,6xHis-F28 was constructed by integrating fragment F28 (see Example 3.F, Fig. 29) into the unique cleavage site for restriction enzyme BamHI of plasmid pDS56/RBSII,6xHis (see Figs. 7 and 8). The resulting plasmid pDS56/RBSII,6xHis-F28 encodes protein P6H28 (Fig. 31), which comprises amino acids 15 to 246 of the human IFN-$\gamma$ receptor, and was used for the production of this protein.

H. Construction of plasmid pDS56/RBSII-F41-6xHis

Plasmid pDS56/RBSII-F41-6xHis was constructed in two steps: 1) a DNA fragment of plasmid pDS56/RBSII-F28-6xHis(A) comprising fragment F28 was mutagenized on the DNA level without affecting the amino acid sequence of the encoded human IFN-$\gamma$ receptor and 2) from the mutagenized DNA, fragment F41 was isolated and integrated into plasmid pDS56/RBSII-6xHis resulting in plasmid pDS56/RBSII-F41-6xHis.

1. Mutagenesis of fragment F28

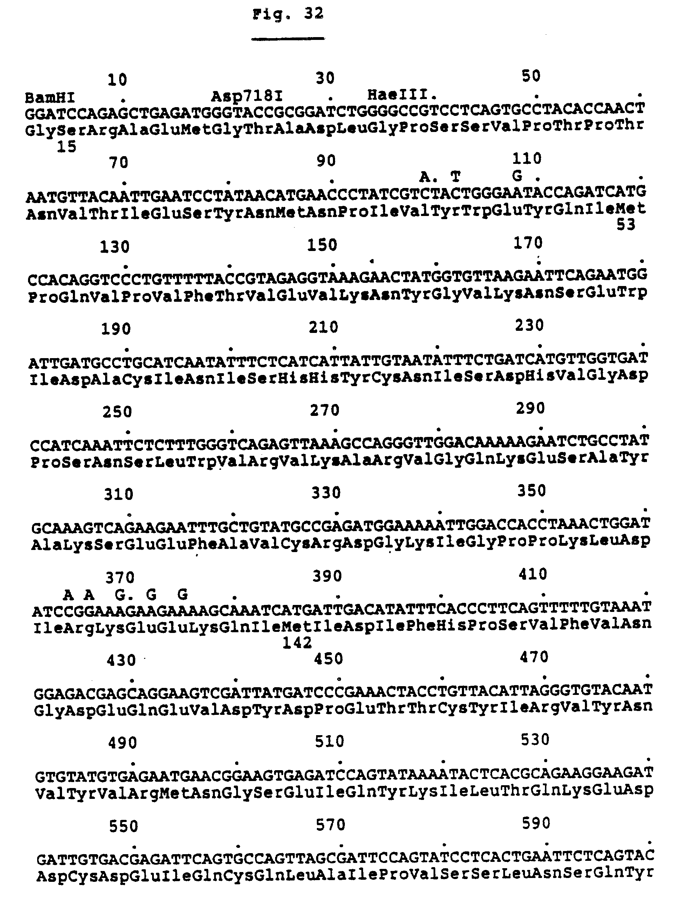

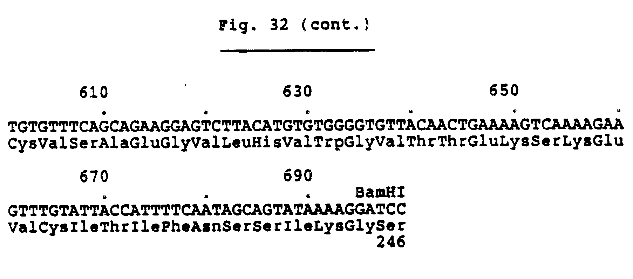

The EcoRI/HindIII fragment of plasmid pDS56/RBSII-F28-6xHis(A) (see Example 3.E) comprising the ribosomal binding site RBSII, fragment F28 encoding amino acids 15 to 246 of the human IFN-γ receptor (see Fig. 29), and the region encoding six histidine residues was integrated into the unique cleavage sites for restriction enzymes EcoRI and HindIII of plasmid M13mp19 (Pharmacia). In the resulting plasmid, the gene for the receptor was simultaneously mutagenized using primers 2 and 3 (Fig. 17). Due to this mutagenesis, some nucleotides in the DNA preceeding the regions encoding amino acids 53 and 142 of the human IFN-γ receptor were exchanged without affecting the amino acid sequence of the receptor (compare Figs. 29 and 32). The resulting plasmid carries fragment F41, which nucleotide sequence and deduced amino acid sequence are shown in Fig. 32.

2. Assembly of plasmid pDS56/RBSII-F41-6xHis

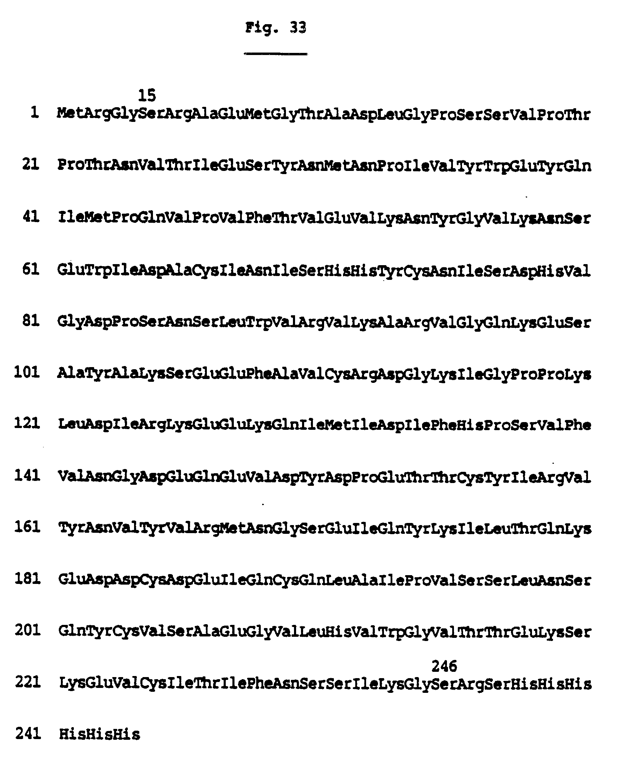

The mutagenized DNA was digested with restriction enzyme BamHI and fragment F41 (Fig. 32) was isolated from the resulting mixture of DNA fragments. Fragment F41 encodes amino acids 15 to 246 of the human IFN-γ receptor and one additional amino acid. Finally, fragment F41 was integrated into the unique cleavage site for BamHI of plasmid pDS56/RBSII-6xHis (see Figs. 9 and 10) resulting in plasmid pDS56/RBSII-F41-6xHis. This plasmid encodes protein P41H6 (Fig. 33), which comprises amino acids 15 to 246 of the human IFN-γ receptor, and was used for the production of this protein. Proteins P28H6A (see Fig. 28) and P41H6A are identical.

I. Construction of plasmid pDS56/RBSII,NcoI-F43-6xHis encoding protein P43H6

Plasmid pDS56/RBSII,NcoI-F43-6xHis was constructed in three steps: 1) fragment F43 was prepared by modifying the 5'-end of fragment F41, 2) fragments XhoI/NcoI and BamHI/XhoI were isolated from plasmids pDS56/RBSII,NcoI and pDS56/RBSII-6xHis, respectively, and 3) all three fragments were assembled resulting in plasmid pDS56/RBSII,NcoI-F43-6xHis.

1. Preparation of fragment F43

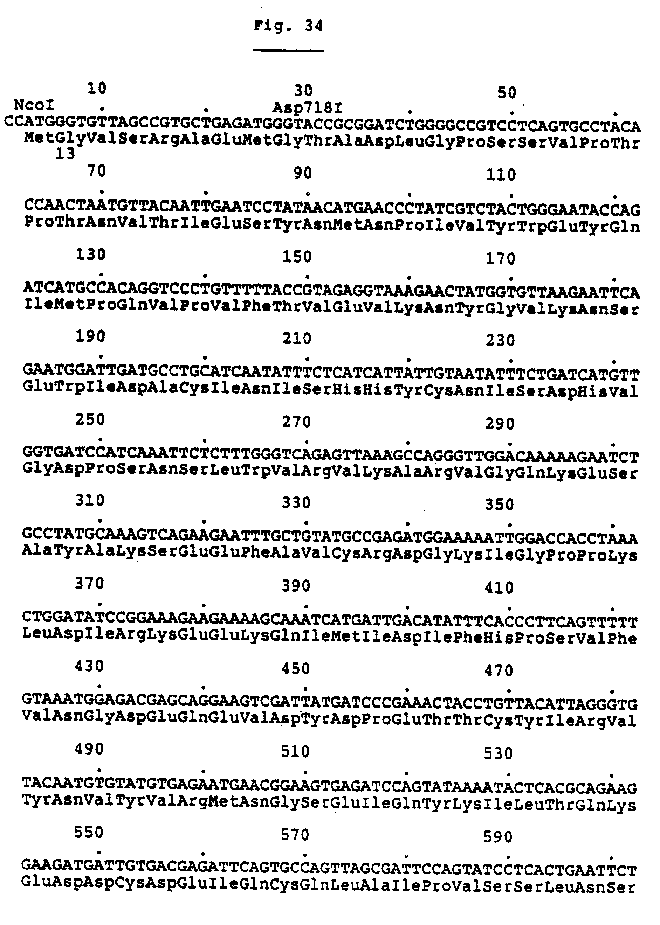

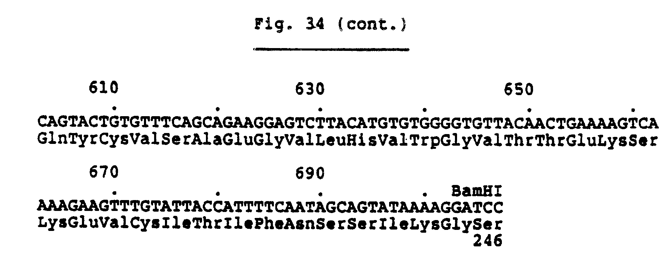

Plasmid pDS56/RBSII-F14-6xHis (see Example 3.H. and Fig. 32) was cut at its unique cleavage site for restriction enzyme Asp718I. To the ends of the resulting DNA fragment a phosphorylated DNA adaptor (adaptor 2, Fig. 17) was ligated, before the DNA was digested with restriction enzymes NcoI and BamHI. Subsequently, fragment F43 was isolated from the mixture of DNA fragments. The nucleotide sequence and the deduced amino acid sequence of F43 are shown in Fig. 34. Fragment F43 encodes amino acids 13 to 246 of the human IFN-γ receptor and one additional methionine residue.

2. Preparation of fragments XhoI/NcoI and BamHI/XhoI

Plasmid pDS56/RBSII,NcoI (see Figs. 3 and 4) was digested with restriction enzymes XhoI and NcoI. From the resulting mixture of DNA fragments, fragment XhoI/NcoI comprising N250PSN250P29 and RBSII,NcoI was isolated. Plasmid pDS56/RBSII-6xHis (see Figs. 9 and 10) was digested with restriction enzymes BamHI and XhoI. From the resulting mixture of DNA fragments, fragment BamHI/XhoI containing the replication region was isolated.

3. Assembly of plasmid pDS56/RBSII,NcoI-F43-6xHis

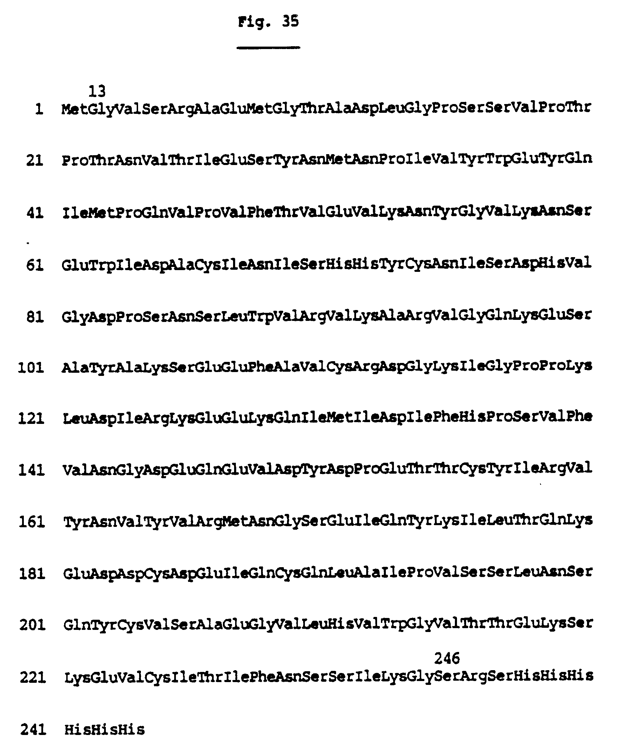

DNA fragments XhoI/NcoI, F43 and BamHI/XhoI were assembled resulting in plasmid pDS56/RBSII,NcoI-F43-6xHis. This plasmid encodes protein P43H6 (Fig. 35), which comprises amino acids 13 to 246 of the human IFN-γ receptor, and was used for the production of this protein.

J. Construction of plasmid pDS56/RBSII,SpHI-F44-6xHis encoding protein P44H6

Plasmid pDS56/RBSII,SphI-F44-6xHis was constructed in three steps: 1) fragment F44 was prepared by modifying the 5'-end of fragment F41, 2) fragment XhoI/SphI was isolated from plasmid pDS56/RBSII,SphI, and 3) fragments F44, XhoI/SphI and BamHI/XhoI were assembled resulting in plasmid pDS56/RBSII,SphI-F44-6xHis.

1. Preparation of fragment F44

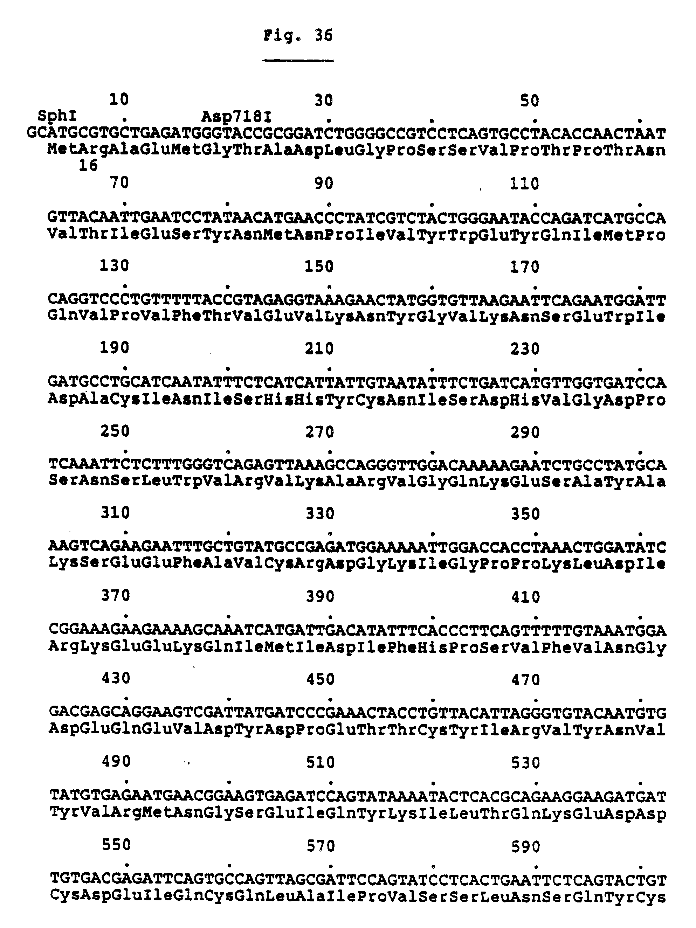

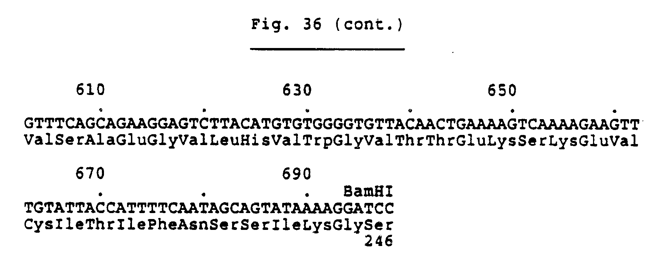

Plasmid pDS56/RBSII-F41-6xHis (see Example 3.H and Fig. 32) was cut at its unique cleavage site for restriction enzyme Asp718I. To the ends of the resulting DNA fragment a phosphorylated adaptor (adaptor 3, Fig. 17) was ligated, before the DNA was digested with restriction enzymes SphI and BamHI. Subsequently, fragment F44 was isolated from the mixture of DNA fragments. The nucleotide sequence and the deduced amino acid sequence of F44 are shown in Fig. 36. Fragment F44 encodes amino acids 16 to 246 of the human IFN-$\gamma$ receptor and one additional methionine residue.

2. Preparation of fragment XhoI/SphI

Plasmid pDS56/RBSII,SphI (see Figs. 5 and 6) was digested with restriction enzymes XhoI and SphI. From the resulting mixture of DNA fragments, fragment XhoI/SphI comprising N250PSN250P29 and RBSII,SphI was isolated.

3. Assembly of plasmid pDS56/RBSII,SphI-F44-6xHis

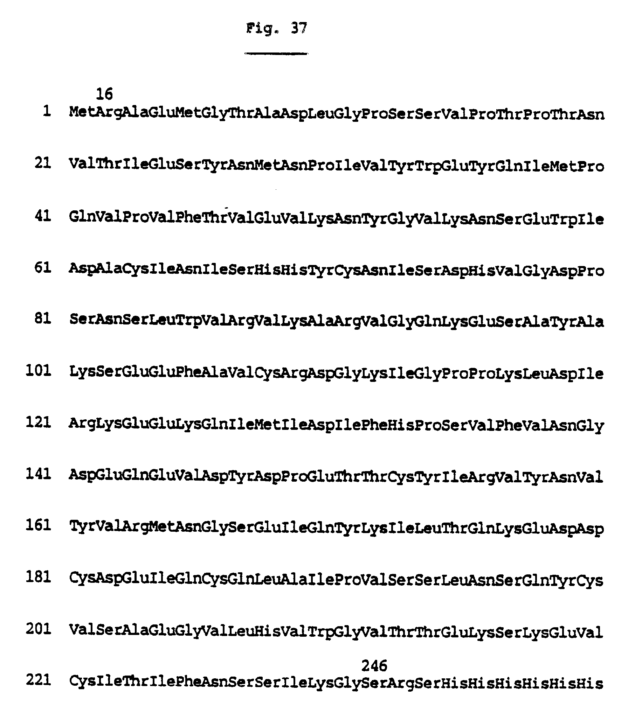

DNA fragments XhoI/SphI, F44 and BamHI/XhoI (see above) were assembled resulting in plasmid pDS56/RBSII,SphI-F44-6xHis. This plasmid encodes protein P44H6 (Fig. 37), which comprises amino acids 16 to 246 of the human IFN-$\gamma$ receptor, and was used for the production of this protein.

K. Construction of plasmid pDS56/RBSII,NcoI-F45-6xHis encoding protein P45H6

Plasmid pDS56/RBSII,NcoI-F45-6xHis was constructed in two steps: 1) fragment F45 was prepared by modifying the 5'-end of fragment F41 and 2) fragments XhoI/NcoI, F45 and BamHI/XhoI were assembled resulting in plasmid pDS56/RBSII,NcoI-F45-6xHis.

1. Preparation of fragment F45

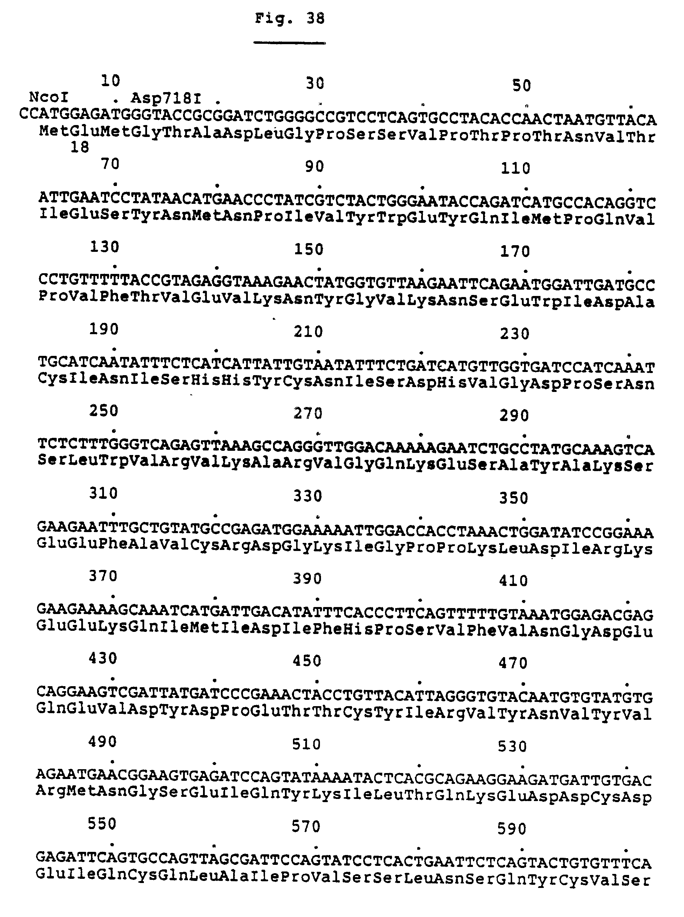

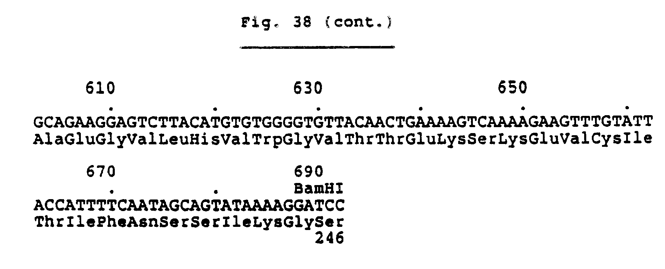

Plasmid pDS56/RBSII-F41-6xHis (see Example 3.H and Fig. 32) was cut at its unique cleavage site for restriction enzyme Asp718I. To the ends of the resulting DNA fragment a phosphorylated adaptor (adaptor 4, Fig. 17) was ligated, before the DNA was digested with restriction enzymes NcoI and BamHI. Subsequently, fragment F45 was isolated from the mixture of DNA fragments. The nucleotide sequence and the deduced amino acid sequence of F45 are shown in Fig. 38. Fragment F45 encodes amino acids 18 to 246 of the human IFN-$\gamma$ receptor and one additional methionine residue.

2. Assembly of plasmid pDS56/RBSII,NcoI-F45-6xHis

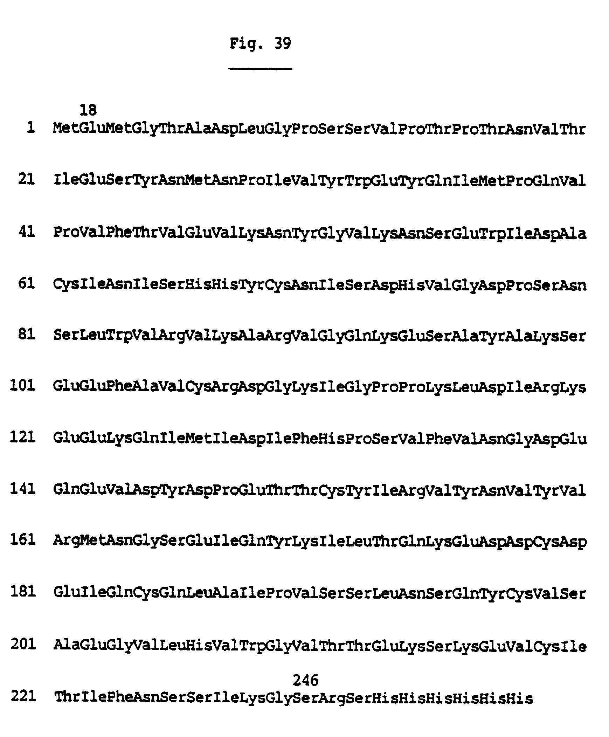

DNA fragments XhoI/NcoI (see above), F45 and BamHI/XhoI (see above) were assembled resulting in plasmid pDS56/RBSII,NcoI-F45-6xHis. This plasmid encodes protein P45H6 (Fig. 39), which comprises amino acids 18 to 246 of the human IFN-$\gamma$ receptor, and was used for the production of this protein.

L. Construction of plasmid pDS56/RBSII,NcoI-F46-6xHis encoding protein P46H6

Plasmid pDS56/RBSII,NcoI-F46-6xHis was constructed in two steps: 1) fragment F46 was prepared by modifying the 5'-end of fragment F41 and 2) fragments XhoI/NcoI, F46 and BamHI/XhoI were assembled resulting in plasmid pDS56/RBSII,NcoI-F46-6xHis.

1. Preparation of fragment F46

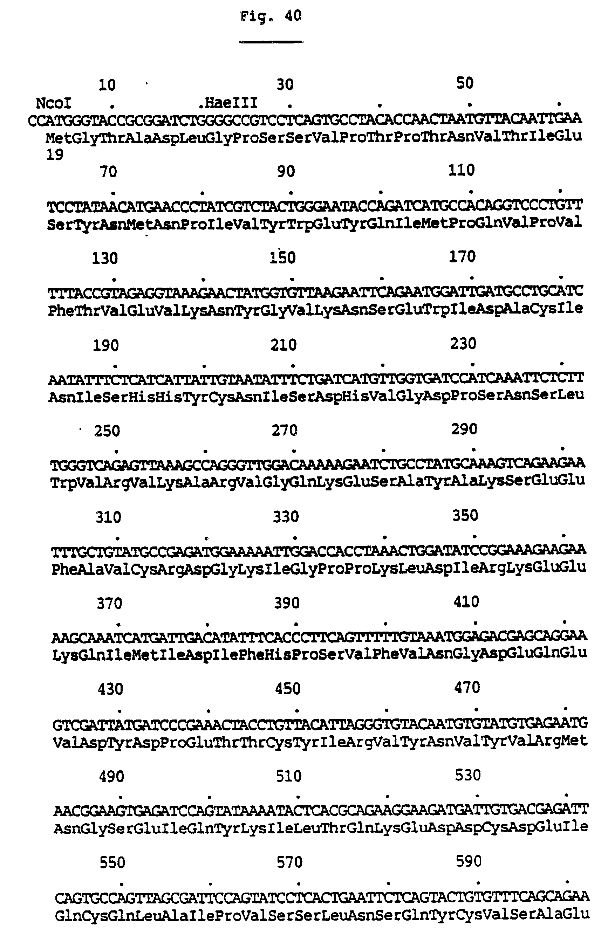

Plasmid pDS56/RBSII-F41-6xHis (see Example 3.H and Fig. 32) was digested with restriction enzyme HaeIII. To the ends of the resulting DNA fragments a phosphorylated adaptor (adaptor 5, Fig. 17) was ligated, before the DNA was digested with restriction enzymes NcoI and BamHI. Subsequently, fragment F46 was isolated from the mixture of DNA fragments. The nucleotide sequence and the deduced amino acid sequence of F46 are shown in Fig. 40. Fragment F46 encodes amino acids 19 to 246 of the human IFN-γ receptor.

2. Assembly of plasmid pDS56/RBSII,NcoI-F46-6xHis

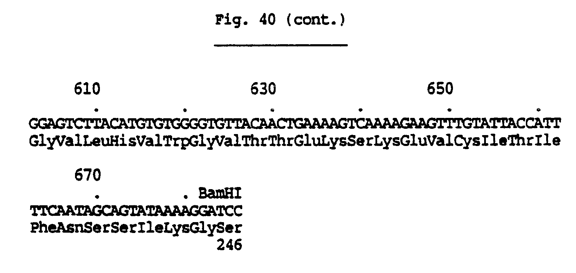

DNA fragments XhoI/NcoI (see Example 3.I), F46 and BamHI/XhoI (see Example 3.I) were assembled resulting in plasmid pDS56/RBSII,NcoI-F46-6xHis. This plasmid encodes protein P46H6 (Fig. 41), which comprises amino acids 19 to 246 of the human IFN-γ receptor, and was used for the production of this protein.

Example 4

Expression of human IFN-γ receptor derivatives in E.coli including the soluble human IFN-γ receptors of the present invention (for short human IFN-γ receptor derivatives)

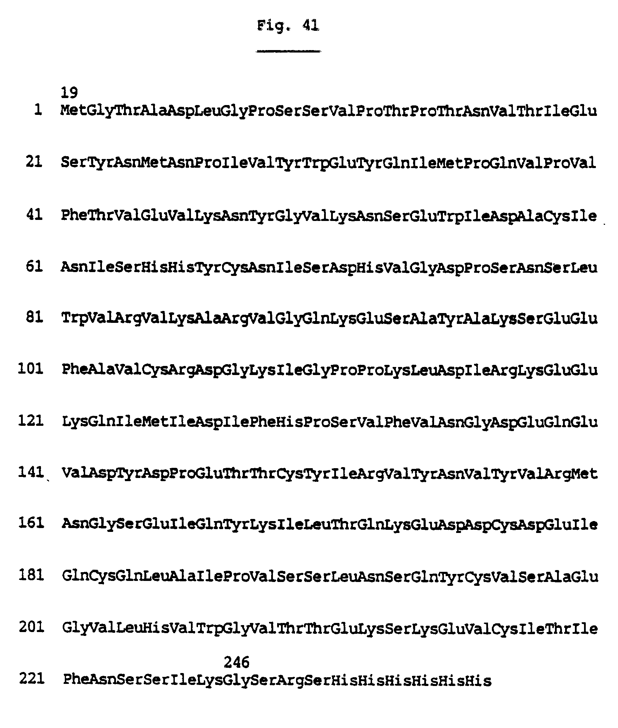

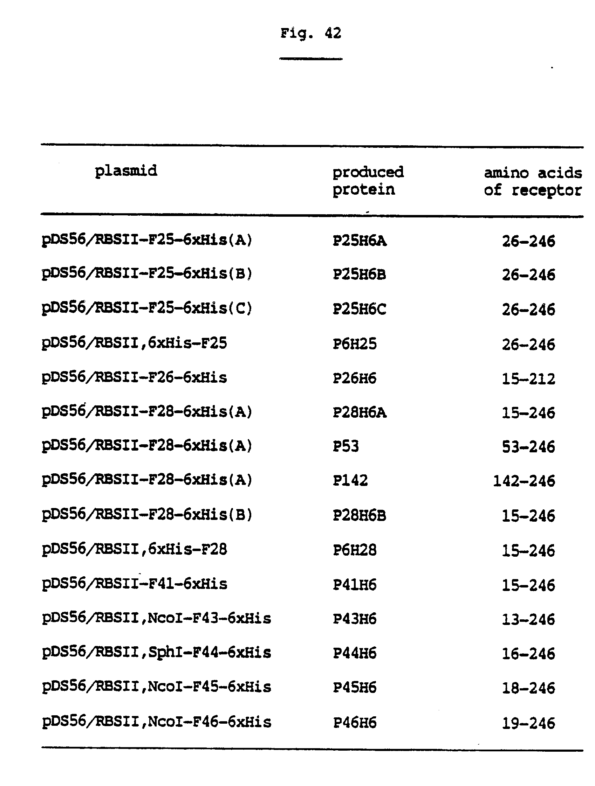

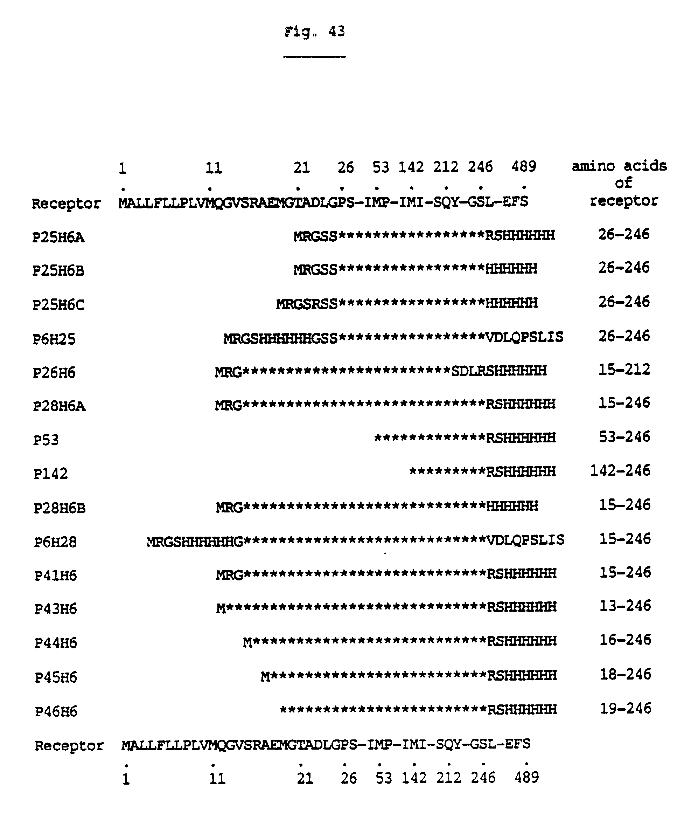

E.coli M15 cells containing plasmid pDMI,1 were transformed independently with the plasmids shown in Fig. 42 and subsequently grown at 37°C in LB medium (10 g bacto tryptone, 5 g yeast extract, 5 g NaCl per litre) containing 100 mg/l ampicillin and 25 mg/l kanamycin. At an optical density at 600 nm of about 0.7 to 1.0 IPTG was added to a final concentration of 2mM. After additional 2.5 to 5 h at 37°C the cells were harvested by centrifugation and analyzed for the presence of the expected derivatives of the human IFN-γ receptor (Fig. 42) by SDS-PAGE (polyacrylamide electrophoresis in presence of sodium dodecylsulfate) under reducing conditions (3% β-mercaptoethanol in the sample buffer) as described in the literature (Laemmli, Nature 227, 680-685 [1970]).
The expected amino acid sequences of the various human IFN-γ receptor derivatives expressed in E.coli are shown in Fig. 43. Also included in this figure are the amino acid sequences of proteins P53 and P142. These proteins are produced in addition to the expected proteins in E.coli cells containing e.g. plasmid pDS56/RBSII-F28-6xHis(A) but are produced to an about 5 fold lower yield in E.coli cells containing plasmid pDS56/RBSII-F41-6xHis. Plasmid pDS56/RBSII-F41-6xHis carries fragment F41, which differs from fragment F28 contained in plasmid pDS56/RBSII-F28-6xHis(A) not by the encoded amino acid sequence but only by some nucleotides preceeding the regions encoding the methionine residues at positions 53 and 142 of the human IFN-γ receptor (see Fig. 32). Therefore, proteins P53 and P142 are the products of internal start of translation within the mRNA and contain the methionine residues at position 53 and 142, respectively, of the receptor as their N-terminal amino acids. Almost all proteins listed in Fig. 42 were produced in E.coli to a level of more than 5 mg/l of culture. The exceptions were proteins P43H6, P44H6, P45H6 and P46H6, which were producted to a level of about 0.1-0.5 mg/l of culture.

Example 5

Characterization of human IFN-γ derivatives expressed in E.coli

The human IFN-γ derivatives expressed in E.coli were extracted from the cells and enriched by Ni-chelate affinity chromatography, before they were analyzed for their recognition by monoclonal antibodies directed against the receptor purified from Raji cells and their ability to bind IFN-γ specifically.

A. Enrichment of the receptor derivatives by Ni-chelate affinity chromatography

10 g E.coli cells containing one of the various receptor derivatives were thawed, before 100 ml of buffer A (6 M guanidine-hydrochloride, 0.1 M sodium phosphate, pH 8.0) were added. After stirring (100 rpm) for 2 h, the suspension was centrifuged for 10 minutes at 10000 xg. The supernatant was loaded at 42 ml/h onto a column (1.6 x 4 cm) containing 8 ml of the NTA-resin described in European Patent Applications Nos. 253 303 and 282 042. Then, the column was washed with 50 ml of buffer A. Subsequently, the column was washed with 30 ml of buffer B (8 M urea, 0.1 M sodium phosphate, 0.01 M Tris, pH 8.0), and then with 50 ml of buffer B, pH 6.5. Finally, the receptor derivative was eluted with buffer B, pH 4.5.

B. Recognition of the receptor derivatives by monoclonal antibodies

First, the isolated receptor derivatives were separated by SDS-PAGE (Laemmli, supra, sample buffer without β-mercaptoethanol) in a 12% polyacrylamide gel. Then, the proteins were electrotransfered from the gel to nitrocellulose paper in a TRANS-BLOT™ CELL (Biorad). This transfer was performed according to the instructions of the manufacturer under cooling with water for 2.5 h in a buffer containing 192 mM glycine, 25 mM Tris, 20% methanol, pH 8.3. Subsequently, the nitrocellulose paper was first incubated for 1.5 h in buffer C (8.1 mM $NaH_2PO_4$, 1.5 mM $KH_2PO_4$, 2.7 mM KCl, 137 mM NaCl, 1% defatted milk-powder, pH 7.2) and then incubated for 4 h in buffer C containing a radiolabelled monoclonal antibody against native human IFN-$\gamma$ receptor as described in Example 10.C. After removal of this solution, the nitrocellulose paper was washed three times for 10 minutes each in buffer C and then three times in buffer C lacking the milk-powder but containing 0.02% Tween 20. After drying of the nitrocellulose paper, proteins recognized by the monoclonal antibody were visualized by autoradiography. As summarized in Fig. 44, all the proteins, with exception of protein P142, were recognized by the monoclonal antibody.

C. Ability of the receptor derivatives to bind IFN-$\gamma$

The ability of the receptor derivatives to bind IFN-$\gamma$ was analyzed by following the same procedure as described under B., except that iodinated IFN-$\gamma$ (see Example 9) replaced the monoclonal antibody. As summarized in Fig. 44, all receptor derivatives comprising at least amino acids 26 to 246 of the human IFN-$\gamma$ receptor were able to bind IFN-$\gamma$, whereas proteins lacking amino acids 1 to 52 or 213 to 489 of the receptor did not bind the ligand.

The receptor derivatives comprising amino acids 26 to 246 of the human IFN-$\gamma$ receptor did not bind IFN-$\gamma$ in experiments, in which the proteins were loaded in sample buffer containing 3% $\beta$-mercaptoethanol onto the polyacrylamide gels.

Example 6

Extraction and purification of soluble human IFN-$\gamma$ receptors from E.coli cells

Protein P41H6 (see Figs. 33, 42 and 43) expressed in E.coli was purified and obtained as a soluble protein. The recovery procedure included the following steps:

(I) Disruption of the cells and extraction of the proteins with guanidine-hydrochloride;

(II) Ni-chelate affinity chromatography (most of the bacterial proteins were removed);

(III) Chromatography on a sizing column in the presence of urea (polymeric forms of protein P41H6 were removed);

(IV) Refolding of the protein under removal of urea; and

(V) Chromatography on an anion exchanger column (smaller fragments and wrong conformational forms of P41H6 were removed).

Step I: Disruption of the cells and extraction of the proteins

100 g of frozen (-70 °C) cells containing protein P41H6 were kept at 4 °C for 16 h. Then, the thawed cells were suspended in 1 liter of buffer E (7 M guanidine-hydrochloride, 0.1 M sodium phosphate, pH 8.0) using a Polytron homogenizer. This suspension was sonicated for 5 minutes with a Branson sonifier (position 8) and then stirred at 22 °C for 1 h. The resulting suspension was kept on ice for 1 h and then centrifuged at 12000 xg for 1 h. Finally, the supernatant containing protein P41H6 was collected.

Step II: Ni-chelate affinity chromatography

A column (5 x 5 cm), containing 100 ml NTA-resin, which was prepared as described in European Patent Applications Publication Nos. 253 303 and 282 042, was loaded with $Ni^{++}$, washed with 0.2 M acetic acid and equilibrated in buffer E at 4 °C (all further operations were carried out at 4 °C). The supernatant of step I was loaded at 50 ml/h onto this column, before the column was washed first with 1 l of buffer E and then with 1 l of buffer F (7 M urea, 0.1 M Tris/HCl, pH 8.0). Proteins still bound to the NTA-resin were eluted with 1 l of buffer G (7 M urea, 0.1 M Tris-maleate, pH 6.3) and, finally, with 1 l of buffer H (7 M urea, 0.1 M Tris-maleate, pH 5.9). The collected fractions were analyzed by SDS-PAGE under reducing and non reducing conditions for presence of protein P41H6. Those fractions containing protein p41H6 mainly in its monomeric form were combined resulting in a volume of about 1 liter.

Step III: Sizing column

The solution of step II with protein P41H6 in buffer H was concentrated under nitrogen pressure to a volume of 20 ml using an ultrafiltration system (Amicon; membrane cut-off 10000 $M_r$). This solution was loaded at a flow-rate of 30 ml/h onto a column (5 x 90 cm) containing 1,8 l of Sephadex G-100 (Pharmacia) equilibrated in buffer I (7 M urea, 0.05 M Tris/HCl, pH 7.5). Then, the column was further developed at the same flow-rate with 700 ml of buffer I. The appearance of protein in the collected fractions was monitored by UV-absorption. Two protein peaks were obtained and analyzed by SDS-PAGE. The first peak appeared after 450 - 500 ml of solution had passed the column and contained mainly polymeric forms of protein P41H6. The second peak appeared after 550 - 600 ml of solution had been collected and contained protein P41H6 in monomeric form together with some additional proteins, e.g. proteins P53 and P142 (see Example 4). Fractions containing protein P41H6 in its monomeric form were combined resulting in a volume of about 100 ml.

Step IV: Refolding

The solution of step III containing protein P41H6 in its monomeric form was adjusted to 10 mM benzamidine-HCl, 1 mM phenylmethane-sulfonyl fluoride (PMSF) and 5 $\mu$g/ml aprotinin by adding solid benzamidine-HCl, a solution of 0,2 M PMSF in DMSO and aprotinin. The resulting solution was then dialyzed (dialysis tubing cut-off 10000 $M_r$) against 3 liters of 100 mM Tris/HCl, 10% glycerol, 10 mM benzamidine, 1 mM PMSF and 5 $\mu$g/ml aprotinin at 4°C with three changes of the dialysis buffer. Subsequently, the solution was centrifuged for 30 minutes at 10000 xg, before the supernatant containing protein P41H6 in its monomeric form was either frozen or directly worked up.

Step V: Chromatography on an ion-exchanger column

The solution of step IV containing protein P41H6 was dialyzed against 50 mM sodium phosphate, 0.2 M NaCl, 1 mM PMSF and 1 mM benzamidine-HCl, pH 7.0. After centrifugation for 30 minutes at 10000 xg, the supernatant was applied at 50 ml/h onto a column (1.5 x 25 cm) containing 45 ml Polybuffer exchanger 94 (Pharmacia) equilibrated in 50 mM sodium phosphate, 200 mM NaCl, 1 mM PMSF and 1 mM benzamidine-HCl, pH 6.0. The column was washed with 200 ml of the same buffer, before the protein was eluted with a gradient from 0.2 M to 0.6 M NaCl in 50 mM sodium-phosphate, pH 6.0. 10 ml fractions were collected and analysed by SDS-PAGE. Fragments containing mainly protein P41H6 in its monomeric form were combined and the resulting fractions were stored at -20°C.

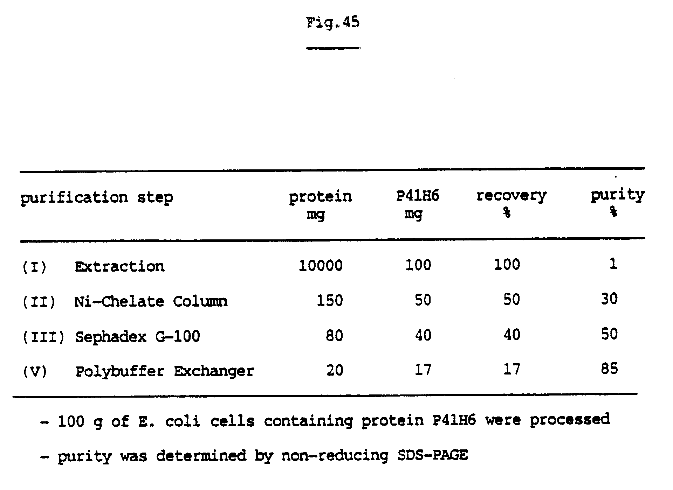

The purification of protein P41H6 described above is summarized in Fig. 45. 17 mg of soluble and monomeric P41H6 with a purity of about 85% were obtained from 100 g of cells.

Proteins P26H6, P25H6C and P28H6A (see Fig. 43) were purified according to the protocol described above, except that step V was omitted. Since only 10 g of cells were processed, the sizes of the columns and the volumes of the solutions were reduced proportionally. All three proteins were obtained with a purity of about 50% as soluble and monomeric molecules.

In contrast to protein P26H6, proteins P25H6C, P28H6A and P41H6 were able to bind IFN-$\gamma$ (see Example 5 and Fig. 44) and inhibited the binding of IFN-$\gamma$ to the human IFN-$\gamma$ receptor present on Raji cells (see Example 10). Furthermore, proteins P25H6C and P28H6A were able to inhibit the antiviral activity caused by IFN-$\gamma$ (see Example 10, protein P41H6 was not included in this test).

Therefore, proteins P25H6C, P28H6A and P41H6 contain that region of the human IFN-$\gamma$ receptor, which is essential for the binding of its ligand, in an active form.

## Example 7

## Description of plasmids used for the expression of receptor derivatives in COS-cells

### A. Principles

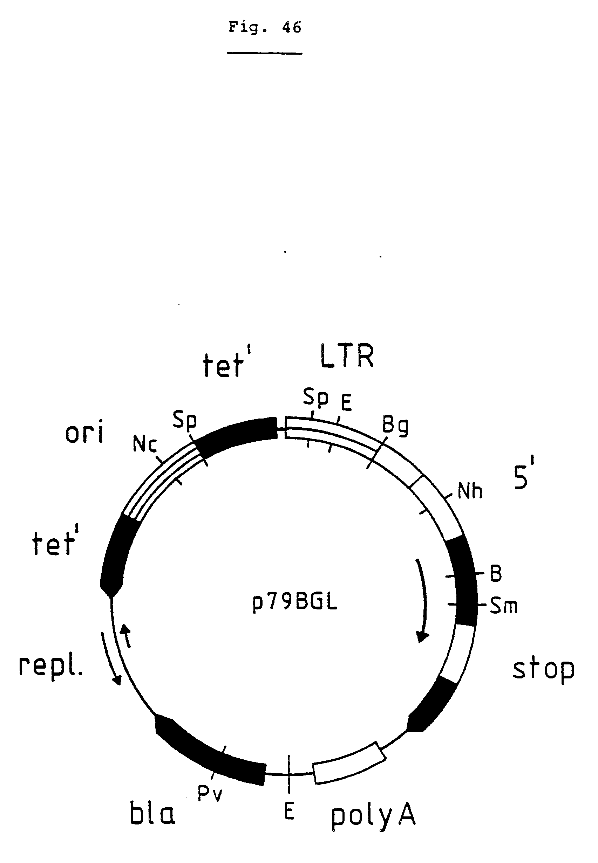

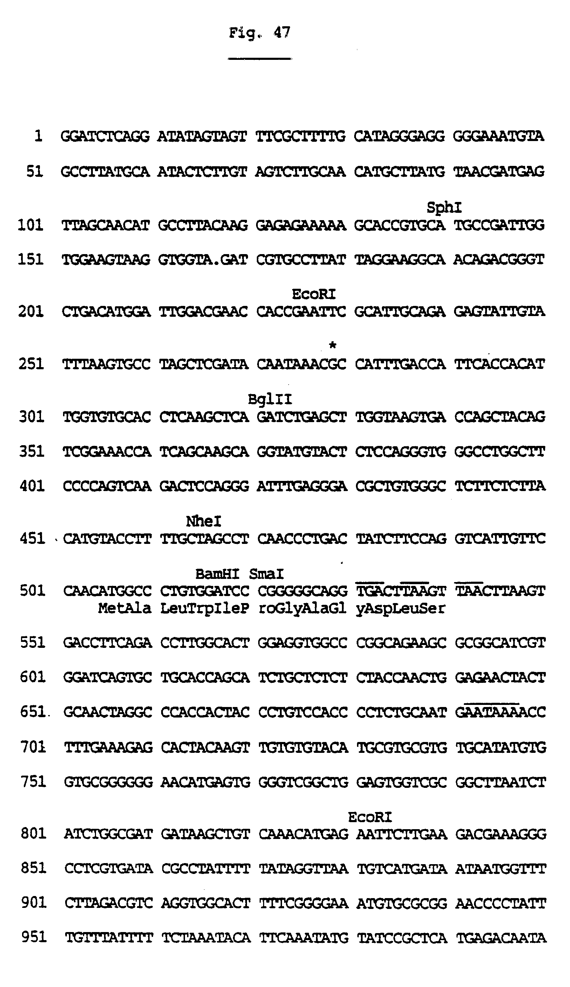

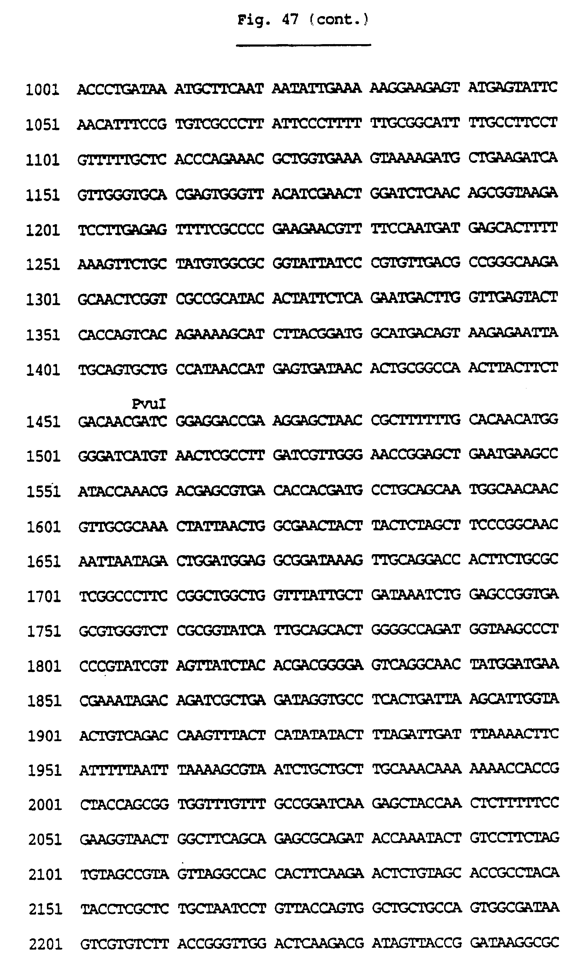

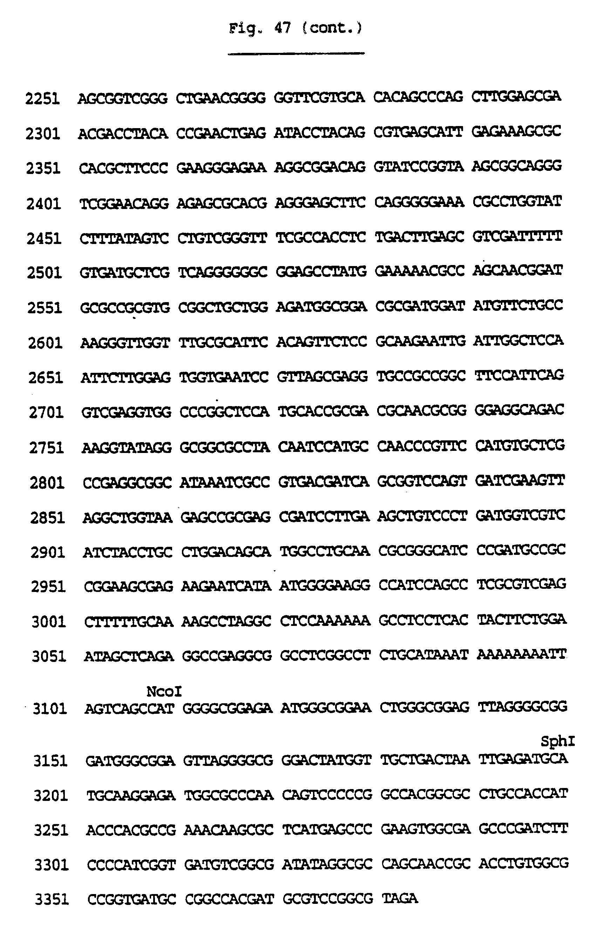

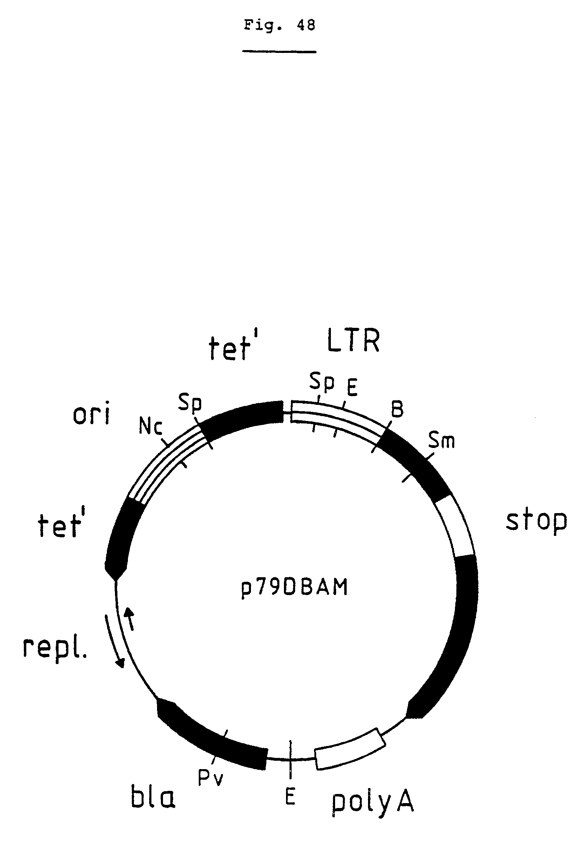

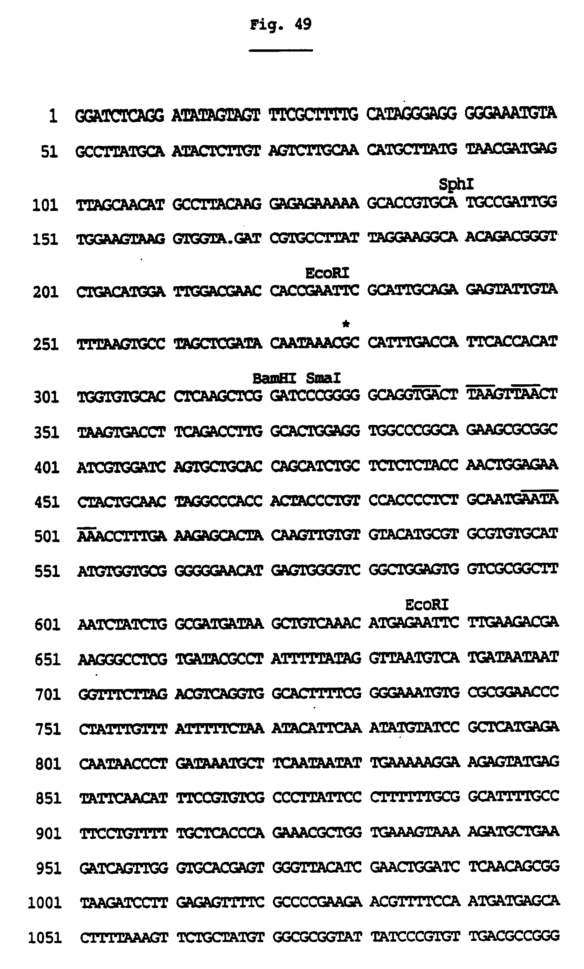

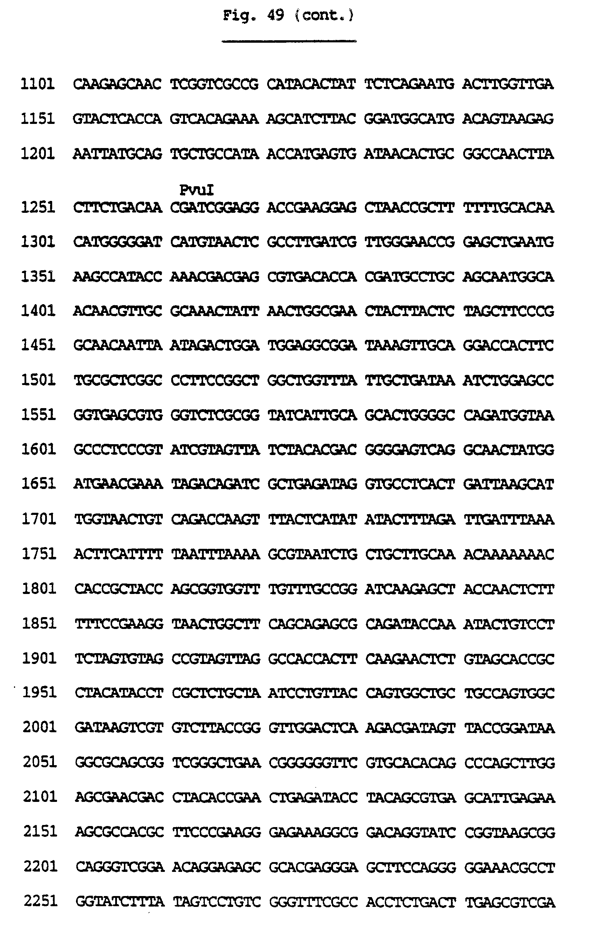

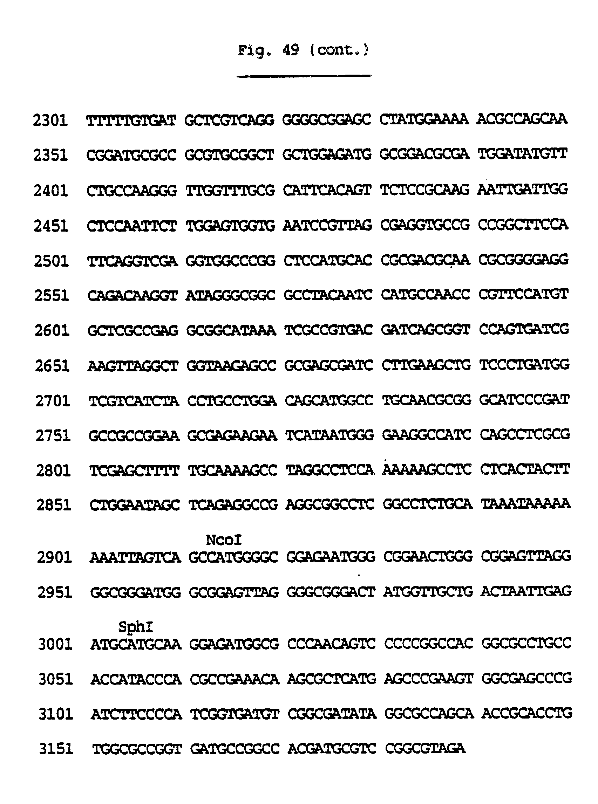

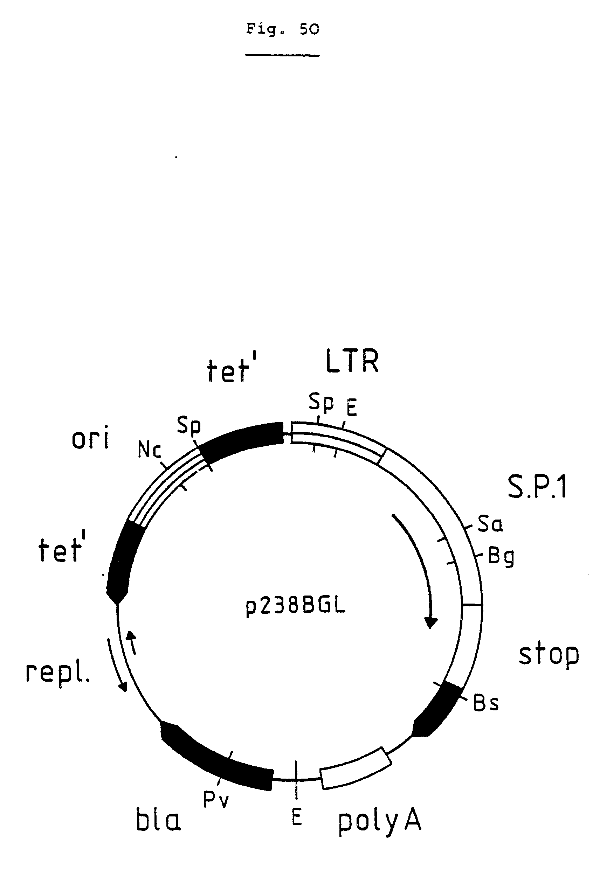

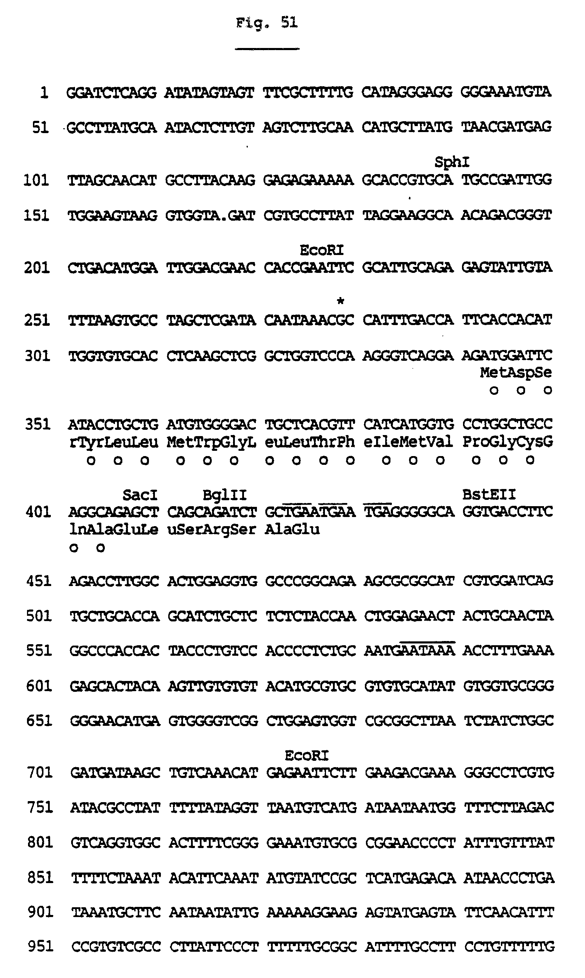

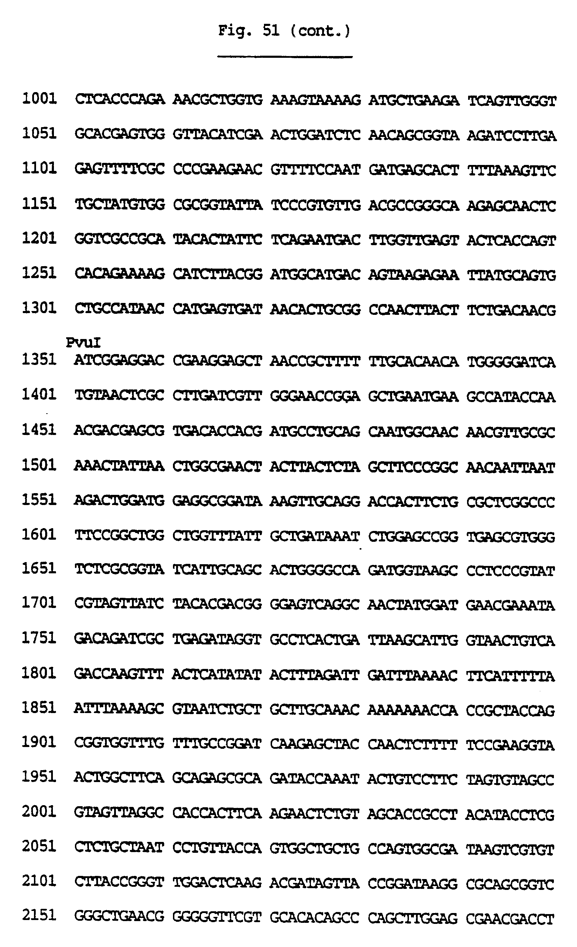

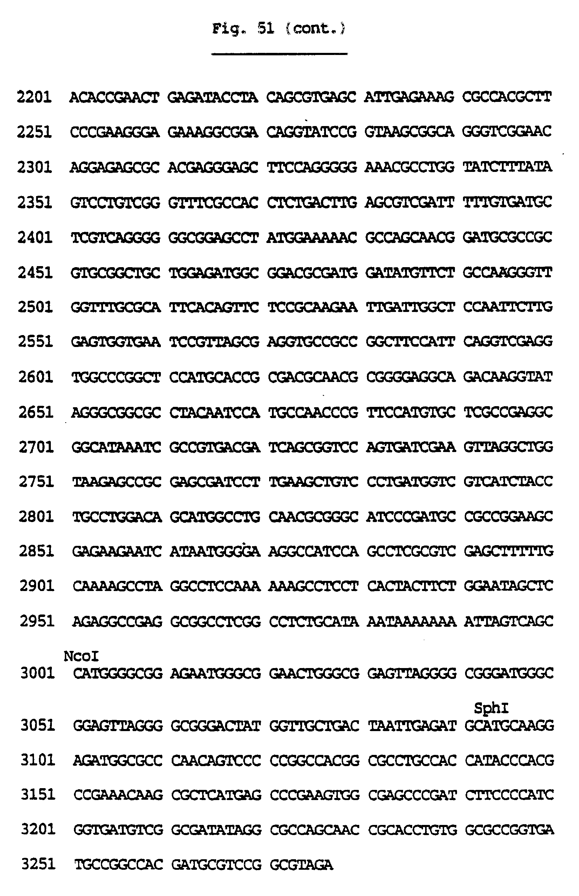

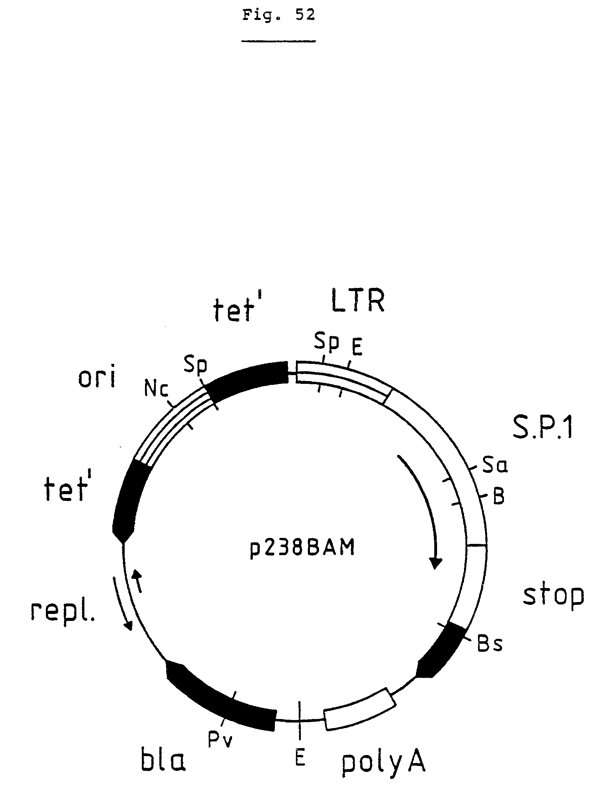

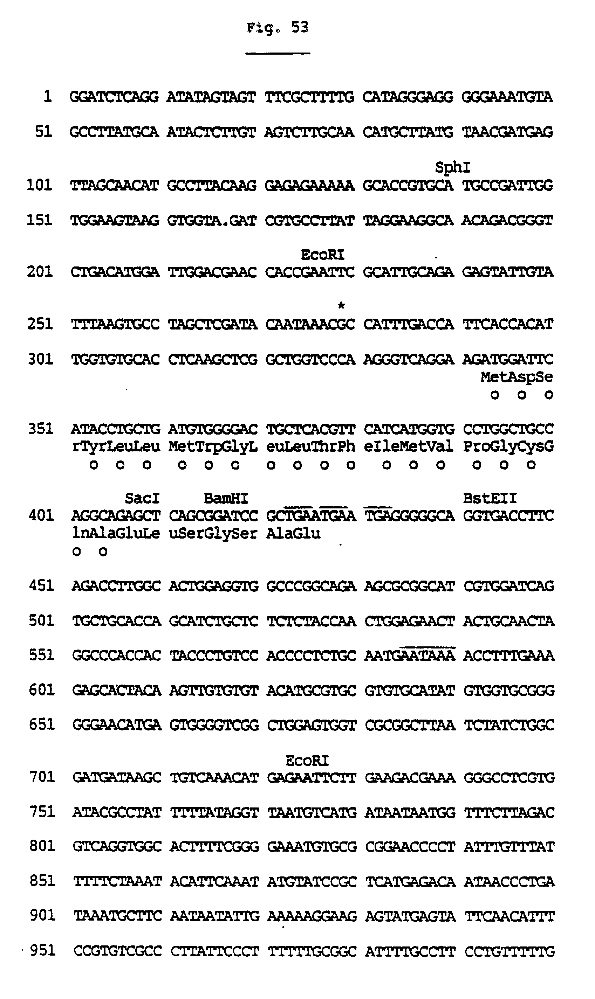

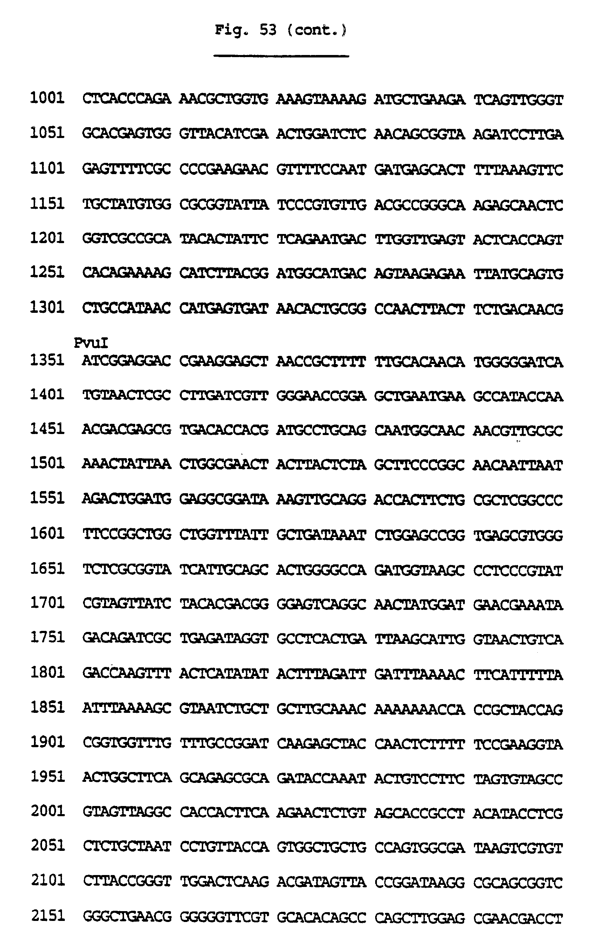

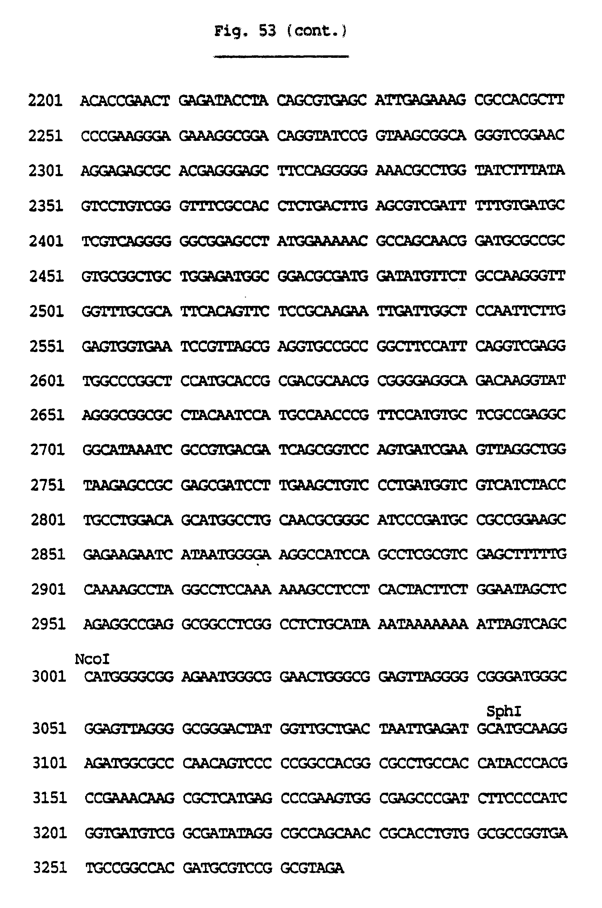

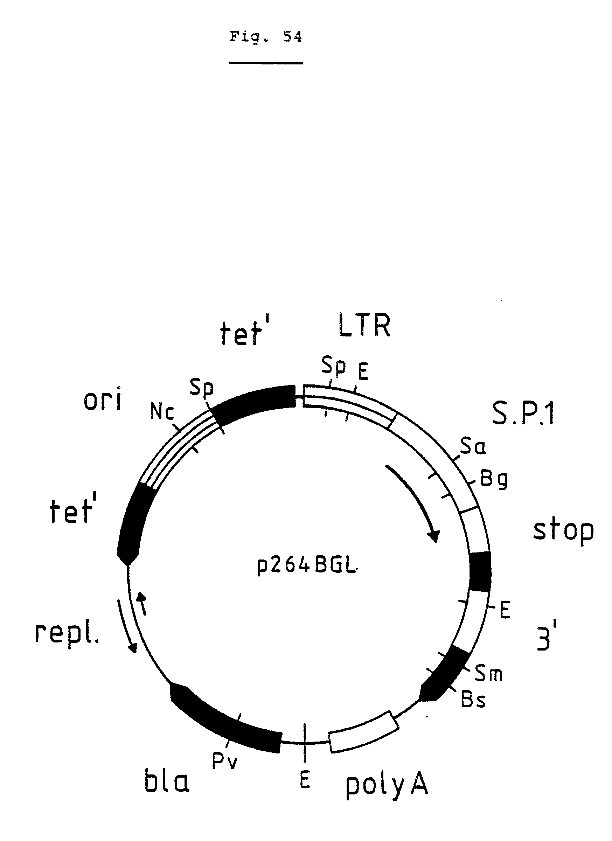

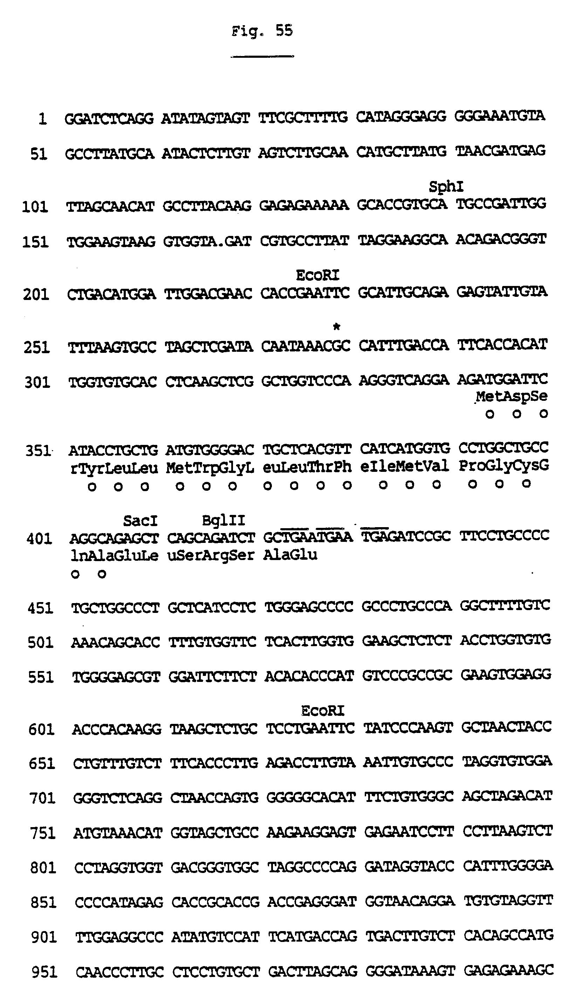

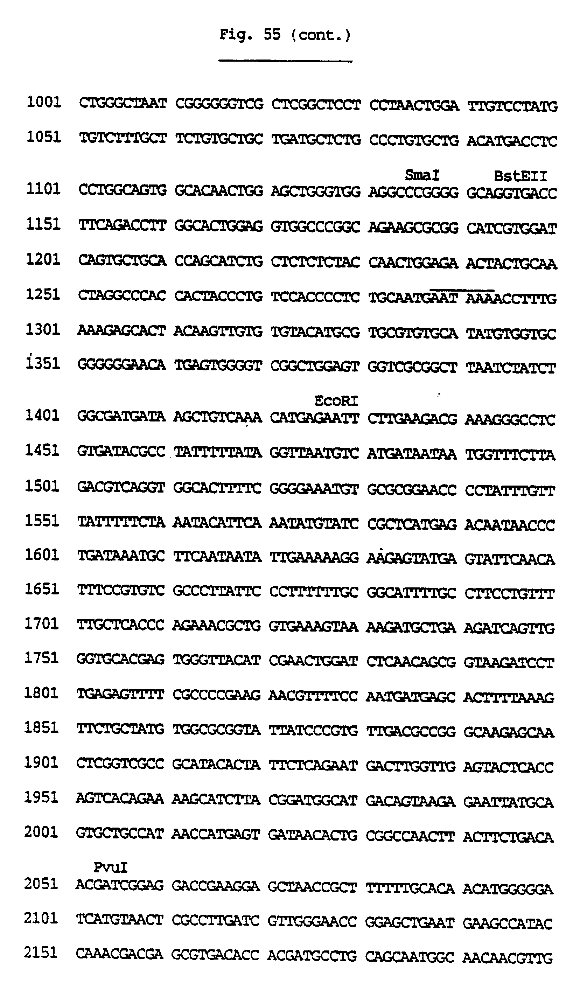

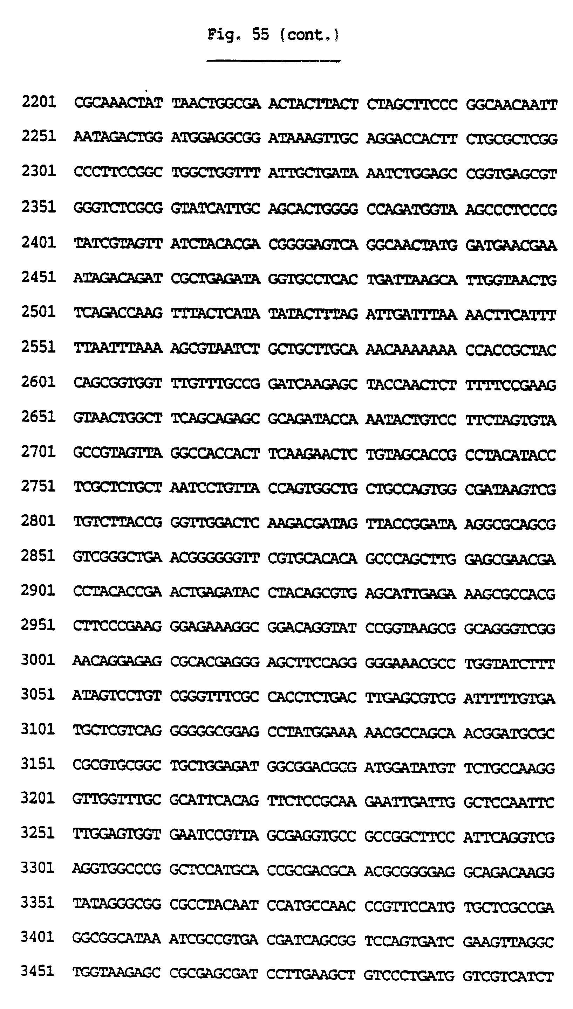

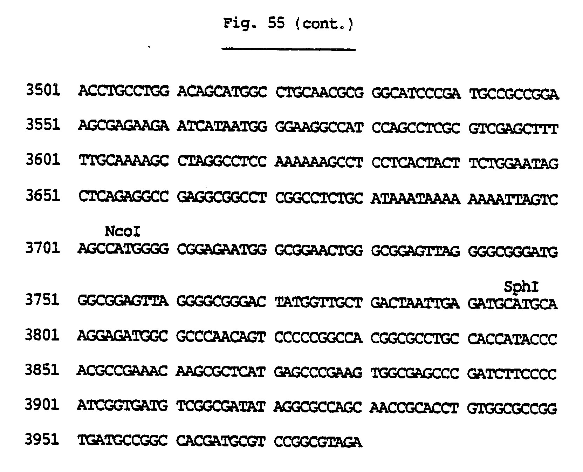

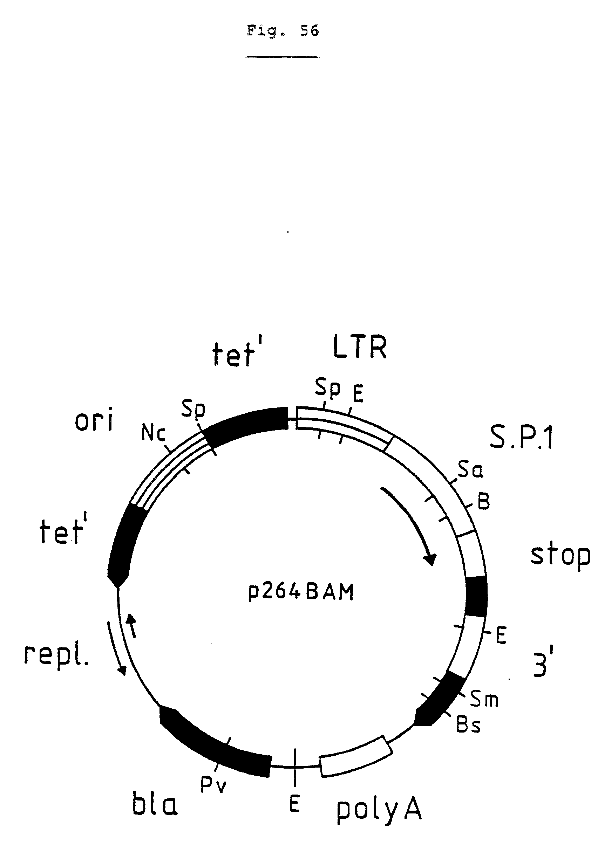

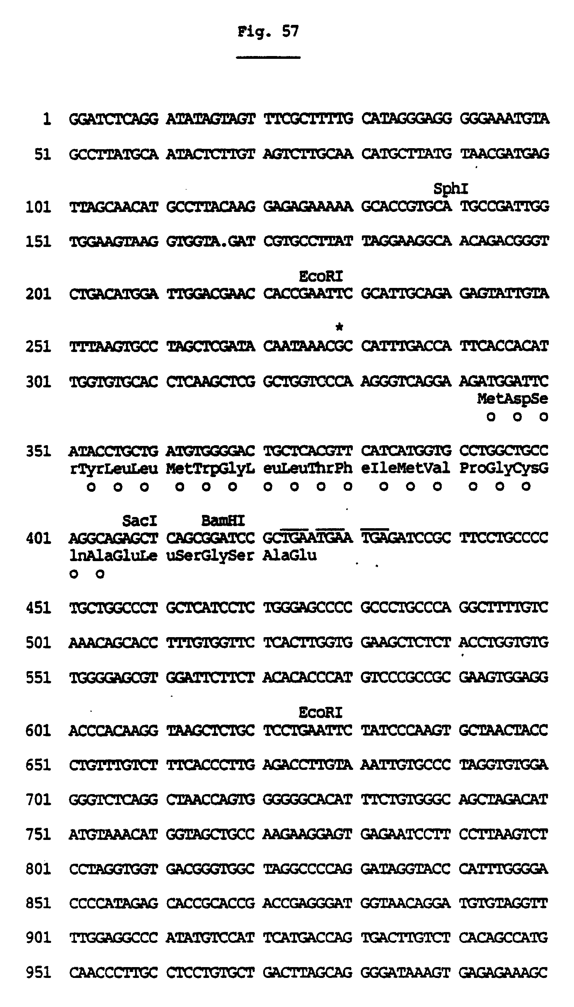

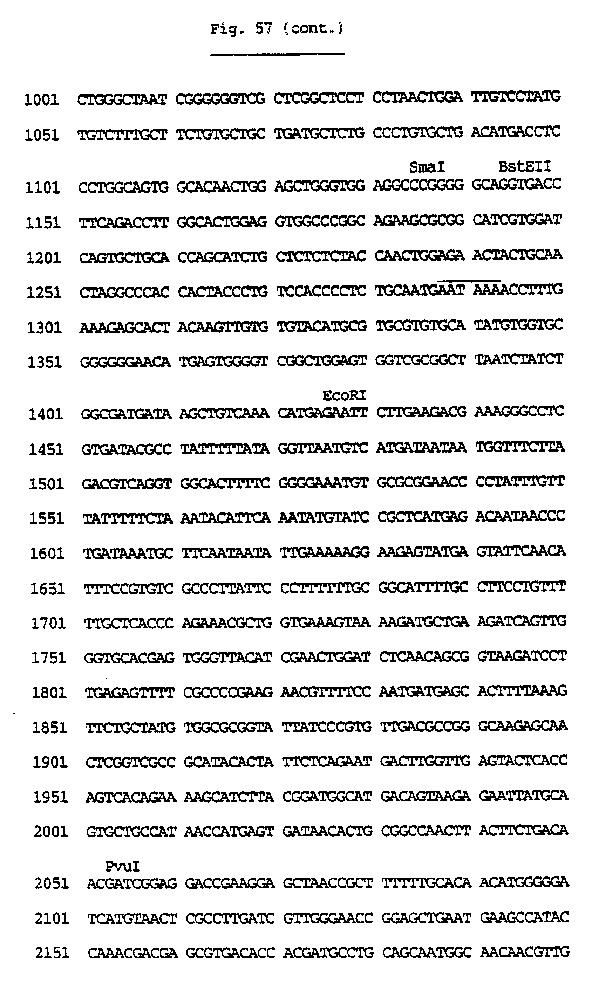

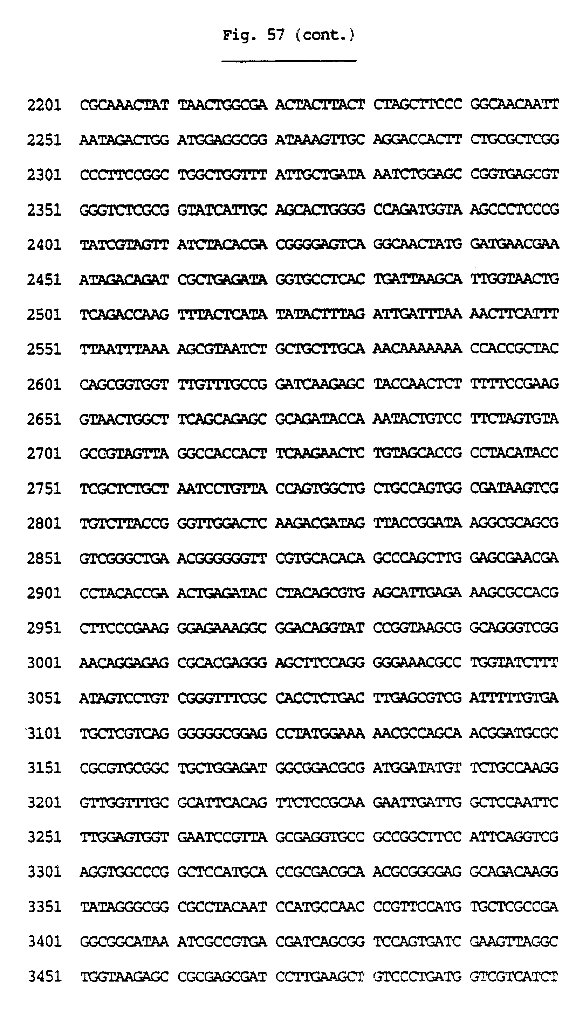

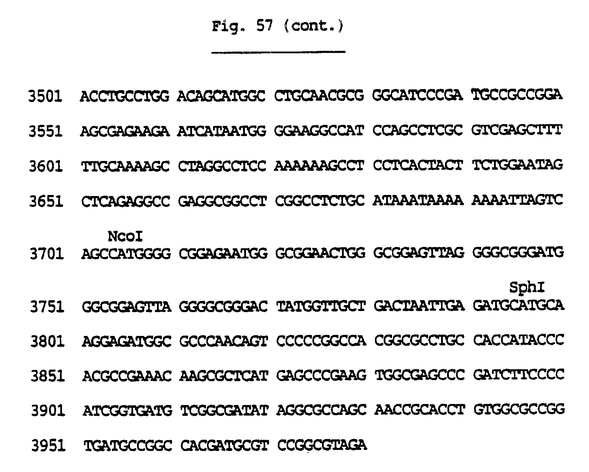

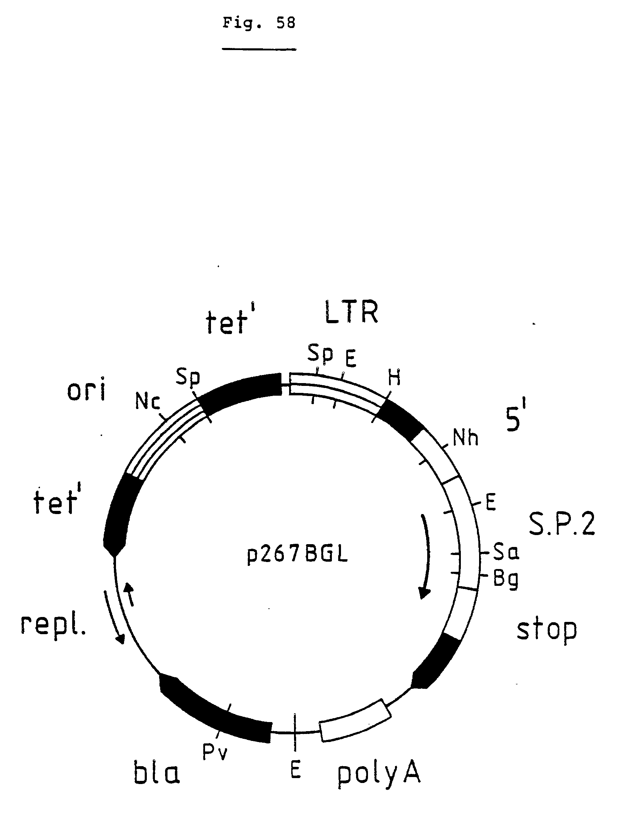

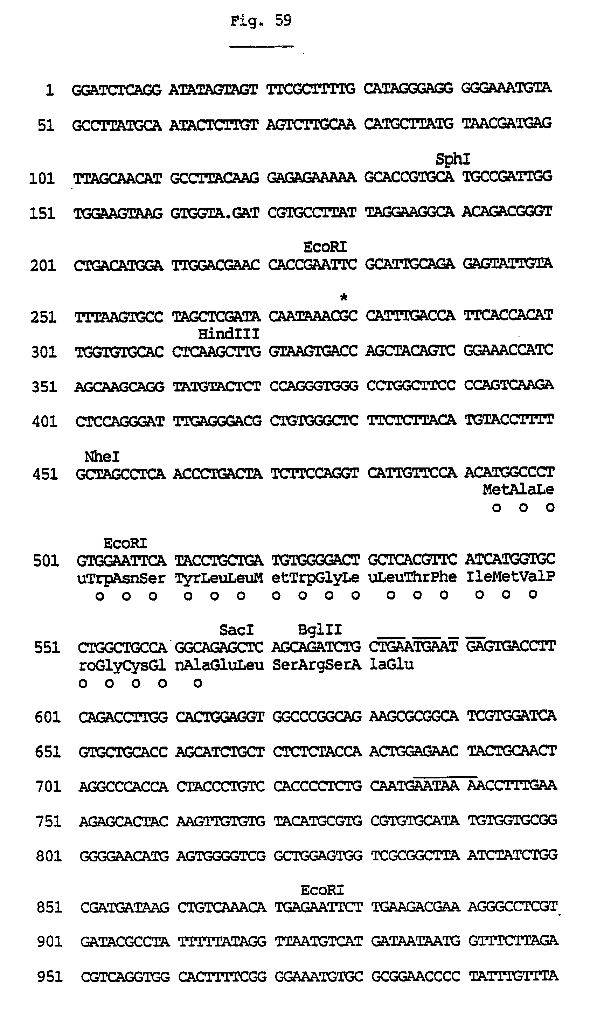

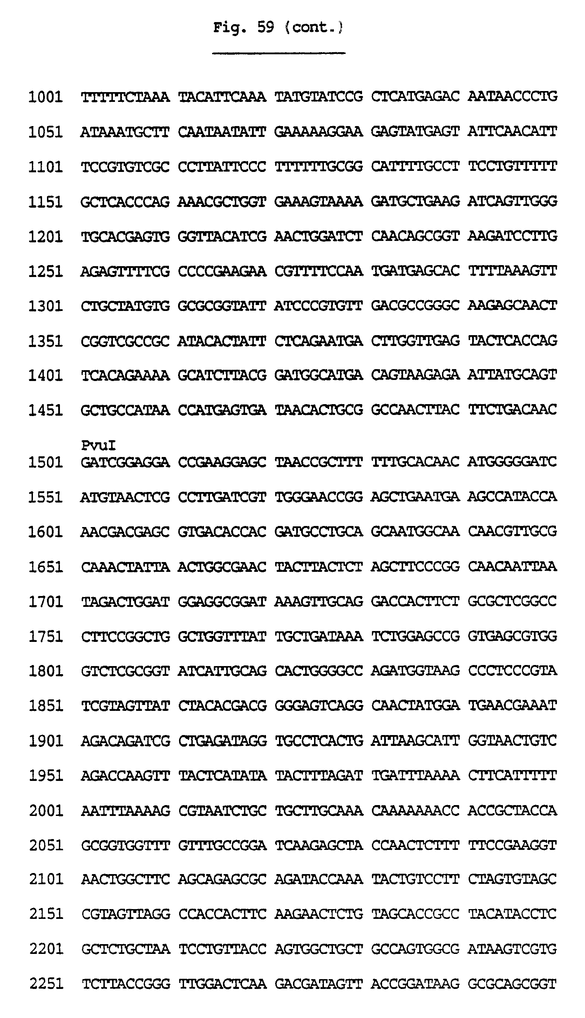

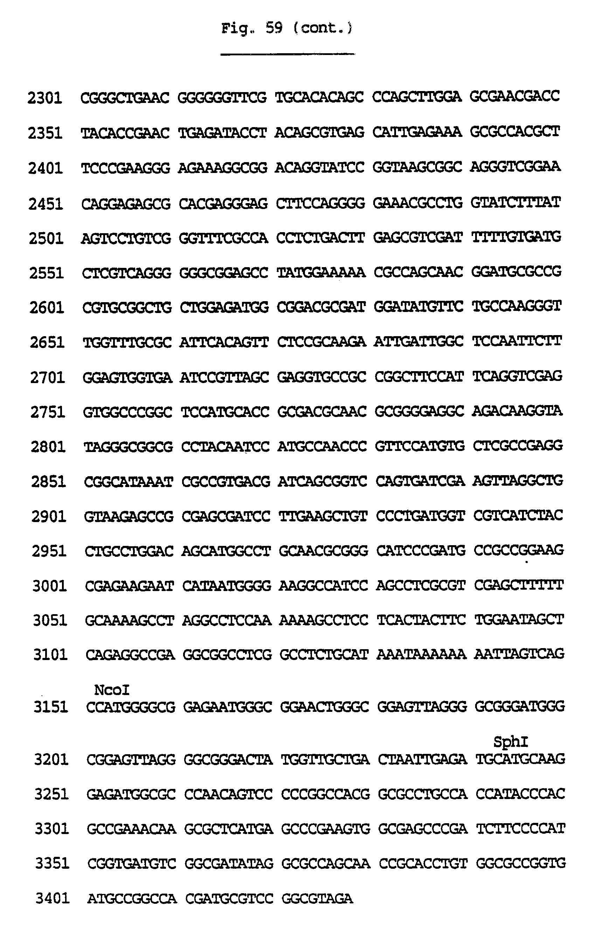

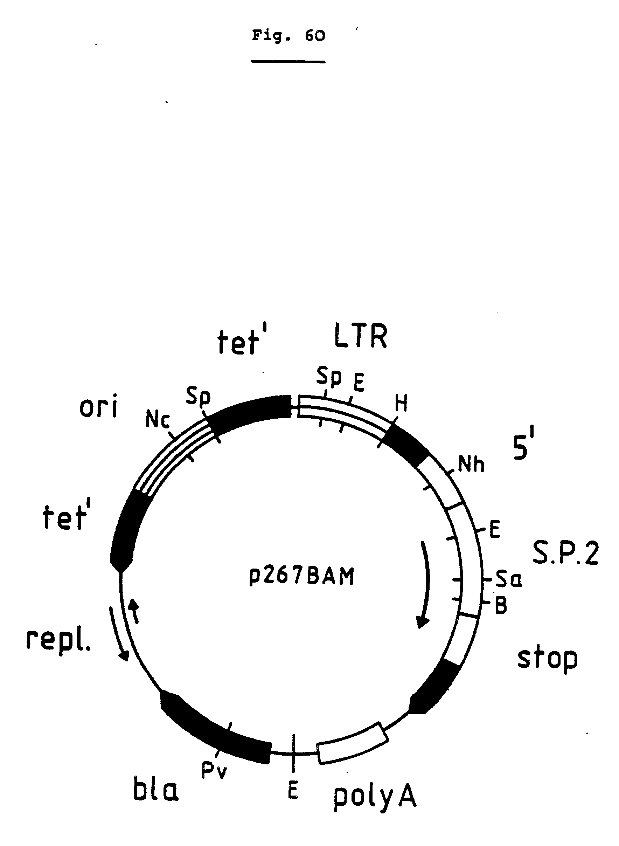

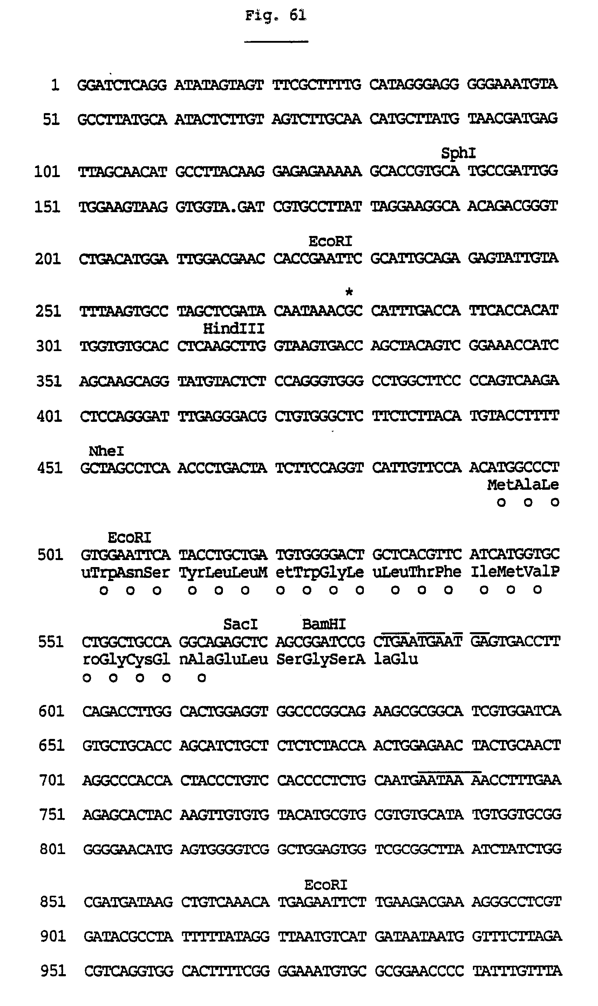

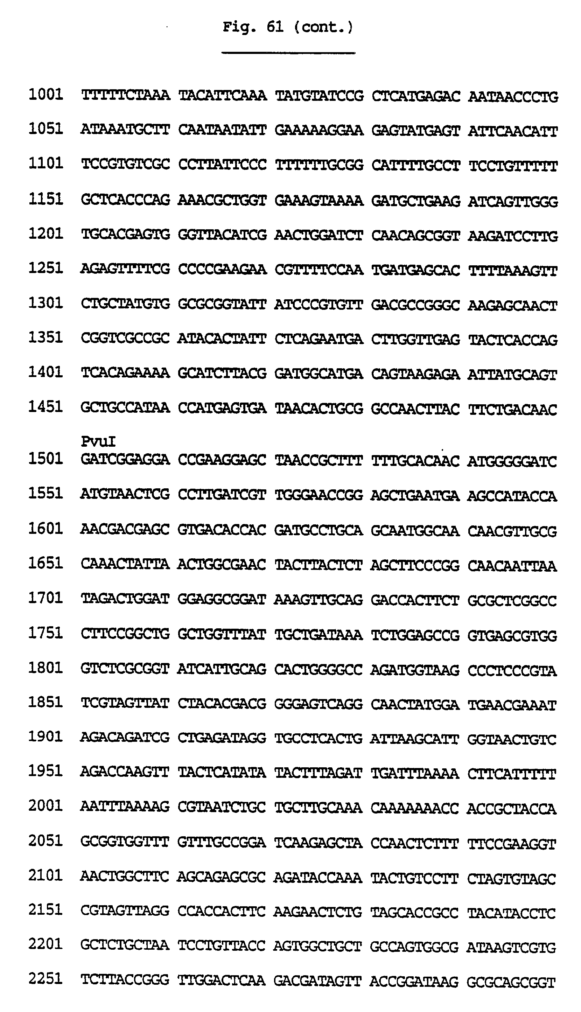

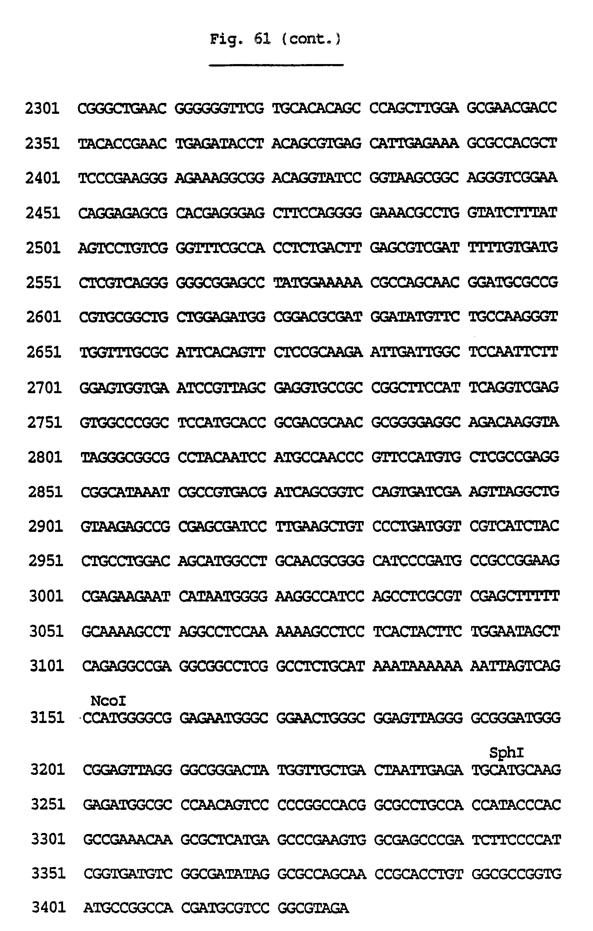

Plasmids p79BGL (Figs. 46 and 47), p79DBAM (Figs. 48 and 49) p238BGL (Figs. 50 and 51), p238BAM (Figs. 52 and 53), p264BGL (Figs. 54 and 55), p264BAM (Figs. 56 and 57), p267BGL (Figs. 58 and 59) and p267BAM (Figs. 60 and 61) were used for the expression of derivatives of the human IFN-$\gamma$ receptor in COS-cells. The following E.coli cells transformed with these plasmids were deposited at the Deutsche Sammlung von Microorganismen in Braunschweig on April 6, 1989, in accordance with the Budapest Treaty:
E.coli M15 (p79BGL), DSM No.: 5296,
E.coli M15 (p79DBAM), DSM No.: 5295,
E.coli M15 (p238BGL), DSM No.: 5294,
E.coli M15 (p238BAM), DSM No.: 5293,
E.coli M15 (p264BGL), DSM No.: 5292,
E.coli M15 (p264BAM), DSM No.: 5291,
E.coli M15 (p267BGL), DSM No.: 5290 and
E.coli M15 (p267BAM), DSM No.: 5289.

The above mentioned vectors all contain a region originally derived from plasmid pBR322 (Bolivar et al., supra; Sutcliffe, supra), which comprises the replication region, the gene for $\beta$-lactamase and parts of the tetracycline resistance gene of this plasmid. Therefore the above mentioned vectors can be stably maintained in E.coli cells grown in the presence of ampicillin. Futhermore, these plasmids contain the replication region originally derived from the simian virus SV40, allowing at least the transient maintenance of these plasmids in monkey COS-1 cells. The additional characteristics of the plasmids used for expression in COS-1 cells are described in the following chapters.

B. Plasmid p79BGL

Plasmid p79BGL (Figs. 46 and 47) carries in addition to the region necessary for its maintenance in E.coli and in COS-1 cells the long terminal repeat (LTR) of rous sarcoma virus. This promoter is followed by the gene for rat preproinsulin, in which parts of the coding region have been replaced by a region encoding several translational stop codons. Therefore, plasmid p79BGL contains downstream of its promoter a 5 - untranslated region, a region encoding a translational initiation codon, a unique cleavage site for restriction enzyme BamHI, several translational stop codons and a polyadenylation signal.

C. Plasmid p79DBAM

Plasmid p79DBAM (Figs. 48 and 49) is similar to plasmid p79BGL but lacks the region between the BglII and BamHI restriction sites of plasmid p79BGL. Therefore, plasmid p79DBAM does not encode a translational initiation codon between its promoter and its unique cleavage site for restriction enzyme BamHI.

D. Plasmid p238BGL

Plasmid p238BGL (Figs. 50 and 51) carries the same replication regions and promoter as plasmid p79BGL. However, in plasmid p238BGL the LTR is followed by: 1) a region encoding the signal peptide of the interleukin-2 receptor (S.P.1), 2) unique cleavage sites for restriction enzymes SacI and BglII, 3) a region encoding several translational stop codons and 4) a polyadenylation signal.

E. Plasmid p238BAM

Plasmid p238BAM (Figs. 52 and 53) is similar to plasmid p238BGL but contains instead of a BglII site a unique cleavage site for restriction enzyme BamHI.

F. Plasmid p264BGL

Plasmid p264BGL (Figs. 54 and 55) carries the same replication regions and promoter as plasmid p79BGL. However, in plasmid p264BGL the LTR is followed by: 1) a region encoding the signal peptide of interleukin-2 receptor (S.P.1), 2) unique cleavage sites for restriction enzymes SacI and BglII, 3) a region encoding several translational stop codons, 4) the 3'intron of the rat preproinsulin gene and 5) a polyadenylation signal.

G. Plasmid p264BAM

Plasmid p264BAM (Figs. 56 and 57) is similar to plasmid p264BGL but contains instead of a BglII site a unique cleavage site for restriction enzyme BamHI.

H. Plasmid p267BGL

Plasmid p267BGL (Figs. 58 and 59) carries the same replication regions and promoter as plasmid p79BGL. However, in plasmid p267BGL the LTR is followed by: 1) the 5'-untranslated region and the 5'-intron of the rat preproinsulin gene, 2) a region encoding the slightly modified signal peptide of the interleukin-2 receptor (S.P.2), 3) unique cleavage sites for restriction enzymes SacI and BglII, 4) a region encoding several translational stop codons and 5) a polyadenylation signal.

I. Plasmid p267BAM

Plasmid p267BAM (Figs. 60 and 61) is similar to plasmid p267BGL but contains instead of a BglII site a unique cleavage site for restriction enzyme BamHI.

Example 8

Construction of plasmids used for expression of receptor derivatives in COS-cells

A. Principles

DNA fragments encoding various parts of the human IFN-$\gamma$ receptor were prepared and integrated first in such a way into some of the plasmids described in Example 2, that they easily could be recovered after amplification of the resulting plasmids. Then, these fragments were integrated into the plasmids suitable for expression in COS-cells.

B. Preparation of DNA fragments F1, F3, F12, F25, F26, F28, F30 and F31

1. Preparation of DNA fragment F1

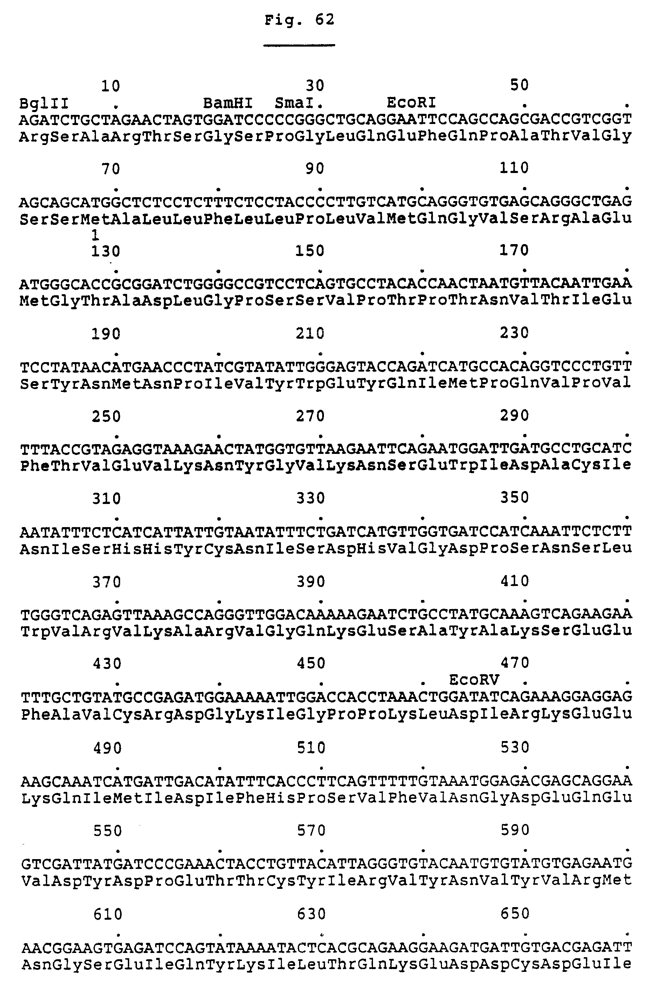

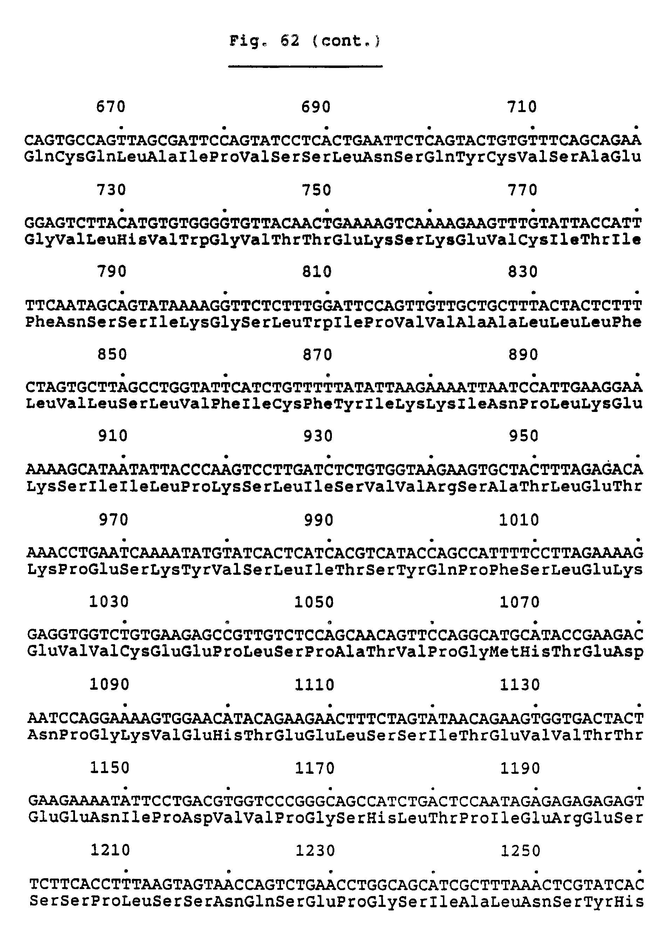

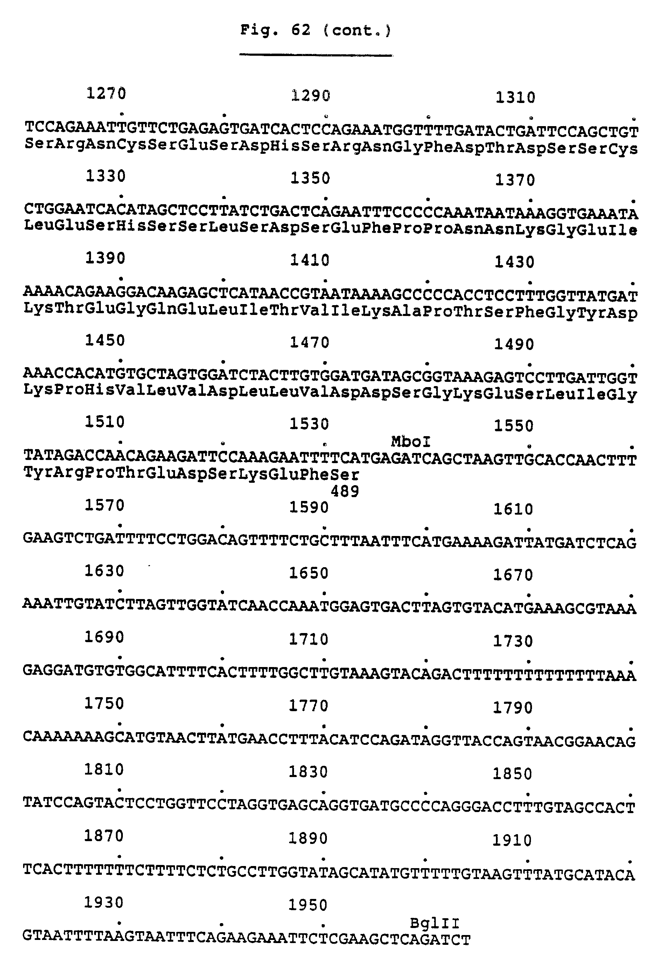

Plasmid pBABLUE was digested with restriction endonucleases XbaI and HindIII (See Fig. 1). The protruding ends of the resulting DNA fragments were filled-in using Klenow enzyme before phosphorylated BglII-linkers (linker 1, Fig. 17) were added. After cleavage with restriction enzyme BglII, fragment F1 was isolated from the resulting mixture of DNA fragments and was integrated into the unique cleavage site for restriction enzyme BglII of plasmid pDHFR-(0/-1)-6xHis (see Figs. 13 and 14) resulting in plasmid pDHFR-F1-6xHis. After amplification, this plasmid was cleaved with restriction enzyme BglII and fragment F1 was isolated. The nucleotide sequence and deduced amino acid sequence of F1 are shown in Fig. 62. Fragment F1 encodes the entire human IFN-$\gamma$ receptor and some additional amino acids. Furthermore, this fragment contains parts of the 3'-untranslated region of the receptor gene.

2. Preparation of DNA fragment F3

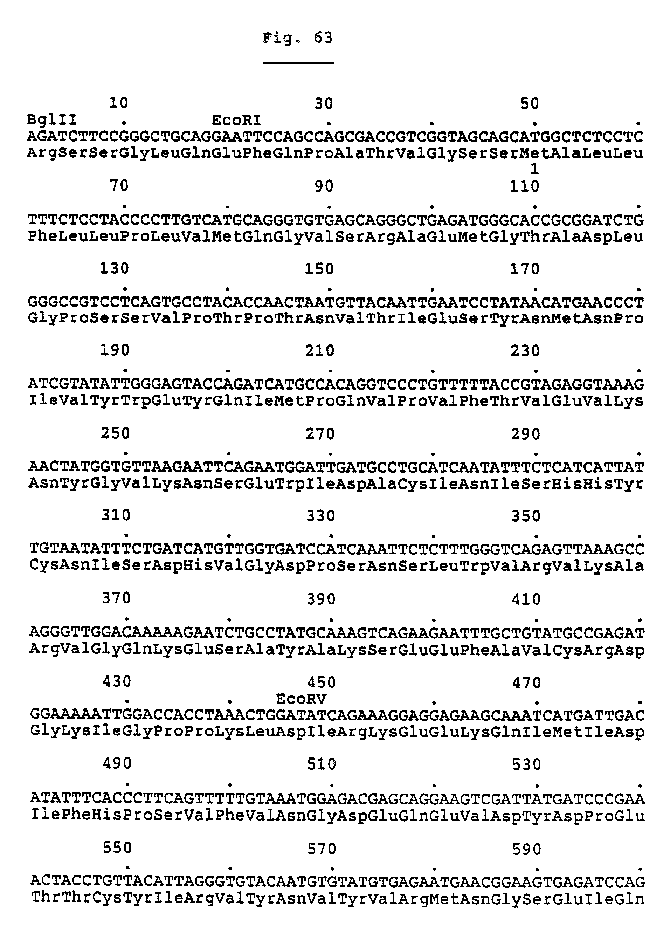

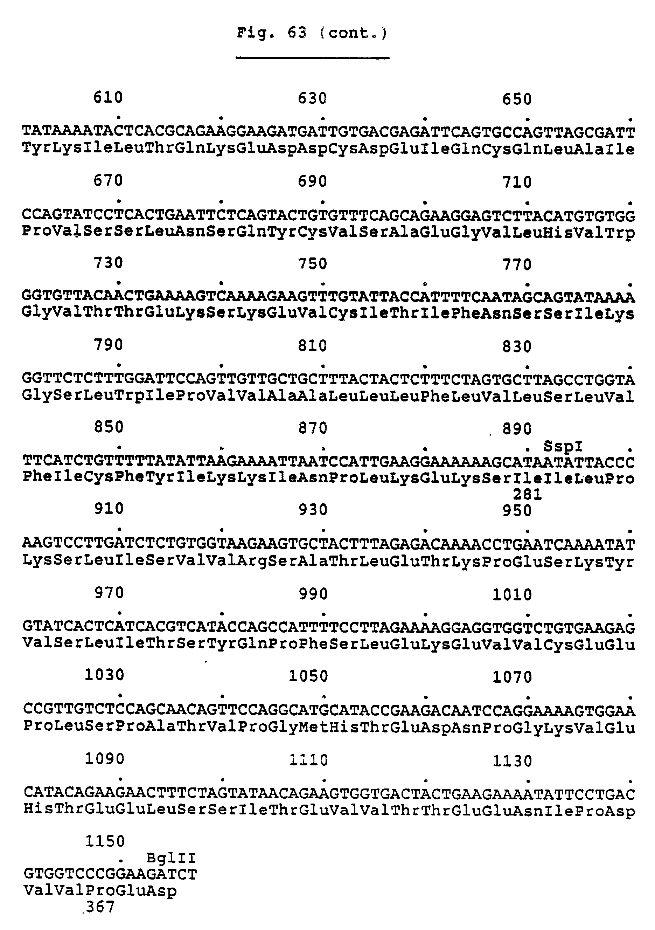

Plasmid pBABLUE was digested with restriction enzyme SmaI (see Fig. 1). To the ends of the resulting DNA fragments BglII-linkers (linker 2, Fig. 17) were added. After cleavage with restriction enzyme BglII, fragment F3 was isolated from the mixture of DNA fragments and integrated into the unique cleavage site for restriction enzyme BglII of plasmid pDHFR-(0/-1)-6xHis (see Figs. 13 and 14) resulting in plasmid pDHFR-F3-6xHis. After amplification, this plasmid was cleaved with restriction enzyme BglII and fragment F3 was isolated. The nucleotide sequence and the deduced amino acid sequence of F3 are shown in Fig. 63. Fragment F3 encodes amino acids 1 to 367 of the human IFN-$\gamma$ receptor and some additional amino acids.

3. Preparation of DNA fragment F12

The preparation of fragment F12 (Fig. 21) is described in Example 3.C.1. Fragment F12 encodes amino acids 26 to 416 of the human IFN-$\gamma$ receptor and some additional amino acids.

4. Preparation of DNA fragment F25

The preparation of fragment F25 (Fig. 23) is described in Example 3.C.3. Fragment F25 encodes amino acids 26 to 246 of the human IFN-$\gamma$ receptor and some additional amino acids.

5. Preparation of DNA fragment F26

The preparation of fragment F26 (Fig. 19) is described in Example 3.B.2. Fragment F26 encodes amino acids 15 to 212 of the human IFN-γ receptor and some additional amino acids.

6. Preparation of DNA fragment F28

The preparation of fragment F28 (Fig. 29) is described in Example 3.F.1. Fragment F28 encodes amino acids 15 to 246 of the human IFN-γ receptor and one additional amino acid.

7. Preparation of DNA fragment F30

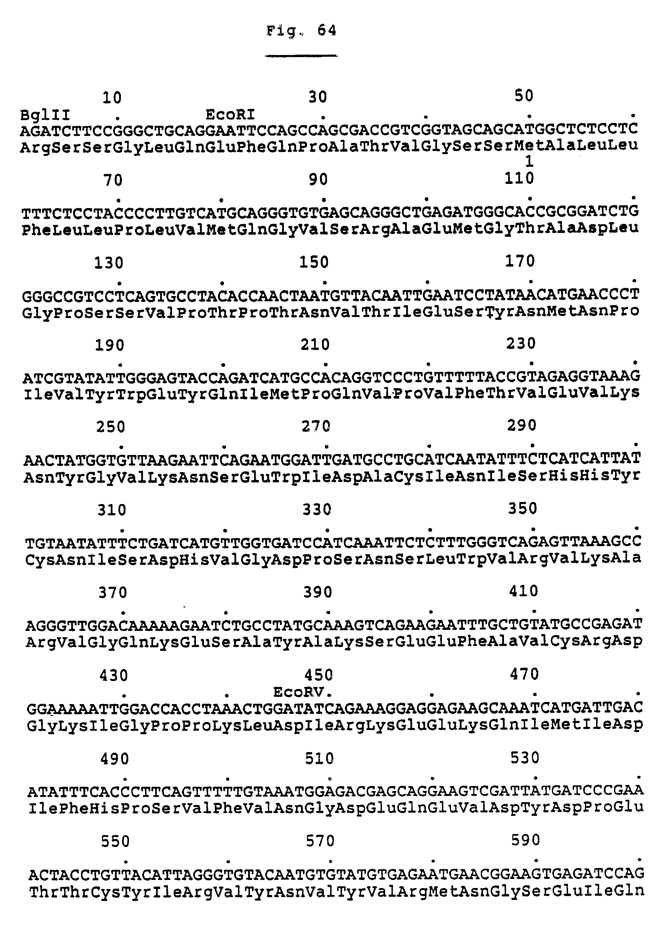

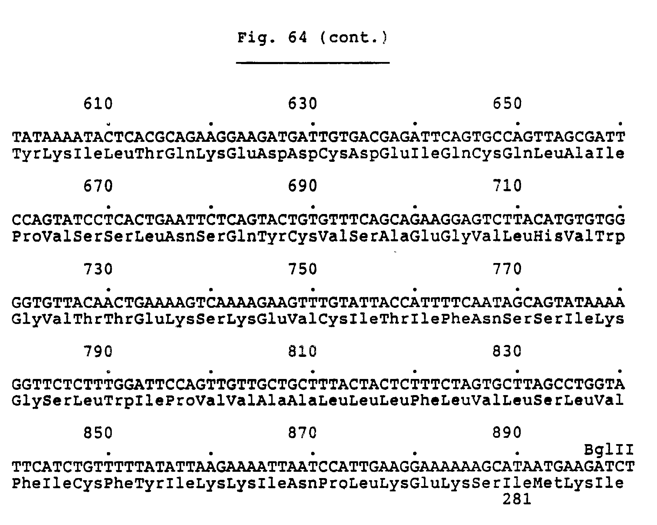

For preparation of fragment F30, plasmid pDHFR-F3-6xHis (see Example 8.B.2) was cleaved with restriction Enzyme SspI (see Figs. 1 and 63). To the ends of the resulting DNA fragments phosphorylated BglII-linkers (linker 3, Fig. 17) were ligated. After cleavage with restriction enzyme BglII, fragment F30 was isolated from the mixture of DNA fragments. The nucleotide sequence and the deduced amino acid sequence of F30 are shown in Fig. 64. Fragment F30 encodes amino acids 1 to 281 of the human IFN-γ receptor and some additional amino acids.

8. Preparation of DNA fragment F31

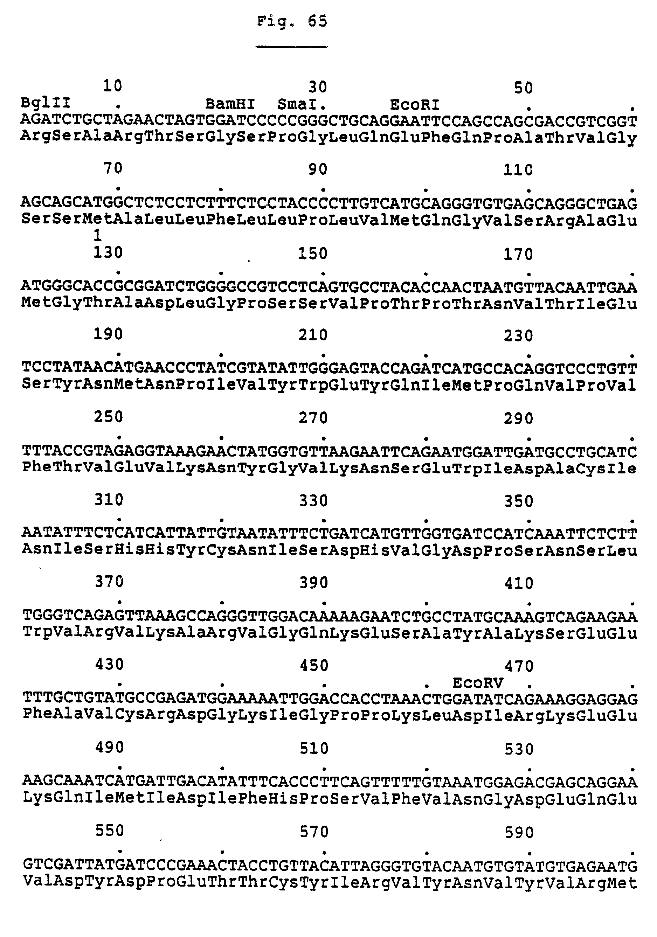

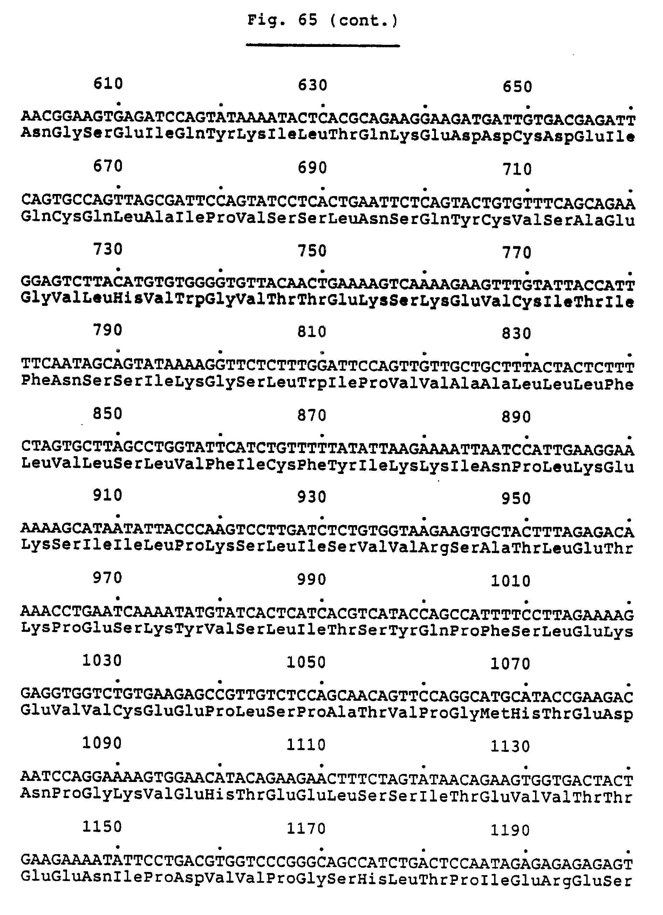

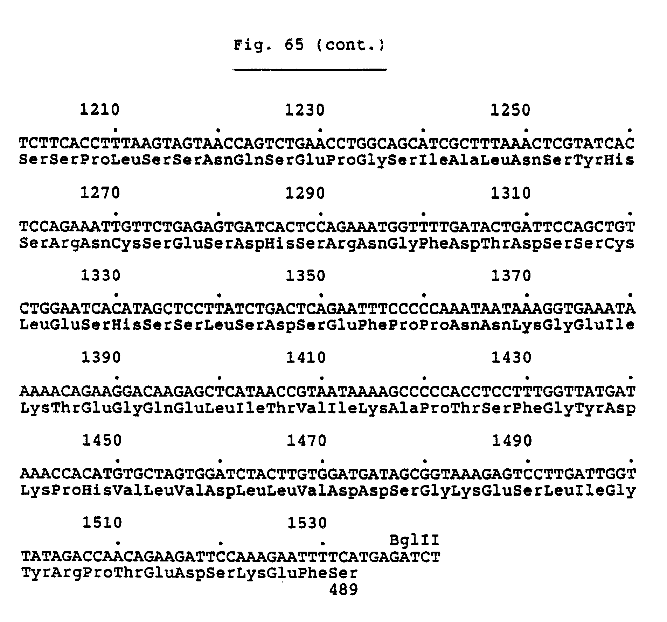

For preparation of fragment F31, plasmid pDHFR-F1-6xHis (see Example 8.B.1) was incubated with restriction enzymes BglII and MboI (see Figs. 1 and 62) and fragment F31 was isolated from the mixture of DNA fragments. The nucleotide sequence and deduced amino acid sequence of F31 are shown in Fig. 65. Fragment F31 encodes the entire human IFN-γ receptor and some additional amino acids. In contrast to fragment F1 (Fig. 62), which also encodes the entire receptor, fragment F31 does not contain the 3'-untranslated region of the receptor gene.

C. Construction of plasmids expressing repressor derivatives, which contain the transmembrane region of the receptor

1. Construction of plasmid p79DBAM-F1

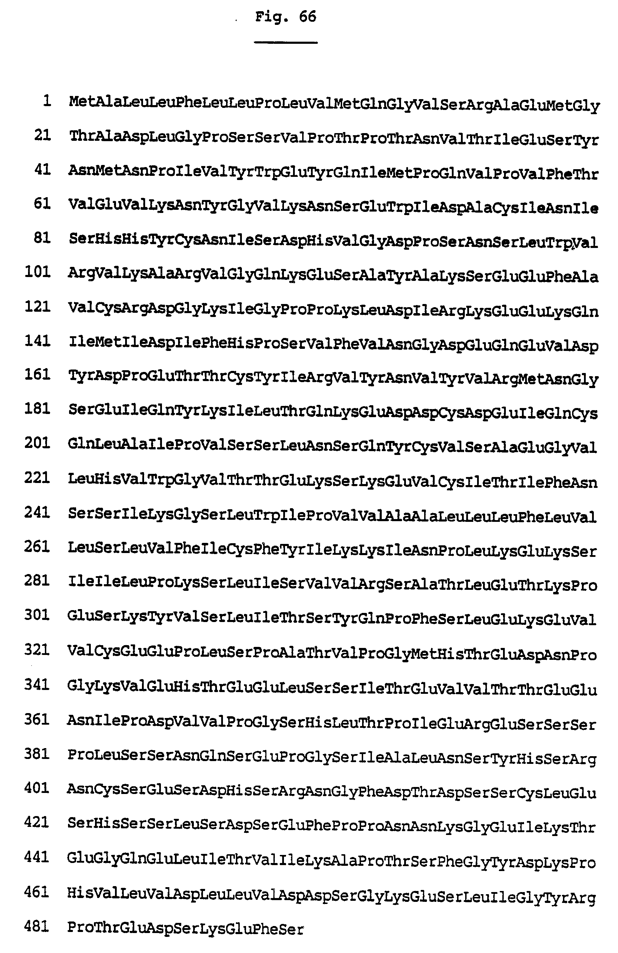

Plasmid p79DBAM-F1 was constructed by integrating fragment F1 (see Example 8.B.1) into the unique cleavage site for restriction enzyme BamHI of plasmid p79DBAM (Figs. 48 and 49). The resulting plasmid p79DBAM-F1 encodes protein PF1 (Fig. 66) comprising the entire human IFN-γ receptor.

2. Construction of plasmid p79DBAM-F3

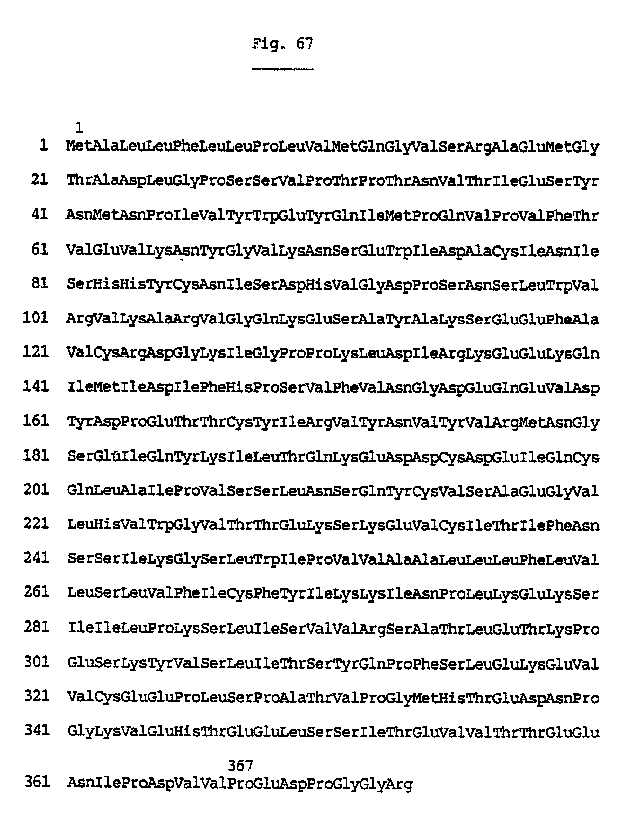

Plasmid p79DBAM-F3 was constructed by integrating fragment F3 (see Example 8.B.2) into the unique cleavage site for restriction enzymes BamHI of plasmid p79DBAM (Figs. 48 and 49). The resulting plasmid p79DBAM-F3 encodes protein PF3 (Fig. 67) and was used for the production of this protein, which comprises amino acids 1 to 367 of the human IFN-γ receptor.

3. Construction of plasmid p79DBGLBAM-F3

Plasmid p79DBGLBAM-F3 was constructed by inserting fragment F3 (see Example 8.B.2) between the unique cleavage sites for restriction enzymes BglII and BamHI of plasmid p79BGL (Figs. 46 and 47), thereby replacing parts of plasmid p79BGL. The resulting plasmid p79DBGLBAM-F3 encodes like plasmid p79DBAM-F3 protein PF3 (Fig. 67) and was used for the expression of this protein, which comprises amino acids 1 to 367 of the human IFN-γ receptor.

4. Construction of plasmid p79DBAM-F12

Plasmid p79DBAM-F12 was constructed by integrating fragment F12 (see Example 8.B.3) into the unique cleavage site for restriction enzyme BamHI of plasmid p79DBAM (Figs. 48 and 49). The resulting plasmid p79DBAM-F12 encodes amino acids 26 to 416 of the human IFN-γ receptor.

5. Construction of plasmid p267BGL-F12

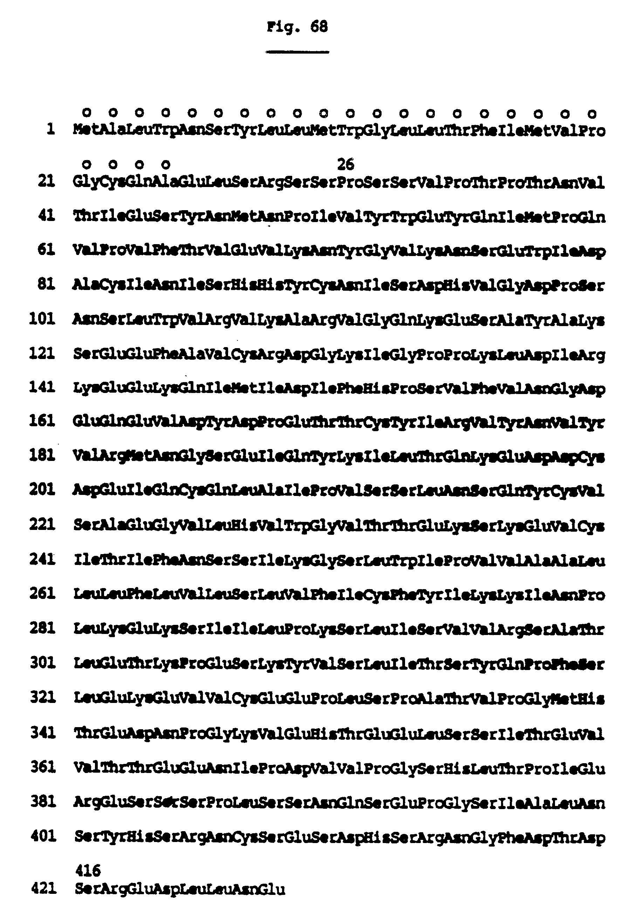

Plasmid p267BGL-F12 was constructed by integrating fragment F12 (see Example 8.B.3) into the unique cleavage site for restriction enzyme BglII of plasmid p267BGL (Figs. 58 and 59). The resulting plasmid p267BGL-F12 encodes protein PBGLF12 (Fig. 68) and was used for the production of this protein, which comprises signal peptide S.P.2 and amino acids 26 to 416 of the human IFN-$\gamma$ receptor.

6. Construction of plasmid p79DBAM-F30

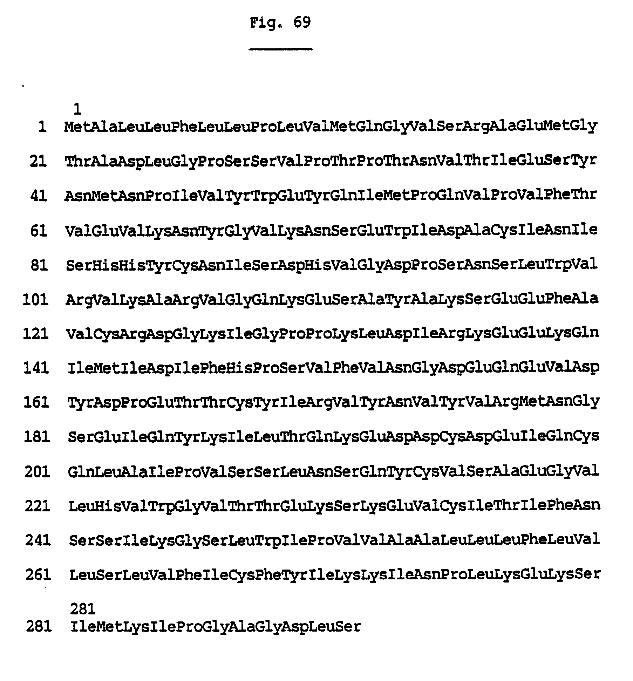

Plasmid p79DBAM-F30 was constructed by integrating fragment F30 (see Example 8.B.7) into the unique cleavage site for restriction enzyme BamHI of plasmid p79DBAM (Figs. 48 and 49). The resulting plasmid p79DBAM-F30 encodes protein PF30 (Fig. 69) and was used for the production of this protein, which comprises amino acids 1 to 281 of the human IFN-$\gamma$ receptor.

7. Construction of plasmid p79DBGLBAM-F30

Plasmid p79DBGLBAM-F30 was constructed by inserting fragment F30 (see Example 8.B.7) between the unique cleavage sites for restriction enzymes BglII and BamHI of plasmid p79BGL (Figs. 46 and 47), thereby replacing parts of plasmid p79BGL. The resulting plasmid p79DBGLBAM-F30 encodes like plasmid p79DBAM-F30 protein PF30 (Fig. 69) and was used for the production of this protein, which comprises amino acids 1 to 281 of the human IFN-$\gamma$ receptor.

8. Construction of plasmid p79DBAM-F31

Plasmid p79DBAM-F31 was constructed by integrating fragment F31 (see Example 8.B.8) into the unique cleavage site for restriction enzyme BamHI of plasmid p79DBAM (Figs. 48 and 49). The resulting plasmid p79DBAM-F31 encodes like plasmid p79DBAM-F1 protein PF1 (Fig. 66), which comprises the entire human IFN-$\gamma$ receptor.

9. Construction of plasmid p79DBAM-F1/F3

Plasmid p79DBAM-F1/F3 was constructed by combining fragment PvuI-F1'-EcoRV of plasmid p79DBAM-F1 (see Example 8.C.1) which contains parts of the gene for $\beta$-lactamase, the replication region, the RSV promoter and the 5'-end of fragment F1 (Fig. 62), with fragment EcoRV-F3'-PvuI of plasmid p79DBAM-F3 (see Example 8.C.2), which contains the 3-end of fragment F3 (Fig. 63), the polyadenylation signal and parts of the gene for $\beta$-lactamase. The resulting plasmid p79DBAM-F1/F3 encodes like plasmids p79DBAM-F3 and p79DBGLBAM-F3 protein PF3 (Fig. 67) and was used for the production of this protein, which comprises amino acids 1 to 367 of the human IFN-$\gamma$ receptor.

10. Construction of plasmid p79DBAM-F1/F30

Plasmid p79DBAM-F1/F30 was constructed by combining fragment PvuI-F1'-EcoRV of plasmid p79DBAM-F1 (see Example 8.C.1) with fragment EcoRV-F30'-PvuI of plasmid p79DBAM-F30 (see Example 8.C.6), which comprises the 3'-end of fragment F30 (Fig. 64), the polyadenylation signal and parts of the gene for $\beta$-lactamase. The resulting plasmid p79DBAM-F1/F30 encodes like plasmids p79DBAM-F30 and p79DBGLBAM-F30 protein PF30 (Fig. 69) and was used for the production of this protein, which comprises amino acids 1 to 281 of the human IFN-$\gamma$ receptor.

11. Construction of plasmid p267BGL/79DBAM-F12/F1

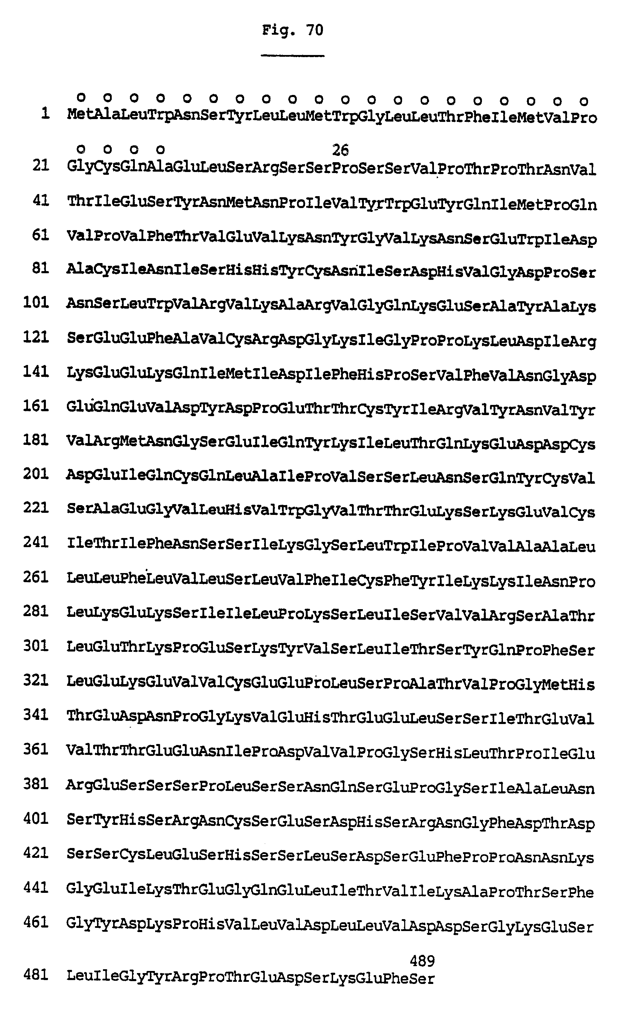

Plasmid p267BGL/79DBAM-F12/F1 was constructed by combining fragment PvuI-F12'-EcoRV of plasmid p267BGL-F12 (see Example 8.C.5), which comprises parts of the gene for $\beta$-lactamase, the replication region, the RSV promoter, the region encoding signal peptide S.P.2 and the 5'-end of fragment F12 (Fig. 21), with fragment EcoRV-F1'-PvuI of plasmid p79DBAM-F1 (see Example 8.C.1), which contains the 3'-end of fragment F1 (Fig. 62), the polyadenylation signal and parts of the gene for $\beta$-lactamase. The resulting plasmid p267BGL/79DBAM-F12/F1 encodes protein PF12F1 (Fig. 70) and was used for production of this protein, which comprises signal peptide S.P.2 and amino acids 26 to 489 of the human IFN-$\gamma$ receptor.

12. Construction of plasmid p267BGL/79DBAM-F12/F31

Plasmid p267BGL/79DBAM-F12/F31 was constructed by combining fragment PvuI-F12'-EcoRV (see Example 8.C.11), with fragment EcoRV-F31'-PvuI of plasmid p79DBAB-F31 (see Example 8.C.8), which comprises the 3'-end of fragment F31 (Fig. 65), the polyadenylation signal and parts of the gene for β-lactamase. The resulting plasmid p267BGL/79DBAM-F12/F31 encodes like plasmid p267BGL/79DBAM-F12/F1 protein PF12F1 (Fig. 70) and was used for production of this protein, which comprises signal peptide S.P.2 and amino acids 26 to 489 of the human IFN-γ receptor.

13. Construction of plasmid p267BGL/79DBAM-F12/F12

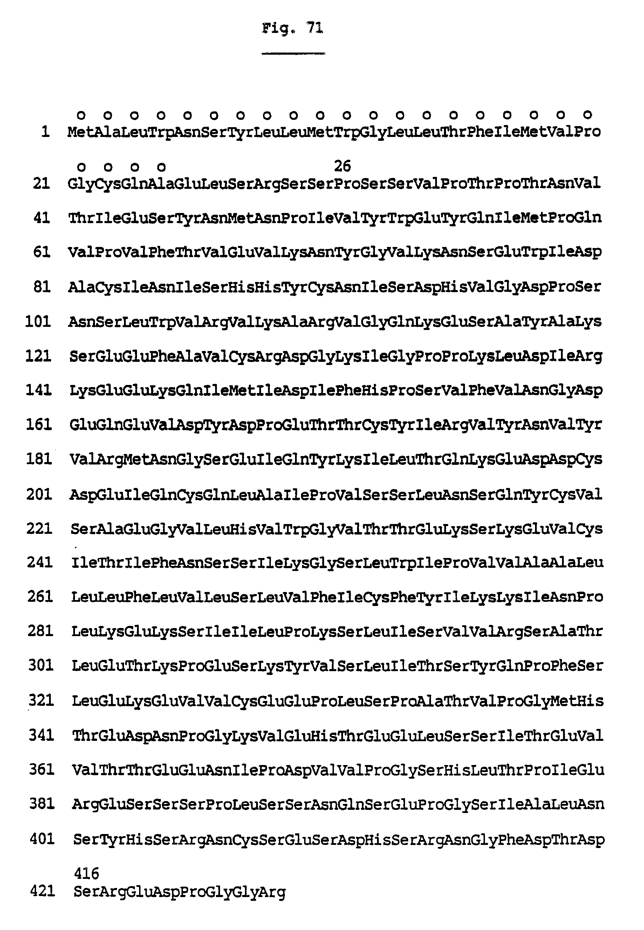

Plasmid p267BGL/79DBAM-F12/F12 was constructed by combining fragment PvuI-F12'-EcoRV (see Example 8.C.11), with fragment EcoRV-F12'-PvuI of plasmid p79DBAM-F12 (see Example 8.C.4), which comprises the 3'-end of fragment F12 (Fig. 21), the polyadenylation signal and parts of the gene for β-lactamase. The resulting plasmid p267BGL/79DBAM-F12/F12 encodes protein PF12F12 (Fig. 71) and was used for the production of this protein, which comprises signal peptide S.P.2 and amino acids 26 to 416 of the human IFN-γ receptor.

14. Construction of plasmid p267BGL/79DBAM-F12/F3

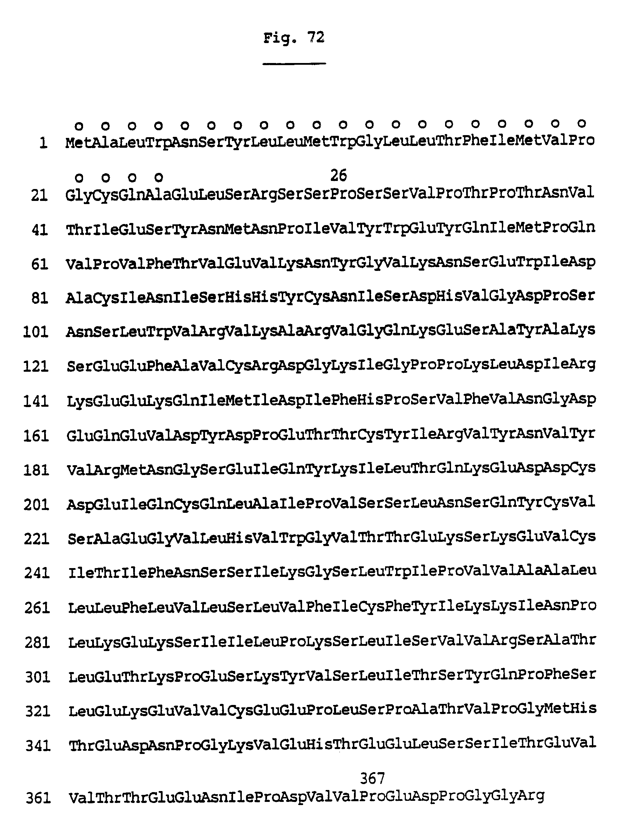

Plasmid p267BGL/79DBAM-F12/F3 was constructed by combining fragment PvuI-F12'-EcoRV (see Example 8.C.11) with fragment EcoRV-F3'-PvuI (see Example 8.C.9). The resulting plasmid p267BGL/79DBAM-F12/F3 encodes protein PF12F3 (Fig. 72) and was used for the production of this protein, which comprises signal peptide S.P.2 and amino acids 26 to 367 of the human IFN-γ receptor.

15. Construction of plasmid p267BGL/79DBAM-F12/F30

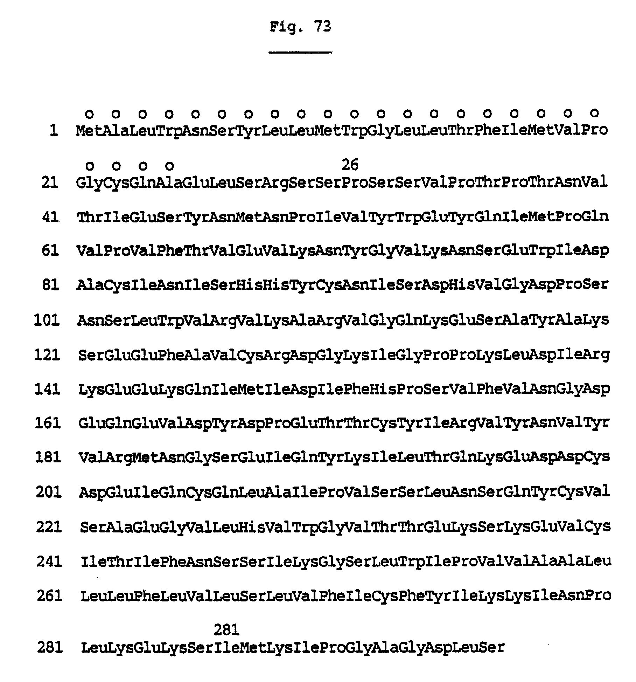

Plasmid p267BGL/79DBAM-F12/F30 was constructed by combining fragment PvuI-F12'-EcoRV (see Example 8.C.11) with fragment EcoRV-F30'-PvuI (see Example 8.C.10). The resulting plasmid p267BGL/79DBAM-F12/F30 encodes protein PF12F30 (Fig. 73) and was used for the production of this protein, which comprises signal peptide S.P.2 and amino acids 26 to 281 of the human IFN-γ receptor.

D. Construction of plasmids used for the production of secreted receptor derivatives in COS-1 cells

1. Principles

Plasmids encoding receptor derivatives expected to be secreted into the medium by COS-1 cells containing these plasmids were constructed by integrating DNA fragments F25 (see Example 3.C.1 and Fig. 23) or F28 (see Example 3.F.2 and Fig. 29) into plasmids described in Example 7. In the resulting plasmids a signal peptide is linked to the information encoded by fragments F25 and F28. Since these fragments encode only amino acids 26 to 246 and 15 to 246 of the human IFN-γ receptor, respectively, but not its transmembrane region, the expressed proteins are expected to be secreted into the medium by COS-cells transfected with these plasmids.

2. Construction of plasmid p238BAM-F28

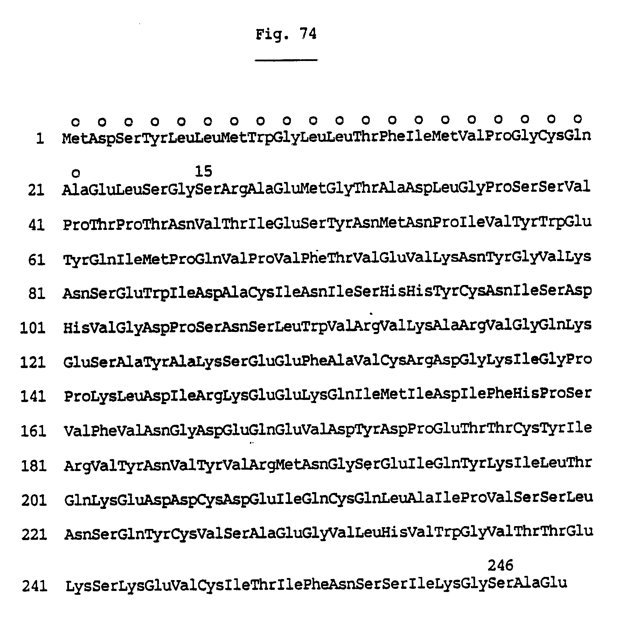

Plasmid p238BAM-F28 was constructed by intergrating fragment F28 into the unique cleavage site for restriction enzyme BamHI of plasmid p238BAM (see Figs. 52 and 53). The resulting plasmid encodes protein P28A (Fig. 74) and was used for the production of this protein, which comprises signal peptide S.P.1 and amino acids 15 to 246 of the human IFN-γ receptor.

3. Construction of plasmid p238BGL-F28

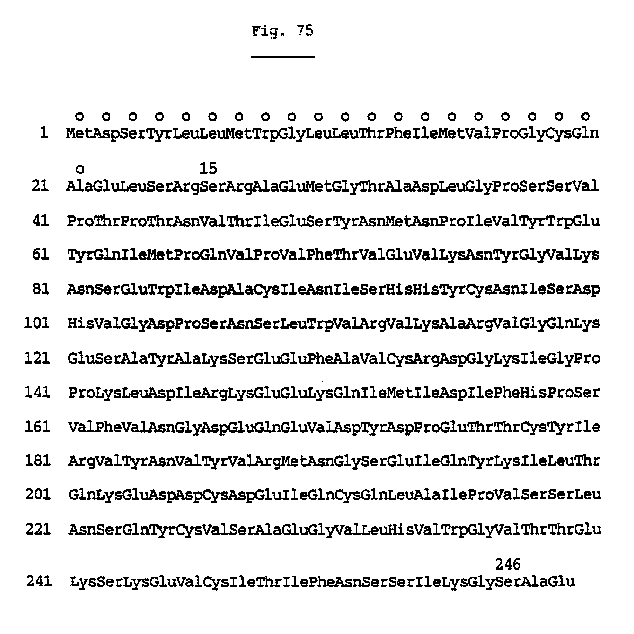

Plasmid p238BGL-F28 was constructed by integrating fragment F28 into the unique cleavage site for restriction enzyme BglII of plasmid p238BGL (see Figs. 50 and 51). The resulting plasmid encodes protein P28B (Fig. 75) and was used for the production of this protein, which comprises signal peptide S.P.1 and amino acids 15 to 246 of the human IFN-γ receptor.

4. Construction of plasmid p264BAM-F28

Plasmid p264BAM-F28 was constructed by integrating fragment F28 into the unique cleavage site for restriction enzyme BamHI of plasmid p264BAM (see Figs. 56 and 57). The resulting plasmid encodes like plasmid p238BAM-F28 protein P28A (Fig. 74) and was used for the production of this protein, which comprises amino acids 15 to 246 of the human IFN-γ receptor.

5. Construction of plasmid p264BGL-F28

Plasmid p264BGL-F28 was constructed by integrating fragment F28 into the unique cleavage site for restriction enzyme BglII of plasmid p264BGL (see Figs. 54 and 55). The resulting plasmid encodes like plasmid p238BGL-F28 protein P28B (Fig. 75) and was used for the production of this protein, which comprises signal peptide S.P.1 and amino acids 15 to 246 of the human IFN-γ receptor.

6. Construction of p267BAM-F28

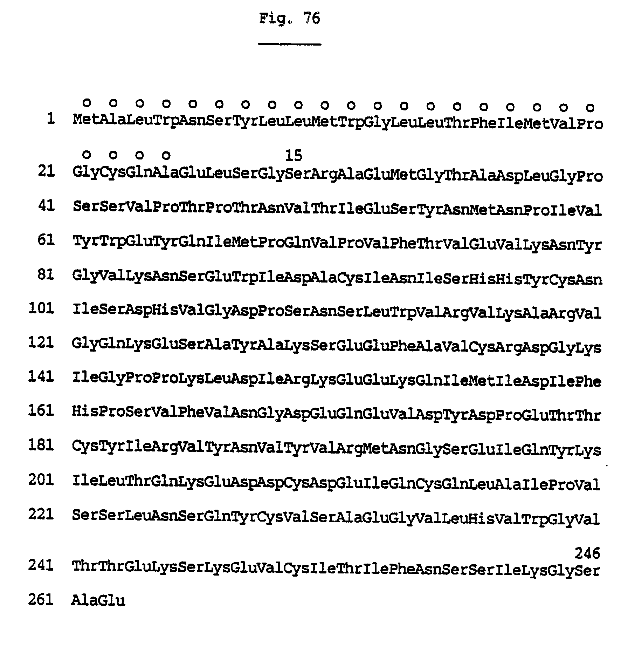

Plasmid p267BAM-F28 was constructed by integrating fragment F28 into the unique cleavage site for restriction enzyme BamHI of plasmid p267BAM (see Figs. 60 and 61). The resulting plasmid encodes protein P28C (Fig. 76) and was used for the production of this protein, which comprises signal peptide S.P.2 and amino acids 15 to 246 of the human IFN-γ receptor.

7. Construction of plasmid p267BGL-F28

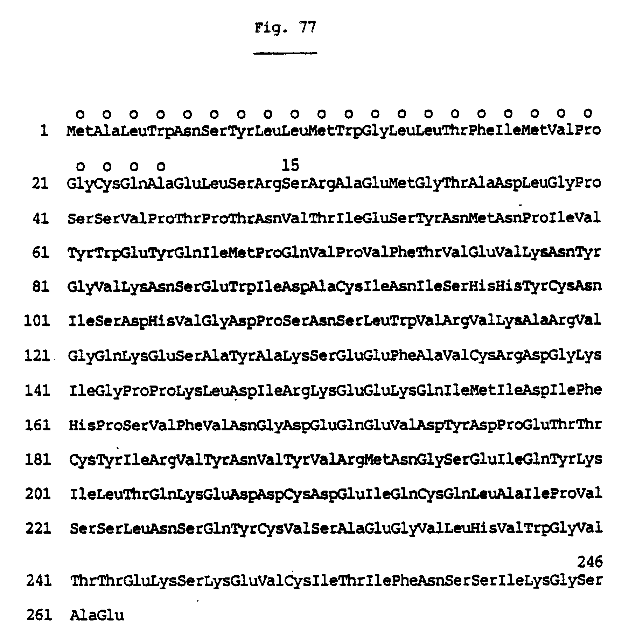

Plasmid p267BGL-F28 was constructed by integrating fragment F28 into the unique cleavage site for restriction enzyme BglII of plasmid p267BGL (see Figs. 58 and 59). The resulting plasmid encodes protein P28D (Fig. 77) and was used for the production of this protein, which comprises signal peptide S.P.2 and amino acids 15 to 246 of the human IFN-γ receptor.

8. Construction of plasmid p238BAM-F25

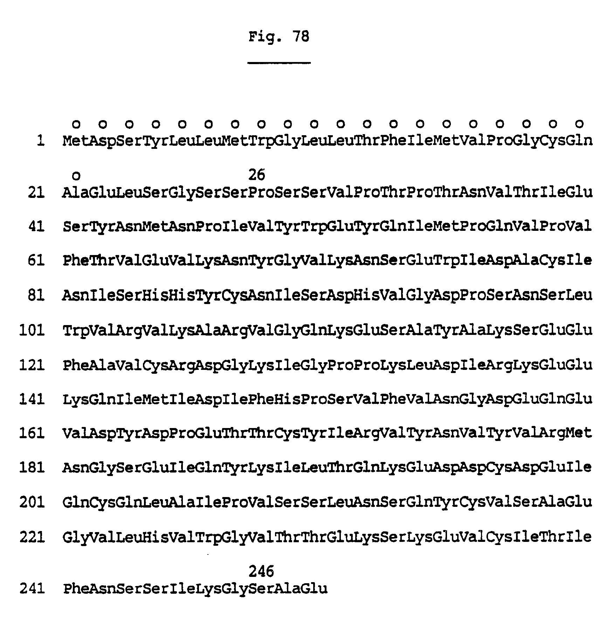

Plasmid p238BAM-F25 was constructed by integrating fragment F25 into the unique cleavage site for restriction enzyme BamHI of plasmid p238BAM (see Figs. 52 and 53). The resulting plasmid encodes protein P25A (Fig. 78) and was used for the production of this protein, which comprises signal peptide S.P.1 and amino acids 26 to 246 of the human IFN-γ receptor.

9. Construction of plasmid p238BGL-F25

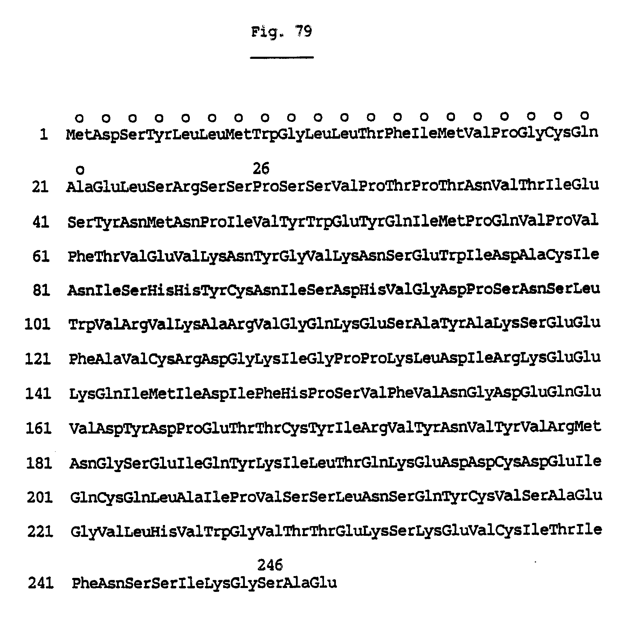

Plasmid p238BGL-F25 was constructed by integrating fragment F25 into the unique cleavage site for restriction enzyme BglII of plasmid p238BGL (see Figs. 50 and 51). The resulting plasmid encodes protein P25B (Fig. 79) and was used for the production of this protein, which comprises signal peptide S.P.1 and amino acids 26 to 246 of the human IFN-γ receptor.

10. Construction of plasmid p264BAM-F25

Plasmid p264BAM-F25 was constructed by integrating fragment F25 into the unique cleavage site for restriction enzyme BamHI of plasmid p264BAM (see Figs. 56 and 57). The resulting plasmid encodes like plasmid p238BAM-F25 protein P25A (Fig. 78) and was used for the production of this protein, which comprises signal peptide S.P.1 and amino acids 26 to 246 of the human IFN-γ receptor.

11. Construction of plasmid p267BAM-F25

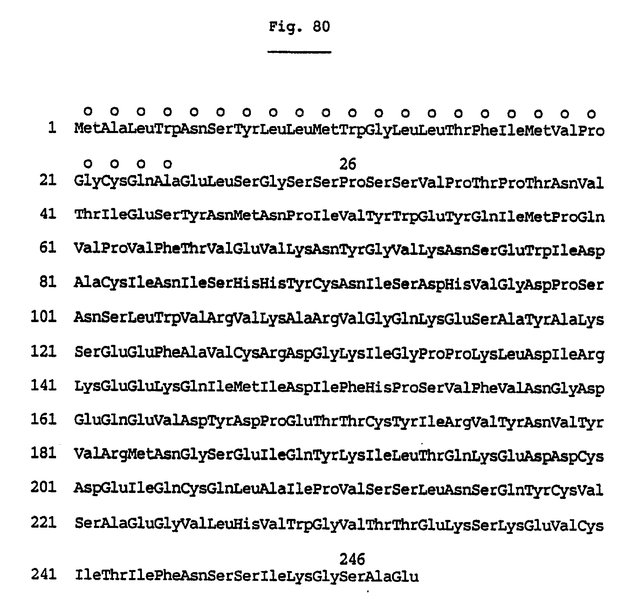

Plasmid p267BAM-F25 was constructed by integrating fragment F25 into the unique cleavage site for restriction enzyme BamHI of plasmid p267BAM (see Figs. 60 and 61). The resulting plasmid encodes protein P25C (Fig. 80) and was used for the production of this protein, which comprises signal peptide S.P.2 and amino acids 26 to 246 of the human IFN-γ receptor.

12. Construction of plasmid p267BGL-F25

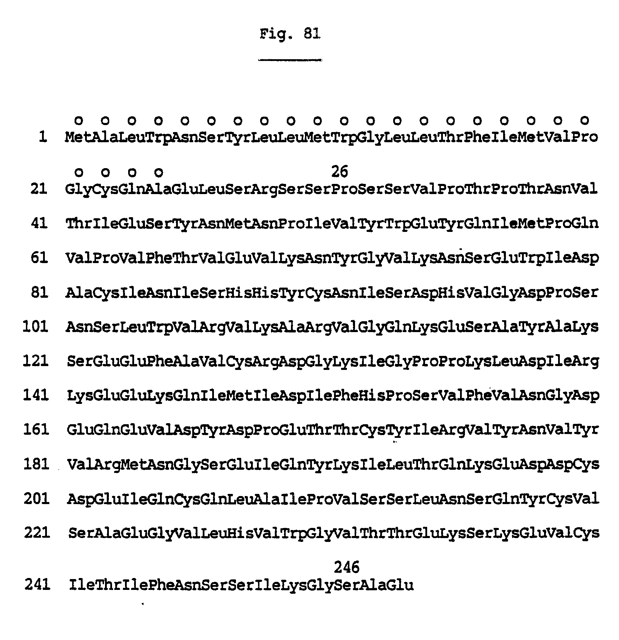

Plasmid p267BGL-F25 was constructed by integrating fragment F25 into the unique cleavage site for restriction enzyme BglII of plasmid p267BGL (see Figs. 58 and 59). The resulting plasmid encodes protein P25D (Fig. 81) and was used for the production of this protein, which comprises signal peptide S.P.2 and amino acids 26 to 246 of the human IFN-$\gamma$ receptor.

Example 9

Expression of membrane bound derivatives of the human IFN-$\gamma$ receptor in COS-cells

A. Principles

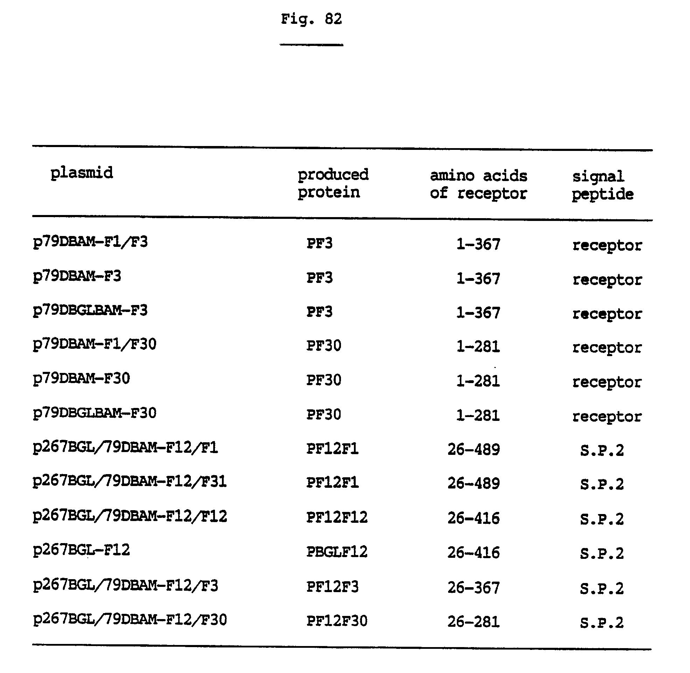

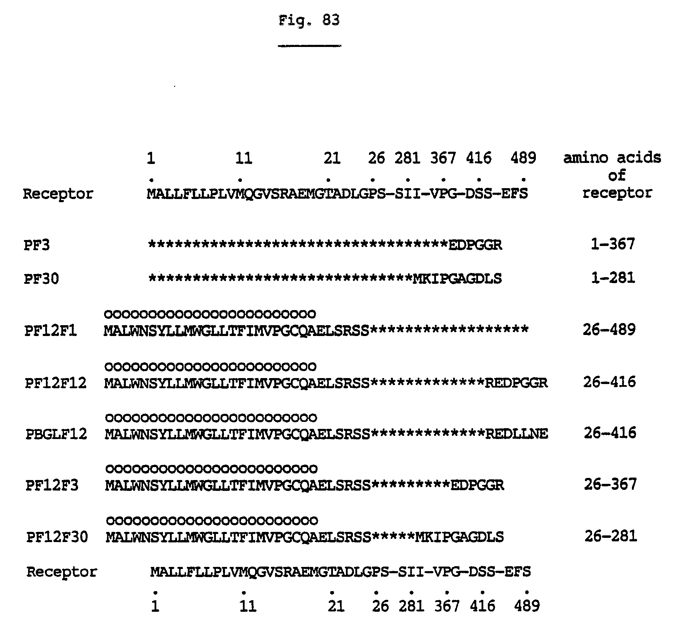

Plasmids encoding derivatives of the human IFN-$\gamma$ receptor, which contain a signal peptide and the transmembrane region of the receptor, were transfected into COS-1 cells. Due to the presence of the transmembrane region, upon expression these receptor derivatives are expected to be present on the surface of the transfected cells. Therefore, the ability of the receptor derivatives to bind IFN-$\gamma$ could be analyzed by measuring the amount of IFN-$\gamma$ binding specifically to the surface of intact cells. The plasmids used in this experiment and the proteins encoded by these plasmids are listed in Fig. 82, whereas the amino acid sequences of these proteins are summarized in Fig. 83.

B. Transfection of COS-1 cells and expression of the receptor derivatives

COS-1 cells (ATCC CRL 1650) were harvested by trypsinization (PBS, 0.25% trypsin), washed by phosphate buffered saline (PBS: 8.1 mM $NaH_2PO_4$, 1.5 mM $KH_2PO_4$, 2.7 mM KCl, 137 mM NaCl, pH 7.2), counted and resuspended in Dulbecco's Modified Eagle Medium (DMEM) from Gibco containing 5% Fetal Bovine Serum (FBS), before $2x10^6$ cells were distributed in 60 mm tissue culture disks (Falcon). After 24 h at 37°C in a carbon dioxide incubator (5% $CO_2$), the 70% confluent cell monolayer was washed with PBS and covered with 1 ml of PBS containing 3 mg/ml DEAE Dextran (Pharmacia) and 5 $\mu$g plasmid DNA. After 30 minutes at 37°C, the transfection cocktail was removed and 4 ml Iscove's Modified Dulbecco's Medium (IMDM) from Gibco containing 10% FBS and 100 $\mu$g chloroquine (Sigma) were distributed in each plate. The plates were incubated for 2.5 h at 37°C in a carbon dioxide incubator, before the medium was aspirated and the cell monolayers were covered with IMDM medium containing 10% FBS and 10% DMSO. After 2.5 minutes at room temperature, the DMSO containing medium was substituted with IMDM medium containing 10% FBS. After incubation for 72 h at 37°C in a carbon dioxide incubator, the transfected cells were tested for their cell surface expression of human IFN-$\gamma$ receptor.

C. Binding of IFN-$\gamma$ to receptor derivatives expressed on the surface of transfected COS-1 cells

COS-1 transfected with DNA encoding membrane anchored receptor derivatives were harvested, washed with PBS, counted and resuspended in HHB buffer (HHB: Hank's balanced salt solution, 15 mM Hepes and 1% Bovine serum albumine) to have $10^7$ cells per ml. A volume of 100 $\mu$l of each cell suspension was distributed in U-bottomed polystyrene microtiter plates (Costar), before 2 ng (200 U) of iodinated human IFN-$\gamma$ ($2x10^4$ dpm/ng) labeled according to Aguet and Merlin (supra) were added. After 90 minutes on ice, the plates were centrifuged for 5 minutes at 100xg, before the supernatant with unbound radioactive IFN-$\gamma$ was discarded and the cells were washed twice with HHBT buffer (HHBT: HHB with 0.01% Triton X-100). The cells were resuspended in 200 $\mu$l HHBT, transferred to flexible U-bottomed PVC microtiter plates (Dynatech) and once more centrifuged. The flexible plates were cut and the radioactivity present in each well was determined.

After subtraction of the radioactivity obtained in experiments, in which COS-1 cells transfected with plasmids not encoding the receptor were analyzed, the resulting values are a measure for the ability of the receptor derivatives to bind IFN-$\gamma$.

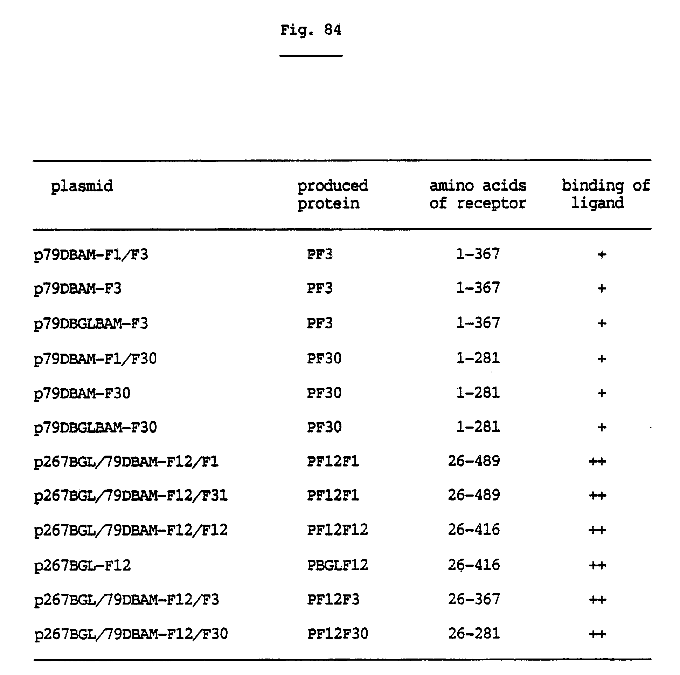

As summarized in Fig. 84, all receptor derivatives were expressed on the surface of the COS-1 cells and were able to bind IFN-$\gamma$. These results demonstrate that 1) the cDNA present in plasmid pBABLUE (see Fig. 1) encodes a signal peptide active at least in COS-1 cells, 2) amino acids 282 to 489 of the human IFN-$\gamma$ receptor are not essential for its expression and its ability to bind IFN-$\gamma$, and 3) amino acids 1 to 26 of the receptor are not essential for the binding of IFN-$\gamma$.

Example 10

Expression of soluble human IFN-γ receptors in COS-1 cells

A. Principle

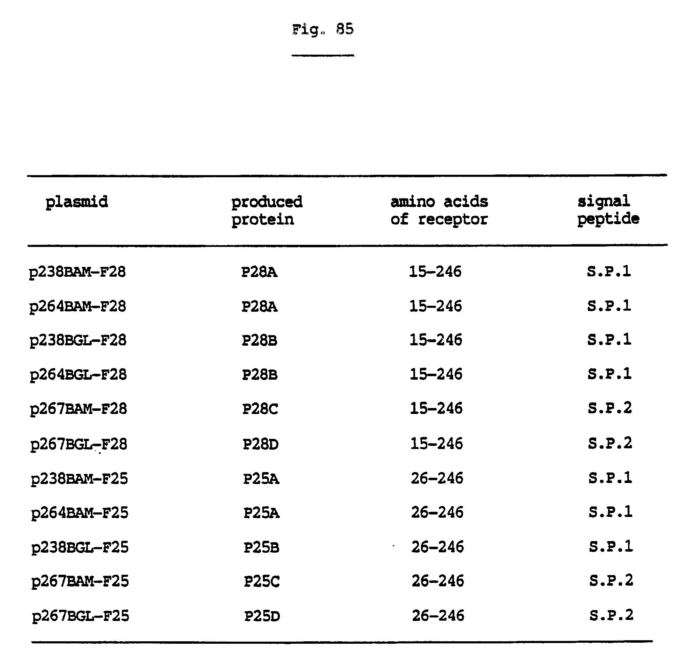

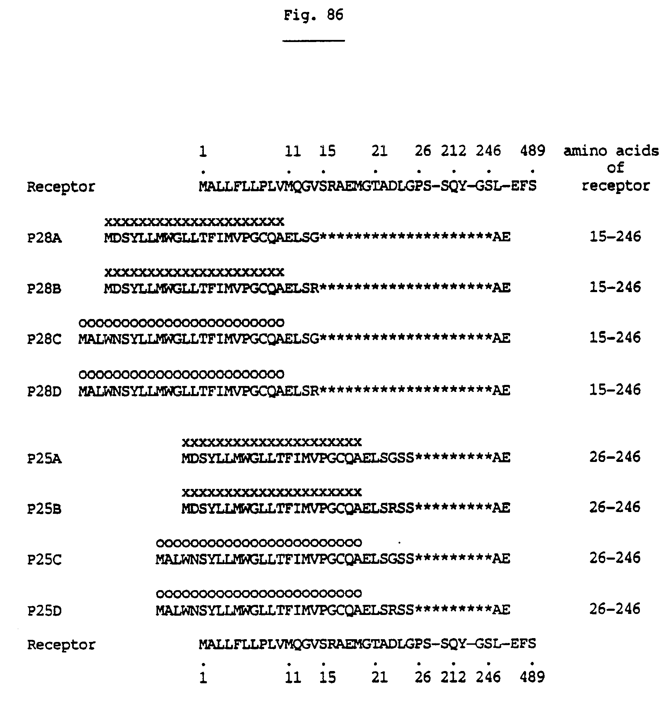

Plasmids encoding derivatives of the human IFN-γ receptor, which contain a signal peptide but lack the transmembrane region of the receptor, were transfected into COS-1 cells. Due to the absence of the transmembrane region, upon expression the receptor derivatives were expected to be secreted into the culture medium. Therefore, the culture medium of the transfected cells was analyzed in a radioimmuno assay (RIA) for the presence of soluble human IFN-γ receptors. Furthermore, the capacity of such molecules to bind IFN-γ was analyzed by testing: 1) their ability to inhibit the binding of IFN-γ to the human IFN-γ receptor present on Raji cells, and 2) their ability to inhibit the antiviral activity caused by IFN-γ. The plasmids used in this experiment and the proteins encoded by these plasmids are listed in Fig. 85, whereas the amino acid sequences of these proteins are summarized in Fig. 86.

B. Transfection of COS-1 cells and expression of soluble human IFN-γ receptors

COS-1 cells were harvested by trypsinization, washed with PBS, counted and resuspended in DMEM 5% FBS medium, before $2x10^6$ cells were distributed in 60 mm tissue culture disks (Falcon). After 24 h at 37°C in a carbon dioxide incubator, the 70% confluent cell monolayer was washed with PBS, before 1 ml of PBS containing 3 mg/ml DEAE Dextran (Pharmacia) and 5 µg plasmid DNA were added. After 30 minutes at 37°C, the transfection cocktail was removed and 4 ml IMDM medium containing 10% FBS and 100 µg chloroquine (Sigma) were added to each plate. The plates were incubated for 2.5 h at 37°C in a carbon dioxide incubator, before the medium was aspirated and the cell monolayers were covered with IMDM medium containing 10% FBS and 10% DMSO. After 2.5 minutes at room temperature, the DMSO containing medium was substituted with IMDM medium containing 10% FBS. After incubation for 72 h at 37°C in a carbon dioxide incubator the supernatants of the transfected cells were harvested and tested for the presence of soluble human IFN-γ receptors.

C. Detection of soluble human IFN-γ receptors

Monoclonal antibodies were made according to Fazekas de St.Groth and Scheidegger (J. Immunol. Methods 35, 1-21 [1980]). Among the monoclonal antibodies obtained against native human IFN-γ receptor protein extracted and then affinity purified according to Aguet and Merlin (supra), the antibody γR38/D12 inhibited IFN-γ binding to cell-bound human IFN-γ receptor but did not precipitate the complexes formed by IFN-γ with its receptor. This antibody reacted with an epitope located in the extracellular region of human IFN-γ receptor, between aa 26-133. Another monoclonal antibody, γR99/1H4 inhibited IFN-γ binding to the cell-bound receptor and precipitated the complexes between IFN-γ and its receptor. This antibody reacted with an epitope located in the extracellular region of human IFN-γ receptor, between as 70-210. The described antibodies were believed to recognize structural epitopes because they bound only the non-reduced form of the human IFN-γ receptor. Both monoclonal antibodies against human IFN-γ receptor were IgG1 and were affinity purified on protein G column (Pharmacia) according to Pharmacia instructions. The antibody γR99/1H4 was radiolabelled by $^{125}I$ in presence of chloramine T according to Hunter (Handbook of Experimental Immunology, D.M. Weir ed., Blackwell Scientific Publications Oxford [1973]). The stock solution of antibody γR38/D12 was diluted with PBS to have 5 µg/ml of pure antibody. A volume of 100 µl of this solution containing 0.5 µg of pure antibody was distributed in each well of PVC flat-bottomed microtiter plates (Dynatech) to coat the plastic. The coating reaction was performed overnight at 4°C. The residual binding capacity of plastic was then blocked by adding to each well 250 µl of PBS containing 3% Bovine serum albumine (BSA) and by incubating the microtiter plates at room temperature for at least 2 h. After blocking, the plates were washed 3 times with PBS and the RIA performed as follow. The supernatants containing soluble human IFN-γ receptor derivatives released by transfected COS-1 cells, were diluted 1:5, 1:50, 1:500, 1:5000 with medium (Optimem Gibco, 2% FBS). The stock solutions of soluble human IFN-γ receptor derivatives (see Example 6) were diluted with medium to have 30 ng/ml, 10 ng/ml, 3 ng/ml, 1 ng/ml, 0.3 ng/ml and 0.1 ng/ml of the soluble receptor. A volume of 100 µl from each dilution was distributed in 3 wells of coated plates and 20 µl of a solution containing 3 ng (180'000 dpm) of iodinated antibody γR99/1H4 were immediately added. After 3 h of incubation at room temperature, with occasional shakings, the plates were washed 6 times with PBS. After the last washing, the plates were air-dried, cut and the radioactivity of each well was determined. The concentration of soluble human IFN-γ receptor released by transfected COS-1 cells was determined on the basis of a standard curve produced with soluble IFN-γ receptor purified from E.coli.

Fig. 87 summarizes the proteins which could be detected in the supernatants of the transfected COS-1 cells.

D. Inhibition of IFN-γ binding to Raji cells by soluble human IFN-γ receptors

The binding capacity of soluble human IFN-γ receptors expressed in E.coli or in COS-1 cells was determined by inhibition of IFN-γ binding to Raji cell surface receptor. The solutions containing the soluble human IFN-γ receptors were serially diluted with PBS and 50 µl of each solution were distributed in triplicate in the wells of polystyrene U-bottomed microtiter plates (Costar). A volume of 50 µl with 2 ng (200 U) iodinated IFN-γ of a preparation with $2x10^4$ dpm/ng was added to each well after dilution in HHB medium. Then 100 µl of HHB medium containing $10^6$ Raji cells (ATCC CCL 86) were added. After incubation for 90 minutes at 0°C, the plates were centrifuged for 5 minute at 100xg, before the supernatant with unbound radioactive IFN-γ was discarded and the cells were washed twice with HHBT buffer. Then the cells were resuspended in 200 µl HHBT, transferred to flexible U-bottomed PVC microtiter plates (Dynatech) and once more centrifuged. The flexible plates were cut and the radioactivity in each well was determined. In Figs 44 and 87 the proteins inhibiting the binding of IFN-γ to Raji cells are summarized.

E. Inhibition of the antiviral activity of IFN-γ by soluble human IFN-γ receptors

The binding capacity of soluble human IFN-γ receptors expressed in E.coli or in COS-1 cells was determined by the inhibition of antiviral activity exerted by IFN-γ. The solutions containing the soluble receptors were serially diluted with Minimal Essential Medium (MEM) from GIBCO additionated with 5% FBS. A volume of 50 µl of each dilution was distributed in triplicate to the wells of tissue culture grade flat-bottomed microtiter plates (Costar). Then 25 µl containing 0.25 pg of IFN-γ (1 U/ml of a preparation of IFN-γ with a specific activity of $10^8$ U/mg) were added to each well. After 1 h at room temperature the IFN-γ activity therein was determined by the cytopathic effect (CPE) reading method according to Armstrong (Methods in Enzymology, S. Pestka ed., vol. 78 page 38, Academic Press New York [1981]). Thus, 50 µl of a WISH cell (ATCC CCL 25) suspension ($4x10^5$ cells/ml) in 5% FBS-containing MEM were placed in each well of the 96-well microplates and incubation was conducted in a carbon dioxide gas incubator (5% $CO_2$) at 37°C. After 24 h the culture supernatants were discarded and 100 µl of MEM medium with 2% FBS and 1000 plaque forming units of Encephalomyocarditis (EMC) Virus (ATCC VR-129B) were added to each well. The plates were incubated for 90 minutes at 37°C, the medium discarded and substituted with 100 µl 2% FBS-containing MEM. About 24 h later, when the cells in wells not containing IFN-γ show 100% CPE, the medium was discarded and the remaining adherent cells were washed with PBS. Then 100 µl of a crystal violet solution (1% crystal violet, 10% ethanol in water) were added to each well. After 5 minutes, the unbound dye was removed by washing with water before the stained monolayers were dried. Finally, the dye associated with the cells was extracted in 100 µl methylcellosolve and quantitated by reading the absorbance at 550 nm in a Titertek Multiskan$^R$ MC (Flow Laboratories). Since the amount of dye is proportional to the number of cells present per well, the obtained absorbance readings are a measure for the protection of IFN-γ against the viral cytopatic effect. The capacity of the soluble human IFN-γ receptors to inhibit this protection is summarized in Figs. 44 and 87.

Example 11

Expression of soluble human IFN-γ receptors using the baculovirus expression system

A. Construction of transfer vector pN57

The baculovirus transfer vector pVL941 (Luckow and Summers 1989 Virology 170, 31-39 [1989]) was digested with BamHI and then treated with calf intestinal alkaline phosphatase. The dephosphorylated vector was then isolated from an agarose gel as described above. The vector was ligated with a fragment encoding the extracellular domain of the IFN-γ receptor (amino acids 1-246 of Fig. 2). E.coli strain HB 101 was transformed and transformants which had the gene in the correct orientation for expression from the polyhedrin promoter were identified by restriction analysis and sequencing. The resulting transfer vector pN57 was isolated from E.coli using standard procedures. E.coli HB 101 cells transformed with transfervector pN57 were deposited at the Deutsche Sammlung von Mikroorganismen in Braunschweig on January 19, 1990 in accordance with the Budapest Treaty, the accession no. being DSM 5751.

B. Transfection of insect cells and isolation of recombinant baculoviruses

The transfer vector pN57 was transfected into Sf9 cells (ATCC CRL1711) together with the DNA of the baculovirus AcMNPV (described in European patent appliction, Publ. No. 127839) Polyhedrin minus viruses were identified and plaques purified using standard techniques (Summers and Smith 1986, "Manual of Methods for Baculovirus and Insect Culture Procedures, Texas Agricultural Experimental Station). One of the isolated recombinant virus was deriguated V57.

C. Detection of soluble human IFN-γ receptor (designated protein N57) in the medium of Sf9-cells infectedwith the recombinant virus V57

Sf9 cells were infected with the recombinant virus V57 at a multiplicity of 10. 72 hours post transfection the cells were removed by centrifugation (2 x 15 minutes, 15000 rpm, 4°C) and the supernatant was analysed for released soluble human IFN-γ receptor using the RIA as described in Example 10 above. Furthermore, the capacity of such molecules to bind IFN-γ was analysed by testing their ability to inhibit the binding of IFN-γ to the human IFN-γ receptor present on Raji cells as described in Example 10.

The result of these tests are summarized in Table 1 below.

Table 1

| Detection of soluble human IFN-γ receptor in insect cell medium and its capacity to bind IFN-γ | | | |
|---|---|---|---|
| Supernatant of | Reciprocal of dilution | Detection by RIA[a] (cpm)[a] | Inhibition of IFN-γ binding to human Raji cells (cpm/$10^6$ cells)[6] |
| Sf9 cells (control) | 50 | 616 | 15749 |
| | 500 | 459 | 17219 |
| Sf9 cells infected with Baculovirus V57 | 10 | | 3956 |
| | 50 | 8226 | |
| | 100 | | 11898 |
| | 300 | | 15894 |
| | 500 | 6061 | |
| | 1000 | | 16251 |

[a] Amount of human soluble IFN-γ receptor captured by mAbs against native IFN-γ receptor as a function of the dilutions of Sf9 cell supernatants.
[b] Amount of radiolabeled IFN-γ bound by human Raji cells as a function of the dilution of Sf9 cell supernatants.

In summary, proteins P25H6C, P28H6A and P41H6 expressed in E.coli, protein P25C expressed in COS-1 cells and protein N57 expressed in the baculovirus-insect cell vector system were able to bind IFN-γ. Therefore, these soluble IFN-γ receptor molecules contain the region of the human IFN-γ receptor, which is essential for the binding of IFN-γ, in an active form and inhibited the biological activity of IFN-γ.

## Claims

**1.** A soluble IFN-γ receptor capable of specifically binding IFN-γ comprising the N-terminal portion of the natural IFN-γ receptor containing at least amino acids 26-246 of the natural IFN-γ receptor sequence and missing the cytoplasmic and transmembrane domains of the natural IFN-γ receptor.

**2.** A soluble IFN-γ receptor of claim 1 selected from the group consisting of P25H6C, P28H6A, P41H6 and P25C characterized by the amino acid sequences given in Figures 43 and 86.

3. A soluble IFN-γ receptor of claim 1 which is protein N57 encoded by transfer vector pN57 (DSM 5751).

4. A DNA sequence coding for a soluble IFN-γ receptor as claimed in claims 1 and 2.

5. A DNA sequence coding for a soluble IFN-γ receptor as claimed in claim 3.

6. An expression vector comprising a DNA sequence as claimed in claim 4 and being capable of directing expression of the DNA sequence in a compatible prokaryotic or mammalian host cell.

7. A prokaryotic or mammalian host cell transformed with an expression vector of claim 6.

8. A recombinant baculovirus comprising a DNA sequence as claimed in claim 5.

9. An insect host cell infected with a recombinant baculovirus of claim 8.

10. A method for the production of a soluble IFN-γ receptor as claimed in claims 1 and 2 comprising culturing a transformed prokaryotic or mammalian host cell in a suitable medium so that said receptor is expressed and purifying and refolding said receptor.

11. A method for the production of a soluble IFN-γ receptor as claimed in claims 1 and 3 comprising culturing an infected insect host cell in a suitable medium so that said receptor is expressed and purifying said receptor from the insect cell medium.

12. A soluble IFN-γ receptor as claimed in claim 1-3 for use as a therapeutically active agent.

13. A soluble IFN-γ receptor as claimed in claims 1-3 for use as a therapeutically active agent for the treatment of autoimmune diseases, chronic inflammations, delayed hypersensitivities, allotransplant rejections and multiple sclerosis.

14. A pharmaceutical composition comprising a soluble IFN-γ receptor as claimed in claims 1-3 and a compatible pharmaceutically acceptable carrier material.

15. The use of a soluble IFN-γ receptor as claimed in claim s 1-3 for the preparation of pharmaceutical compositions.

16. The use of a soluble IFN-γ receptor as claimed in claims 1-3 for the preparation of pharmaceutical compositions for the treatment of autoimmune diseases, chronic inflammations, delayed hypersensitivities, allotransplant rejections and multiple sclerosis.

17. The use of a soluble IFN-γ receptor as claimed in claims 1-3 for identifying IFN-γ agonists or antagonists.

**Patentansprüche**

1. Ein löslicher IFN-γ Rezeptor, der befähigt ist, IFN-γ spezifisch zu binden und der den N-terminalen Teil des natürlichen IFN-γ Rezeptorsumfasst, welcher wenigstens die Aminosäuren 26-246 des natürlichen IFN-γ Rezeptors enthält,und dem die cytoplasmatische und die durch die Membran reichende Regionen des natürlichen IFN-γ Rezeptors fehlen.

2. Ein löslicher IFN-γ Rezeptor gemäss Anspruch 1, der aus der Gruppe bestehend aus P25H6C, P28H6A, P41H6 und P25C ausgewählt ist und durch die in den Abbildungen 43 und 86 gezeigten Aminosäuresequenzen charakterisiert ist.

3. Ein löslicher IFN-γ Rezepter gemäss Anspruch 1, der Protein N57 ist und von dem Transfervektor pN57 (DSM 5751) kodiert wird.

4. Eine DNS-Sequenz, die einen löslichen IFN-γ Rezeptor gemäss den Ansprüchen 1 und 2 kodiert.

**5.** Eine DNS-Sequenz, die einen löslichen IFN-γ Rezeptor gemäss Anspruch 3 kodiert.

**6.** Ein Expressionsvektor, der eine DNS-Sequenz gemäss Anspruch 4 kodiert und der befähigt ist, die Ausprägung der DNS-Sequenz in einer verträglichen prokaryotischen Wirtszelle oder Säugerwirtszelle zu bewirken.

**7.** Eine prokaryotische Wirtszelle oder Säugerwirtszelle, die mit einem Expressionsvektor gemäss Anspruch 6 transformiert ist.

**8.** Ein rekombinanter Baculovirus, der eine DNS-Sequenz gemäss Anspruch 5 enthält.

**9.** Eine Insektenwirtszelle, die mit einem rekombinanten Baculovirus gemäss Anspruch 8 infiziert ist.

**10.** Ein Verfahren zur Herstellung eines löslichen IFN-γ Rezeptors gemäss den Ansprüchen 1 und 2, dadurch gekennzeichnet, dass man eine transformierte prokaryotische Wirtszelle oder Säugerwirtszelle in einem geeigneten Medium kultiviert, so dass besagter Rezeptor ausgeprägt wird, und besagten Rezeptor reinigt und rückfaltet.

**11.** Ein Verfahren zur Herstellung eines löslichen IFN-γ Rezeptors gemäss den Ansprüchen 1 und 3, dadurch gekennzeichnet, dass man eine infizierte Insektenwirtszelle in einem geeigneten Medium kuliviert, so dass besagter Rezeptor ausgeprägt wird, und besagten Rezeptor aus dem Insektenzellmedium aufreinigt.

**12.** Ein löslicher IFN-γ Rezeptor gemäss den Ansprüchen 1-3 zur Verwendung als ein therapeutisch wirksames Mittel.

**13.** Ein löslicher IFN-γ Rezeptor gemäss den Ansprüchen 1-3 zur Verwendung als ein therapeutisch wirksames Mittel zur Behandlung von Autoimmunkrankheiten, chronischen Entzündungen, verzögerten Ueberempfindlichkeiten, Allotransplantat-Abstossungen und Multiple Sklerose.

**14.** Eine pharmazeutische Zusammensetzung, enthaltend einen löslichen IFN-γ Rezeptor gemäss den Ansprüchen 1-3 und ein verträgliches pharmazeutisch annehmbares Trägermaterial.

**15.** Die Verwendung eines löslichen IFN-γ Rezeptors gemäss den Ansprüchen 1-3 zur Herstellung von pharmazeutischen Zusammensetzungen.

**16.** Die Verwendung eines löslichen IFN-γ Rezeptors gemäss den Ansprüchen 1-3 zur Herstellung von pharmazeutischen Zusammensetzungen zur Behandlung von Autoimmunkrankheiten, chronischen Entzündungen, verzögerten Ueberempfindlichkeiten, Allotransplantat-Abstossungen und Multiple Sklerose.

**17.** Die Verwendung eines löslichen IFN-γ Rezeptors gemäss den Ansprüchen 1-3 zur Identifizierung von IFN-γ Agonisten oder Antagonisten.

**Revendications**

**1.** Récepteur soluble de l'IFN-γ capable de se lier de manière spécifique à l'IFN-γ comportant la partie N-terminale du récepteur naturel de l'IFN-γ contenant au moins les acides aminés 26 à 246 de la séquence du récepteur naturel de l'IFN-γ et n'ayant pas les domaines cytoplasmique et transmembranaire du récepteur naturel de l'IFN-γ.

**2.** Récepteur soluble de l'IFN-γ selon la revendication 1, sélectionné parmi le groupe composé de P25H6C, P28H6A, P41H6 et P25C, caractérisé par les séquences d'acides aminés données sur les figures 43 et 86.

**3.** Récepteur soluble de l'IFN-γ selon la revendication 1, qui est la protéine N57 codée par le vecteur de transfert pN57 (DSM 5751).

**4.** Séquence d'ADN codant pour un récepteur soluble de l'IFN-γ selon les revendications 1 et 2.

**5.** Séquence d'ADN codant pour un récepteur soluble de l'IFN-$\gamma$ selon la revendication 3.

**6.** Vecteur d'expression comportant une séquence d'ADN selon la revendication 4 et étant capable de diriger l'expression de la séquence d'ADN dans une cellule hôte compatible de procaryote ou de mammifère .

**7.** Cellule hôte de procaryote ou de mammifère transformée par un vecteur d'expression selon la revendication 6.

**8.** Baculovirus recombinant comportant une séquence d'ADN selon la revendication 5.

**9.** Cellule hôte d'insecte infectée par un baculovirus recombinant selon la revendication 8.

**10.** Procédé pour la production d'un récepteur soluble de l'IFN-$\gamma$ selon les revendications 1 et 2, comportant la mise en culture d'une cellule hôte de procaryote ou de mammifère transformée dans un milieu approprié de sorte à ce que ledit récepteur soit exprimé et la purification et le repliement dudit récepteur.

**11.** Procédé pour la production d'un récepteur soluble de l'IFN-$\gamma$ selon les revendications 1 et 3, comportant la mise en culture d'une cellule hôte d'insecte infectée dans un milieu approprié de sorte à ce que ledit récepteur soit exprimé et la purification dudit récepteur à partir du milieu de culture de cellules d'insecte .

**12.** Récepteur soluble de l'IFN-$\gamma$ selon les revendications 1 à 3, destiné à une utilisation comme agent actif de manière thérapeutique.

**13.** Récepteur soluble de l'IFN-$\gamma$ selon les revendications 1 à 3, destiné à une utilisation comme agent actif de manière thérapeutique pour le traitement de maladies auto-immunes, d'inflammations chroniques, d'hypersensibilités retardées, de rejets d'allo-transplantation et de scléroses multiples.

**14.** Composition pharmaceutique comportant un récepteur soluble de l'IFN-$\gamma$ selon les revendications 1 à 3, et un matériel de support compatible acceptable de manière pharmaceutique.

**15.** Utilisation d'un récepteur soluble de l'IFN-$\gamma$ selon les revendications 1 à 3 pour la préparation de compositions pharmaceutiques.

**16.** Utilisation d'un récepteur soluble de l'IFN-$\gamma$ selon les revendications 1 à 3, pour la préparation de compositions pharmaceutiques destinées au traitement de maladies auto-immunes, d'inflammations chroniques, d'hypersensibilités retardées, de rejets d'allotransplantation et de scléroses multiples.

**17.** Utilisation d'un récepteur soluble de l'IFN-$\gamma$ selon les revendications 1 à 3, pour l'identification d'agonistes ou d'antagonistes à l'IFN-$\gamma$.

## Fig. 1

```
        10                  30                  50
SacI     .         .   Xba .      BamHI  SmaI  .     EcoRI.
GAGCTCCACCGCGGTGGCGGCCGCTCTAGAACTAGTGGATCCCCCGGGCTGCAGGAATTC

        70                  90                 110
         .         .         .         .         .         .
CAGCCAGCGACCGTCGGTAGCAGCATGGCTCTCCTCTTTCTCCTACCCCTTGTCATGCAG
                        MetAlaLeuLeuPheLeuLeuProLeuValMetGln
                         1
       130                 150                 170
         .         .         .  HaeIII   .         .         .
GGTGTGAGCAGGGCTGAGATGGGCACCGCGGATCTGGGGCCGTCCTCAGTGCCTACACCA
GlyValSerArgAlaGluMetGlyThrAlaAspLeuGlyProSerSerValProThrPro
     15                                 26
       190                 210                 230
         .         .         .         .         .         .
ACTAATGTTACAATTGAATCCTATAACATGAACCCTATCGTATATTGGAGTACCAGATC
ThrAsnValThrIleGluSerTyrAsnMetAsnProIleValTyrTrpGluTyrGlnIle

       250                 270                 290
        PpuMI      .         .         .        .EcoRI     .
ATGCCACAGGTCCCTGTTTTTACCGTAGAGGTAAAGAACTATGGTGTTAAGAATTCAGAA
MetProGlnValProValPheThrValGluValLysAsnTyrGlyValLysAsnSerGlu

       310                 330                 350
         .         .         .         .         .         .
TGGATTGATGCCTGCATCAATATTTCTCATCATTATTGTAATATTTCTGATCATGTTGGT
TrpIleAspAlaCysIleAsnIleSerHisHisTyrCysAsnIleSerAspHisValGly

       370                 390                 410
         .         .         .         .         .         .
GATCCATCAAATTCTCTTTGGGTCAGAGTTAAAGCCAGGGTTGGACAAAAAGAATCTGCC
AspProSerAsnSerLeuTrpValArgValLysAlaArgValGlyGlnLysGluSerAla

       430                 450                 470
         .         .         .         .         .         .
TATGCAAAGTCAGAAGAATTTGCTGTATGCCGAGATGGAAAAATTGGACCACCTAAACTG
TyrAlaLysSerGluGluPheAlaValCysArgAspGlyLysIleGlyProProLysLeu

       490                 510                 530
EcoRV    .         .         .         .         .         .
GATATCAGAAAGGAGGAGAAGCAAATCATGATTGACATATTTCACCCTTCAGTTTTTGTA
AspIleArgLysGluGluLysGlnIleMetIleAspIlePheHisProSerValPheVal

       550                 570                 590
         .         .         .         .         .         .
AATGGAGACGAGCAGGAAGTCGATTATGATCCCGAAACTACCTGTTACATTAGGGTGTAC
AsnGlyAspGluGlnGluValAspTyrAspProGluThrThrCysTyrIleArgValTyr

       610                 630                 650
         .         .         .         .         .         .
AATGTGTATGTGAGAATGAACGGAAGTGAGATCCAGTATAAAATACTCACGCAGAAGGAA
AsnValTyrValArgMetAsnGlySerGluIleGlnTyrLysIleLeuThrGlnLysGlu
```

Fig. 1 (cont.)

```
670                 690                 710
.         .         .         .         .EcoRI   ScaI
GATGATTGTGACGAGATTCAGTGCCAGTTAGCGATTCCAGTATCCTCACTGAATTCTCAG
AspAspCysAspGluIleGlnCysGlnLeuAlaIleProValSerSerLeuAsnSerGln
                                                         212
730                 750                 770
.         .         .         .         .         .
TACTGTGTTTCAGCAGAAGGAGTCTTACATGTGTGGGGTGTTACAACTGAAAAGTCAAAA
TyrCysValSerAlaGluGlyValLeuHisValTrpGlyValThrThrGluLysSerLys

790                 810                 830
.         .         .         .         .         .
GAAGTTTGTATTACCATTTTCAATAGCAGTATAAAAGGTTCTCTTTGGATTCCAGTTGTT
GluValCysIleThrIlePheAsnSerSerIleLysGlySerLeuTrpIleProValVal
                                          246
850                 870                 890
.         .         .         .         .         .
GCTGCTTTACTACTCTTTCTAGTGCTTAGCCTGGTATTCATCTGTTTTTATATTAAGAAA
AlaAlaLeuLeuLeuPheLeuValLeuSerLeuValPheIleCysPheTyrIleLysLys

910                 930                 950
.         .        SspI         .         .         .
ATTAATCCATTGAAGGAAAAAAGCATAATATTACCCAAGTCCTTGATCTCTGTGGTAAGA
IleAsnProLeuLysGluLysSerIleIleLeuProLysSerLeuIleSerValValArg
                           281
970                 990                 1010
.         .         .         .         .         .
AGTGCTACTTTAGAGACAAAACCTGAATCAAAATATGTATCACTCATCACGTCATACCAG
SerAlaThrLeuGluThrLysProGluSerLysTyrValSerLeuIleThrSerTyrGln

1030                1050                1070
.         .         .         .         .         .
CCATTTTCCTTAGAAAAGGAGGTGGTCTGTGAAGAGCCGTTGTCTCCAGCAACAGTTCCA
ProPheSerLeuGluLysGluValValCysGluGluProLeuSerProAlaThrValPro

1090                1110                1130
.         .         .         .         .         .
GGCATGCATACCGAAGACAATCCAGGAAAAGTGGAACATACAGAAGAACTTTCTAGTATA
GlyMetHisThrGluAspAsnProGlyLysValGluHisThrGluGluLeuSerSerIle

1150                1170                1190
.         .         .         .    SmaI .         .
ACAGAAGTGGTGACTACTGAAGAAAATATTCCTGACGTGGTCCCGGGCAGCCATCTGACT
ThrGluValValThrThrGluGluAsnIleProAspValValProGlySerHisLeuThr
                                          367
1210                1230                1250
.         .         .         .         .         .
CCAATAGAGAGAGAGAGAGTTCTTCACCTTTAAGTAGTAACCAGTCTGAACCTGGCAGCATC
ProIleGluArgGluSerSerSerProLeuSerSerAsnGlnSerGluProGlySerIle

1270                1290                1310
.         .         .         .         .         .
GCTTTAAACTCGTATCACTCCAGAAATTGTTCTGAGAGTGATCACTCCAGAAATGGTTTT
AlaLeuAsnSerTyrHisSerArgAsnCysSerGluSerAspHisSerArgAsnGlyPhe
```

Fig. 1 (cont.)

```
      1330                 1350                 1370
       . PvuII   .         .         .         .         .
GATACTGATTCCAGCTGTCTGGAATCACATAGCTCCTTATCTGACTCAGAATTTCCCCCA
AspThrAspSerSerCysLeuGluSerHisSerSerLeuSerAspSerGluPheProPro
        416
      1390                 1410                 1430
         .         .         .         .         .         .
AATAATAAAGGTGAAATAAAAACAGAAGGACAAGAGCTCATAACCGTAATAAAAGCCCCC
AsnAsnLysGlyGluIleLysThrGluGlyGlnGluLeuIleThrValIleLysAlaPro

      1450                 1470                 1490
         .         .         .         .         .         .
ACCTCCTTTGGTTATGATAAACCACATGTGCTAGTGGATCTACTTGTGGATGATAGCGGT
ThrSerPheGlyTyrAspLysProHisValLeuValAspLeuLeuValAspAspSerGly

      1510                 1530                 1550
         .         .         .         .         .  MboI .
AAAGAGTCCTTGATTGGTTATAGACCAACAGAAGATTCCAAAGAATTTTCATGAGATCAG
LysGluSerLeuIleGlyTyrArgProThrGluAspSerLysGluPheSer
                                                489
      1570                 1590                 1610
         .         .         .         .         .         .
CTAAGTTGCACCAACTTTGAAGTCTGATTTTCCTGGACAGTTTTCTGCTTTAATTTCATG

      1630                 1650                 1670
         .         .         .         .         .         .
AAAAGATTATGATCTCAGAAATTGTATCTTAGTTGGTATCAACCAAATGGAGTGACTTAG

      1690                 1710                 1730
         .         .         .         .         .         .
TGTACATGAAAGCGTAAAGAGGATGTGTGGCATTTTCACTTTTGGCTTGTAAAGTACAGA

      1750                 1770                 1790
         .         .         .         .         .         .
CTTTTTTTTTTTTTTAAACAAAAAAAGCATGTAACTTATGAACCTTTACATCCAGATAGG

      1810                 1830                 1850
         .         .  ScaI .         .         .         .
TTACCAGTAACGGAACAGTATCCAGTACTCCTGGTTCCTAGGTGAGCAGGTGATGCCCCA

      1870                 1890                 1910
         .         .         .         .         .         .
GGGACCTTTGTAGCCACTTCACTTTTTTTCTTTTCTCTGCCTTGGTATAGCATATGTTTT

      1930                 1950                 1970
         .         .         .         .         .HindIII  .
TGTAAGTTTATGCATACAGTAATTTTAAGTAATTTCAGAAGAAATTCTCGAAGCTTTTCA

      1990                 2010                 2030
         .         .         .         .         .         .
AAATTGGACTTAAAATCTAATTCAAACTAATAGAATTAATGGAATATGTAAATAGAAACG
```

Fig. 1 (cont.)

```
      2050                2070                2090
         .         .         .         .         .         .
TGTATATTTTTTATGAAACATTACAGTTAGAGATTTTTAAATAAAGAATTTTAAAACTCA

      2110                2130                2150
         .         .         .EcoRI EcoRV HindIII.         .
AAAAAAAAAAAAAAAAAAAAAAAAAAAAAGGAATTCGATATCAAGCTTATCGATACCGTC

      2170
    XhoI .         Asp718I
GACCTCGAGGGGGGGCCCGGTACC
```

Fig. 2

```
     o  o  o  o  o  o  o  o  o  o  o  o  o  o  o  o
  1  MetAlaLeuLeuPheLeuLeuProLeuValMetGlnGlyValSerArgAlaGluMetGly

 21  ThrAlaAspLeuGlyProSerSerValProThrProThrAsnValThrIleGluSerTyr

 41  AsnMetAsnProIleValTyrTrpGluTyrGlnIleMetProGlnValProValPheThr
                                                      x
 61  ValGluValLysAsnTyrGlyValLysAsnSerGluTrpIleAspAlaCysIleAsnIle
                 x
 81  SerHisHisTyrCysAsnIleSerAspHisValGlyAspProSerAsnSerLeuTrpVal

101  ArgValLysAlaArgValGlyGlnLysGluSerAlaTyrAlaLysSerGluGluPheAla
        x
121  ValCysArgAspGlyLysIleGlyProProLysLeuAspIleArgLysGluGluLysGln

141  IleMetIleAspIlePheHisProSerValPheValAsnGlyAspGluGlnGluValAsp
                       x
161  TyrAspProGluThrThrCysTyrIleArgValTyrAsnValTyrValArgMetAsnGly
                                               x              x
181  SerGluIleGlnTyrLysIleLeuThrGlnLysGluAspAspCysAspGluIleGlnCys
                                            x
201  GlnLeuAlaIleProValSerSerLeuAsnSerGlnTyrCysValSerAlaGluGlyVal
                                               x
221  LeuHisValTrpGlyValThrThrGluLysSerLysGluValCysIleThrIlePheAsn

                       *  *  *  *  *  *  *  *  *  *  *  *  *  *
241  SerSerIleLysGlySerLeuTrpIleProValValAlaAlaLeuLeuLeuPheLeuVal

        *  *  *  *  *  *  *  x
261  LeuSerLeuValPheIleCysPheTyrIleLysLysIleAsnProLeuLysGluLysSer

281  IleIleLeuProLysSerLeuIleSerValValArgSerAlaThrLeuGluThrLysPro

301  GluSerLysTyrValSerLeuIleThrSerTyrGlnProPheSerLeuGluLysGluVal
        x
321  ValCysGluGluProLeuSerProAlaThrValProGlyMetHisThrGluAspAsnPro

341  GlyLysValGluHisThrGluGluLeuSerSerIleThrGluValValThrThrGluGlu

361  AsnIleProAspValValProGlySerHisLeuThrProIleGluArgGluSerSerSer

381  ProLeuSerSerAsnGlnSerGluProGlySerIleAlaLeuAsnSerTyrHisSerArg
        x                                                  x
401  AsnCysSerGluSerAspHisSerArgAsnGlyPheAspThrAspSerSerCysLeuGlu

421  SerHisSerSerLeuSerAspSerGluPheProProAsnAsnLysGlyGluIleLysThr

441  GluGlyGlnGluLeuIleThrValIleLysAlaProThrSerPheGlyTyrAspLysPro

461  HisValLeuValAspLeuLeuValAspAspSerGlyLysGluSerLeuIleGlyTyrArg

481  ProThrGluAspSerLysGluPheSer
```

Fig. 3

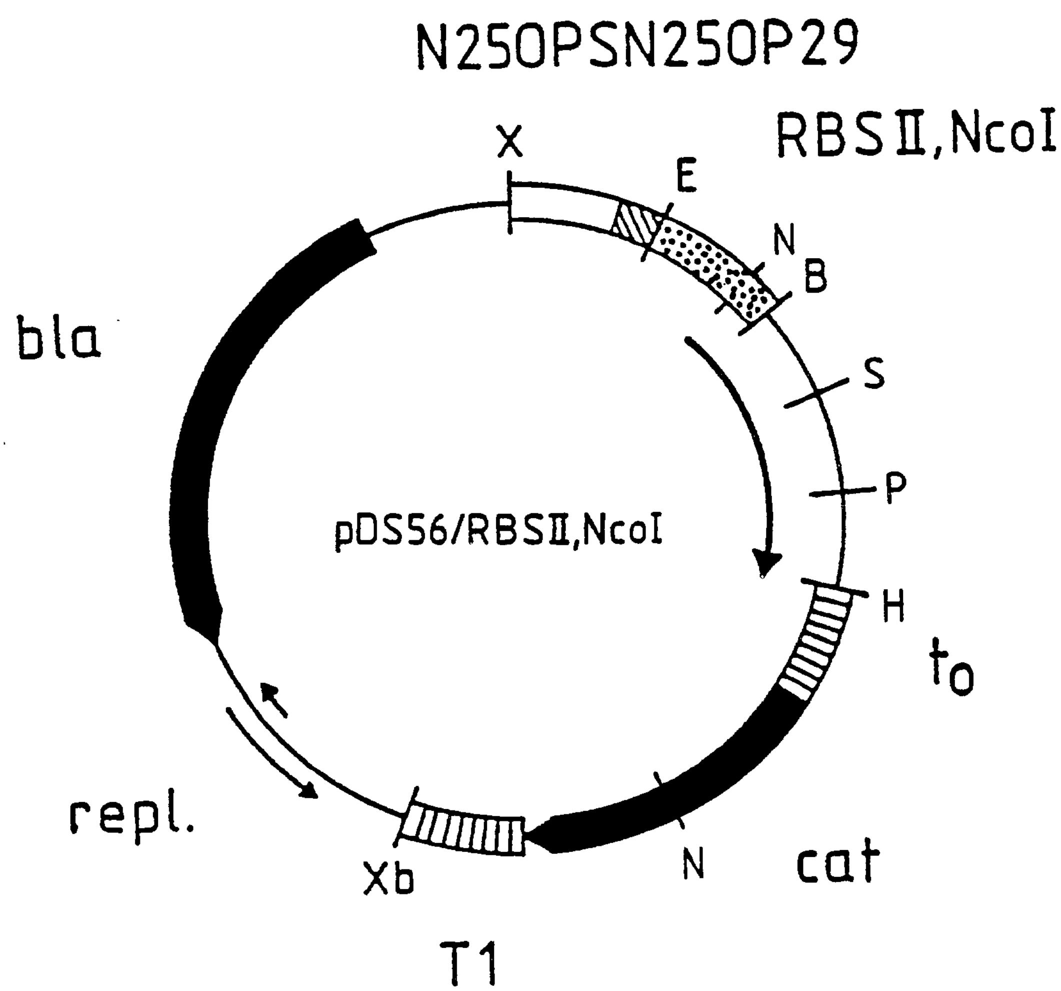
N25OPSN25OP29
RBSII,NcoI
X
E
N
B
S
P
H
to
cat
N
Xb
T1
repl.
bla
pDS56/RBSII,NcoI

Fig. 4

```
        XhoI
   1    CTCGAGAAAT CATAAAAAAT TTATTTGCTT TGTGAGCGGA TAACAATTAT

                                                   EcoRI
  51    AATAGATTCA ATTGTGAGCG GATAACAATT TCACACAGAA TTCATTAAAG

                      NcoI     BamHI  SalI   PstI     HindIII
 101    AGGAGAAATT AACCATGGGA GGATCCGTCG ACCTGCAGCC AAGCTTAATT
                     MetArg GlySerValA spLeuGlnPr oSerLeuIle

 151    AGCTGAGCTT GGACTCCTGT TGATAGATCC AGTAATGACC TCAGAACTCC
        Ser

 201    ATCTGGATTT GTTCAGAACG CTCGGTTGCC GCCGGGCGTT TTTTATTGGT

 251    GAGAATCCAA GCTAGCTTGG CGAGATTTTC AGGAGCTAAG GAAGCTAAAA

 301    TGGAGAAAAA AATCACTGGA TATACCACCG TTGATATATC CCAATGGCAT

 351    CGTAAAGAAC ATTTTGAGGC ATTTCAGTCA GTTGCTCAAT GTACCTATAA

 401    CCAGACCGTT CAGCTGGATA TTACGGCCTT TTTAAAGACC GTAAAGAAAA

 451    ATAAGCACAA GTTTTATCCG GCCTTTATTC ACATTCTTGC CCGCCTGATG

 501    AATGCTCATC CGGAATTTCG TATGGCAATG AAAGACGGTG AGCTGGTGAT

 551    ATGGGATAGT GTTCACCCTT GTTACACCGT TTTCCATGAG CAAACTGAAA

 601    CGTTTTCATC GCTCTGGAGT GAATACCACG ACGATTTCCG GCAGTTTCTA

 651    CACATATATT CGCAAGATGT GGCGTGTTAC GGTGAAAACC TGGCCTATTT

 701    CCCTAAAGGG TTTATTGAGA ATATGTTTTT CGTCTCAGCC AATCCCTGGG

 751    TGAGTTTCAC CAGTTTTGAT TTAAACGTGG CCAATATGGA CAACTTCTTC

                        NcoI
 801    GCCCCCGTTT TCACCATGGG CAAATATTAT ACGCAAGGCG ACAAGGTGCT

 851    GATGCCGCTG GCGATTCAGG TTCATCATGC CGTCTGTGAT GGCTTCCATG

 901    TCGGCAGAAT GCTTAATGAA TTACAACAGT ACTGCGATGA GTGGCAGGGC

 951    GGGGCGTAAT TTTTTTAAGG CAGTTATTGG TGCCCTTAAA CGCCTGGGGT

1001    AATGACTCTC TAGCTTGAGG CATCAAATAA AACGAAAGGC TCAGTCGAAA

1051    GACTGGGCCT TTCGTTTTAT CTGTTGTTTG TCGGTGAACG CTCTCCTGAG

                              XbaI
1101    TAGGACAAAT CCGCCGCTCT AGAGCTGCCT CGCGCGTTTC GGTGATGACG
```

Fig. 4 (cont.)

1151 GTGAAAACCT CTGACACATG CAGCTCCCGG AGACGGTCAC AGCTTGTCTG
1201 TAAGCGGATG CCGGGAGCAG ACAAGCCCGT CAGGGCGCGT CAGCGGGTGT
1251 TGGCGGGTGT CGGGGCGCAG CCATGACCCA GTCACGTAGC GATAGCGGAG
1301 TGTATACTGG CTTAACTATG CGGCATCAGA GCAGATTGTA CTGAGAGTGC
1351 ACCATATGCG GTGTGAAATA CCGCACAGAT GCGTAAGGAG AAAATACCGC
1401 ATCAGGCGCT CTTCCGCTTC CTCGCTCACT GACTCGCTGC GCTCGGTCTG
1451 TCGGCTGCGG CGAGCGGTAT CAGCTCACTC AAAGGCGGTA ATACGGTTAT
1501 CCACAGAATC AGGGGATAAC GCAGGAAAGA ACATGTGAGC AAAAGGCCAG
1551 CAAAAGGCCA GGAACCGTAA AAAGGCCGCG TTGCTGGCGT TTTTCCATAG
1601 GCTCCGCCCC CCTGACGAGC ATCACAAAAA TCGACGCTCA AGTCAGAGGT
1651 GGCGAAACCC GACAGGACTA TAAAGATACC AGGCGTTTCC CCCTGGAAGC
1701 TCCCTCGTGC GCTCTCCTGT TCCGACCCTG CCGCTTACCG GATACCTGTC
1751 CGCCTTTCTC CCTTCGGGAA GCGTGGCGCT TTCTCAATGC TCACGCTGTA
1801 GGTATCTCAG TTCGGTGTAG GTCGTTCGCT CCAAGCTGGG CTGTGTGCAC
1851 GAACCCCCCG TTCAGCCCGA CCGCTGCGCC TTATCCGGTA ACTATCGTCT
1901 TGAGTCCAAC CCGGTAAGAC ACGACTTATC GCCACTGGCA GCAGCCACTG
1951 GTAACAGGAT TAGCAGAGCG AGGTATGTAG GCGGTGCTAC AGAGTTCTTG
2001 AAGTGGTGGC CTAACTACGG CTACACTAGA AGGACAGTAT TTGGTATCTG
2051 CGCTCTGCTG AAGCCAGTTA CCTTCGGAAA AAGAGTTGGT AGCTCTTGAT
2101 CCGGCAAACA AACCACCGCT GGTAGCGGTG GTTTTTTTGT TTGCAAGCAG
2151 CAGATTACGC GCAGAAAAAA AGGATCTCAA GAAGATCCTT TGATCTTTTC
2201 TACGGGGTCT GACGCTCAGT GGAACGAAAA CTCACGTTAA GGGATTTTGG
2251 TCATGAGATT ATCAAAAAGG ATCTTCACCT AGATCCTTTT AAATTAAAAA
2301 TGAAGTTTTA AATCAATCTA AAGTATATAT GAGTAAACTT GGTCTGACAG
2351 TTACCAATGC TTAATCAGTG AGGCACCTAT CTCAGCGATC TGTCTATTTC
2401 GTTCATCCAT AGCTGCCTGA CTCCCCGTCG TGTAGATAAC TACGATACGG
2451 GAGGGCTTAC CATCTGGCCC CAGTGCTGCA ATGATACCGC GAGACCCACG

Fig. 4 (cont.)

2501 CTCACCGGCT CCAGATTTAT CAGCAATAAA CCAGCCAGCC GGAAGGGCCG
2551 AGCGCAGAAG TGGTCCTGCA ACTTTATCCG CCTCCATCCA GTCTATTAAT
2601 TGTTGCCGGG AAGCTAGAGT AAGTAGTTCG CCAGTTAATA GTTTGCGCAA
2651 CGTTGTTGCC ATTGCTACAG GCATCGTGGT GTCACGCTCG TCGTTTGGTA
2701 TGGCTTCATT CAGCTCCGGT TCCCAACGAT CAAGGCGAGT TACATGATCC
2751 CCCATGTTGT GCAAAAAAGC GGTTAGCTCC TTCGGTCCTC CGATCGTTGT
2801 CAGAAGTAAG TTGGCCGCAG TGTTATCACT CATGGTTATG GCAGCACTGC
2851 ATAATTCTCT TACTGTCATG CCATCCGTAA GATGCTTTTC TGTGACTGGT
2901 GAGTACTCAA CCAAGTCATT CTGAGAATAG TGTATGCGGC GACCGAGTTG
2951 CTCTTGCCCG GCGTCAATAC GGGATAATAC CGCGCCACAT AGCAGAACTT
3001 TAAAAGTGCT CATCATTGGA AAACGTTCTT CGGGGCGAAA ACTCTCAAGG
3051 ATCTTACCGC TGTTGAGATC CAGTTCGATG TAACCCACTC GTGCACCCAA
3101 CTGATCTTCA GCATCTTTTA CTTTCACCAG CGTTTCTGGG TGAGCAAAAA
3151 CAGGAAGGCA AAATGCCGCA AAAAAGGGAA TAAGGGCGAC ACGGAAATGT
3201 TGAATACTCA TACTCTTCCT TTTTCAATAT TATTGAAGCA TTTATCAGGG
3251 TTATTGTCTC ATGAGCGGAT ACATATTTGA ATGTATTTAG AAAAATAAAC
3301 AAATAGGGGT TCCGCGCACA TTTCCCCGAA AAGTGCCACC TGACGTCTAA
3351 GAAACCATTA TTATCATGAC ATTAACCTAT AAAAATAGGC GTATCACGAG
3401 GCCCTTTCGT CTTCAC

Fig. 5

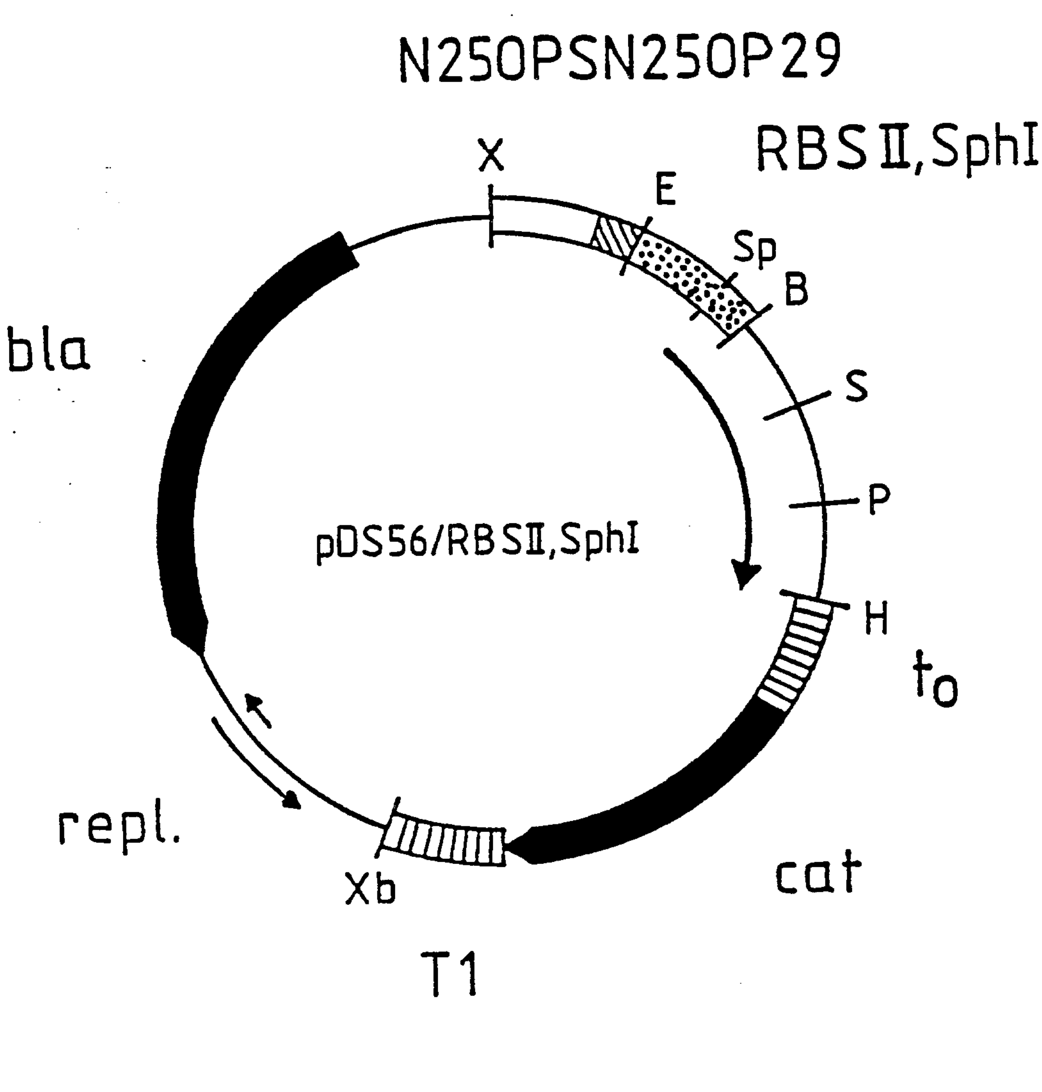
N25OPSN25OP29
RBSII,SphI
X
E
Sp
B
S
P
H
to
bla
pDS56/RBSII,SphI
repl.
Xb
T1
cat

Fig. 6

```
      XhoI
   1  CTCGAGAAAT CATAAAAAAT TTATTTGCTT TGTGAGCGGA TAACAATTAT

                                                EcoRI
  51  AATAGATTCA ATTGTGAGCG GATAACAATT TCACACAGAA TTCATTAAAG

                       SphI     BamHI  SalI    PstI     HindIII
 101  AGGAGAAATT AAGCATGCGA GGATCCGTCG ACCTGCAGCC AAGCTTAATT
                    MetArg GlySerValA spLeuGlnPr oSerLeuIle

 151  AGCTGAGCTT GGACTCCTGT TGATAGATCC AGTAATGACC TCAGAACTCC
      Ser

 201  ATCTGGATTT GTTCAGAACG CTCGGTTGCC GCCGGGCGTT TTTTATTGGT

 251  GAGAATCCAA GCTAGCTTGG CGAGATTTTC AGGAGCTAAG GAAGCTAAAA

 301  TGGAGAAAAA AATCACTGGA TATACCACCG TTGATATATC CCAATGGCAT

 351  CGTAAAGAAC ATTTTGAGGC ATTTCAGTCA GTTGCTCAAT GTACCTATAA

 401  CCAGACCGTT CAGCTGGATA TTACGGCCTT TTTAAAGACC GTAAAGAAAA

 451  ATAAGCACAA GTTTTATCCG GCCTTTATTC ACATTCTTGC CCGCCTGATG

 501  AATGCTCATC CGGAATTTCG TATGGCAATG AAAGACGGTG AGCTGGTGAT

 551  ATGGGATAGT GTTCACCCTT GTTACACCGT TTTCCATGAG CAAACTGAAA

 601  CGTTTTCATC GCTCTGGAGT GAATACCACG ACGATTTCCG GCAGTTTCTA

 651  CACATATATT CGCAAGATGT GGCGTGTTAC GGTGAAAACC TGGCCTATTT

 701  CCCTAAAGGG TTTATTGAGA ATATGTTTTT CGTCTCAGCC AATCCCTGGG

 751  TGAGTTTCAC CAGTTTTGAT TTAAACGTGG CCAATATGGA CAACTTCTTC

 801  GCCCCCGTTT TCACCATGGG CAAATATTAT ACGCAAGGCG ACAAGGTGCT

 851  GATGCCGCTG GCGATTCAGG TTCATCATGC CGTCTGTGAT GGCTTCCATG

 901  TCGGCAGAAT GCTTAATGAA TTACAACAGT ACTGCGATGA GTGGCAGGGC

 951  GGGGCGTAAT TTTTTTAAGG CAGTTATTGG TGCCCTTAAA CGCCTGGGGT

1001  AATGACTCTC TAGCTTGAGG CATCAAATAA AACGAAAGGC TCAGTCGAAA

1051  GACTGGGCCT TTCGTTTTAT CTGTTGTTTG TCGGTGAACG CTCTCCTGAG

                                XbaI
1101  TAGGACAAAT CCGCCGCTCT AGAGCTGCCT CGCGCGTTTC GGTGATGACG
```

Fig. 6 (cont.)

1151 GTGAAAACCT CTGACACATG CAGCTCCCGG AGACGGTCAC AGCTTGTCTG
1201 TAAGCGGATG CCGGGAGCAG ACAAGCCCGT CAGGGCGCGT CAGCGGGTGT
1251 TGGCGGGTGT CGGGGCGCAG CCATGACCCA GTCACGTAGC GATAGCGGAG
1301 TGTATACTGG CTTAACTATG CGGCATCAGA GCAGATTGTA CTGAGAGTGC
1351 ACCATATGCG GTGTGAAATA CCGCACAGAT GCGTAAGGAG AAAATACCGC
1401 ATCAGGCGCT CTTCCGCTTC CTCGCTCACT GACTCGCTGC GCTCGGTCTG
1451 TCGGCTGCGG CGAGCGGTAT CAGCTCACTC AAAGGCGGTA ATACGGTTAT
1501 CCACAGAATC AGGGGATAAC GCAGGAAAGA ACATGTGAGC AAAAGGCCAG
1551 CAAAAGGCCA GGAACCGTAA AAAGGCCGCG TTGCTGGCGT TTTTCCATAG
1601 GCTCCGCCCC CCTGACGAGC ATCACAAAAA TCGACGCTCA AGTCAGAGGT
1651 GGCGAAACCC GACAGGACTA TAAAGATACC AGGCGTTTCC CCCTGGAAGC
1701 TCCCTCGTGC GCTCTCCTGT TCCGACCCTG CCGCTTACCG GATACCTGTC
1751 CGCCTTTCTC CCTTCGGGAA GCGTGGCGCT TTCTCAATGC TCACGCTGTA
1801 GGTATCTCAG TTCGGTGTAG GTCGTTCGCT CCAAGCTGGG CTGTGTGCAC
1851 GAACCCCCCG TTCAGCCCGA CCGCTGCGCC TTATCCGGTA ACTATCGTCT
1901 TGAGTCCAAC CCGGTAAGAC ACGACTTATC GCCACTGGCA GCAGCCACTG
1951 GTAACAGGAT TAGCAGAGCG AGGTATGTAG GCGGTGCTAC AGAGTTCTTG
2001 AAGTGGTGGC CTAACTACGG CTACACTAGA AGGACAGTAT TTGGTATCTG
2051 CGCTCTGCTG AAGCCAGTTA CCTTCGGAAA AAGAGTTGGT AGCTCTTGAT
2101 CCGGCAAACA AACCACCGCT GGTAGCGGTG GTTTTTTTGT TTGCAAGCAG
2151 CAGATTACGC GCAGAAAAAA AGGATCTCAA GAAGATCCTT TGATCTTTTC
2201 TACGGGGTCT GACGCTCAGT GGAACGAAAA CTCACGTTAA GGGATTTTGG
2251 TCATGAGATT ATCAAAAAGG ATCTTCACCT AGATCCTTTT AAATTAAAAA
2301 TGAAGTTTTA AATCAATCTA AAGTATATAT GAGTAAACTT GGTCTGACAG
2351 TTACCAATGC TTAATCAGTG AGGCACCTAT CTCAGCGATC TGTCTATTTC
2401 GTTCATCCAT AGCTGCCTGA CTCCCCGTCG TGTAGATAAC TACGATACGG
2451 GAGGGCTTAC CATCTGGCCC CAGTGCTGCA ATGATACCGC GAGACCCACG

Fig. 6 (cont.)

2501 CTCACCGGCT CCAGATTTAT CAGCAATAAA CCAGCCAGCC GGAAGGGCCG

2551 AGCGCAGAAG TGGTCCTGCA ACTTTATCCG CCTCCATCCA GTCTATTAAT

2601 TGTTGCCGGG AAGCTAGAGT AAGTAGTTCG CCAGTTAATA GTTTGCGCAA

2651 CGTTGTTGCC ATTGCTACAG GCATCGTGGT GTCACGCTCG TCGTTTGGTA

2701 TGGCTTCATT CAGCTCCGGT TCCCAACGAT CAAGGCGAGT TACATGATCC

2751 CCCATGTTGT GCAAAAAAGC GGTTAGCTCC TTCGGTCCTC CGATCGTTGT

2801 CAGAAGTAAG TTGGCCGCAG TGTTATCACT CATGGTTATG GCAGCACTGC

2851 ATAATTCTCT TACTGTCATG CCATCCGTAA GATGCTTTTC TGTGACTGGT

2901 GAGTACTCAA CCAAGTCATT CTGAGAATAG TGTATGCGGC GACCGAGTTG

2951 CTCTTGCCCG GCGTCAATAC GGGATAATAC CGCGCCACAT AGCAGAACTT

3001 TAAAAGTGCT CATCATTGGA AAACGTTCTT CGGGGCGAAA ACTCTCAAGG

3051 ATCTTACCGC TGTTGAGATC CAGTTCGATG TAACCCACTC GTGCACCCAA

3101 CTGATCTTCA GCATCTTTTA CTTTCACCAG CGTTTCTGGG TGAGCAAAAA

3151 CAGGAAGGCA AAATGCCGCA AAAAAGGGAA TAAGGGCGAC ACGGAAATGT

3201 TGAATACTCA TACTCTTCCT TTTTCAATAT TATTGAAGCA TTTATCAGGG

3251 TTATTGTCTC ATGAGCGGAT ACATATTTGA ATGTATTTAG AAAAATAAAC

3301 AAATAGGGGT TCCGCGCACA TTTCCCCGAA AAGTGCCACC TGACGTCTAA

3351 GAAACCATTA TTATCATGAC ATTAACCTAT AAAAATAGGC GTATCACGAG

3401 GCCCTTTCGT CTTCAC

Fig. 7

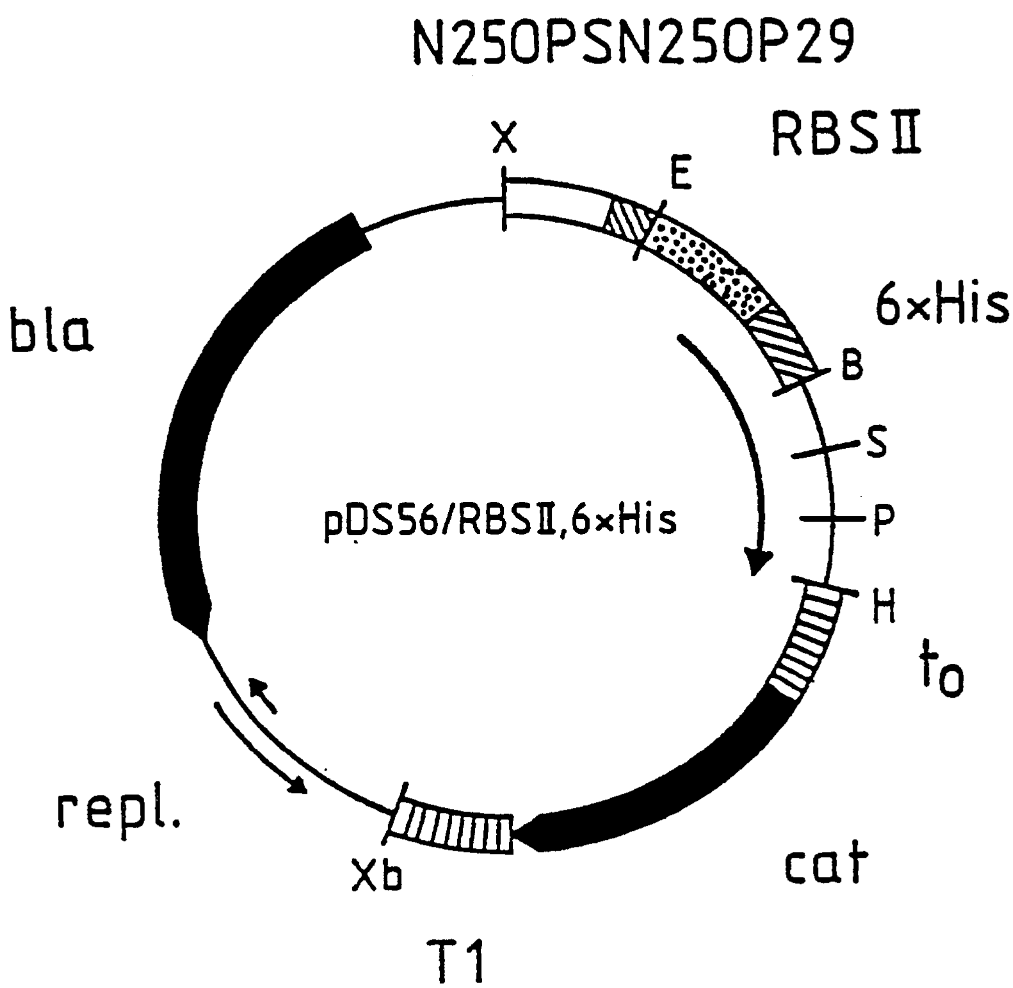
N250PSN250P29
X
E
RBSII
6xHis
B
S
P
H
$t_o$
bla
pDS56/RBSII,6xHis
repl.
Xb
cat
T1

## Fig. 8

```
      XhoI
   1  CTCGAGAAAT CATAAAAAAT TTATTTGCTT TGTGAGCGGA TAACAATTAT

                                               EcoRI
  51  AATAGATTCA ATTGTGAGCG GATAACAATT TCACACAGAA TTCATTAAAG

                                                   BamHI
 101  AGGAGAAATT AACTATGAGA GGATCGCATC ACCATCACCA TCACGGATCC
                     MetArg GlySerHisH isHisHisHi sHisGlySer

      SalI  PstI    HindIII
 151  GTCGACCTGC AGCCAAGCTT AATTAGCTGA GCTTGGACTC CTGTTGATAG
      ValAspLeuG lnProSerLe uIleSer

 201  ATCCAGTAAT GACCTCAGAA CTCCATCTGG ATTTGTTCAG AACGCTCGGT

 251  TGCCGCCGGG CGTTTTTTAT TGGTGAGAAT CCAAGCTAGC TTGGCGAGAT

 301  TTTCAGGAGC TAAGGAAGCT AAAATGGAGA AAAAAATCAC TGGATATACC

 351  ACCGTTGATA TATCCCAATG GCATCGTAAA GAACATTTTG AGGCATTTCA

 401  GTCAGTTGCT CAATGTACCT ATAACCAGAC CGTTCAGCTG GATATTACGG

 451  CCTTTTTAAA GACCGTAAAG AAAAATAAGC ACAAGTTTTA TCCGGCCTTT

 501  ATTCACATTC TTGCCCGCCT GATGAATGCT CATCCGGAAT TTCGTATGGC

 551  AATGAAAGAC GGTGAGCTGG TGATATGGGA TAGTGTTCAC CCTTGTTACA

 601  CCGTTTTCCA TGAGCAAACT GAAACGTTTT CATCGCTCTG GAGTGAATAC

 651  CACGACGATT TCCGGCAGTT TCTACACATA TATTCGCAAG ATGTGGCGTG

 701  TTACGGTGAA AACCTGGCCT ATTTCCCTAA AGGGTTTATT GAGAATATGT

 751  TTTTCGTCTC AGCCAATCCC TGGGTGAGTT TCACCAGTTT TGATTTAAAC

 801  GTGGCCAATA TGGACAACTT CTTCGCCCCC GTTTTCACCA TGGGCAAATA

 851  TTATACGCAA GGCGACAAGG TGCTGATGCC GCTGGCGATT CAGGTTCATC

 901  ATGCCGTCTG TGATGGCTTC CATGTCGGCA GAATGCTTAA TGAATTACAA

 951  CAGTACTGCG ATGAGTGGCA GGGCGGGGCG TAATTTTTTT AAGGCAGTTA

1001  TTGGTGCCCT TAAACGCCTG GGGTAATGAC TCTCTAGCTT GAGGCATCAA

1051  ATAAAACGAA AGGCTCAGTC GAAAGACTGG GCCTTTCGTT TTATCTGTTG
```

Fig. 8 (cont.)

XbaI

1101 TTTGTCGGTG AACGCTCTCC TGAGTAGGAC AAATCCGCCG CTCTAGAGCT

1151 GCCTCGCGCG TTTCGGTGAT GACGGTGAAA ACCTCTGACA CATGCAGCTC

1201 CCGGAGACGG TCACAGCTTG TCTGTAAGCG GATGCCGGGA GCAGACAAGC

1251 CCGTCAGGGC GCGTCAGCGG GTGTTGGCGG GTGTCGGGGC GCAGCCATGA

1301 CCCAGTCACG TAGCGATAGC GGAGTGTATA CTGGCTTAAC TATGCGGCAT

1351 CAGAGCAGAT TGTACTGAGA GTGCACCATA TGCGGTGTGA AATACCGCAC

1401 AGATGCGTAA GGAGAAAATA CCGCATCAGG CGCTCTTCCG CTTCCTCGCT

1451 CACTGACTCG CTGCGCTCGG TCTGTCGGCT GCGGCGAGCG GTATCAGCTC

1501 ACTCAAAGGC GGTAATACGG TTATCCACAG AATCAGGGGA TAACGCAGGA

1551 AAGAACATGT GAGCAAAAGG CCAGCAAAAG GCCAGGAACC GTAAAAAGGC

1601 CGCGTTGCTG GCGTTTTTCC ATAGGCTCCG CCCCCCTGAC GAGCATCACA

1651 AAAATCGACG CTCAAGTCAG AGGTGGCGAA ACCCGACAGG ACTATAAAGA

1701 TACCAGGCGT TTCCCCCTGG AAGCTCCCTC GTGCGCTCTC CTGTTCCGAC

1751 CCTGCCGCTT ACCGGATACC TGTCCGCCTT TCTCCCTTCG GGAAGCGTGG

1801 CGCTTTCTCA ATGCTCACGC TGTAGGTATC TCAGTTCGGT GTAGGTCGTT

1851 CGCTCCAAGC TGGGCTGTGT GCACGAACCC CCCGTTCAGC CCGACCGCTG

1901 CGCCTTATCC GGTAACTATC GTCTTGAGTC CAACCCGGTA AGACACGACT

1951 TATCGCCACT GGCAGCAGCC ACTGGTAACA GGATTAGCAG AGCGAGGTAT

2001 GTAGGCGGTG CTACAGAGTT CTTGAAGTGG TGGCCTAACT ACGGCTACAC

2051 TAGAAGGACA GTATTTGGTA TCTGCGCTCT GCTGAAGCCA GTTACCTTCG

2101 GAAAAAGAGT TGGTAGCTCT TGATCCGGCA AACAAACCAC CGCTGGTAGC

2151 GGTGGTTTTT TTGTTTGCAA GCAGCAGATT ACGCGCAGAA AAAAAGGATC

2201 TCAAGAAGAT CCTTTGATCT TTTCTACGGG GTCTGACGCT CAGTGGAACG

2251 AAAACTCACG TTAAGGGATT TTGGTCATGA GATTATCAAA AAGGATCTTC

Fig. 8 (cont.)

2301 ACCTAGATCC TTTTAAATTA AAAATGAAGT TTTAAATCAA TCTAAAGTAT
2351 ATATGAGTAA ACTTGGTCTG ACAGTTACCA ATGCTTAATC AGTGAGGCAC
2401 CTATCTCAGC GATCTGTCTA TTTCGTTCAT CCATAGCTGC CTGACTCCCC
2451 GTCGTGTAGA TAACTACGAT ACGGGAGGGC TTACCATCTG GCCCCAGTGC
2501 TGCAATGATA CCGCGAGACC CACGCTCACC GGCTCCAGAT TTATCAGCAA
2551 TAAACCAGCC AGCCGGAAGG GCCGAGCGCA GAAGTGGTCC TGCAACTTTA
2601 TCCGCCTCCA TCCAGTCTAT TAATTGTTGC CGGGAAGCTA GAGTAAGTAG
2651 TTCGCCAGTT AATAGTTTGC GCAACGTTGT TGCCATTGCT ACAGGCATCG
2701 TGGTGTCACG CTCGTCGTTT GGTATGGCTT CATTCAGCTC CGGTTCCCAA
2751 CGATCAAGGC GAGTTACATG ATCCCCCATG TTGTGCAAAA AAGCGGTTAG
2801 CTCCTTCGGT CCTCCGATCG TTGTCAGAAG TAAGTTGGCC GCAGTGTTAT
2851 CACTCATGGT TATGGCAGCA CTGCATAATT CTCTTACTGT CATGCCATCC
2901 GTAAGATGCT TTTCTGTGAC TGGTGAGTAC TCAACCAAGT CATTCTGAGA
2951 ATAGTGTATG CGGCGACCGA GTTGCTCTTG CCCGGCGTCA ATACGGGATA
3001 ATACCGCGCC ACATAGCAGA ACTTTAAAAG TGCTCATCAT TGGAAAACGT
3051 TCTTCGGGGC GAAAACTCTC AAGGATCTTA CCGCTGTTGA GATCCAGTTC
3101 GATGTAACCC ACTCGTGCAC CCAACTGATC TTCAGCATCT TTTACTTTCA
3151 CCAGCGTTTC TGGGTGAGCA AAAACAGGAA GGCAAAATGC CGCAAAAAAG
3201 GGAATAAGGG CGACACGGAA ATGTTGAATA CTCATACTCT TCCTTTTTCA
3251 ATATTATTGA AGCATTTATC AGGGTTATTG TCTCATGAGC GGATACATAT
3301 TTGAATGTAT TTAGAAAAAT AAACAAATAG GGGTTCCGCG CACATTTCCC
3351 CGAAAAGTGC CACCTGACGT CTAAGAAACC ATTATTATCA TGACATTAAC
3401 CTATAAAAAT AGGCGTATCA CGAGGCCCTT TCGTCTTCAC

Fig. 9

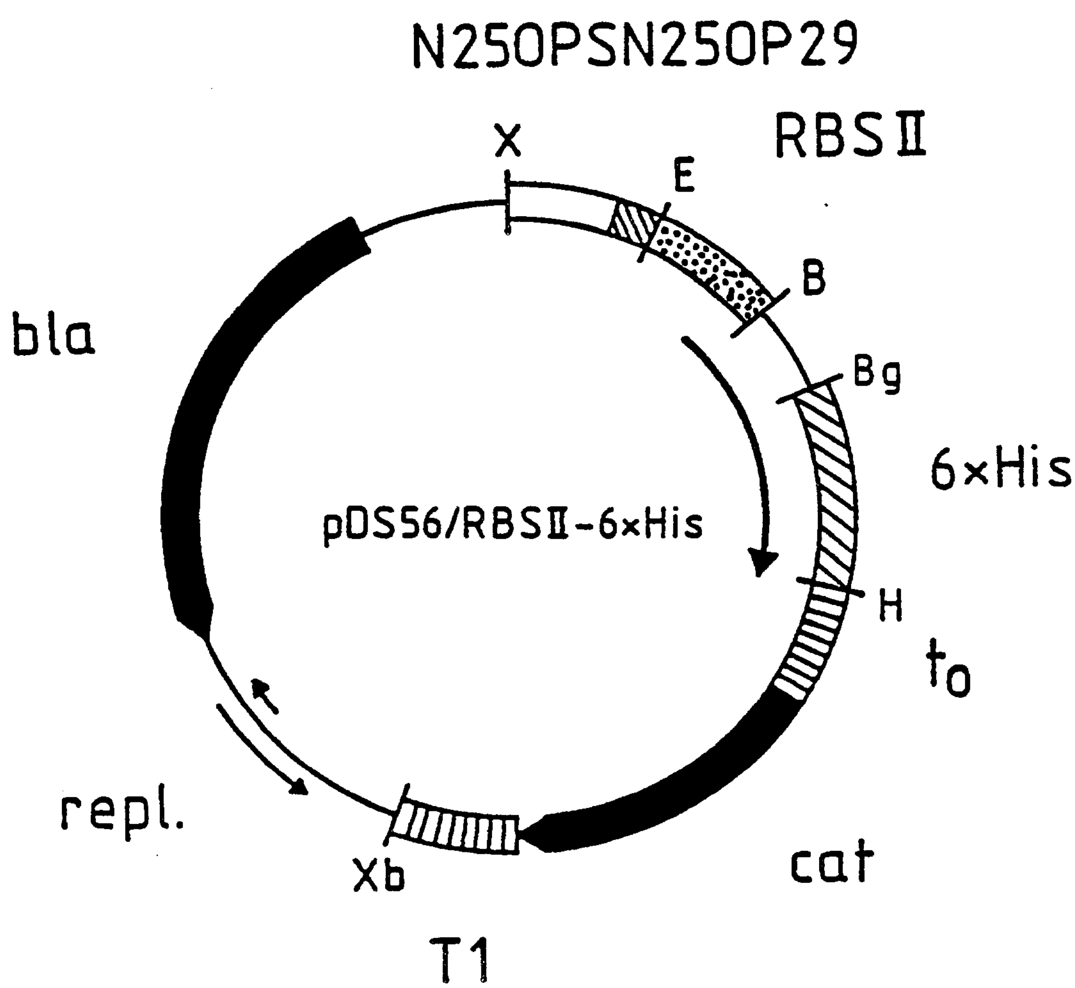
N25OPSN25OP29
X
E
RBSII
B
Bg
6×His
H
to
cat
T1
Xb
repl.
bla
pDS56/RBSII-6×His

Fig. 10

```
      XhoI
   1  CTCGAGAAAT CATAAAAAAT TTATTTGCTT TGTGAGCGGA TAACAATTAT

                                                 EcoRI
  51  AATAGATTCA ATTGTGAGCG GATAACAATT TCACACAGAA TTCATTAAAG

                            BamHI  BglII
 101  AGGAGAAATT AACTATGAGA GGATCCAGAT CTCATCACCA TCACCATCAC
                      MetArg GlySerArgS erHisHisHi sHisHisHis

      HindIII
 151  TAAGCTTAAT TAGCTGAGCT TGGACTCCTG TTGATAGATC CAGTAATGAC

 201  CTCAGAACTC CATCTGGATT TGTTCAGAAC GCTCGGTTGC CGCCGGGCGT

 251  TTTTTATTGG TGAGAATCCA AGCTAGCTTG GCGAGATTTT CAGGAGCTAA

 301  GGAAGCTAAA ATGGAGAAAA AAATCACTGG ATATACCACC GTTGATATAT

 351  CCCAATGGCA TCGTAAAGAA CATTTTGAGG CATTTCAGTC AGTTGCTCAA

 401  TGTACCTATA ACCAGACCGT TCAGCTGGAT ATTACGGCCT TTTTAAAGAC

 451  CGTAAAGAAA AATAAGCACA AGTTTTATCC GGCCTTTATT CACATTCTTG

 501  CCCGCCTGAT GAATGCTCAT CCGGAATTTC GTATGGCAAT GAAAGACGGT

 551  GAGCTGGTGA TATGGGATAG TGTTCACCCT TGTTACACCG TTTTCCATGA

 601  GCAAACTGAA ACGTTTTCAT CGCTCTGGAG TGAATACCAC GACGATTTCC

 651  GGCAGTTTCT ACACATATAT TCGCAAGATG TGGCGTGTTA CGGTGAAAAC

 701  CTGGCCTATT TCCCTAAAGG GTTTATTGAG AATATGTTTT TCGTCTCAGC

 751  CAATCCCTGG GTGAGTTTCA CCAGTTTTGA TTTAAACGTG GCCAATATGG

 801  ACAACTTCTT CGCCCCCGTT TTCACCATGG GCAAATATTA TACGCAAGGC

 851  GACAAGGTGC TGATGCCGCT GGCGATTCAG GTTCATCATG CCGTCTGTGA

 901  TGGCTTCCAT GTCGGCAGAA TGCTTAATGA ATTACAACAG TACTGCGATG

 951  AGTGGCAGGG CGGGGCGTAA TTTTTTTAAG GCAGTTATTG GTGCCCTTAA

1001  ACGCCTGGGG TAATGACTCT CTAGCTTGAG GCATCAAATA AAACGAAAGG

1051  CTCAGTCGAA AGACTGGGCC TTTCGTTTTA TCTGTTGTTT GTCGGTGAAC

                                        XbaI
1101  GCTCTCCTGA GTAGGACAAA TCCGCCGCTC TAGAGCTGCC TCGCGCGTTT
```

Fig. 10 (cont.)

1151 CGGTGATGAC GGTGAAAACC TCTGACACAT GCAGCTCCCG GAGACGGTCA

1201 CAGCTTGTCT GTAAGCGGAT GCCGGGAGCA GACAAGCCCG TCAGGGCGCG

1251 TCAGCGGGTG TTGGCGGGTG TCGGGGCGCA GCCATGACCC AGTCACGTAG

1301 CGATAGCGGA GTGTATACTG GCTTAACTAT GCGGCATCAG AGCAGATTGT

1351 ACTGAGAGTG CACCATATGC GGTGTGAAAT ACCGCACAGA TGCGTAAGGA

1401 GAAAATACCG CATCAGGCGC TCTTCCGCTT CCTCGCTCAC TGACTCGCTG

1451 CGCTCGGTCT GTCGGCTGCG GCGAGCGGTA TCAGCTCACT CAAAGGCGGT

1501 AATACGGTTA TCCACAGAAT CAGGGGATAA CGCAGGAAAG AACATGTGAG

1551 CAAAAGGCCA GCAAAAGGCC AGGAACCGTA AAAAGGCCGC GTTGCTGGCG

1601 TTTTTCCATA GGCTCCGCCC CCCTGACGAG CATCACAAAA ATCGACGCTC

1651 AAGTCAGAGG TGGCGAAACC CGACAGGACT ATAAAGATAC CAGGCGTTTC

1701 CCCCTGGAAG CTCCCTCGTG CGCTCTCCTG TTCCGACCCT GCCGCTTACC

1751 GGATACCTGT CCGCCTTTCT CCCTTCGGGA AGCGTGGCGC TTTCTCAATG

1801 CTCACGCTGT AGGTATCTCA GTTCGGTGTA GGTCGTTCGC TCCAAGCTGG

1851 GCTGTGTGCA CGAACCCCCC GTTCAGCCCG ACCGCTGCGC CTTATCCGGT

1901 AACTATCGTC TTGAGTCCAA CCCGGTAAGA CACGACTTAT CGCCACTGGC

1951 AGCAGCCACT GGTAACAGGA TTAGCAGAGC GAGGTATGTA GGCGGTGCTA

2001 CAGAGTTCTT GAAGTGGTGG CCTAACTACG GCTACACTAG AAGGACAGTA

2051 TTTGGTATCT GCGCTCTGCT GAAGCCAGTT ACCTTCGGAA AAAGAGTTGG

2101 TAGCTCTTGA TCCGGCAAAC AAACCACCGC TGGTAGCGGT GGTTTTTTTG

2151 TTTGCAAGCA GCAGATTACG CGCAGAAAAA AAGGATCTCA AGAAGATCCT

2201 TTGATCTTTT CTACGGGGTC TGACGCTCAG TGGAACGAAA ACTCACGTTA

2251 AGGGATTTTG GTCATGAGAT TATCAAAAAG GATCTTCACC TAGATCCTTT

2301 TAAATTAAAA ATGAAGTTTT AAATCAATCT AAAGTATATA TGAGTAAACT

2351 TGGTCTGACA GTTACCAATG CTTAATCAGT GAGGCACCTA TCTCAGCGAT

Fig. 10 (cont.)

2401 CTGTCTATTT CGTTCATCCA TAGCTGCCTG ACTCCCCGTC GTGTAGATAA
2451 CTACGATACG GGAGGGCTTA CCATCTGGCC CCAGTGCTGC AATGATACCG
2501 CGAGACCCAC GCTCACCGGC TCCAGATTTA TCAGCAATAA ACCAGCCAGC
2551 CGGAAGGGCC GAGCGCAGAA GTGGTCCTGC AACTTTATCC GCCTCCATCC
2601 AGTCTATTAA TTGTTGCCGG GAAGCTAGAG TAAGTAGTTC GCCAGTTAAT
2651 AGTTTGCGCA ACGTTGTTGC CATTGCTACA GGCATCGTGG TGTCACGCTC
2701 GTCGTTTGGT ATGGCTTCAT TCAGCTCCGG TTCCCAACGA TCAAGGCGAG
2751 TTACATGATC CCCCATGTTG TGCAAAAAAG CGGTTAGCTC CTTCGGTCCT
2801 CCGATCGTTG TCAGAAGTAA GTTGGCCGCA GTGTTATCAC TCATGGTTAT
2851 GGCAGCACTG CATAATTCTC TTACTGTCAT GCCATCCGTA AGATGCTTTT
2901 CTGTGACTGG TGAGTACTCA ACCAAGTCAT TCTGAGAATA GTGTATGCGG
2951 CGACCGAGTT GCTCTTGCCC GGCGTCAATA CGGGATAATA CCGCGCCACA
3001 TAGCAGAACT TTAAAAGTGC TCATCATTGG AAAACGTTCT TCGGGGCGAA
3051 AACTCTCAAG GATCTTACCG CTGTTGAGAT CCAGTTCGAT GTAACCCACT
3101 CGTGCACCCA ACTGATCTTC AGCATCTTTT ACTTTCACCA GCGTTTCTGG
3151 GTGAGCAAAA ACAGGAAGGC AAAATGCCGC AAAAAAGGGA ATAAGGGCGA
3201 CACGGAAATG TTGAATACTC ATACTCTTCC TTTTTCAATA TTATTGAAGC
3251 ATTTATCAGG GTTATTGTCT CATGAGCGGA TACATATTTG AATGTATTTA
3301 GAAAAATAAA CAAATAGGGG TTCCGCGCAC ATTTCCCCGA AAAGTGCCAC
3351 CTGACGTCTA AGAAACCATT ATTATCATGA CATTAACCTA TAAAAATAGG
3401 CGTATCACGA GGCCCTTTCG TCTTCAC

Fig. 11

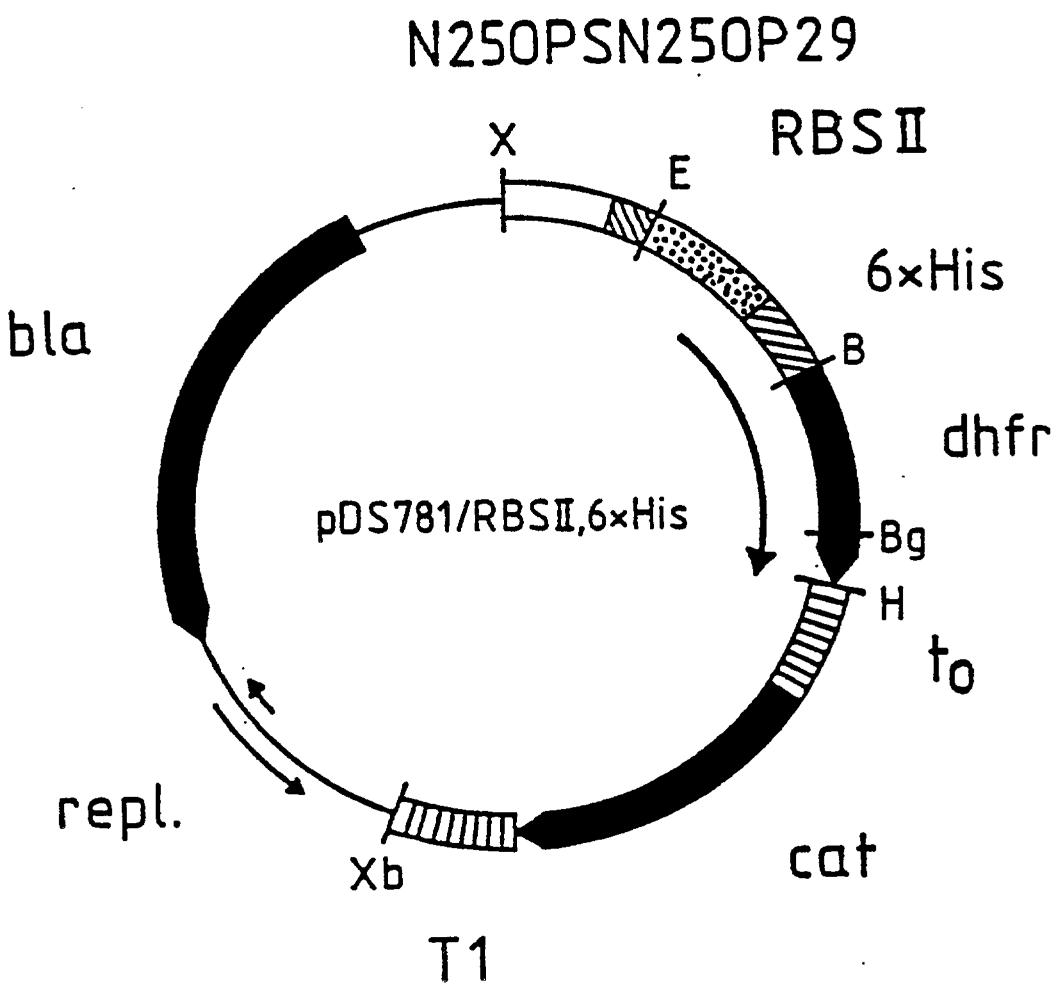
N25OPSN25OP29
X
E
RBSII
6×His
B
dhfr
Bg
H
to
cat
T1
Xb
repl.
bla
pDS781/RBSII,6×His

## Fig. 12

```
     XhoI
  1  CTCGAGAAATCATAAAAAATTTATTTGCTTTGTGAGCGGATAACAATTATAATAGATTCA

                                             EcoRI
 61  ATTGTGAGCGGATAACAATTTCACACAGAATTCATTAAAGAGGAGAAATTAACTATGAGA
                                                           MetArg

                                       BamHI
121  GGATCGCATCACCATCACCATCACGGATCCGGCATCATGGTTCGACCATTGAACTGCATC
     GlySerHisHisHisHisHisHisGlySerGlyIleMetValArgProLeuAsnCysIle

181  GTCGCCGTGTCCCAAAATATGGGATTGGCAAAGAACGGAGACCTACCCTGGCCTCCGCTC
     ValAlaValSerGlnAsnMetGlyIleGlyLysAsnGlyAspLeuProTrpProProLeu

241  AGGAACGAGTTCAAGTACTTCCAAAGAATGACCACAACCTCTTCAGTGGAAGGTAAACAG
     ArgAsnGluPheLysTyrPheGlnArgMetThrThrThrSerSerValGluGlyLysGln

301  AATCTGGTGATTATGGGTAGGAAAACCTGGTTCTCCATTCCTGAGAAGAATCGACCTTTA
     AsnLeuValIleMetGlyArgLysThrTrpPheSerIleProGluLysAsnArgProLeu

361  AAGGACAGAATTAATATAGTTCTCAGTAGAGAACTCAAAGAACCACCACGAGGAGCTCAT
     LysAspArgIleAsnIleValLeuSerArgGluLeuLysGluProProArgGlyAlaHis

421  TTTCTTGCCAAAAGTTTGGATGATGCCTTAAGACTTATTGAACAACCGGAATTGGCAAGT
     PheLeuAlaLysSerLeuAspAspAlaLeuArgLeuIleGluGlnProGluLeuAlaSer

481  AAAGTAGACATGGTTTGGATAGTCGGAGGCAGTTCTGTTTACCAGGAAGCCATGAATCAA
     LysValAspMetValTrpIleValGlyGlySerSerValTyrGlnGluAlaMetAsnGln

541  CCAGGCCACCTTAGACTCTTTGTGACAAGGATCATGCAGGAATTTGAAAGTGACACGTTT
     ProGlyHisLeuArgLeuPheValThrArgIleMetGlnGluPheGluSerAspThrPhe

601  TTCCCAGAAATTGATTTGGGGAAATATAAACTTCTCCCAGAATACCCAGGCGTCCTCTCT
     PheProGluIleAspLeuGlyLysTyrLysLeuLeuProGluTyrProGlyValLeuSer

661  GAGGTCCAGGAGGAAAAAGGCATCAAGTATAAGTTTGAAGTCTACGAGAAGAAAGGTTCC
     GluValGlnGluGluLysGlyIleLysTyrLysPheGluValTyrGluLysLysGlySer

     BglII  HindIII
721  AGATCTTAAGCTTAATTAGCTGAGCTTGGACTCCTGTTGATAGATCCAGTAATGACCTCA
     ArgSer

781  GAACTCCATCTGGATTTGTTCAGAACGCTCGGTTGCCGCCGGGCGTTTTTTATTGGTGAG

841  AATCCAAGCTAGCTTGGCGAGATTTTCAGGAGCTAAGGAAGCTAAAATGGAGAAAAAAAT

901  CACTGGATATACCACCGTTGATATATCCCAATGGCATCGTAAAGAACATTTTGAGGCATT

961  TCAGTCAGTTGCTCAATGTACCTATAACCAGACCGTTCAGCTGGATATTACGGCCTTTTT
```

Fig. 12 (cont.)

1021 AAAGACCGTAAAGAAAAATAAGCACAAGTTTTATCCGGCCTTTATTCACATTCTTGCCCG

1081 CCTGATGAATGCTCATCCGGAATTTCGTATGGCAATGAAAGACGGTGAGCTGGTGATATG

1141 GGATAGTGTTCACCCTTGTTACACCGTTTTCCATGAGCAAACTGAAACGTTTTCATCGCT

1201 CTGGAGTGAATACCACGACGATTTCCGGCAGTTTCTACACATATATTCGCAAGATGTGGC

1261 GTGTTACGGTGAAAACCTGGCCTATTTCCCTAAAGGGTTTATTGAGAATATGTTTTTCGT

1321 CTCAGCCAATCCCTGGGTGAGTTTCACCAGTTTTGATTTAAACGTGGCCAATATGGACAA

1381 CTTCTTCGCCCCCGTTTTCACCATGGGCAAATATTATACGCAAGGCGACAAGGTGCTGAT

1441 GCCGCTGGCGATTCAGGTTCATCATGCCGTCTGTGATGGCTTCCATGTCGGCAGAATGCT

1501 TAATGAATTACAACAGTACTGCGATGAGTGGCAGGGCGGGGCGTAATTTTTTTAAGGCAG

1561 TTATTGGTGCCCTTAAACGCCTGGGGTAATGACTCTCTAGCTTGAGGCATCAAATAAAAC

1621 GAAAGGCTCAGTCGAAAGACTGGGCCTTTCGTTTTATCTGTTGTTTGTCGGTGAACGCTC

XbaI

1681 TCCTGAGTAGGACAAATCCGCCGCTCTAGAGCTGCCTCGCGCGTTTCGGTGATGACGGTG

1741 AAAACCTCTGACACATGCAGCTCCCGGAGACGGTCACAGCTTGTCTGTAAGCGGATGCCG

1801 GGAGCAGACAAGCCCGTCAGGGCGCGTCAGCGGGTGTTGGCGGGTGTCGGGGCGCAGCCA

1861 TGACCCAGTCACGTAGCGATAGCGGAGTGTATACTGGCTTAACTATGCGGCATCAGAGCA

1921 GATTGTACTGAGAGTGCACCATATGCGGTGTGAAATACCGCACAGATGCGTAAGGAGAAA

1981 ATACCGCATCAGGCGCTCTTCCGCTTCCTCGCTCACTGACTCGCTGCGCTCGGTCTGTCG

2041 GCTGCGGCGAGCGGTATCAGCTCACTCAAAGGCGGTAATACGGTTATCCACAGAATCAGG

2101 GGATAACGCAGGAAAGAACATGTGAGCAAAAGGCCAGCAAAAGGCCAGGAACCGTAAAAA

2161 GGCCGCGTTGCTGGCGTTTTTCCATAGGCTCCGCCCCCCTGACGAGCATCACAAAAATCG

2221 ACGCTCAAGTCAGAGGTGGCGAAACCCGACAGGACTATAAAGATACCAGGCGTTTCCCCC

2281 TGGAAGCTCCCTCGTGCGCTCTCCTGTTCCGACCCTGCCGCTTACCGGATACCTGTCCGC

2341 CTTTCTCCCTTCGGGAAGCGTGGCGCTTTCTCAATGCTCACGCTGTAGGTATCTCAGTTC

2401 GGTGTAGGTCGTTCGCTCCAAGCTGGGCTGTGTGCACGAACCCCCCGTTCAGCCCGACCG

Fig. 12 (cont.)

2461 CTGCGCCTTATCCGGTAACTATCGTCTTGAGTCCAACCCGGTAAGACACGACTTATCGCC

2521 ACTGGCAGCAGCCACTGGTAACAGGATTAGCAGAGCGAGGTATGTAGGCGGTGCTACAGA

2581 GTTCTTGAAGTGGTGGCCTAACTACGGCTACACTAGAAGGACAGTATTTGGTATCTGCGC

2641 TCTGCTGAAGCCAGTTACCTTCGGAAAAAGAGTTGGTAGCTCTTGATCCGGCAAACAAAC

2701 CACCGCTGGTAGCGGTGGTTTTTTTGTTTGCAAGCAGCAGATTACGCGCAGAAAAAAAGG

2761 ATCTCAAGAAGATCCTTTGATCTTTTCTACGGGGTCTGACGCTCAGTGGAACGAAAACTC

2821 ACGTTAAGGGATTTTGGTCATGAGATTATCAAAAAGGATCTTCACCTAGATCCTTTTAAA

2881 TTAAAAATGAAGTTTTAAATCAATCTAAAGTATATATGAGTAAACTTGGTCTGACAGTTA

2941 CCAATGCTTAATCAGTGAGGCACCTATCTCAGCGATCTGTCTATTTCGTTCATCCATAGC

3001 TGCCTGACTCCCCGTCGTGTAGATAACTACGATACGGGAGGGCTTACCATCTGGCCCCAG

3061 TGCTGCAATGATACCGCGAGACCCACGCTCACCGGCTCCAGATTTATCAGCAATAAACCA

3121 GCCAGCCGGAAGGGCCGAGCGCAGAAGTGGTCCTGCAACTTTATCCGCCTCCATCCAGTC

3181 TATTAATTGTTGCCGGGAAGCTAGAGTAAGTAGTTCGCCAGTTAATAGTTTGCGCAACGT

3241 TGTTGCCATTGCTACAGGCATCGTGGTGTCACGCTCGTCGTTTGGTATGGCTTCATTCAG

3301 CTCCGGTTCCCAACGATCAAGGCGAGTTACATGATCCCCCATGTTGTGCAAAAAAGCGGT

3361 TAGCTCCTTCGGTCCTCCGATCGTTGTCAGAAGTAAGTTGGCCGCAGTGTTATCACTCAT

3421 GGTTATGGCAGCACTGCATAATTCTCTTACTGTCATGCCATCCGTAAGATGCTTTTCTGT

3481 GACTGGTGAGTACTCAACCAAGTCATTCTGAGAATAGTGTATGCGGCGACCGAGTTGCTC

3541 TTGCCCGGCGTCAATACGGGATAATACCGCGCCACATAGCAGAACTTTAAAAGTGCTCAT

3601 CATTGGAAAACGTTCTTCGGGGCGAAAACTCTCAAGGATCTTACCGCTGTTGAGATCCAG

3661 TTCGATGTAACCCACTCGTGCACCCAACTGATCTTCAGCATCTTTTACTTTCACCAGCGT

3721 TTCTGGGTGAGCAAAAACAGGAAGGCAAAATGCCGCAAAAAAGGGAATAAGGGCGACACG

3781 GAAATGTTGAATACTCATACTCTTCCTTTTTCAATATTATTGAAGCATTTATCAGGGTTA

3841 TTGTCTCATGAGCGGATACATATTTGAATGTATTTAGAAAAATAAACAAATAGGGGTTCC

3901 GCGCACATTTCCCCGAAAAGTGCCACCTGACGTCTAAGAAACCATTATTATCATGACATT

3961 AACCTATAAAAATAGGCGTATCACGAGGCCCTTTCGTCTTCAC

Fig. 13

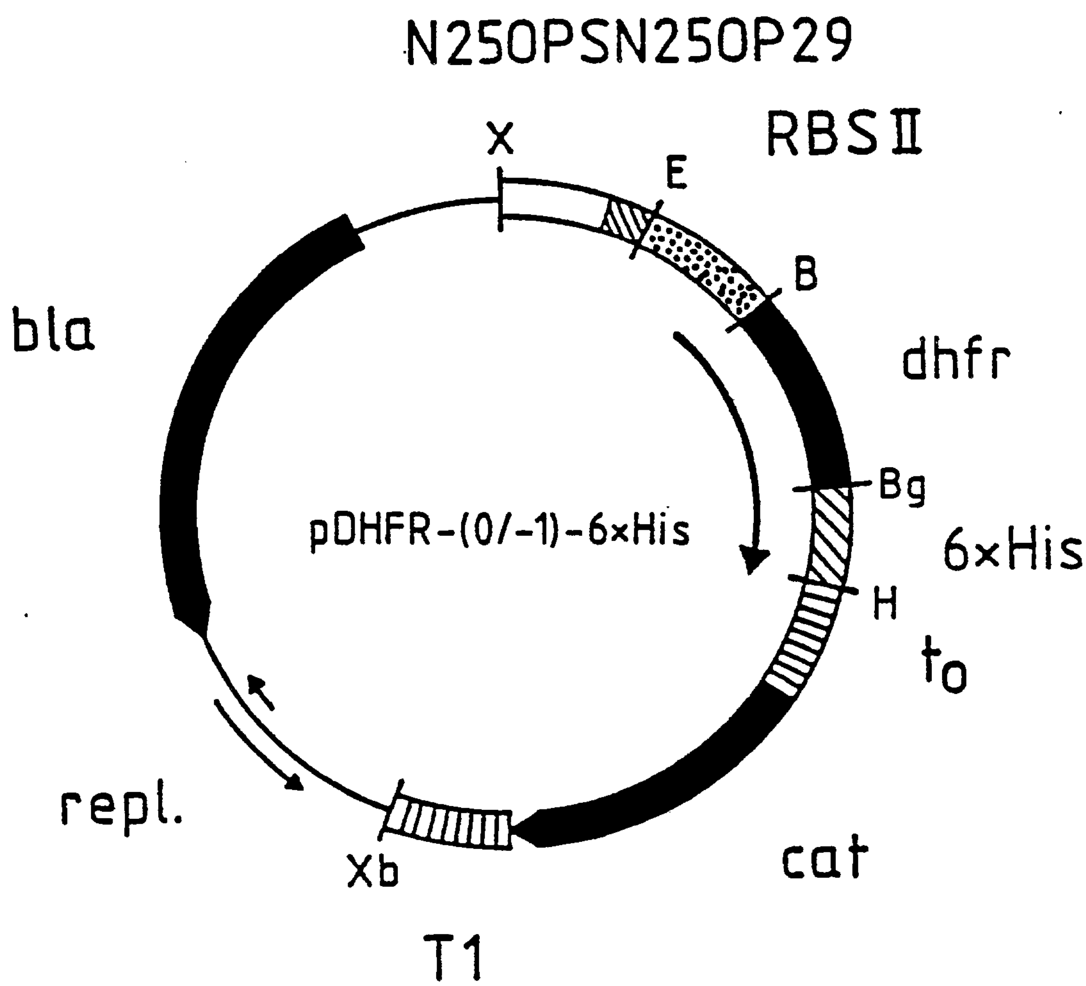
N25OPSN25OP29
X
E
RBSII
B
bla
dhfr
Bg
pDHFR-(0/-1)-6xHis
6xHis
H
to
repl.
Xb
cat
T1

Fig. 14

```
   XhoI
1  CTCGAGAAATCATAAAAAATTTATTTGCTTTGTGAGCGGATAACAATTATAATAGATTCA

                                  EcoRI
61  ATTGTGAGCGGATAACAATTTCACACAGAATTCATTAAAGAGGAGAAATTAACTATGAGA
                                                          MetArg

    BamHI
121 GGATCCGGCATCATGGTTCGACCATTGAACTGCATCGTCGCCGTGTCCCAAAATATGGGG
    GlySerGlyIleMetValArgProLeuAsnCysIleValAlaValSerGlnAsnMetGly

181 ATTGGCAAGAACGGAGACCTACCCTGGCCTCCGCTCAGGAACGAGTTCAAGTACTTCCAA
    IleGlyLysAsnGlyAspLeuProTrpProProLeuArgAsnGluPheLysTyrPheGln

241 AGAATGACCACAACCTCTTCAGTGGAAGGTAAACAGAATCTGGTGATTATGGGTAGGAAA
    ArgMetThrThrThrSerSerValGluGlyLysGlnAsnLeuValIleMetGlyArgLys

301 ACCTGGTTCTCCATTCCTGAGAAGAATCGACCTTTAAAGGACAGAATTAATATAGTTCTC
    ThrTrpPheSerIleProGluLysAsnArgProLeuLysAspArgIleAsnIleValLeu

361 AGTAGAGAACTCAAAGAACCACCACGAGGAGCTCATTTTCTTGCCAAAAGTTTGGATGAT
    SerArgGluLeuLysGluProProArgGlyAlaHisPheLeuAlaLysSerLeuAspAsp

421 GCCTTAAGACTTATTGAACAACCGGAATTGGCAAGTAAAGTAGACATGGTTTGGATAGTC
    AlaLeuArgLeuIleGluGlnProGluLeuAlaSerLysValAspMetValTrpIleVal

481 GGAGGCAGTTCTGTTTACCAGGAAGCCATGAATCAACCAGGCCACCTTAGACTCTTTGTG
    GlyGlySerSerValTyrGlnGluAlaMetAsnGlnProGlyHisLeuArgLeuPheVal

541 ACAAGGATCATGCAGGAATTTGAAAGTGACACGTTTTTCCCAGAAATTGATTTGGGGAAA
    ThrArgIleMetGlnGluPheGluSerAspThrPhePheProGluIleAspLeuGlyLys

601 TATAAACTTCTCCCAGAATACCCAGGCGTCCTCTCTGAGGTCCAGGAGGAAAAAGGCATC
    TyrLysLeuLeuProGluTyrProGlyValLeuSerGluValGlnGluGluLysGlyIle

                                          BglII
661 AAGTATAAGTTTGAAGTCTACGAGAAGAAAGGTTCCAGATCTTCGATCTCATCACCATCA
    LysTyrLysPheGluValTyrGluLysLysGlySerArgSerSerIleSerSerProSer

            HindIII
721 CCATCACTAAGCTTAATTAGCTGAGCTTGGACTCCTGTTGATAGATCCAGTAATGACCTC
    ProSerLeuSerLeuIleSer

781 AGAACTCCATCTGGATTTGTTCAGAACGCTCGGTTGCCGCCGGGCGTTTTTTATTGGTGA

841 GAATCCAAGCTAGCTTGGCGAGATTTTCAGGAGCTAAGGAAGCTAAAATGGAGAAAAAAA

901 TCACTGGATATACCACCGTTGATATATCCCAATGGCATCGTAAAGAACATTTTGAGGCAT

961 TTCAGTCAGTTGCTCAATGTACCTATAACCAGACCGTTCAGCTGGATATTACGGCCTTTT
```

**Fig. 14 (cont.)**

1021 TAAAGACCGTAAAGAAAAATAAGCACAAGTTTTATCCGGCCTTTATTCACATTCTTGCCC

1081 GCCTGATGAATGCTCATCCGGAATTTCGTATGGCAATGAAAGACGGTGAGCTGGTGATAT

1141 GGGATAGTGTTCACCCTTGTTACACCGTTTTCCATGAGCAAACTGAAACGTTTTCATCGC

1201 TCTGGAGTGAATACCACGACGATTTCCGGCAGTTTCTACACATATATTCGCAAGATGTGG

1261 CGTGTTACGGTGAAAACCTGGCCTATTTCCCTAAAGGGTTTATTGAGAATATGTTTTTCG

1321 TCTCAGCCAATCCCTGGGTGAGTTTCACCAGTTTTGATTTAAACGTGGCCAATATGGACA

1381 ACTTCTTCGCCCCCGTTTTCACCATGGGCAAATATTATACGCAAGGCGACAAGGTGCTGA

1441 TGCCGCTGGCGATTCAGGTTCATCATGCCGTCTGTGATGGCTTCCATGTCGGCAGAATGC

1501 TTAATGAATTACAACAGTACTGCGATGAGTGGCAGGGCGGGGCGTAATTTTTTTAAGGCA

1561 GTTATTGGTGCCCTTAAACGCCTGGGGTAATGACTCTCTAGCTTGAGGCATCAAATAAAA

1621 CGAAAGGCTCAGTCGAAAGACTGGGCCTTTCGTTTTATCTGTTGTTTGTCGGTGAACGCT

XbaI

1681 CTCCTGAGTAGGACAAATCCGCCGCTCTAGAGCTGCCTCGCGCGTTTCGGTGATGACGGT

1741 GAAAACCTCTGACACATGCAGCTCCCGGAGACGGTCACAGCTTGTCTGTAAGCGGATGCC

1801 GGGAGCAGACAAGCCCGTCAGGGCGCGTCAGCGGGTGTTGGCGGGTGTCGGGGCGCAGCC

1861 ATGACCCAGTCACGTAGCGATAGCGGAGTGTATACTGGCTTAACTATGCGGCATCAGAGC

1921 AGATTGTACTGAGAGTGCACCATATGCGGTGTGAAATACCGCACAGATGCGTAAGGAGAA

1981 AATACCGCATCAGGCGCTCTTCCGCTTCCTCGCTCACTGACTCGCTGCGCTCGGTCTGTC

2041 GGCTGCGGCGAGCGGTATCAGCTCACTCAAAGGCGGTAATACGGTTATCCACAGAATCAG

2101 GGGATAACGCAGGAAAGAACATGTGAGCAAAAGGCCAGCAAAAGGCCAGGAACCGTAAAA

2161 AGGCCGCGTTGCTGGCGTTTTTCCATAGGCTCCGCCCCCCTGACGAGCATCACAAAAATC

2221 GACGCTCAAGTCAGAGGTGGCGAAACCCGACAGGACTATAAAGATACCAGGCGTTTCCCC

2281 CTGGAAGCTCCCTCGTGCGCTCTCCTGTTCCGACCCTGCCGCTTACCGGATACCTGTCCG

2341 CCTTTCTCCCTTCGGGAAGCGTGGCGCTTTCTCAATGCTCACGCTGTAGGTATCTCAGTT

2401 CGGTGTAGGTCGTTCGCTCCAAGCTGGGCTGTGTGCACGAACCCCCCGTTCAGCCCGACC

Fig. 14 (cont.)

2461 GCTGCGCCTTATCCGGTAACTATCGTCTTGAGTCCAACCCGGTAAGACACGACTTATCGC

2521 CACTGGCAGCAGCCACTGGTAACAGGATTAGCAGAGCGAGGTATGTAGGCGGTGCTACAG

2581 AGTTCTTGAAGTGGTGGCCTAACTACGGCTACACTAGAAGGACAGTATTTGGTATCTGCG

2641 CTCTGCTGAAGCCAGTTACCTTCGGAAAAAGAGTTGGTAGCTCTTGATCCGGCAAACAAA

2701 CCACCGCTGGTAGCGGTGGTTTTTTTGTTTGCAAGCAGCAGATTACGCGCAGAAAAAAAG

2761 GATCTCAAGAAGATCCTTTGATCTTTTCTACGGGGTCTGACGCTCAGTGGAACGAAAACT

2821 CACGTTAAGGGATTTTGGTCATGAGATTATCAAAAAGGATCTTCACCTAGATCCTTTTAA

2881 ATTAAAAATGAAGTTTTAAATCAATCTAAAGTATATATGAGTAAACTTGGTCTGACAGTT

2941 ACCAATGCTTAATCAGTGAGGCACCTATCTCAGCGATCTGTCTATTTCGTTCATCCATAG

3001 CTGCCTGACTCCCCGTCGTGTAGATAACTACGATACGGGAGGGCTTACCATCTGGCCCCA

3061 GTGCTGCAATGATACCGCGAGACCCACGCTCACCGGCTCCAGATTTATCAGCAATAAACC

3121 AGCCAGCCGGAAGGGCCGAGCGCAGAAGTGGTCCTGCAACTTTATCCGCCTCCATCCAGT

3181 CTATTAATTGTTGCCGGGAAGCTAGAGTAAGTAGTTCGCCAGTTAATAGTTTGCGCAACG

3241 TTGTTGCCATTGCTACAGGCATCGTGGTGTCACGCTCGTCGTTTGGTATGGCTTCATTCA

3301 GCTCCGGTTCCCAACGATCAAGGCGAGTTACATGATCCCCCATGTTGTGCAAAAAAGCGG

3361 TTAGCTCCTTCGGTCCTCCGATCGTTGTCAGAAGTAAGTTGGCCGCAGTGTTATCACTCA

3421 TGGTTATGGCAGCACTGCATAATTCTCTTACTGTCATGCCATCCGTAAGATGCTTTTCTG

3481 TGACTGGTGAGTACTCAACCAAGTCATTCTGAGAATAGTGTATGCGGCGACCGAGTTGCT

3541 CTTGCCCGGCGTCAATACGGGATAATACCGCGCCACATAGCAGAACTTTAAAAGTGCTCA

3601 TCATTGGAAAACGTTCTTCGGGGCGAAAACTCTCAAGGATCTTACCGCTGTTGAGATCCA

3661 GTTCGATGTAACCCACTCGTGCACCCAACTGATCTTCAGCATCTTTTACTTTCACCAGCG

3721 TTTCTGGGTGAGCAAAAACAGGAAGGCAAAATGCCGCAAAAAAGGGAATAAGGGCGACAC

3781 GGAAATGTTGAATACTCATACTCTTCCTTTTTCAATATTATTGAAGCATTTATCAGGGTT

3841 ATTGTCTCATGAGCGGATACATATTTGAATGTATTTAGAAAAATAAACAAATAGGGGTTC

3901 CGCGCACATTTCCCCGAAAAGTGCCACCTGACGTCTAAGAAACCATTATTATCATGACAT

3961 TAACCTATAAAAATAGGCGTATCACGAGGCCCTTTCGTCTTCAC

Fig. 15

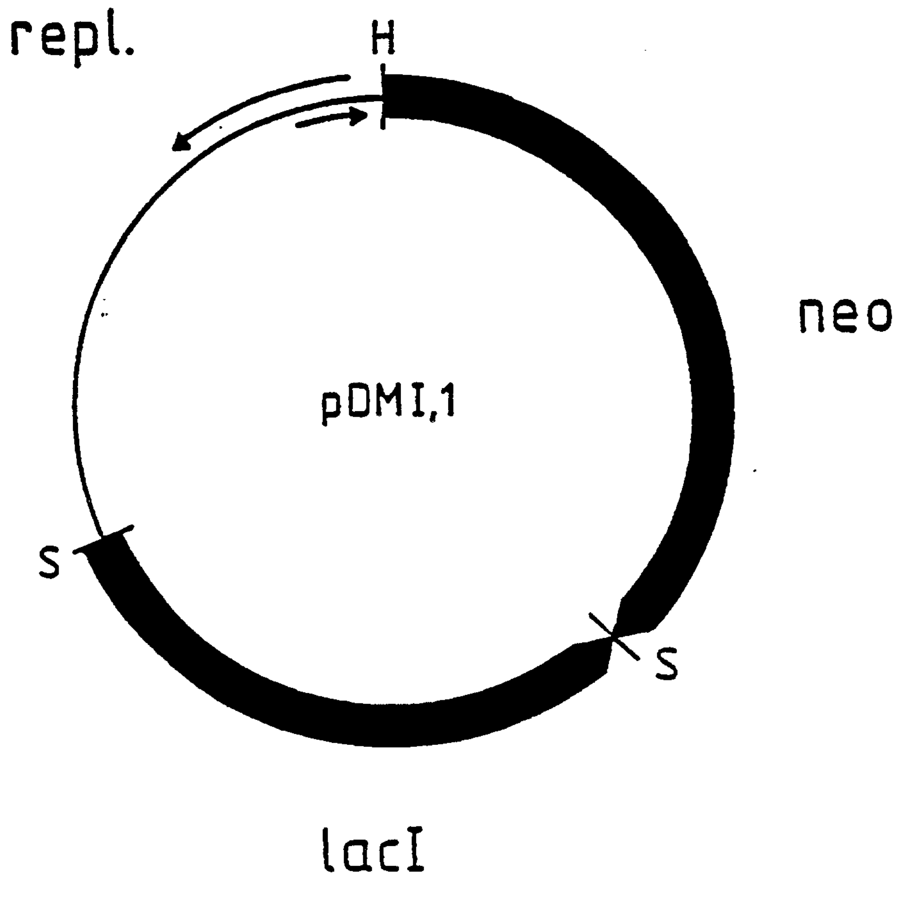

Fig. 16

```
        HindIII
   1    AAGCTTCACG CTGCCGCAAG CACTCAGGGC GCAAGGGCTG CTAAAGGAAG

  51    CGGAACACGT AGAAAGCCAG TCCGCAGAAA CGGTGCTGAC CCCGGATGAA

 101    TGTCAGCTAC TGGGCTATCT GGACAAGGGA AAACGCAAGC GCAAAGAGAA

 151    AGCAGGTAGC TTGCAGTGGG CTTACATGGC GATAGCTAGA CTGGGCGGTT

 201    TTATGGACAG CAAGCGAACC GGAATTGCCA GCTGGGGCGC CCTCTGGTAA

 251    GGTTGGGAAG CCCTGCAAAG TAAACTGGAT GGCTTTCTTG CCGCCAAGGA

 301    TCTGATGGCG CAGGGGATCA AGATCTGATC AAGAGACAGG ATGAGGATCG

 351    TTTCGCATGA TTGAACAAGA TGGATTGCAC GCAGGTTCTC CGGCCGCTTG
                Met

 401    GGTGGAGAGG CTATTCGGCT ATGACTGGGC ACAACAGACA ATCGGCTGCT

 451    CTGATGCCGC CGTGTTCCGG CTGTCAGCGC AGGGGCGCCC GGTTCTTTTT

 501    GTCAAGACCG ACCTGTCCGG TGCCCTGAAT GAACTGCAGG ACGAGGCAGC

 551    GCGGCTATCG TGGCTGGCCA CGACGGGCGT TCCTTGCGCA GCTGTGCTCG

 601    ACGTTGTCAC TGAAGCGGGA AGGGACTGGC TGCTATTGGG CGAAGTGCCG

 651    GGGCAGGATC TCCTGTCATC TCACCTTGCT CCTGCCGAGA AAGTATCCAT

 701    CATGGCTGAT GCAATGCGGC GGCTGCATAC GCTTGATCCG GCTACCTGCC

 751    CATTCGACCA CCAAGCGAAA CATCGCATCG AGCGAGCACG TACTCGGATG

 801    GAAGCCGGTC TTGTCGATCA GGATGATCTG GACGAAGAGC ATCAGGGGCT

 851    CGCGCCAGCC GAACTGTTCG CCAGGCTCAA GGCGCGCATG CCCGACGGCG

 901    AGGATCTCGT CGTGACCCAT GGCGATGCCT GCTTGCCGAA TATCATGGTG

 951    GAAAATGGCC GCTTTTCTGG ATTCATCGAC TGTGGCCGGC TGGGTGTGGC

1001    GGACCGCTAT CAGGACATAG CGTTGGCTAC CCGTGATATT GCTGAAGAGC

1051    TTGGCGGCGA ATGGGCTGAC CGCTTCCTCG TGCTTTACGG TATCGCCGCT

1101    CCCGATTCGC AGCGCATCGC CTTCTATCGC CTTCTTGACG AGTTCTTCTG
                                                      Phe

1151    AGCGGGACTC TGGGGTTCGA AATGACCGAC CAAGCGACGC CCAACCTGCC
```

Fig. 16 (cont.)

1201 ATCACGAGAT TTCGATTCCA CCGCCGCCTT CTATGAAAGG TTGGGCTTCG

1251 GAATCGTTTT CCGGGACGCC GGCTGGATGA TCCTCCAGCG CGGGGATCTC

1301 ATGCTGGAGT TCTTCGCCCA CCCCGGGCTC GATCCCCTCG CGAGTTGGTT

1351 CAGCTGCTGC CTGAGGCTGG ACGACCTCGC GGAGTTCTAC CGGCAGTGCA

1401 AATCCGTCGG CATCCAGGAA ACCAGCAGCG GCTATCCGCG CATCCATGCC

SalI

1451 CCCGAACTGC AGGAGTGGGG AGGCACGATG GCCGCTTTGG TCGACAATTC

1501 GCGCTAACTT ACATTAATTG CGTTGCGCTC ACTGCCCGCT TTCCAGTCGG

(Gln)

1551 GAAACCTGTC GTGCCAGCTG CATTAATGAA TCGGCCAACG CGCGGGGAGA

1601 GGCGGTTTGC GTATTGGGCG CCAGGGTGGT TTTTCTTTTC ACCAGTGAGA

1651 CGGGCAACAG CTGATTGCCC TTCACCGCCT GGCCCTGAGA GAGTTGCAGC

1701 AAGCGGTCCA CGCTGGTTTG CCCCAGCAGG CGAAAATCCT GTTTGATGGT

1751 GGTTAACGGC GGGATATAAC ATGAGCTGTC TTCGGTATCG TCGTATCCCA

1801 CTACCGAGAT ATCCGCACCA ACGCGCAGCC CGGACTCGGT AATGGCGCGC

1851 ATTGCGCCCA GCGCCATCTG ATCGTTGGCA ACCAGCATCG CAGTGGGAAC

1901 GATGCCCTCA TTCAGCATTT GCATGGTTTG TTGAAAACCG GACATGGCAC

1951 TCCAGTCGCC TTCCCGTTCC GCTATCGGCT GAATTTGATT GCGAGTGAGA

2001 TATTTATGCC AGCCAGCCAG ACGCAGACGC GCCGAGACAG AACTTAATGG

2051 GCCCGCTAAC AGCGCGATTT GCTGGTGACC CAATGCGACC AGATGCTCCA

2101 CGCCCAGTCG CGTACCGTCT TCATGGGAGA AAATAATACT GTTGATGGGT

2151 GTCTGGTCAG AGACATCAAG AAATAACGCC GGAACATTAG TGCAGGCAGC

2201 TTCCACAGCA ATGGCATCCT GGTCATCCAG CGGATAGTTA ATGATCAGCC

2251 CACTGACGCG TTGCGCGAGA AGATTGTGCA CCGCCGCTTT ACAGGCTTCG

2301 ACGCCGCTTC GTTCTACCAT CGACACCACC ACGCTGGCAC CCAGTTGATC

2351 GGCGCGAGAT TTAATCGCCG CGACAATTTG CGACGGCGCG TGCAGGGCCA

2401 GACTGGAGGT GGCAACGCCA ATCAGCAACG ACTGTTTGCC CGCCAGTTGT

Fig. 16 (cont.)

```
2451 TGTGCCACGC GGTTGGGAAT GTAATTCAGC TCCGCCATCG CCGCTTCCAC
2501 TTTTTCCCGC GTTTTCGCAG AAACGTGGCT GGCCTGGTTC ACCACGCGGG
2551 AAACGGTCTG ATAAGAGACA CCGGCATACT CTGCGACATC GTATAACGTT
2601 ACTGGTTTCA CATTCACCAC CCTGAATTGA CTCTCTTCCG GGCGCTATCA
               (Me t)
2651 TGCCATACCG CGAAAGGTTT TGCACCATTC GATGGTGTCA ACGTAAATGC
                                  SalI
2701 ATGCCGCTTC GCCTTCGCGC GCGAATTGTC GACCCTGTCC CTCCTGTTCA
2751 GCTACTGACG GGGTGGTGCG TAACGGCAAA AGCACCGCCG GACATCAGCG
2801 CTAGCGGAGT GTATACTGGC TTACTATGTT GGCACTGATG AGGGTGTCAG
2851 TGAAGTGCTT CATGTGGCAG GAGAAAAAAG GCTGCACCGG TGCGTCAGCA
2901 GAATATGTGA TACAGGATAT ATTCCGCTTC CTCGCTCACT GACTCGCTAC
2951 GCTCGGTCGT TCGACTGCGG CGAGCGGAAA TGGCTTACGA ACGGGGCGGA
3001 GATTTCCTGG AAGATGCCAG GAAGATACTT AACAGGGAAG TGAGAGGGCC
3051 GCGGCAAAGC CGTTTTTCCA TAGGCTCCGC CCCCCTGACA AGCATCACGA
3101 AATCTGACGC TCAAATCAGT GGTGGCGAAA CCCGACAGGA CTATAAAGAT
3151 ACCAGGCGTT TCCCCTGGCG GCTCCCTCGT GCGCTCTCCT GTTCCTGCCT
3201 TTCGGTTTAC CGGTGTCATT CCGCTGTTAT GGCCGCGTTT GTCTCATTCC
3251 ACGCCTGACA CTCAGTTCCG GGTAGGCAGT TCGCTCCAAG CTGGACTGTA
3301 TGCACGAACC CCCCGTTCAG TCCGACCGCT GCGCCTTATC CGGTAACTAT
3351 CGTCTTGAGT CCAACCCGGA AAGACATGCA AAAGCACCAC TGGCAGCAGC
3401 CACTGGTAAT TGATTTAGAG GAGTTAGTCT TGAAGTCATG CGCCGGTTAA
3451 GGCTAAACTG AAAGGACAAG TTTTGGTGAC TGCGCTCCTC CAAGCCAGTT
3501 ACCTCGGTTC AAAGAGTTGG TAGCTCAGAG AACCTTCGAA AAACCGCCCT
3551 GCAAGGCGGT TTTTTCGTTT TCAGAGCAAG AGATTACGCG CAGACCAAAA
3601 CGATCTCAAG AAGATCATCT TATTAATCAG ATAAAATATT TCTAGATTTC
3651 AGTGCAATTT ATCTCTTCAA ATGTAGCACC TGAAGTCAGC CCCATACGAT
3701 ATAAGTTGTT AATTCTCATG TTTGACAGCT TATCATCGAT
```

Fig. 17

```
                BglII
linker 1:      CAGATCTG
               GTCTAGAC

                  BglII
linker 2:      GGAAGATCTTCC
               CCTTCTAGAAGG

                 BglII
linker 3:      GAAGATCTTC
               CTTCTAGAAG

                            BamHI
primer 1:      GCAGTATAAAAGGATCCCTTTGGATTCC

primer 2:      GAACCCTATCGTCTACTGGGAATACCAGATCATGCC

primer 3:      CCTAAACTGGATATCCGGAAAGAAGAAAAGCAAATCATGATTG

               BamHI
adaptor 1:      GATCCAGAGCTGAGATGGGTACCGCGGATCTGGGG
                    GTCTCGACTCTACCCATGGCGCCTAGACCCC

               NcoI                         Asp718I
adaptor 2:      CATGGGTGTTAGCCGTGCTGAGATGG
                    CCACAATCGGCACGACTCTACCCATG

               SphI               Asp718I
adaptor 3:          CGTGCTGAGATGG
                GTACGCACGACTCTACCCATG

               NcoI        Asp718I
adaptor 4:      CATGGAGATGG
                    CTCTACCCATG

               NcoI
adaptor 5:      CATGGGTACCGCGGATCTGGGG
                    CCATGGCGCCTAGACCCC
```

Fig. 18

```
        10                30                50
BglII    .       BamHI  SmaI.      EcoRI       .    .
AGATCTGCTAGAACTAGTGGATCCCCCGGGCTGCAGGAATTCCAGCCAGCGACCGTCGGT
ArgSerAlaArgThrSerGlySerProGlyLeuGlnGluPheGlnProAlaThrValGly

        70                90                110
         .        .        .        .        .         .
AGCAGCATGGCTCTCCTCTTTCTCCTACCCCTTGTCATGCAGGGTGTGAGCAGGGCTGAG
SerSerMetAlaLeuLeuPheLeuLeuProLeuValMetGlnGlyValSerArgAlaGlu
      1
        130               150               170
         .       HaeIII    .        .        .         .
ATGGGCACCGCGGATCTGGGGCCGTCCTCAGTGCCTACACCAACTAATGTTACAATTGAA
MetGlyThrAlaAspLeuGlyProSerSerValProThrProThrAsnValThrIleGlu
                     26
        190               210               230
         .        .        .        .        .         .
TCCTATAACATGAACCCTATCGTATATTGGGAGTACCAGATCATGCCACAGGTCCCTGTT
SerTyrAsnMetAsnProIleValTyrTrpGluTyrGlnIleMetProGlnValProVal

        250               270               290
         .        .        .        .        .         .
TTTACCGTAGAGGTAAAGAACTATGGTGTTAAGAATTCAGAATGGATTGATGCCTGCATC
PheThrValGluValLysAsnTyrGlyValLysAsnSerGluTrpIleAspAlaCysIle

        310               330               350
         .        .        .        .        .         .
AATATTTCTCATCATTATTGTAATATTTCTGATCATGTTGGTGATCCATCAAATTCTCTT
AsnIleSerHisHisTyrCysAsnIleSerAspHisValGlyAspProSerAsnSerLeu

        370               390               410
         .        .        .        .        .         .
TGGGTCAGAGTTAAAGCCAGGGTTGGACAAAAAGAATCTGCCTATGCAAAGTCAGAAGAA
TrpValArgValLysAlaArgValGlyGlnLysGluSerAlaTyrAlaLysSerGluGlu

        430               450               470
         .        .        .        .        .         .
TTTGCTGTATGCCGAGATGGAAAAATTGGACCACCTAAACTGGATATCAGAAAGGAGGAG
PheAlaValCysArgAspGlyLysIleGlyProProLysLeuAspIleArgLysGluGlu

        490               510               530
         .        .        .        .        .         .
AAGCAAATCATGATTGACATATTTCACCCTTCAGTTTTTGTAAATGGAGACGAGCAGGAA
LysGlnIleMetIleAspIlePheHisProSerValPheValAsnGlyAspGluGlnGlu

        550               570               590
         .        .        .        .        .         .
GTCGATTATGATCCCGAAACTACCTGTTACATTAGGGTGTACAATGTGTATGTGAGAATG
ValAspTyrAspProGluThrThrCysTyrIleArgValTyrAsnValTyrValArgMet
```

Fig. 18 (cont.)

```
        610                 630                 650
          .         .         .         .         .         .
AACGGAAGTGAGATCCAGTATAAAATACTCACGCAGAAGGAAGATGATTGTGACGAGATT
AsnGlySerGluIleGlnTyrLysIleLeuThrGlnLysGluAspAspCysAspGluIle

        670                 690                 710
          .         .         .         .    BglII
CAGTGCCAGTTAGCGATTCCAGTATCCTCACTGAATTCTCAGTCAGATCT
GlnCysGlnLeuAlaIleProValSerSerLeuAsnSerGlnSerAsp
                                        212
```

Fig. 19

```
        10                            30                            50
BamHI          .             .             .   HaeIII.        .             .
GGATCCAGAGCTGAGATGGGTACCGCGGATCTGGGGCCGTCCTCAGTGCCTACACCAACT
GlySerArgAlaGluMetGlyThrAlaAspLeuGlyProSerSerValProThrProThr
   15                                       26
        70                            90                           110
               .             .             .             .             .
AATGTTACAATTGAATCCTATAACATGAACCCTATCGTATATTGGGAGTACCAGATCATG
AsnValThrIleGluSerTyrAsnMetAsnProIleValTyrTrpGluTyrGlnIleMet

       130                           150                           170
    PpuMI      .             .             .             .             .
CCACAGGTCCCTGTTTTTACCGTAGAGGTAAAGAACTATGGTGTTAAGAATTCAGAATGG
ProGlnValProValPheThrValGluValLysAsnTyrGlyValLysAsnSerGluTrp

       190                           210                           230
               .             .             .             .             .
ATTGATGCCTGCATCAATATTTCTCATCATTATTGTAATATTTCTGATCATGTTGGTGAT
IleAspAlaCysIleAsnIleSerHisHisTyrCysAsnIleSerAspHisValGlyAsp

       250                           270                           290
               .             .             .             .             .
CCATCAAATTCTCTTTGGGTCAGAGTTAAAGCCAGGGTTGGACAAAAAGAATCTGCCTAT
ProSerAsnSerLeuTrpValArgValLysAlaArgValGlyGlnLysGluSerAlaTyr

       310                           330                           350
               .             .             .             .             .
GCAAAGTCAGAAGAATTTGCTGTATGCCGAGATGGAAAAATTGGACCACCTAAACTGGAT
AlaLysSerGluGluPheAlaValCysArgAspGlyLysIleGlyProProLysLeuAsp

       370                           390                           410
               .             .             .             .             .
ATCAGAAAGGAGGAGAAGCAAATCATGATTGACATATTTCACCCTTCAGTTTTTGTAAAT
IleArgLysGluGluLysGlnIleMetIleAspIlePheHisProSerValPheValAsn

       430                           450                           470
               .             .             .             .             .
GGAGACGAGCAGGAAGTCGATTATGATCCCGAAACTACCTGTTACATTAGGGTGTACAAT
GlyAspGluGlnGluValAspTyrAspProGluThrThrCysTyrIleArgValTyrAsn

       490                           510                           530
               .             .             .             .             .
GTGTATGTGAGAATGAACGGAAGTGAGATCCAGTATAAAATACTCACGCAGAAGGAAGAT
ValTyrValArgMetAsnGlySerGluIleGlnTyrLysIleLeuThrGlnLysGluAsp

       550                           570                           590
               .             .             .             .             .
GATTGTGACGAGATTCAGTGCCAGTTAGCGATTCCAGTATCCTCACTGAATTCTCAGTCA
AspCysAspGluIleGlnCysGlnLeuAlaIleProValSerSerLeuAsnSerGlnSer
                                                         212

BglII
GATCT
Asp
```

Fig. 20

15
1 MetArgGlySerArgAlaGluMetGlyThrAlaAspLeuGlyProSerSerValProThr

21 ProThrAsnValThrIleGluSerTyrAsnMetAsnProIleValTyrTrpGluTyrGln

41 IleMetProGlnValProValPheThrValGluValLysAsnTyrGlyValLysAsnSer

61 GluTrpIleAspAlaCysIleAsnIleSerHisHisTyrCysAsnIleSerAspHisVal

81 GlyAspProSerAsnSerLeuTrpValArgValLysAlaArgValGlyGlnLysGluSer

101 AlaTyrAlaLysSerGluGluPheAlaValCysArgAspGlyLysIleGlyProProLys

121 LeuAspIleArgLysGluGluLysGlnIleMetIleAspIlePheHisProSerValPhe

141 ValAsnGlyAspGluGlnGluValAspTyrAspProGluThrThrCysTyrIleArgVal

161 TyrAsnValTyrValArgMetAsnGlySerGluIleGlnTyrLysIleLeuThrGlnLys

181 GluAspAspCysAspGluIleGlnCysGlnLeuAlaIleProValSerSerLeuAsnSer

212
201 GlnSerAspLeuArgSerHisHisHisHisHisHis

Fig. 21

```
         10                    30                    50
BglII    .         .         .         .         .         .
AGATCTTCCCCGTCCTCAGTGCCTACACCAACTAATGTTACAATTGAATCCTATAACATG
ArgSerSerProSerSerValProThrProThrAsnValThrIleGluSerTyrAsnMet
         26
         70                    90                    110
         .         .         .         .         .         .
AACCCTATCGTATATTGGGAGTACCAGATCATGCCACAGGTCCCTGTTTTTACCGTAGAG
AsnProIleValTyrTrpGluTyrGlnIleMetProGlnValProValPheThrValGlu

         130                   150                   170
         .         .         .         .         .         .
GTAAAGAACTATGGTGTTAAGAATTCAGAATGGATTGATGCCTGCATCAATATTTCTCAT
ValLysAsnTyrGlyValLysAsnSerGluTrpIleAspAlaCysIleAsnIleSerHis

         190                   210                   230
         .         .         .         .         .         .
CATTATTGTAATATTTCTGATCATGTTGGTGATCCATCAAATTCTCTTTGGGTCAGAGTT
HisTyrCysAsnIleSerAspHisValGlyAspProSerAsnSerLeuTrpValArgVal

         250                   270                   290
         .         .         .         .         .         .
AAAGCCAGGGTTGGACAAAAAGAATCTGCCTATGCAAAGTCAGAAGAATTTGCTGTATGC
LysAlaArgValGlyGlnLysGluSerAlaTyrAlaLysSerGluGluPheAlaValCys

         310                   330                   350
         .         .         .EcoRV    .         .         .
CGAGATGGAAAAATTGGACCACCTAAACTGGATATCAGAAAGGAGGAGAAGCAAATCATG
ArgAspGlyLysIleGlyProProLysLeuAspIleArgLysGluGluLysGlnIleMet

         370                   390                   410
         .         .         .         .         .         .
ATTGACATATTTCACCCTTCAGTTTTTGTAAATGGAGACGAGCAGGAAGTCGATTATGAT
IleAspIlePheHisProSerValPheValAsnGlyAspGluGlnGluValAspTyrAsp

         430                   450                   470
         .         .         .         .         .         .
CCCGAAACTACCTGTTACATTAGGGTGTACAATGTGTATGTGAGAATGAACGGAAGTGAG
ProGluThrThrCysTyrIleArgValTyrAsnValTyrValArgMetAsnGlySerGlu

         490                   510                   530
         .         .         .         .         .         .
ATCCAGTATAAAATACTCACGCAGAAGGAAGATGATTGTGACGAGATTCAGTGCCAGTTA
IleGlnTyrLysIleLeuThrGlnLysGluAspAspCysAspGluIleGlnCysGlnLeu

         550                   570                   590
         .         .         .         .         .         .
GCGATTCCAGTATCCTCACTGAATTCTCAGTACTGTGTTTCAGCAGAAGGAGTCTTACAT
AlaIleProValSerSerLeuAsnSerGlnTyrCysValSerAlaGluGlyValLeuHis

         610                   630                   650
         .         .         .         .         .         .
GTGTGGGGTGTTACAACTGAAAAGTCAAAAGAAGTTTGTATTACCATTTTCAATAGCAGT
ValTrpGlyValThrThrGluLysSerLysGluValCysIleThrIlePheAsnSerSer
```

Fig. 21 (cont.)

```
        670                 690                 710
         .         .         .         .         .         .
ATAAAAGGTTCTCTTTGGATTCCAGTTGTTGCTGCTTTACTACTCTTTCTAGTGCTTAGC
IleLysGlySerLeuTrpIleProValValAlaAlaLeuLeuLeuPheLeuValLeuSer
        246
        730                 750                 770
         .         .         .         .         .         .
CTGGTATTCATCTGTTTTTATATTAAGAAAATTAATCCATTGAAGGAAAAAAGCATAATA
LeuValPheIleCysPheTyrIleLysLysIleAsnProLeuLysGluLysSerIleIle

        790                 810                 830
         .         .         .         .         .         .
TTACCCAAGTCCTTGATCTCTGTGGTAAGAAGTGCTACTTTAGAGACAAAACCTGAATCA
LeuProLysSerLeuIleSerValValArgSerAlaThrLeuGluThrLysProGluSer

        850                 870                 890
         .         .         .         .         .         .
AAATATGTATCACTCATCACGTCATACCAGCCATTTTCCTTAGAAAAGGAGGTGGTCTGT
LysTyrValSerLeuIleThrSerTyrGlnProPheSerLeuGluLysGluValValCys

        910                 930                 950
         .         .         .         .         .         .
GAAGAGCCGTTGTCTCCAGCAACAGTTCCAGGCATGCATACCGAAGACAATCCAGGAAAA
GluGluProLeuSerProAlaThrValProGlyMetHisThrGluAspAsnProGlyLys

        970                 990                1010
         .         .         .         .         .         .
GTGGAACATACAGAAGAACTTTCTAGTATAACAGAAGTGGTGACTACTGAAGAAAATATT
ValGluHisThrGluGluLeuSerSerIleThrGluValValThrThrGluGluAsnIle

       1030                1050                1070
         .         .         .         .         .         .
CCTGACGTGGTCCCGGGCAGCCATCTGACTCCAATAGAGAGAGAGAGTTCTTCACCTTTA
ProAspValValProGlySerHisLeuThrProIleGluArgGluSerSerSerProLeu

       1090                1110                1130
         .         .         .         .         .         .
AGTAGTAACCAGTCTGAACCTGGCAGCATCGCTTTAAACTCGTATCACTCCAGAAATTGT
SerSerAsnGlnSerGluProGlySerIleAlaLeuAsnSerTyrHisSerArgAsnCys

       1150                1170                1190
         .         .         .         .           BglII
TCTGAGAGTGATCACTCCAGAAATGGTTTTGATACTGATTCCAGGGAAGATCT
SerGluSerAspHisSerArgAsnGlyPheAspThrAspSerArgGluAsp
                                             416
```

Fig. 22

```
        10                  30                  50
BglII    .         .         .         .         .         .
AGATCTTCCCCGTCCTCAGTGCCTACACCAACTAATGTTACAATTGAATCCTATAACATG
ArgSerSerProSerSerValProThrProThrAsnValThrIleGluSerTyrAsnMet
         26
        70                  90                 110
         .         .         .         .         .         .
AACCCTATCGTATATTGGGAGTACCAGATCATGCCACAGGTCCCTGTTTTTACCGTAGAG
AsnProIleValTyrTrpGluTyrGlnIleMetProGlnValProValPheThrValGlu

       130                 150                 170
         .         .         .         .         .         .
GTAAAGAACTATGGTGTTAAGAATTCAGAATGGATTGATGCCTGCATCAATATTTCTCAT
ValLysAsnTyrGlyValLysAsnSerGluTrpIleAspAlaCysIleAsnIleSerHis

       190                 210                 230
         .         .         .         .         .         .
CATTATTGTAATATTTCTGATCATGTTGGTGATCCATCAAATTCTCTTTGGGTCAGAGTT
HisTyrCysAsnIleSerAspHisValGlyAspProSerAsnSerLeuTrpValArgVal

       250                 270                 290
         .         .         .         .         .         .
AAAGCCAGGGTTGGACAAAAAGAATCTGCCTATGCAAAGTCAGAAGAATTTGCTGTATGC
LysAlaArgValGlyGlnLysGluSerAlaTyrAlaLysSerGluGluPheAlaValCys

       310                 330                 350
         .         .         .         .         .         .
CGAGATGGAAAAATTGGACCACCTAAACTGGATATCAGAAAGGAGGAGAAGCAAATCATG
ArgAspGlyLysIleGlyProProLysLeuAspIleArgLysGluGluLysGlnIleMet

       370                 390                 410
         .         .         .         .         .         .
ATTGACATATTTCACCCTTCAGTTTTTGTAAATGGAGACGAGCAGGAAGTCGATTATGAT
IleAspIlePheHisProSerValPheValAsnGlyAspGluGlnGluValAspTyrAsp

       430                 450                 470
         .         .         .         .         .         .
CCCGAAACTACCTGTTACATTAGGGTGTACAATGTGTATGTGAGAATGAACGGAAGTGAG
ProGluThrThrCysTyrIleArgValTyrAsnValTyrValArgMetAsnGlySerGlu

       490                 510                 530
         .         .         .         .         .         .
ATCCAGTATAAAATACTCACGCAGAAGGAAGATGATTGTGACGAGATTCAGTGCCAGTTA
IleGlnTyrLysIleLeuThrGlnLysGluAspAspCysAspGluIleGlnCysGlnLeu

       550                 570                 590
         .         .         .         .         .         .
GCGATTCCAGTATCCTCACTGAATTCTCAGTACTGTGTTTCAGCAGAAGGAGTCTTACAT
AlaIleProValSerSerLeuAsnSerGlnTyrCysValSerAlaGluGlyValLeuHis

       610                 630                 650
         .         .         .         .         .         .
GTGTGGGGTGTTACAACTGAAAAGTCAAAAGAAGTTTGTATTACCATTTTCAATAGCAGT
ValTrpGlyValThrThrGluLysSerLysGluValCysIleThrIlePheAsnSerSer
```

Fig. 22 (cont.)

```
670                 690                 710
 BamHI      .         .         .         .         .
ATAAAAGGATCCCTTTGGATTCCAGTTGTTGCTGCTTTACTACTCTTTCTAGTGCTTAGC
IleLysGlySerLeuTrpIleProValValAlaAlaLeuLeuLeuPheLeuValLeuSer
   246
  730                 750                 770
      .         .         .         .         .         .
CTGGTATTCATCTGTTTTTATATTAAGAAAATTAATCCATTGAAGGAAAAAAGCATAATA
LeuValPheIleCysPheTyrIleLysLysIleAsnProLeuLysGluLysSerIleIle

  790                 810                 830
      .         .         .         .         .         .
TTACCCAAGTCCTTGATCTCTGTGGTAAGAAGTGCTACTTTAGAGACAAAACCTGAATCA
LeuProLysSerLeuIleSerValValArgSerAlaThrLeuGluThrLysProGluSer

  850                 870                 890
      .         .         .         .         .         .
AAATATGTATCACTCATCACGTCATACCAGCCATTTTCCTTAGAAAAGGAGGTGGTCTGT
LysTyrValSerLeuIleThrSerTyrGlnProPheSerLeuGluLysGluValValCys

  910                 930                 950
      .         .         .         .         .         .
GAAGAGCCGTTGTCTCCAGCAACAGTTCCAGGCATGCATACCGAAGACAATCCAGGAAAA
GluGluProLeuSerProAlaThrValProGlyMetHisThrGluAspAsnProGlyLys

  970                 990                 1010
      .         .         .         .         .         .
GTGGAACATACAGAAGAACTTTCTAGTATAACAGAAGTGGTGACTACTGAAGAAAATATT
ValGluHisThrGluGluLeuSerSerIleThrGluValValThrThrGluGluAsnIle

 1030                1050                1070
      .         .         .         .         .         .
CCTGACGTGGTCCCGGGCAGCCATCTGACTCCAATAGAGAGAGAGAGTTCTTCACCTTTA
ProAspValValProGlySerHisLeuThrProIleGluArgGluSerSerSerProLeu

 1090                1110                1130
      .         .         .         .         .         .
AGTAGTAACCAGTCTGAACCTGGCAGCATCGCTTTAAACTCGTATCACTCCAGAAATTGT
SerSerAsnGlnSerGluProGlySerIleAlaLeuAsnSerTyrHisSerArgAsnCys

 1150                1170                1190
      .         .         .         .          BglII
TCTGAGAGTGATCACTCCAGAAATGGTTTTGATACTGATTCCAGGGAAGATCT
SerGluSerAspHisSerArgAsnGlyPheAspThrAspSerArgGluAsp
                                         416
```

Fig. 23

```
      10                  30                  50
BglII  .         .         .         .         .         .
AGATCTTCCCCGTCCTCAGTGCCTACACCAACTAATGTTACAATTGAATCCTATAACATG
ArgSerSerProSerSerValProThrProThrAsnValThrIleGluSerTyrAsnMet
      26
      70                  90                  110
       .         .         .    PpuMI          .         .
AACCCTATCGTATATTGGGAGTACCAGATCATGCCACAGGTCCCTGTTTTTACCGTAGAG
AsnProIleValTyrTrpGluTyrGlnIleMetProGlnValProValPheThrValGlu

      130                 150                 170
       .         .         .         .         .         .
GTAAAGAACTATGGTGTTAAGAATTCAGAATGGATTGATGCCTGCATCAATATTTCTCAT
ValLysAsnTyrGlyValLysAsnSerGluTrpIleAspAlaCysIleAsnIleSerHis

      190                 210                 230
       .         .         .         .         .         .
CATTATTGTAATATTTCTGATCATGTTGGTGATCCATCAAATTCTCTTTGGGTCAGAGTT
HisTyrCysAsnIleSerAspHisValGlyAspProSerAsnSerLeuTrpValArgVal

      250                 270                 290
       .         .         .         .         .         .
AAAGCCAGGGTTGGACAAAAAGAATCTGCCTATGCAAAGTCAGAAGAATTTGCTGTATGC
LysAlaArgValGlyGlnLysGluSerAlaTyrAlaLysSerGluGluPheAlaValCys

      310                 330                 350
       .         .         .         .         .         .
CGAGATGGAAAAATTGGACCACCTAAACTGGATATCAGAAAGGAGGAGAAGCAAATCATG
ArgAspGlyLysIleGlyProProLysLeuAspIleArgLysGluGluLysGlnIleMet

      370                 390                 410
       .         .         .         .         .         .
ATTGACATATTTCACCCTTCAGTTTTTGTAAATGGAGACGAGCAGGAAGTCGATTATGAT
IleAspIlePheHisProSerValPheValAsnGlyAspGluGlnGluValAspTyrAsp

      430                 450                 470
       .         .         .         .         .         .
CCCGAAACTACCTGTTACATTAGGGTGTACAATGTGTATGTGAGAATGAACGGAAGTGAG
ProGluThrThrCysTyrIleArgValTyrAsnValTyrValArgMetAsnGlySerGlu

      490                 510                 530
       .         .         .         .         .         .
ATCCAGTATAAAATACTCACGCAGAAGGAAGATGATTGTGACGAGATTCAGTGCCAGTTA
IleGlnTyrLysIleLeuThrGlnLysGluAspAspCysAspGluIleGlnCysGlnLeu

      550                 570                 590
       .         .         .         .         .         .
GCGATTCCAGTATCCTCACTGAATTCTCAGTACTGTGTTTCAGCAGAAGGAGTCTTACAT
AlaIleProValSerSerLeuAsnSerGlnTyrCysValSerAlaGluGlyValLeuHis
```

Fig. 23 (cont.)

```
       610                 630                 650
         .         .         .         .         .         .
GTGTGGGGTGTTACAACTGAAAAGTCAAAAGAAGTTTGTATTACCATTTTCAATAGCAGT
ValTrpGlyValThrThrGluLysSerLysGluValCysIleThrIlePheAsnSerSer

       670
       BamHI
ATAAAAGGATCC
IleLysGlySer
         246
```

Fig. 24

26
1 MetArgGlySerSerProSerSerValProThrProThrAsnValThrIleGluSerTyr

21 AsnMetAsnProIleValTyrTrpGluTyrGlnIleMetProGlnValProValPheThr

41 ValGluValLysAsnTyrGlyValLysAsnSerGluTrpIleAspAlaCysIleAsnIle

61 SerHisHisTyrCysAsnIleSerAspHisValGlyAspProSerAsnSerLeuTrpVal

81 ArgValLysAlaArgValGlyGlnLysGluSerAlaTyrAlaLysSerGluGluPheAla

101 ValCysArgAspGlyLysIleGlyProProLysLeuAspIleArgLysGluGluLysGln

121 IleMetIleAspIlePheHisProSerValPheValAsnGlyAspGluGlnGluValAsp

141 TyrAspProGluThrThrCysTyrIleArgValTyrAsnValTyrValArgMetAsnGly

161 SerGluIleGlnTyrLysIleLeuThrGlnLysGluAspAspCysAspGluIleGlnCys

181 GlnLeuAlaIleProValSerSerLeuAsnSerGlnTyrCysValSerAlaGluGlyVal

201 LeuHisValTrpGlyValThrThrGluLysSerLysGluValCysIleThrIlePheAsn

246
221 SerSerIleLysGlySerArgSerHisHisHisHisHisHis

Fig. 25

26
1 MetArgGlySerSerProSerSerValProThrProThrAsnValThrIleGluSerTyr

21 AsnMetAsnProIleValTyrTrpGluTyrGlnIleMetProGlnValProValPheThr

41 ValGluValLysAsnTyrGlyValLysAsnSerGluTrpIleAspAlaCysIleAsnIle

61 SerHisHisTyrCysAsnIleSerAspHisValGlyAspProSerAsnSerLeuTrpVal

81 ArgValLysAlaArgValGlyGlnLysGluSerAlaTyrAlaLysSerGluGluPheAla

101 ValCysArgAspGlyLysIleGlyProProLysLeuAspIleArgLysGluGluLysGln

121 IleMetIleAspIlePheHisProSerValPheValAsnGlyAspGluGlnGluValAsp

141 TyrAspProGluThrThrCysTyrIleArgValTyrAsnValTyrValArgMetAsnGly

161 SerGluIleGlnTyrLysIleLeuThrGlnLysGluAspAspCysAspGluIleGlnCys

181 GlnLeuAlaIleProValSerSerLeuAsnSerGlnTyrCysValSerAlaGluGlyVal

201 LeuHisValTrpGlyValThrThrGluLysSerLysGluValCysIleThrIlePheAsn

246
221 SerSerIleLysGlySerHisHisHisHisHisHis

Fig. 26

26
1 MetArgGlySerArgSerSerProSerSerValProThrProThrAsnValThrIleGlu

21 SerTyrAsnMetAsnProIleValTyrTrpGluTyrGlnIleMetProGlnValProVal

41 PheThrValGluValLysAsnTyrGlyValLysAsnSerGluTrpIleAspAlaCysIle

61 AsnIleSerHisHisTyrCysAsnIleSerAspHisValGlyAspProSerAsnSerLeu

81 TrpValArgValLysAlaArgValGlyGlnLysGluSerAlaTyrAlaLysSerGluGlu

101 PheAlaValCysArgAspGlyLysIleGlyProProLysLeuAspIleArgLysGluGlu

121 LysGlnIleMetIleAspIlePheHisProSerValPheValAsnGlyAspGluGlnGlu

141 ValAspTyrAspProGluThrThrCysTyrIleArgValTyrAsnValTyrValArgMet

161 AsnGlySerGluIleGlnTyrLysIleLeuThrGlnLysGluAspAspCysAspGluIle

181 GlnCysGlnLeuAlaIleProValSerSerLeuAsnSerGlnTyrCysValSerAlaGlu

201 GlyValLeuHisValTrpGlyValThrThrGluLysSerLysGluValCysIleThrIle

246
221 PheAsnSerSerIleLysGlySerHisHisHisHisHisHis

Fig. 27

26

1 MetArgGlySerHisHisHisHisHisHisGlySerSerProSerSerValProThrPro

21 ThrAsnValThrIleGluSerTyrAsnMetAsnProIleValTyrTrpGluTyrGlnIle

41 MetProGlnValProValPheThrValGluValLysAsnTyrGlyValLysAsnSerGlu

61 TrpIleAspAlaCysIleAsnIleSerHisHisTyrCysAsnIleSerAspHisValGly

81 AspProSerAsnSerLeuTrpValArgValLysAlaArgValGlyGlnLysGluSerAla

101 TyrAlaLysSerGluGluPheAlaValCysArgAspGlyLysIleGlyProProLysLeu

121 AspIleArgLysGluGluLysGlnIleMetIleAspIlePheHisProSerValPheVal

141 AsnGlyAspGluGlnGluValAspTyrAspProGluThrThrCysTyrIleArgValTyr

161 AsnValTyrValArgMetAsnGlySerGluIleGlnTyrLysIleLeuThrGlnLysGlu

181 AspAspCysAspGluIleGlnCysGlnLeuAlaIleProValSerSerLeuAsnSerGln

201 TyrCysValSerAlaGluGlyValLeuHisValTrpGlyValThrThrGluLysSerLys

246

221 GluValCysIleThrIlePheAsnSerSerIleLysGlySerValAspLeuGlnProSer

241 LeuIleSer

Fig. 28

15

1 MetArgGlySerArgAlaGluMetGlyThrAlaAspLeuGlyProSerSerValProThr

21 ProThrAsnValThrIleGluSerTyrAsnMetAsnProIleValTyrTrpGluTyrGln

41 IleMetProGlnValProValPheThrValGluValLysAsnTyrGlyValLysAsnSer

61 GluTrpIleAspAlaCysIleAsnIleSerHisHisTyrCysAsnIleSerAspHisVal

81 GlyAspProSerAsnSerLeuTrpValArgValLysAlaArgValGlyGlnLysGluSer

101 AlaTyrAlaLysSerGluGluPheAlaValCysArgAspGlyLysIleGlyProProLys

121 LeuAspIleArgLysGluGluLysGlnIleMetIleAspIlePheHisProSerValPhe

141 ValAsnGlyAspGluGlnGluValAspTyrAspProGluThrThrCysTyrIleArgVal

161 TyrAsnValTyrValArgMetAsnGlySerGluIleGlnTyrLysIleLeuThrGlnLys

181 GluAspAspCysAspGluIleGlnCysGlnLeuAlaIleProValSerSerLeuAsnSer

201 GlnTyrCysValSerAlaGluGlyValLeuHisValTrpGlyValThrThrGluLysSer

246

221 LysGluValCysIleThrIlePheAsnSerSerIleLysGlySerArgSerHisHisHis

241 HisHisHis

Fig. 29

```
     10                     30                     50
BamHI      .          .          .          .          .          .
GGATCCAGAGCTGAGATGGGTACCGCGGATCTGGGGCCGTCCTCAGTGCCTACACCAACT
GlySerArgAlaGluMetGlyThrAlaAspLeuGlyProSerSerValProThrProThr
   15
     70                     90                    110
           .          .          .          .          .          .
AATGTTACAATTGAATCCTATAACATGAACCCTATCGTATATTGGGAGTACCAGATCATG
AsnValThrIleGluSerTyrAsnMetAsnProIleValTyrTrpGluTyrGlnIleMet

    130                    150                    170
   PpuMI                .          .          .          .
CCACAGGTCCCTGTTTTTACCGTAGAGGTAAAGAACTATGGTGTTAAGAATTCAGAATGG
ProGlnValProValPheThrValGluValLysAsnTyrGlyValLysAsnSerGluTrp

    190                    210                    230
           .          .          .          .          .          .
ATTGATGCCTGCATCAATATTTCTCATCATTATTGTAATATTTCTGATCATGTTGGTGAT
IleAspAlaCysIleAsnIleSerHisHisTyrCysAsnIleSerAspHisValGlyAsp

    250                    270                    290
           .          .          .          .          .          .
CCATCAAATTCTCTTTGGGTCAGAGTTAAAGCCAGGGTTGGACAAAAAGAATCTGCCTAT
ProSerAsnSerLeuTrpValArgValLysAlaArgValGlyGlnLysGluSerAlaTyr

    310                    330                    350
           .          .          .          .          .          .
GCAAAGTCAGAAGAATTTGCTGTATGCCGAGATGGAAAAATTGGACCACCTAAACTGGAT
AlaLysSerGluGluPheAlaValCysArgAspGlyLysIleGlyProProLysLeuAsp

    370                    390                    410
           .          .          .          .          .          .
ATCAGAAAGGAGGAGAAGCAAATCATGATTGACATATTTCACCCTTCAGTTTTTGTAAAT
IleArgLysGluGluLysGlnIleMetIleAspIlePheHisProSerValPheValAsn

    430                    450                    470
           .          .          .          .          .          .
GGAGACGAGCAGGAAGTCGATTATGATCCCGAAACTACCTGTTACATTAGGGTGTACAAT
GlyAspGluGlnGluValAspTyrAspProGluThrThrCysTyrIleArgValTyrAsn

    490                    510                    530
           .          .          .          .          .          .
GTGTATGTGAGAATGAACGGAAGTGAGATCCAGTATAAAATACTCACGCAGAAGGAAGAT
ValTyrValArgMetAsnGlySerGluIleGlnTyrLysIleLeuThrGlnLysGluAsp

    550                    570                    590
           .          .          .          .          .          .
GATTGTGACGAGATTCAGTGCCAGTTAGCGATTCCAGTATCCTCACTGAATTCTCAGTAC
AspCysAspGluIleGlnCysGlnLeuAlaIleProValSerSerLeuAsnSerGlnTyr
```

Fig. 29 (cont.)

```
        610                 630                 650
         .         .         .         .         .         .
TGTGTTTCAGCAGAAGGAGTCTTACATGTGTGGGGTGTTACAACTGAAAAGTCAAAAGAA
CysValSerAlaGluGlyValLeuHisValTrpGlyValThrThrGluLysSerLysGlu

        670                 690
         .         .         .    BamHI
GTTTGTATTACCATTTTCAATAGCAGTATAAAAGGATCC
ValCysIleThrIlePheAsnSerSerIleLysGlySer
                                    246
```

Fig. 30

15
1 MetArgGlySerArgAlaGluMetGlyThrAlaAspLeuGlyProSerSerValProThr

21 ProThrAsnValThrIleGluSerTyrAsnMetAsnProIleValTyrTrpGluTyrGln

41 IleMetProGlnValProValPheThrValGluValLysAsnTyrGlyValLysAsnSer

61 GluTrpIleAspAlaCysIleAsnIleSerHisHisTyrCysAsnIleSerAspHisVal

81 GlyAspProSerAsnSerLeuTrpValArgValLysAlaArgValGlyGlnLysGluSer

101 AlaTyrAlaLysSerGluGluPheAlaValCysArgAspGlyLysIleGlyProProLys

121 LeuAspIleArgLysGluGluLysGlnIleMetIleAspIlePheHisProSerValPhe

141 ValAsnGlyAspGluGlnGluValAspTyrAspProGluThrThrCysTyrIleArgVal

161 TyrAsnValTyrValArgMetAsnGlySerGluIleGlnTyrLysIleLeuThrGlnLys

181 GluAspAspCysAspGluIleGlnCysGlnLeuAlaIleProValSerSerLeuAsnSer

201 GlnTyrCysValSerAlaGluGlyValLeuHisValTrpGlyValThrThrGluLysSer

246
221 LysGluValCysIleThrIlePheAsnSerSerIleLysGlySerHisHisHisHisHis

241 His

Fig. 31

15
1 MetArgGlySerHisHisHisHisHisHisGlySerArgAlaGluMetGlyThrAlaAsp

21 LeuGlyProSerSerValProThrProThrAsnValThrIleGluSerTyrAsnMetAsn

41 ProIleValTyrTrpGluTyrGlnIleMetProGlnValProValPheThrValGluVal

61 LysAsnTyrGlyValLysAsnSerGluTrpIleAspAlaCysIleAsnIleSerHisHis

81 TyrCysAsnIleSerAspHisValGlyAspProSerAsnSerLeuTrpValArgValLys

101 AlaArgValGlyGlnLysGluSerAlaTyrAlaLysSerGluGluPheAlaValCysArg

121 AspGlyLysIleGlyProProLysLeuAspIleArgLysGluGluLysGlnIleMetIle

141 AspIlePheHisProSerValPheValAsnGlyAspGluGlnGluValAspTyrAspPro

161 GluThrThrCysTyrIleArgValTyrAsnValTyrValArgMetAsnGlySerGluIle

181 GlnTyrLysIleLeuThrGlnLysGluAspAspCysAspGluIleGlnCysGlnLeuAla

201 IleProValSerSerLeuAsnSerGlnTyrCysValSerAlaGluGlyValLeuHisVal

221 TrpGlyValThrThrGluLysSerLysGluValCysIleThrIlePheAsnSerSerIle

246
241 LysGlySerValAspLeuGlnProSerLeuIleSer

Fig. 32

```
     10                            30                             50
BamHI     .          Asp718I       .   HaeIII.                    .        .
GGATCCAGAGCTGAGATGGGTACCGCGGATCTGGGGCCGTCCTCAGTGCCTACACCAACT
GlySerArgAlaGluMetGlyThrAlaAspLeuGlyProSerSerValProThrProThr
  15
     70                            90                            110
     .             .               .         A. T         G .          .
AATGTTACAATTGAATCCTATAACATGAACCCTATCGTCTACTGGGAATACCAGATCATG
AsnValThrIleGluSerTyrAsnMetAsnProIleValTyrTrpGluTyrGlnIleMet
                                                                  53
     130                           150                           170
     .             .               .             .             .
CCACAGGTCCCTGTTTTTACCGTAGAGGTAAAGAACTATGGTGTTAAGAATTCAGAATGG
ProGlnValProValPheThrValGluValLysAsnTyrGlyValLysAsnSerGluTrp

     190                           210                           230
     .             .               .             .             .
ATTGATGCCTGCATCAATATTTCTCATCATTATTGTAATATTTCTGATCATGTTGGTGAT
IleAspAlaCysIleAsnIleSerHisHisTyrCysAsnIleSerAspHisValGlyAsp

     250                           270                           290
     .             .               .             .             .
CCATCAAATTCTCTTTGGGTCAGAGTTAAAGCCAGGGTTGGACAAAAAGAATCTGCCTAT
ProSerAsnSerLeuTrpValArgValLysAlaArgValGlyGlnLysGluSerAlaTyr

     310                           330                           350
     .             .               .             .             .
GCAAAGTCAGAAGAATTTGCTGTATGCCGAGATGGAAAAATTGGACCACCTAAACTGGAT
AlaLysSerGluGluPheAlaValCysArgAspGlyLysIleGlyProProLysLeuAsp

     370                           390                           410
  A A  G. G  G     .               .             .             .
ATCCGGAAAGAAGAAAAGCAAATCATGATTGACATATTTCACCCTTCAGTTTTTGTAAAT
IleArgLysGluGluLysGlnIleMetIleAspIlePheHisProSerValPheValAsn
                              142
     430                           450                           470
     .             .               .             .             .
GGAGACGAGCAGGAAGTCGATTATGATCCCGAAACTACCTGTTACATTAGGGTGTACAAT
GlyAspGluGlnGluValAspTyrAspProGluThrThrCysTyrIleArgValTyrAsn

     490                           510                           530
     .             .               .             .             .
GTGTATGTGAGAATGAACGGAAGTGAGATCCAGTATAAAATACTCACGCAGAAGGAAGAT
ValTyrValArgMetAsnGlySerGluIleGlnTyrLysIleLeuThrGlnLysGluAsp

     550                           570                           590
     .             .               .             .             .
GATTGTGACGAGATTCAGTGCCAGTTAGCGATTCCAGTATCCTCACTGAATTCTCAGTAC
AspCysAspGluIleGlnCysGlnLeuAlaIleProValSerSerLeuAsnSerGlnTyr
```

Fig. 32 (cont.)

```
        610                 630                 650
         .         .         .         .         .         .
TGTGTTTCAGCAGAAGGAGTCTTACATGTGTGGGGTGTTACAACTGAAAAGTCAAAAGAA
CysValSerAlaGluGlyValLeuHisValTrpGlyValThrThrGluLysSerLysGlu

        670                 690
         .         .         .    BamHI
GTTTGTATTACCATTTTCAATAGCAGTATAAAAGGATCC
ValCysIleThrIlePheAsnSerSerIleLysGlySer
                                    246
```

Fig. 33

15
1 MetArgGlySerArgAlaGluMetGlyThrAlaAspLeuGlyProSerSerValProThr

21 ProThrAsnValThrIleGluSerTyrAsnMetAsnProIleValTyrTrpGluTyrGln

41 IleMetProGlnValProValPheThrValGluValLysAsnTyrGlyValLysAsnSer

61 GluTrpIleAspAlaCysIleAsnIleSerHisHisTyrCysAsnIleSerAspHisVal

81 GlyAspProSerAsnSerLeuTrpValArgValLysAlaArgValGlyGlnLysGluSer

101 AlaTyrAlaLysSerGluGluPheAlaValCysArgAspGlyLysIleGlyProProLys

121 LeuAspIleArgLysGluGluLysGlnIleMetIleAspIlePheHisProSerValPhe

141 ValAsnGlyAspGluGlnGluValAspTyrAspProGluThrThrCysTyrIleArgVal

161 TyrAsnValTyrValArgMetAsnGlySerGluIleGlnTyrLysIleLeuThrGlnLys

181 GluAspAspCysAspGluIleGlnCysGlnLeuAlaIleProValSerSerLeuAsnSer

201 GlnTyrCysValSerAlaGluGlyValLeuHisValTrpGlyValThrThrGluLysSer

246
221 LysGluValCysIleThrIlePheAsnSerSerIleLysGlySerArgSerHisHisHis

241 HisHisHis

Fig. 34

```
        10                     30                     50
 NcoI    .          .          Asp718I       .          .          .
CCATGGGTGTTAGCCGTGCTGAGATGGGTACCGCGGATCTGGGGCCGTCCTCAGTGCCTACA
  MetGlyValSerArgAlaGluMetGlyThrAlaAspLeuGlyProSerSerValProThr
    13
        70                     90                     110
        .          .          .          .          .          .
  CCAACTAATGTTACAATTGAATCCTATAACATGAACCCTATCGTCTACTGGGAATACCAG
  ProThrAsnValThrIleGluSerTyrAsnMetAsnProIleValTyrTrpGluTyrGln

        130                    150                    170
        .          .          .          .          .          .
  ATCATGCCACAGGTCCCTGTTTTTACCGTAGAGGTAAAGAACTATGGTGTTAAGAATTCA
  IleMetProGlnValProValPheThrValGluValLysAsnTyrGlyValLysAsnSer

        190                    210                    230
        .          .          .          .          .          .
  GAATGGATTGATGCCTGCATCAATATTTCTCATCATTATTGTAATATTTCTGATCATGTT
  GluTrpIleAspAlaCysIleAsnIleSerHisHisTyrCysAsnIleSerAspHisVal

        250                    270                    290
        .          .          .          .          .          .
  GGTGATCCATCAAATTCTCTTTGGGTCAGAGTTAAAGCCAGGGTTGGACAAAAAGAATCT
  GlyAspProSerAsnSerLeuTrpValArgValLysAlaArgValGlyGlnLysGluSer

        310                    330                    350
        .          .          .          .          .          .
  GCCTATGCAAAGTCAGAAGAATTTGCTGTATGCCGAGATGGAAAAATTGGACCACCTAAA
  AlaTyrAlaLysSerGluGluPheAlaValCysArgAspGlyLysIleGlyProProLys

        370                    390                    410
        .          .          .          .          .          .
  CTGGATATCCGGAAAGAAGAAAAGCAAATCATGATTGACATATTTCACCCTTCAGTTTTT
  LeuAspIleArgLysGluGluLysGlnIleMetIleAspIlePheHisProSerValPhe

        430                    450                    470
        .          .          .          .          .          .
  GTAAATGGAGACGAGCAGGAAGTCGATTATGATCCCGAAACTACCTGTTACATTAGGGTG
  ValAsnGlyAspGluGlnGluValAspTyrAspProGluThrThrCysTyrIleArgVal

        490                    510                    530
        .          .          .          .          .          .
  TACAATGTGTATGTGAGAATGAACGGAAGTGAGATCCAGTATAAAATACTCACGCAGAAG
  TyrAsnValTyrValArgMetAsnGlySerGluIleGlnTyrLysIleLeuThrGlnLys

        550                    570                    590
        .          .          .          .          .          .
  GAAGATGATTGTGACGAGATTCAGTGCCAGTTAGCGATTCCAGTATCCTCACTGAATTCT
  GluAspAspCysAspGluIleGlnCysGlnLeuAlaIleProValSerSerLeuAsnSer
```

Fig. 34 (cont.)

```
     610                 630                 650
       .         .         .         .         .         .
CAGTACTGTGTTTCAGCAGAAGGAGTCTTACATGTGTGGGGTGTTACAACTGAAAAGTCA
GlnTyrCysValSerAlaGluGlyValLeuHisValTrpGlyValThrThrGluLysSer

     670                 690
       .         .         .         .  BamHI
AAAGAAGTTTGTATTACCATTTTCAATAGCAGTATAAAAGGATCC
LysGluValCysIleThrIlePheAsnSerSerIleLysGlySer
                                          246
```

Fig. 35

13

1 MetGlyValSerArgAlaGluMetGlyThrAlaAspLeuGlyProSerSerValProThr

21 ProThrAsnValThrIleGluSerTyrAsnMetAsnProIleValTyrTrpGluTyrGln

41 IleMetProGlnValProValPheThrValGluValLysAsnTyrGlyValLysAsnSer

61 GluTrpIleAspAlaCysIleAsnIleSerHisHisTyrCysAsnIleSerAspHisVal

81 GlyAspProSerAsnSerLeuTrpValArgValLysAlaArgValGlyGlnLysGluSer

101 AlaTyrAlaLysSerGluGluPheAlaValCysArgAspGlyLysIleGlyProProLys

121 LeuAspIleArgLysGluGluLysGlnIleMetIleAspIlePheHisProSerValPhe

141 ValAsnGlyAspGluGlnGluValAspTyrAspProGluThrThrCysTyrIleArgVal

161 TyrAsnValTyrValArgMetAsnGlySerGluIleGlnTyrLysIleLeuThrGlnLys

181 GluAspAspCysAspGluIleGlnCysGlnLeuAlaIleProValSerSerLeuAsnSer

201 GlnTyrCysValSerAlaGluGlyValLeuHisValTrpGlyValThrThrGluLysSer

246

221 LysGluValCysIleThrIlePheAsnSerSerIleLysGlySerArgSerHisHisHis

241 HisHisHis

Fig. 36

```
       10                         30                         50
  SphI  .          Asp718I         .            .            .            .
GCATGCGTGCTGAGATGGGTACCGCGGATCTGGGGCCGTCCTCAGTGCCTACACCAACTAAT
   MetArgAlaGluMetGlyThrAlaAspLeuGlyProSerSerValProThrProThrAsn
     16
       70                         90                         110
        .            .            .            .            .            .
   GTTACAATTGAATCCTATAACATGAACCCTATCGTCTACTGGGAATACCAGATCATGCCA
   ValThrIleGluSerTyrAsnMetAsnProIleValTyrTrpGluTyrGlnIleMetPro

       130                        150                        170
        .            .            .            .            .            .
   CAGGTCCCTGTTTTTACCGTAGAGGTAAAGAACTATGGTGTTAAGAATTCAGAATGGATT
   GlnValProValPheThrValGluValLysAsnTyrGlyValLysAsnSerGluTrpIle

       190                        210                        230
        .            .            .            .            .            .
   GATGCCTGCATCAATATTTCTCATCATTATTGTAATATTTCTGATCATGTTGGTGATCCA
   AspAlaCysIleAsnIleSerHisHisTyrCysAsnIleSerAspHisValGlyAspPro

       250                        270                        290
        .            .            .            .            .            .
   TCAAATTCTCTTTGGGTCAGAGTTAAAGCCAGGGTTGGACAAAAAGAATCTGCCTATGCA
   SerAsnSerLeuTrpValArgValLysAlaArgValGlyGlnLysGluSerAlaTyrAla

       310                        330                        350
        .            .            .            .            .            .
   AAGTCAGAAGAATTTGCTGTATGCCGAGATGGAAAAATTGGACCACCTAAACTGGATATC
   LysSerGluGluPheAlaValCysArgAspGlyLysIleGlyProProLysLeuAspIle

       370                        390                        410
        .            .            .            .            .            .
   CGGAAAGAAGAAAAGCAAATCATGATTGACATATTTCACCCTTCAGTTTTTGTAAATGGA
   ArgLysGluGluLysGlnIleMetIleAspIlePheHisProSerValPheValAsnGly

       430                        450                        470
        .            .            .            .            .            .
   GACGAGCAGGAAGTCGATTATGATCCCGAAACTACCTGTTACATTAGGGTGTACAATGTG
   AspGluGlnGluValAspTyrAspProGluThrThrCysTyrIleArgValTyrAsnVal

       490                        510                        530
        .            .            .            .            .            .
   TATGTGAGAATGAACGGAAGTGAGATCCAGTATAAAATACTCACGCAGAAGGAAGATGAT
   TyrValArgMetAsnGlySerGluIleGlnTyrLysIleLeuThrGlnLysGluAspAsp

       550                        570                        590
        .            .            .            .            .            .
   TGTGACGAGATTCAGTGCCAGTTAGCGATTCCAGTATCCTCACTGAATTCTCAGTACTGT
   CysAspGluIleGlnCysGlnLeuAlaIleProValSerSerLeuAsnSerGlnTyrCys
```

Fig. 36 (cont.)

```
     610                 630                 650
      .         .         .         .         .         .
GTTTCAGCAGAAGGAGTCTTACATGTGTGGGGTGTTACAACTGAAAAGTCAAAAGAAGTT
ValSerAlaGluGlyValLeuHisValTrpGlyValThrThrGluLysSerLysGluVal

     670                 690
      .         .         .  BamHI
TGTATTACCATTTTCAATAGCAGTATAAAAGGATCC
CysIleThrIlePheAsnSerSerIleLysGlySer
                                 246
```

Fig. 37

16
1 MetArgAlaGluMetGlyThrAlaAspLeuGlyProSerSerValProThrProThrAsn

21 ValThrIleGluSerTyrAsnMetAsnProIleValTyrTrpGluTyrGlnIleMetPro

41 GlnValProValPheThrValGluValLysAsnTyrGlyValLysAsnSerGluTrpIle

61 AspAlaCysIleAsnIleSerHisHisTyrCysAsnIleSerAspHisValGlyAspPro

81 SerAsnSerLeuTrpValArgValLysAlaArgValGlyGlnLysGluSerAlaTyrAla

101 LysSerGluGluPheAlaValCysArgAspGlyLysIleGlyProProLysLeuAspIle

121 ArgLysGluGluLysGlnIleMetIleAspIlePheHisProSerValPheValAsnGly

141 AspGluGlnGluValAspTyrAspProGluThrThrCysTyrIleArgValTyrAsnVal

161 TyrValArgMetAsnGlySerGluIleGlnTyrLysIleLeuThrGlnLysGluAspAsp

181 CysAspGluIleGlnCysGlnLeuAlaIleProValSerSerLeuAsnSerGlnTyrCys

201 ValSerAlaGluGlyValLeuHisValTrpGlyValThrThrGluLysSerLysGluVal

246
221 CysIleThrIlePheAsnSerSerIleLysGlySerArgSerHisHisHisHisHisHis

Fig. 38

```
       10                    30                    50
NcoI     . Asp718I .          .          .          .          .
CCATGGAGATGGGTACCGCGGATCTGGGGCCGTCCTCAGTGCCTACACCAACTAATGTTACA
  MetGluMetGlyThrAlaAspLeuGlyProSerSerValProThrProThrAsnValThr
    18
       70                    90                    110
       .          .          .          .          .          .
  ATTGAATCCTATAACATGAACCCTATCGTCTACTGGGAATACCAGATCATGCCACAGGTC
  IleGluSerTyrAsnMetAsnProIleValTyrTrpGluTyrGlnIleMetProGlnVal

      130                   150                   170
       .          .          .          .          .          .
  CCTGTTTTTACCGTAGAGGTAAAGAACTATGGTGTTAAGAATTCAGAATGGATTGATGCC
  ProValPheThrValGluValLysAsnTyrGlyValLysAsnSerGluTrpIleAspAla

      190                   210                   230
       .          .          .          .          .          .
  TGCATCAATATTTCTCATCATTATTGTAATATTTCTGATCATGTTGGTGATCCATCAAAT
  CysIleAsnIleSerHisHisTyrCysAsnIleSerAspHisValGlyAspProSerAsn

      250                   270                   290
       .          .          .          .          .          .
  TCTCTTTGGGTCAGAGTTAAAGCCAGGGTTGGACAAAAAGAATCTGCCTATGCAAAGTCA
  SerLeuTrpValArgValLysAlaArgValGlyGlnLysGluSerAlaTyrAlaLysSer

      310                   330                   350
       .          .          .          .          .          .
  GAAGAATTTGCTGTATGCCGAGATGGAAAAATTGGACCACCTAAACTGGATATCCGGAAA
  GluGluPheAlaValCysArgAspGlyLysIleGlyProProLysLeuAspIleArgLys

      370                   390                   410
       .          .          .          .          .          .
  GAAGAAAAGCAAATCATGATTGACATATTTCACCCTTCAGTTTTTGTAAATGGAGACGAG
  GluGluLysGlnIleMetIleAspIlePheHisProSerValPheValAsnGlyAspGlu

      430                   450                   470
       .          .          .          .          .          .
  CAGGAAGTCGATTATGATCCCGAAACTACCTGTTACATTAGGGTGTACAATGTGTATGTG
  GlnGluValAspTyrAspProGluThrThrCysTyrIleArgValTyrAsnValTyrVal

      490                   510                   530
       .          .          .          .          .          .
  AGAATGAACGGAAGTGAGATCCAGTATAAAATACTCACGCAGAAGGAAGATGATTGTGAC
  ArgMetAsnGlySerGluIleGlnTyrLysIleLeuThrGlnLysGluAspAspCysAsp

      550                   570                   590
       .          .          .          .          .          .
  GAGATTCAGTGCCAGTTAGCGATTCCAGTATCCTCACTGAATTCTCAGTACTGTGTTTCA
  GluIleGlnCysGlnLeuAlaIleProValSerSerLeuAsnSerGlnTyrCysValSer
```

Fig. 38 (cont.)

```
    610                 630                 650
     .         .         .         .         .         .
GCAGAAGGAGTCTTACATGTGTGGGGTGTTACAACTGAAAAGTCAAAAGAAGTTTGTATT
AlaGluGlyValLeuHisValTrpGlyValThrThrGluLysSerLysGluValCysIle

    670                 690
     .         .        BamHI
ACCATTTTCAATAGCAGTATAAAAGGATCC
ThrIlePheAsnSerSerIleLysGlySer
                             246
```

Fig. 39

18
1 MetGluMetGlyThrAlaAspLeuGlyProSerSerValProThrProThrAsnValThr

21 IleGluSerTyrAsnMetAsnProIleValTyrTrpGluTyrGlnIleMetProGlnVal

41 ProValPheThrValGluValLysAsnTyrGlyValLysAsnSerGluTrpIleAspAla

61 CysIleAsnIleSerHisHisTyrCysAsnIleSerAspHisValGlyAspProSerAsn

81 SerLeuTrpValArgValLysAlaArgValGlyGlnLysGluSerAlaTyrAlaLysSer

101 GluGluPheAlaValCysArgAspGlyLysIleGlyProProLysLeuAspIleArgLys

121 GluGluLysGlnIleMetIleAspIlePheHisProSerValPheValAsnGlyAspGlu

141 GlnGluValAspTyrAspProGluThrThrCysTyrIleArgValTyrAsnValTyrVal

161 ArgMetAsnGlySerGluIleGlnTyrLysIleLeuThrGlnLysGluAspAspCysAsp

181 GluIleGlnCysGlnLeuAlaIleProValSerSerLeuAsnSerGlnTyrCysValSer

201 AlaGluGlyValLeuHisValTrpGlyValThrThrGluLysSerLysGluValCysIle

246
221 ThrIlePheAsnSerSerIleLysGlySerArgSerHisHisHisHisHisHis

Fig. 40

```
        10      .              30                   50
NcoI     .          .HaeIII     .         .         .         .
CCATGGGTACCGCGGATCTGGGGCCGTCCTCAGTGCCTACACCAACTAATGTTACAATTGAA
  MetGlyThrAlaAspLeuGlyProSerSerValProThrProThrAsnValThrIleGlu
  19
        70                  90                  110
         .         .         .         .         .         .
  TCCTATAACATGAACCCTATCGTCTACTGGGAATACCAGATCATGCCACAGGTCCCTGTT
  SerTyrAsnMetAsnProIleValTyrTrpGluTyrGlnIleMetProGlnValProVal

       130                 150                  170
         .         .         .         .         .         .
  TTTACCGTAGAGGTAAAGAACTATGGTGTTAAGAATTCAGAATGGATTGATGCCTGCATC
  PheThrValGluValLysAsnTyrGlyValLysAsnSerGluTrpIleAspAlaCysIle

       190                 210                  230
         .         .         .         .         .         .
  AATATTTCTCATCATTATTGTAATATTTCTGATCATGTTGGTGATCCATCAAATTCTCTT
  AsnIleSerHisHisTyrCysAsnIleSerAspHisValGlyAspProSerAsnSerLeu

     · 250                 270                  290
         .         .         .         .         .         .
  TGGGTCAGAGTTAAAGCCAGGGTTGGACAAAAAGAATCTGCCTATGCAAAGTCAGAAGAA
  TrpValArgValLysAlaArgValGlyGlnLysGluSerAlaTyrAlaLysSerGluGlu

       310                 330                  350
         .         .         .         .         .         .
  TTTGCTGTATGCCGAGATGGAAAAATTGGACCACCTAAACTGGATATCCGGAAAGAAGAA
  PheAlaValCysArgAspGlyLysIleGlyProProLysLeuAspIleArgLysGluGlu

       370                 390                  410
         .         .         .         .         .         .
  AAGCAAATCATGATTGACATATTTCACCCTTCAGTTTTTGTAAATGGAGACGAGCAGGAA
  LysGlnIleMetIleAspIlePheHisProSerValPheValAsnGlyAspGluGlnGlu

       430                 450                  470
         .         .         .         .         .         .
  GTCGATTATGATCCCGAAACTACCTGTTACATTAGGGTGTACAATGTGTATGTGAGAATG
  ValAspTyrAspProGluThrThrCysTyrIleArgValTyrAsnValTyrValArgMet

       490                 510                  530
         .         .         .         .         .         .
  AACGGAAGTGAGATCCAGTATAAAATACTCACGCAGAAGGAAGATGATTGTGACGAGATT
  AsnGlySerGluIleGlnTyrLysIleLeuThrGlnLysGluAspAspCysAspGluIle

       550                 570                  590
         .         .         .         .         .         .
  CAGTGCCAGTTAGCGATTCCAGTATCCTCACTGAATTCTCAGTACTGTGTTTCAGCAGAA
  GlnCysGlnLeuAlaIleProValSerSerLeuAsnSerGlnTyrCysValSerAlaGlu
```

Fig. 40 (cont.)

```
    610                 630                 650
     .         .         .         .         .         .
GGAGTCTTACATGTGTGGGGTGTTACAACTGAAAAGTCAAAAGAAGTTTGTATTACCATT
GlyValLeuHisValTrpGlyValThrThrGluLysSerLysGluValCysIleThrIle

    670
     .         . BamHI
TTCAATAGCAGTATAAAAGGATCC
PheAsnSerSerIleLysGlySer
                     246
```

Fig. 41

19
1 MetGlyThrAlaAspLeuGlyProSerSerValProThrProThrAsnValThrIleGlu

21 SerTyrAsnMetAsnProIleValTyrTrpGluTyrGlnIleMetProGlnValProVal

41 PheThrValGluValLysAsnTyrGlyValLysAsnSerGluTrpIleAspAlaCysIle

61 AsnIleSerHisHisTyrCysAsnIleSerAspHisValGlyAspProSerAsnSerLeu

81 TrpValArgValLysAlaArgValGlyGlnLysGluSerAlaTyrAlaLysSerGluGlu

101 PheAlaValCysArgAspGlyLysIleGlyProProLysLeuAspIleArgLysGluGlu

121 LysGlnIleMetIleAspIlePheHisProSerValPheValAsnGlyAspGluGlnGlu

141 ValAspTyrAspProGluThrThrCysTyrIleArgValTyrAsnValTyrValArgMet

161 AsnGlySerGluIleGlnTyrLysIleLeuThrGlnLysGluAspAspCysAspGluIle

181 GlnCysGlnLeuAlaIleProValSerSerLeuAsnSerGlnTyrCysValSerAlaGlu

201 GlyValLeuHisValTrpGlyValThrThrGluLysSerLysGluValCysIleThrIle

246
221 PheAsnSerSerIleLysGlySerArgSerHisHisHisHisHisHis

Fig. 42

| plasmid | produced protein | amino acids of receptor |
|---|---|---|
| pDS56/RBSII-F25-6xHis(A) | P25H6A | 26-246 |
| pDS56/RBSII-F25-6xHis(B) | P25H6B | 26-246 |
| pDS56/RBSII-F25-6xHis(C) | P25H6C | 26-246 |
| pDS56/RBSII,6xHis-F25 | P6H25 | 26-246 |
| pDS56/RBSII-F26-6xHis | P26H6 | 15-212 |
| pDS56/RBSII-F28-6xHis(A) | P28H6A | 15-246 |
| pDS56/RBSII-F28-6xHis(A) | P53 | 53-246 |
| pDS56/RBSII-F28-6xHis(A) | P142 | 142-246 |
| pDS56/RBSII-F28-6xHis(B) | P28H6B | 15-246 |
| pDS56/RBSII,6xHis-F28 | P6H28 | 15-246 |
| pDS56/RBSII-F41-6xHis | P41H6 | 15-246 |
| pDS56/RBSII,NcoI-F43-6xHis | P43H6 | 13-246 |
| pDS56/RBSII,SphI-F44-6xHis | P44H6 | 16-246 |
| pDS56/RBSII,NcoI-F45-6xHis | P45H6 | 18-246 |
| pDS56/RBSII,NcoI-F46-6xHis | P46H6 | 19-246 |

Fig. 43

| | 1 · 11 · 21 · 26 · 53 · 142 · 212 · 246 · 489 | amino acids of receptor |
|---|---|---|
| Receptor | MALLFLLPLVMQGVSRAEMGTADLGPS–IMP–IMI–SQY–GSL–EFS | |
| P25H6A | MRGSS*****************RSHHHHHH | 26–246 |
| P25H6B | MRGSS*****************HHHHHH | 26–246 |
| P25H6C | MRGSRSS*****************HHHHHH | 26–246 |
| P6H25 | MRGSHHHHHHGSS*****************VDLQPSLIS | 26–246 |
| P26H6 | MRG************************SDLRSHHHHHH | 15–212 |
| P28H6A | MRG****************************RSHHHHHH | 15–246 |
| P53 | *************RSHHHHHH | 53–246 |
| P142 | *********RSHHHHHH | 142–246 |
| P28H6B | MRG****************************HHHHHH | 15–246 |
| P6H28 | MRGSHHHHHHG****************************VDLQPSLIS | 15–246 |
| P41H6 | MRG****************************RSHHHHHH | 15–246 |
| P43H6 | M******************************RSHHHHHH | 13–246 |
| P44H6 | M***************************RSHHHHHH | 16–246 |
| P45H6 | M*************************RSHHHHHH | 18–246 |
| P46H6 | ************************RSHHHHHH | 19–246 |
| Receptor | MALLFLLPLVMQGVSRAEMGTADLGPS–IMP–IMI–SQY–GSL–EFS | |
| | 1 · 11 · 21 · 26 · 53 · 142 · 212 · 246 · 489 | |

Fig. 44

| protein | amino acids of receptor | binding of mAb | binding of ligand | inhibition of ligand binding to Raji cells | inhibition of antiviral activity of ligand |
|---|---|---|---|---|---|
| P25H6A | 26-246 | + | + | n.t. | n.t. |
| P25H6B | 26-246 | + | + | n.t. | n.t. |
| P25H6C | 26-246 | + | + | + | + |
| P6H25 | 26-246 | + | + | n.t. | n.t. |
| P26H6 | 15-212 | + | - | - | - |
| P28H6A | 15-246 | + | + | + | + |
| P53 | 53-246 | + | - | n.t. | n.t. |
| P142 | 142-246 | - | - | n.t. | n.t. |
| P28H6B | 15-246 | + | + | n.t. | n.t. |
| P6H28 | 15-246 | + | + | n.t. | n.t. |
| P41H6 | 15-246 | + | + | + | + |
| P43H6 | 13-246 | + | + | n.t. | n.t. |
| P44H6 | 16-246 | + | + | n.t. | n.t. |
| P45H6 | 18-246 | + | + | n.t. | n.t. |
| P46H6 | 19-246 | + | + | n.t. | n.t. |

Fig.45

| purification step | protein mg | P41H6 mg | recovery % | purity % |
|---|---|---|---|---|
| (I) Extraction | 10000 | 100 | 100 | 1 |
| (II) Ni-Chelate Column | 150 | 50 | 50 | 30 |
| (III) Sephadex G-100 | 80 | 40 | 40 | 50 |
| (V) Polybuffer Exchanger | 20 | 17 | 17 | 85 |

- 100 g of E. coli cells containing protein P41H6 were processed
- purity was determined by non-reducing SDS-PAGE

Fig. 46

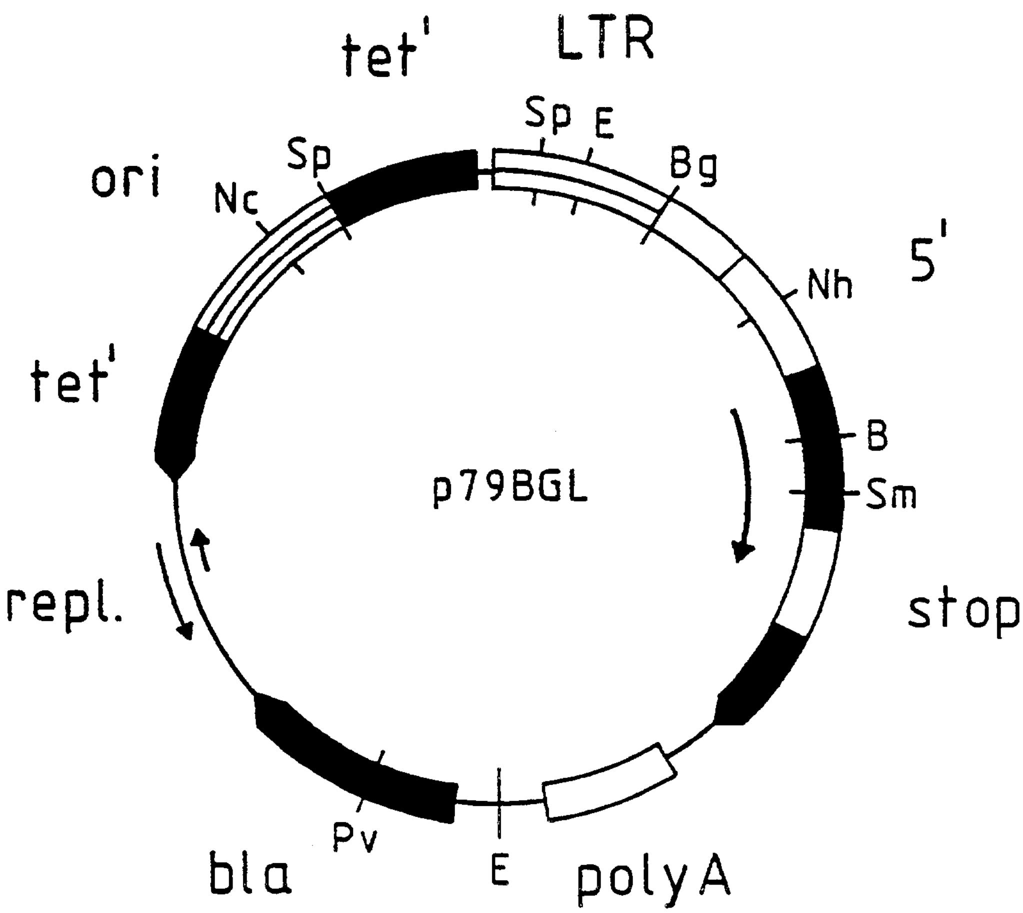
tet'
LTR
Sp
E
ori
Sp
Bg
Nc
5'
Nh
tet'
B
p79BGL
Sm
repl.
stop
Pv
bla
E
polyA

Fig. 47

```
  1  GGATCTCAGG ATATAGTAGT TTCGCTTTTG CATAGGGAGG GGGAAATGTA

 51  GCCTTATGCA ATACTCTTGT AGTCTTGCAA CATGCTTATG TAACGATGAG

                                                 SphI
101  TTAGCAACAT GCCTTACAAG GAGAGAAAAA GCACCGTGCA TGCCGATTGG

151  TGGAAGTAAG GTGGTA.GAT CGTGCCTTAT TAGGAAGGCA ACAGACGGGT

                                EcoRI
201  CTGACATGGA TTGGACGAAC CACCGAATTC GCATTGCAGA GAGTATTGTA

                                       *
251  TTTAAGTGCC TAGCTCGATA CAATAAACGC CATTTGACCA TTCACCACAT

                           BglII
301  TGGTGTGCAC CTCAAGCTCA GATCTGAGCT TGGTAAGTGA CCAGCTACAG

351  TCGGAAACCA TCAGCAAGCA GGTATGTACT CTCCAGGGTG GGCCTGGCTT

401  CCCCAGTCAA GACTCCAGGG ATTTGAGGGA CGCTGTGGGC TCTTCTCTTA

                  NheI
451  CATGTACCTT TTGCTAGCCT CAACCCTGAC TATCTTCCAG GTCATTGTTC

                  BamHI SmaI
501  CAACATGGCC CTGTGGATCC CGGGGGCAGG TGACTTAAGT TAACTTAAGT
          MetAla LeuTrpIleP roGlyAlaGl yAspLeuSer

551  GACCTTCAGA CCTTGGCACT GGAGGTGGCC CGGCAGAAGC GCGGCATCGT

601  GGATCAGTGC TGCACCAGCA TCTGCTCTCT CTACCAACTG GAGAACTACT

651  GCAACTAGGC CCACCACTAC CCTGTCCACC CCTCTGCAAT GAATAAAACC

701  TTTGAAAGAG CACTACAAGT TGTGTGTACA TGCGTGCGTG TGCATATGTG

751  GTGCGGGGGG AACATGAGTG GGGTCGGCTG GAGTGGTCGC GGCTTAATCT

                                        EcoRI
801  ATCTGGCGAT GATAAGCTGT CAAACATGAG AATTCTTGAA GACGAAAGGG

851  CCTCGTGATA CGCCTATTTT TATAGGTTAA TGTCATGATA ATAATGGTTT

901  CTTAGACGTC AGGTGGCACT TTTCGGGGAA ATGTGCGCGG AACCCCTATT

951  TGTTTATTTT TCTAAATACA TTCAAATATG TATCCGCTCA TGAGACAATA
```

Fig. 47 (cont.)

1001 ACCCTGATAA ATGCTTCAAT AATATTGAAA AAGGAAGAGT ATGAGTATTC

1051 AACATTTCCG TGTCGCCCTT ATTCCCTTTT TTGCGGCATT TTGCCTTCCT

1101 GTTTTTGCTC ACCCAGAAAC GCTGGTGAAA GTAAAAGATG CTGAAGATCA

1151 GTTGGGTGCA CGAGTGGGTT ACATCGAACT GGATCTCAAC AGCGGTAAGA

1201 TCCTTGAGAG TTTTCGCCCC GAAGAACGTT TTCCAATGAT GAGCACTTTT

1251 AAAGTTCTGC TATGTGGCGC GGTATTATCC CGTGTTGACG CCGGGCAAGA

1301 GCAACTCGGT CGCCGCATAC ACTATTCTCA GAATGACTTG GTTGAGTACT

1351 CACCAGTCAC AGAAAAGCAT CTTACGGATG GCATGACAGT AAGAGAATTA

1401 TGCAGTGCTG CCATAACCAT GAGTGATAAC ACTGCGGCCA ACTTACTTCT

PvuI

1451 GACAACGATC GGAGGACCGA AGGAGCTAAC CGCTTTTTTG CACAACATGG

1501 GGGATCATGT AACTCGCCTT GATCGTTGGG AACCGGAGCT GAATGAAGCC

1551 ATACCAAACG ACGAGCGTGA CACCACGATG CCTGCAGCAA TGGCAACAAC

1601 GTTGCGCAAA CTATTAACTG GCGAACTACT TACTCTAGCT TCCCGGCAAC

1651 AATTAATAGA CTGGATGGAG GCGGATAAAG TTGCAGGACC ACTTCTGCGC

1701 TCGGCCCTTC CGGCTGGCTG GTTTATTGCT GATAAATCTG GAGCCGGTGA

1751 GCGTGGGTCT CGCGGTATCA TTGCAGCACT GGGGCCAGAT GGTAAGCCCT

1801 CCCGTATCGT AGTTATCTAC ACGACGGGGA GTCAGGCAAC TATGGATGAA

1851 CGAAATAGAC AGATCGCTGA GATAGGTGCC TCACTGATTA AGCATTGGTA

1901 ACTGTCAGAC CAAGTTTACT CATATATACT TTAGATTGAT TTAAAACTTC

1951 ATTTTTAATT TAAAAGCGTA ATCTGCTGCT TGCAAACAAA AAAACCACCG

2001 CTACCAGCGG TGGTTTGTTT GCCGGATCAA GAGCTACCAA CTCTTTTTCC

2051 GAAGGTAACT GGCTTCAGCA GAGCGCAGAT ACCAAATACT GTCCTTCTAG

2101 TGTAGCCGTA GTTAGGCCAC CACTTCAAGA ACTCTGTAGC ACCGCCTACA

2151 TACCTCGCTC TGCTAATCCT GTTACCAGTG GCTGCTGCCA GTGGCGATAA

2201 GTCGTGTCTT ACCGGGTTGG ACTCAAGACG ATAGTTACCG GATAAGGCGC

Fig. 47 (cont.)

2251 AGCGGTCGGG CTGAACGGGG GGTTCGTGCA CACAGCCCAG CTTGGAGCGA

2301 ACGACCTACA CCGAACTGAG ATACCTACAG CGTGAGCATT GAGAAAGCGC

2351 CACGCTTCCC GAAGGGAGAA AGGCGGACAG GTATCCGGTA AGCGGCAGGG

2401 TCGGAACAGG AGAGCGCACG AGGGAGCTTC CAGGGGGAAA CGCCTGGTAT

2451 CTTTATAGTC CTGTCGGGTT TCGCCACCTC TGACTTGAGC GTCGATTTTT

2501 GTGATGCTCG TCAGGGGGGC GGAGCCTATG GAAAAACGCC AGCAACGGAT

2551 GCGCCGCGTG CGGCTGCTGG AGATGGCGGA CGCGATGGAT ATGTTCTGCC

2601 AAGGGTTGGT TTGCGCATTC ACAGTTCTCC GCAAGAATTG ATTGGCTCCA

2651 ATTCTTGGAG TGGTGAATCC GTTAGCGAGG TGCCGCCGGC TTCCATTCAG

2701 GTCGAGGTGG CCCGGCTCCA TGCACCGCGA CGCAACGCGG GGAGGCAGAC

2751 AAGGTATAGG GCGGCGCCTA CAATCCATGC CAACCCGTTC CATGTGCTCG

2801 CCGAGGCGGC ATAAATCGCC GTGACGATCA GCGGTCCAGT GATCGAAGTT

2851 AGGCTGGTAA GAGCCGCGAG CGATCCTTGA AGCTGTCCCT GATGGTCGTC

2901 ATCTACCTGC CTGGACAGCA TGGCCTGCAA CGCGGGCATC CCGATGCCGC

2951 CGGAAGCGAG AAGAATCATA ATGGGGAAGG CCATCCAGCC TCGCGTCGAG

3001 CTTTTTGCAA AAGCCTAGGC CTCCAAAAAA GCCTCCTCAC TACTTCTGGA

3051 ATAGCTCAGA GGCCGAGGCG GCCTCGGCCT CTGCATAAAT AAAAAAAATT

NcoI

3101 AGTCAGCCAT GGGGCGGAGA ATGGGCGGAA CTGGGCGGAG TTAGGGGCGG

SphI

3151 GATGGGCGGA GTTAGGGGCG GGACTATGGT TGCTGACTAA TTGAGATGCA

3201 TGCAAGGAGA TGGCGCCCAA CAGTCCCCCG GCCACGGCGC CTGCCACCAT

3251 ACCCACGCCG AAACAAGCGC TCATGAGCCC GAAGTGGCGA GCCCGATCTT

3301 CCCCATCGGT GATGTCGGCG ATATAGGCGC CAGCAACCGC ACCTGTGGCG

3351 CCGGTGATGC CGGCCACGAT GCGTCCGGCG TAGA

Fig. 48

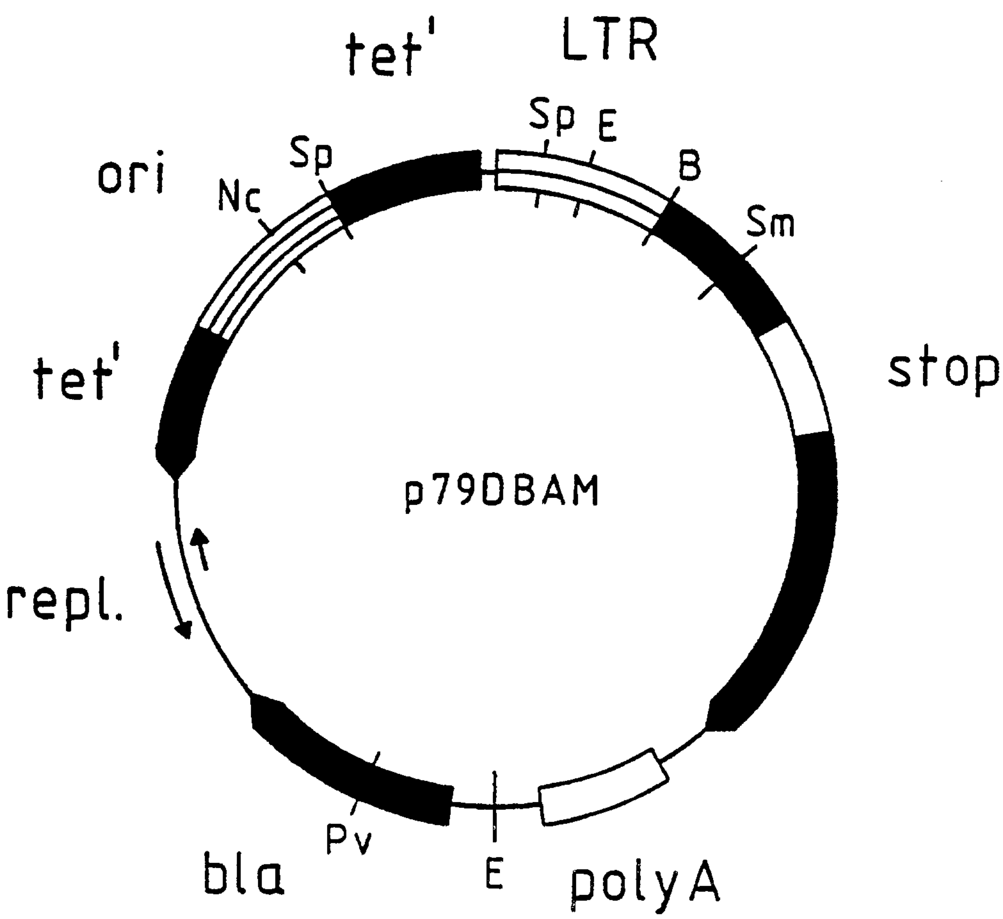

Fig. 49

```
1    GGATCTCAGG ATATAGTAGT TTCGCTTTTG CATAGGGAGG GGGAAATGTA
51   GCCTTATGCA ATACTCTTGT AGTCTTGCAA CATGCTTATG TAACGATGAG
                                             SphI
101  TTAGCAACAT GCCTTACAAG GAGAGAAAAA GCACCGTGCA TGCCGATTGG
151  TGGAAGTAAG GTGGTA.GAT CGTGCCTTAT TAGGAAGGCA ACAGACGGGT
                          EcoRI
201  CTGACATGGA TTGGACGAAC CACCGAATTC GCATTGCAGA GAGTATTGTA
                                *
251  TTTAAGTGCC TAGCTCGATA CAATAAACGC CATTTGACCA TTCACCACAT
                     BamHI SmaI
301  TGGTGTGCAC CTCAAGCTCG GATCCCGGGG GCAGGTGACT TAAGTTAACT
351  TAAGTGACCT TCAGACCTTG GCACTGGAGG TGGCCCGGCA GAAGCGCGGC
401  ATCGTGGATC AGTGCTGCAC CAGCATCTGC TCTCTCTACC AACTGGAGAA
451  CTACTGCAAC TAGGCCCACC ACTACCCTGT CCACCCCTCT GCAATGAATA
501  AAACCTTTGA AAGAGCACTA CAAGTTGTGT GTACATGCGT GCGTGTGCAT
551  ATGTGGTGCG GGGGGAACAT GAGTGGGGTC GGCTGGAGTG GTCGCGGCTT
                                       EcoRI
601  AATCTATCTG GCGATGATAA GCTGTCAAAC ATGAGAATTC TTGAAGACGA
651  AAGGGCCTCG TGATACGCCT ATTTTTATAG GTTAATGTCA TGATAATAAT
701  GGTTTCTTAG ACGTCAGGTG GCACTTTTCG GGGAAATGTG CGCGGAACCC
751  CTATTTGTTT ATTTTTCTAA ATACATTCAA ATATGTATCC GCTCATGAGA
801  CAATAACCCT GATAAATGCT TCAATAATAT TGAAAAAGGA AGAGTATGAG
851  TATTCAACAT TTCCGTGTCG CCCTTATTCC CTTTTTTGCG GCATTTTGCC
901  TTCCTGTTTT TGCTCACCCA GAAACGCTGG TGAAAGTAAA AGATGCTGAA
951  GATCAGTTGG GTGCACGAGT GGGTTACATC GAACTGGATC TCAACAGCGG
1001 TAAGATCCTT GAGAGTTTTC GCCCCGAAGA ACGTTTTCCA ATGATGAGCA
1051 CTTTTAAAGT TCTGCTATGT GGCGCGGTAT TATCCCGTGT TGACGCCGGG
```

Fig. 49 (cont.)

1101 CAAGAGCAAC TCGGTCGCCG CATACACTAT TCTCAGAATG ACTTGGTTGA

1151 GTACTCACCA GTCACAGAAA AGCATCTTAC GGATGGCATG ACAGTAAGAG

1201 AATTATGCAG TGCTGCCATA ACCATGAGTG ATAACACTGC GGCCAACTTA

PvuI

1251 CTTCTGACAA CGATCGGAGG ACCGAAGGAG CTAACCGCTT TTTTGCACAA

1301 CATGGGGGAT CATGTAACTC GCCTTGATCG TTGGGAACCG GAGCTGAATG

1351 AAGCCATACC AAACGACGAG CGTGACACCA CGATGCCTGC AGCAATGGCA

1401 ACAACGTTGC GCAAACTATT AACTGGCGAA CTACTTACTC TAGCTTCCCG

1451 GCAACAATTA ATAGACTGGA TGGAGGCGGA TAAAGTTGCA GGACCACTTC

1501 TGCGCTCGGC CCTTCCGGCT GGCTGGTTTA TTGCTGATAA ATCTGGAGCC

1551 GGTGAGCGTG GGTCTCGCGG TATCATTGCA GCACTGGGGC CAGATGGTAA

1601 GCCCTCCCGT ATCGTAGTTA TCTACACGAC GGGGAGTCAG GCAACTATGG

1651 ATGAACGAAA TAGACAGATC GCTGAGATAG GTGCCTCACT GATTAAGCAT

1701 TGGTAACTGT CAGACCAAGT TTACTCATAT ATACTTTAGA TTGATTTAAA

1751 ACTTCATTTT TAATTTAAAA GCGTAATCTG CTGCTTGCAA ACAAAAAAAC

1801 CACCGCTACC AGCGGTGGTT TGTTTGCCGG ATCAAGAGCT ACCAACTCTT

1851 TTTCCGAAGG TAACTGGCTT CAGCAGAGCG CAGATACCAA ATACTGTCCT

1901 TCTAGTGTAG CCGTAGTTAG GCCACCACTT CAAGAACTCT GTAGCACCGC

1951 CTACATACCT CGCTCTGCTA ATCCTGTTAC CAGTGGCTGC TGCCAGTGGC

2001 GATAAGTCGT GTCTTACCGG GTTGGACTCA AGACGATAGT TACCGGATAA

2051 GGCGCAGCGG TCGGGCTGAA CGGGGGGTTC GTGCACACAG CCCAGCTTGG

2101 AGCGAACGAC CTACACCGAA CTGAGATACC TACAGCGTGA GCATTGAGAA

2151 AGCGCCACGC TTCCCGAAGG GAGAAAGGCG GACAGGTATC CGGTAAGCGG

2201 CAGGGTCGGA ACAGGAGAGC GCACGAGGGA GCTTCCAGGG GGAAACGCCT

2251 GGTATCTTTA TAGTCCTGTC GGGTTTCGCC ACCTCTGACT TGAGCGTCGA

Fig. 49 (cont.)

2301 TTTTTGTGAT GCTCGTCAGG GGGGCGGAGC CTATGGAAAA ACGCCAGCAA

2351 CGGATGCGCC GCGTGCGGCT GCTGGAGATG GCGGACGCGA TGGATATGTT

2401 CTGCCAAGGG TTGGTTTGCG CATTCACAGT TCTCCGCAAG AATTGATTGG

2451 CTCCAATTCT TGGAGTGGTG AATCCGTTAG CGAGGTGCCG CCGGCTTCCA

2501 TTCAGGTCGA GGTGGCCCGG CTCCATGCAC CGCGACGCAA CGCGGGGAGG

2551 CAGACAAGGT ATAGGGCGGC GCCTACAATC CATGCCAACC CGTTCCATGT

2601 GCTCGCCGAG GCGGCATAAA TCGCCGTGAC GATCAGCGGT CCAGTGATCG

2651 AAGTTAGGCT GGTAAGAGCC GCGAGCGATC CTTGAAGCTG TCCCTGATGG

2701 TCGTCATCTA CCTGCCTGGA CAGCATGGCC TGCAACGCGG GCATCCCGAT

2751 GCCGCCGGAA GCGAGAAGAA TCATAATGGG GAAGGCCATC CAGCCTCGCG

2801 TCGAGCTTTT TGCAAAAGCC TAGGCCTCCA AAAAAGCCTC CTCACTACTT

2851 CTGGAATAGC TCAGAGGCCG AGGCGGCCTC GGCCTCTGCA TAAATAAAAA

NcoI

2901 AAATTAGTCA GCCATGGGGC GGAGAATGGG CGGAACTGGG CGGAGTTAGG

2951 GGCGGGATGG GCGGAGTTAG GGGCGGGACT ATGGTTGCTG ACTAATTGAG

SphI

3001 ATGCATGCAA GGAGATGGCG CCCAACAGTC CCCCGGCCAC GGCGCCTGCC

3051 ACCATACCCA CGCCGAAACA AGCGCTCATG AGCCCGAAGT GGCGAGCCCG

3101 ATCTTCCCCA TCGGTGATGT CGGCGATATA GGCGCCAGCA ACCGCACCTG

3151 TGGCGCCGGT GATGCCGGCC ACGATGCGTC CGGCGTAGA

Fig. 50

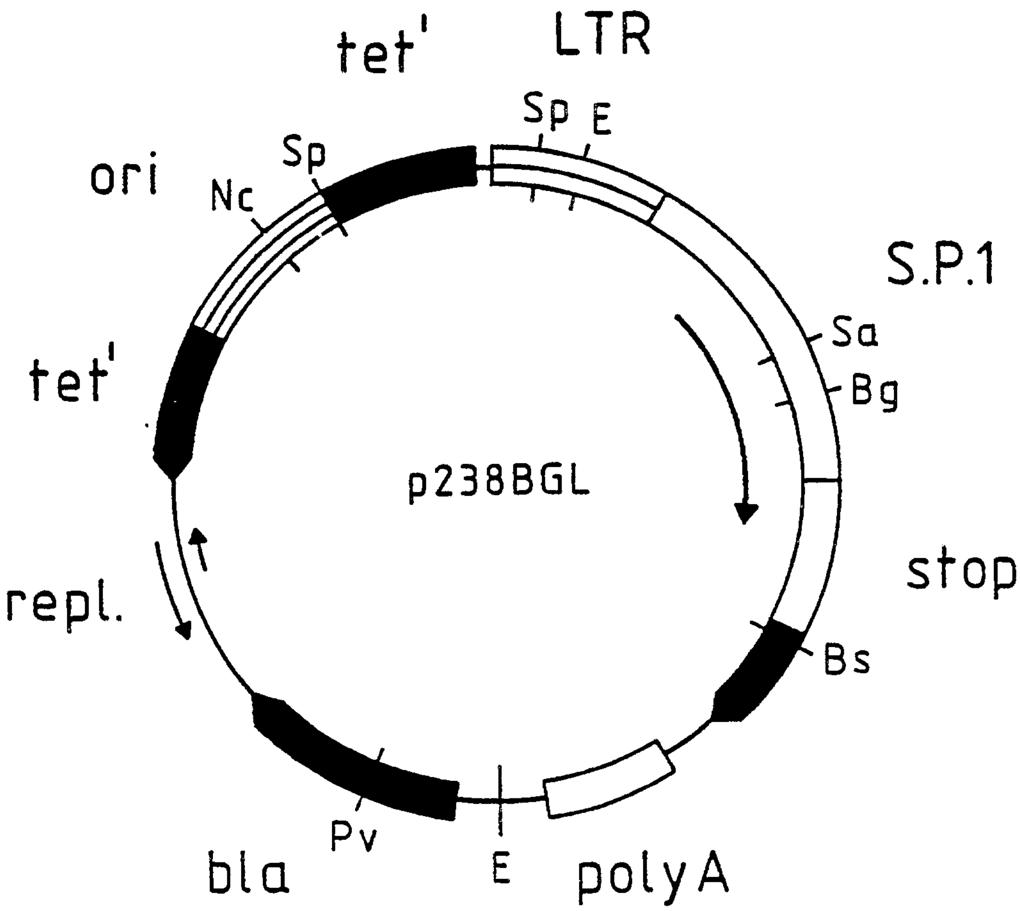

tet'
LTR
Sp
E
ori
Nc
Sp
S.P.1
Sa
Bg
tet'
p238BGL
stop
repl.
Bs
bla
Pv
E
polyA

Fig. 51

```
                                                                  SphI
1    GGATCTCAGG ATATAGTAGT TTCGCTTTTG CATAGGGAGG GGGAAATGTA

51   GCCTTATGCA ATACTCTTGT AGTCTTGCAA CATGCTTATG TAACGATGAG

                                                  SphI
101  TTAGCAACAT GCCTTACAAG GAGAGAAAAA GCACCGTGCA TGCCGATTGG

151  TGGAAGTAAG GTGGTA.GAT CGTGCCTTAT TAGGAAGGCA ACAGACGGGT

                                 EcoRI
201  CTGACATGGA TTGGACGAAC CACCGAATTC GCATTGCAGA GAGTATTGTA

                                    *
251  TTTAAGTGCC TAGCTCGATA CAATAAACGC CATTTGACCA TTCACCACAT

301  TGGTGTGCAC CTCAAGCTCG GCTGGTCCCA AGGGTCAGGA AGATGGATTC
                                                  MetAspSe
                                                   o  o  o

351  ATACCTGCTG ATGTGGGGAC TGCTCACGTT CATCATGGTG CCTGGCTGCC
     rTyrLeuLeu MetTrpGlyL euLeuThrPh eIleMetVal ProGlyCysG
      o  o  o    o  o  o   o  o  o  o  o  o  o    o  o  o

         SacI     BglII                           BstEII
401  AGGCAGAGCT CAGCAGATCT GCTGAATGAA TGAGGGGGCA GGTGACCTTC
     lnAlaGluLe uSerArgSer AlaGlu
     o  o

451  AGACCTTGGC ACTGGAGGTG GCCCGGCAGA AGCGCGGCAT CGTGGATCAG

501  TGCTGCACCA GCATCTGCTC TCTCTACCAA CTGGAGAACT ACTGCAACTA

551  GGCCCACCAC TACCCTGTCC ACCCCTCTGC AATGAATAAA ACCTTTGAAA

601  GAGCACTACA AGTTGTGTGT ACATGCGTGC GTGTGCATAT GTGGTGCGGG

651  GGGAACATGA GTGGGGTCGG CTGGAGTGGT CGCGGCTTAA TCTATCTGGC

                                EcoRI
701  GATGATAAGC TGTCAAACAT GAGAATTCTT GAAGACGAAA GGGCCTCGTG

751  ATACGCCTAT TTTTATAGGT TAATGTCATG ATAATAATGG TTTCTTAGAC

801  GTCAGGTGGC ACTTTTCGGG GAAATGTGCG CGGAACCCCT ATTTGTTTAT

851  TTTTCTAAAT ACATTCAAAT ATGTATCCGC TCATGAGACA ATAACCCTGA

901  TAAATGCTTC AATAATATTG AAAAAGGAAG AGTATGAGTA TTCAACATTT

951  CCGTGTCGCC CTTATTCCCT TTTTTGCGGC ATTTTGCCTT CCTGTTTTTG
```

Fig. 51 (cont.)

1001 CTCACCCAGA AACGCTGGTG AAAGTAAAAG ATGCTGAAGA TCAGTTGGGT
1051 GCACGAGTGG GTTACATCGA ACTGGATCTC AACAGCGGTA AGATCCTTGA
1101 GAGTTTTCGC CCCGAAGAAC GTTTTCCAAT GATGAGCACT TTTAAAGTTC
1151 TGCTATGTGG CGCGGTATTA TCCCGTGTTG ACGCCGGGCA AGAGCAACTC
1201 GGTCGCCGCA TACACTATTC TCAGAATGAC TTGGTTGAGT ACTCACCAGT
1251 CACAGAAAAG CATCTTACGG ATGGCATGAC AGTAAGAGAA TTATGCAGTG
1301 CTGCCATAAC CATGAGTGAT AACACTGCGG CCAACTTACT TCTGACAACG

PvuI
1351 ATCGGAGGAC CGAAGGAGCT AACCGCTTTT TTGCACAACA TGGGGGATCA
1401 TGTAACTCGC CTTGATCGTT GGGAACCGGA GCTGAATGAA GCCATACCAA
1451 ACGACGAGCG TGACACCACG ATGCCTGCAG CAATGGCAAC AACGTTGCGC
1501 AAACTATTAA CTGGCGAACT ACTTACTCTA GCTTCCCGGC AACAATTAAT
1551 AGACTGGATG GAGGCGGATA AAGTTGCAGG ACCACTTCTG CGCTCGGCCC
1601 TTCCGGCTGG CTGGTTTATT GCTGATAAAT CTGGAGCCGG TGAGCGTGGG
1651 TCTCGCGGTA TCATTGCAGC ACTGGGGCCA GATGGTAAGC CCTCCCGTAT
1701 CGTAGTTATC TACACGACGG GGAGTCAGGC AACTATGGAT GAACGAAATA
1751 GACAGATCGC TGAGATAGGT GCCTCACTGA TTAAGCATTG GTAACTGTCA
1801 GACCAAGTTT ACTCATATAT ACTTTAGATT GATTTAAAAC TTCATTTTTA
1851 ATTTAAAAGC GTAATCTGCT GCTTGCAAAC AAAAAAACCA CCGCTACCAG
1901 CGGTGGTTTG TTTGCCGGAT CAAGAGCTAC CAACTCTTTT TCCGAAGGTA
1951 ACTGGCTTCA GCAGAGCGCA GATACCAAAT ACTGTCCTTC TAGTGTAGCC
2001 GTAGTTAGGC CACCACTTCA AGAACTCTGT AGCACCGCCT ACATACCTCG
2051 CTCTGCTAAT CCTGTTACCA GTGGCTGCTG CCAGTGGCGA TAAGTCGTGT
2101 CTTACCGGGT TGGACTCAAG ACGATAGTTA CCGGATAAGG CGCAGCGGTC
2151 GGGCTGAACG GGGGGTTCGT GCACACAGCC CAGCTTGGAG CGAACGACCT

Fig. 51 (cont.)

2201 ACACCGAACT GAGATACCTA CAGCGTGAGC ATTGAGAAAG CGCCACGCTT
2251 CCCGAAGGGA GAAAGGCGGA CAGGTATCCG GTAAGCGGCA GGGTCGGAAC
2301 AGGAGAGCGC ACGAGGGAGC TTCCAGGGGG AAACGCCTGG TATCTTTATA
2351 GTCCTGTCGG GTTTCGCCAC CTCTGACTTG AGCGTCGATT TTTGTGATGC
2401 TCGTCAGGGG GGCGGAGCCT ATGGAAAAAC GCCAGCAACG GATGCGCCGC
2451 GTGCGGCTGC TGGAGATGGC GGACGCGATG GATATGTTCT GCCAAGGGTT
2501 GGTTTGCGCA TTCACAGTTC TCCGCAAGAA TTGATTGGCT CCAATTCTTG
2551 GAGTGGTGAA TCCGTTAGCG AGGTGCCGCC GGCTTCCATT CAGGTCGAGG
2601 TGGCCCGGCT CCATGCACCG CGACGCAACG CGGGGAGGCA GACAAGGTAT
2651 AGGGCGGCGC CTACAATCCA TGCCAACCCG TTCCATGTGC TCGCCGAGGC
2701 GGCATAAATC GCCGTGACGA TCAGCGGTCC AGTGATCGAA GTTAGGCTGG
2751 TAAGAGCCGC GAGCGATCCT TGAAGCTGTC CCTGATGGTC GTCATCTACC
2801 TGCCTGGACA GCATGGCCTG CAACGCGGGC ATCCCGATGC CGCCGGAAGC
2851 GAGAAGAATC ATAATGGGGA AGGCCATCCA GCCTCGCGTC GAGCTTTTTG
2901 CAAAAGCCTA GGCCTCCAAA AAAGCCTCCT CACTACTTCT GGAATAGCTC
2951 AGAGGCCGAG GCGGCCTCGG CCTCTGCATA AATAAAAAAA ATTAGTCAGC

NcoI
3001 CATGGGGCGG AGAATGGGCG GAACTGGGCG GAGTTAGGGG CGGGATGGGC

SphI
3051 GGAGTTAGGG GCGGGACTAT GGTTGCTGAC TAATTGAGAT GCATGCAAGG
3101 AGATGGCGCC CAACAGTCCC CCGGCCACGG CGCCTGCCAC CATACCCACG
3151 CCGAAACAAG CGCTCATGAG CCCGAAGTGG CGAGCCCGAT CTTCCCCATC
3201 GGTGATGTCG GCGATATAGG CGCCAGCAAC CGCACCTGTG GCGCCGGTGA
3251 TGCCGGCCAC GATGCGTCCG GCGTAGA

Fig. 52

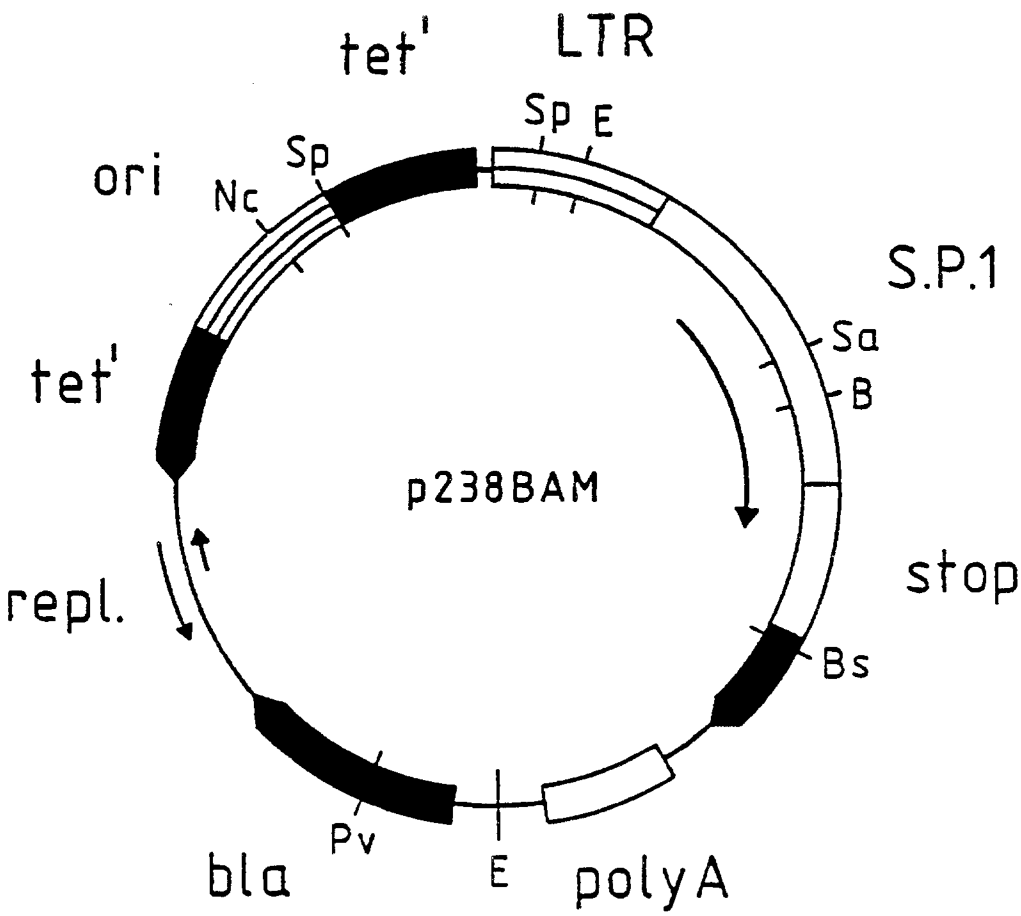
tet'
LTR
Sp
E
ori
Nc
Sp
S.P.1
Sa
B
tet'
p238BAM
stop
repl.
Bs
bla
Pv
E
polyA

Fig. 53

```
                                                                      SphI
  1 GGATCTCAGG ATATAGTAGT TTCGCTTTTG CATAGGGAGG GGGAAATGTA

 51 GCCTTATGCA ATACTCTTGT AGTCTTGCAA CATGCTTATG TAACGATGAG

101 TTAGCAACAT GCCTTACAAG GAGAGAAAAA GCACCGTGCA TGCCGATTGG

151 TGGAAGTAAG GTGGTA.GAT CGTGCCTTAT TAGGAAGGCA ACAGACGGGT

                                  EcoRI
201 CTGACATGGA TTGGACGAAC CACCGAATTC GCATTGCAGA GAGTATTGTA

                                   *
251 TTTAAGTGCC TAGCTCGATA CAATAAACGC CATTTGACCA TTCACCACAT

301 TGGTGTGCAC CTCAAGCTCG GCTGGTCCCA AGGGTCAGGA AGATGGATTC
                                                 MetAspSe
                                                  o  o  o

351 ATACCTGCTG ATGTGGGGAC TGCTCACGTT CATCATGGTG CCTGGCTGCC
    rTyrLeuLeu MetTrpGlyL euLeuThrPh eIleMetVal ProGlyCysG
     o  o  o    o  o  o    o  o  o  o  o  o  o   o  o  o

        SacI     BamHI                             BstEII
401 AGGCAGAGCT CAGCGGATCC GCTGAATGAA TGAGGGGGCA GGTGACCTTC
    lnAlaGluLe uSerGlySer AlaGlu
    o  o

451 AGACCTTGGC ACTGGAGGTG GCCCGGCAGA AGCGCGGCAT CGTGGATCAG

501 TGCTGCACCA GCATCTGCTC TCTCTACCAA CTGGAGAACT ACTGCAACTA

551 GGCCCACCAC TACCCTGTCC ACCCCTCTGC AATGAATAAA ACCTTTGAAA

601 GAGCACTACA AGTTGTGTGT ACATGCGTGC GTGTGCATAT GTGGTGCGGG

651 GGGAACATGA GTGGGGTCGG CTGGAGTGGT CGCGGCTTAA TCTATCTGGC

                                EcoRI
701 GATGATAAGC TGTCAAACAT GAGAATTCTT GAAGACGAAA GGGCCTCGTG

751 ATACGCCTAT TTTTATAGGT TAATGTCATG ATAATAATGG TTTCTTAGAC

801 GTCAGGTGGC ACTTTTCGGG GAAATGTGCG CGGAACCCCT ATTTGTTTAT

851 TTTTCTAAAT ACATTCAAAT ATGTATCCGC TCATGAGACA ATAACCCTGA

901 TAAATGCTTC AATAATATTG AAAAAGGAAG AGTATGAGTA TTCAACATTT

951 CCGTGTCGCC CTTATTCCCT TTTTTGCGGC ATTTTGCCTT CCTGTTTTTG
```

Fig. 53 (cont.)

1001 CTCACCCAGA AACGCTGGTG AAAGTAAAAG ATGCTGAAGA TCAGTTGGGT

1051 GCACGAGTGG GTTACATCGA ACTGGATCTC AACAGCGGTA AGATCCTTGA

1101 GAGTTTTCGC CCCGAAGAAC GTTTTCCAAT GATGAGCACT TTTAAAGTTC

1151 TGCTATGTGG CGCGGTATTA TCCCGTGTTG ACGCCGGGCA AGAGCAACTC

1201 GGTCGCCGCA TACACTATTC TCAGAATGAC TTGGTTGAGT ACTCACCAGT

1251 CACAGAAAAG CATCTTACGG ATGGCATGAC AGTAAGAGAA TTATGCAGTG

1301 CTGCCATAAC CATGAGTGAT AACACTGCGG CCAACTTACT TCTGACAACG

PvuI

1351 ATCGGAGGAC CGAAGGAGCT AACCGCTTTT TTGCACAACA TGGGGGATCA

1401 TGTAACTCGC CTTGATCGTT GGGAACCGGA GCTGAATGAA GCCATACCAA

1451 ACGACGAGCG TGACACCACG ATGCCTGCAG CAATGGCAAC AACGTTGCGC

1501 AAACTATTAA CTGGCGAACT ACTTACTCTA GCTTCCCGGC AACAATTAAT

1551 AGACTGGATG GAGGCGGATA AAGTTGCAGG ACCACTTCTG CGCTCGGCCC

1601 TTCCGGCTGG CTGGTTTATT GCTGATAAAT CTGGAGCCGG TGAGCGTGGG

1651 TCTCGCGGTA TCATTGCAGC ACTGGGGCCA GATGGTAAGC CCTCCCGTAT

1701 CGTAGTTATC TACACGACGG GGAGTCAGGC AACTATGGAT GAACGAAATA

1751 GACAGATCGC TGAGATAGGT GCCTCACTGA TTAAGCATTG GTAACTGTCA

1801 GACCAAGTTT ACTCATATAT ACTTTAGATT GATTTAAAAC TTCATTTTTA

1851 ATTTAAAAGC GTAATCTGCT GCTTGCAAAC AAAAAAACCA CCGCTACCAG

1901 CGGTGGTTTG TTTGCCGGAT CAAGAGCTAC CAACTCTTTT TCCGAAGGTA

1951 ACTGGCTTCA GCAGAGCGCA GATACCAAAT ACTGTCCTTC TAGTGTAGCC

2001 GTAGTTAGGC CACCACTTCA AGAACTCTGT AGCACCGCCT ACATACCTCG

2051 CTCTGCTAAT CCTGTTACCA GTGGCTGCTG CCAGTGGCGA TAAGTCGTGT

2101 CTTACCGGGT TGGACTCAAG ACGATAGTTA CCGGATAAGG CGCAGCGGTC

2151 GGGCTGAACG GGGGGTTCGT GCACACAGCC CAGCTTGGAG CGAACGACCT

Fig. 53 (cont.)

2201 ACACCGAACT GAGATACCTA CAGCGTGAGC ATTGAGAAAG CGCCACGCTT

2251 CCCGAAGGGA GAAAGGCGGA CAGGTATCCG GTAAGCGGCA GGGTCGGAAC

2301 AGGAGAGCGC ACGAGGGAGC TTCCAGGGGG AAACGCCTGG TATCTTTATA

2351 GTCCTGTCGG GTTTCGCCAC CTCTGACTTG AGCGTCGATT TTTGTGATGC

2401 TCGTCAGGGG GGCGGAGCCT ATGGAAAAAC GCCAGCAACG GATGCGCCGC

2451 GTGCGGCTGC TGGAGATGGC GGACGCGATG GATATGTTCT GCCAAGGGTT

2501 GGTTTGCGCA TTCACAGTTC TCCGCAAGAA TTGATTGGCT CCAATTCTTG

2551 GAGTGGTGAA TCCGTTAGCG AGGTGCCGCC GGCTTCCATT CAGGTCGAGG

2601 TGGCCCGGCT CCATGCACCG CGACGCAACG CGGGGAGGCA GACAAGGTAT

2651 AGGGCGGCGC CTACAATCCA TGCCAACCCG TTCCATGTGC TCGCCGAGGC

2701 GGCATAAATC GCCGTGACGA TCAGCGGTCC AGTGATCGAA GTTAGGCTGG

2751 TAAGAGCCGC GAGCGATCCT TGAAGCTGTC CCTGATGGTC GTCATCTACC

2801 TGCCTGGACA GCATGGCCTG CAACGCGGGC ATCCCGATGC CGCCGGAAGC

2851 GAGAAGAATC ATAATGGGGA AGGCCATCCA GCCTCGCGTC GAGCTTTTTG

2901 CAAAAGCCTA GGCCTCCAAA AAAGCCTCCT CACTACTTCT GGAATAGCTC

2951 AGAGGCCGAG GCGGCCTCGG CCTCTGCATA AATAAAAAAA ATTAGTCAGC

NcoI

3001 CATGGGGCGG AGAATGGGCG GAACTGGGCG GAGTTAGGGG CGGGATGGGC

SphI

3051 GGAGTTAGGG GCGGGACTAT GGTTGCTGAC TAATTGAGAT GCATGCAAGG

3101 AGATGGCGCC CAACAGTCCC CCGGCCACGG CGCCTGCCAC CATACCCACG

3151 CCGAAACAAG CGCTCATGAG CCCGAAGTGG CGAGCCCGAT CTTCCCCATC

3201 GGTGATGTCG GCGATATAGG CGCCAGCAAC CGCACCTGTG GCGCCGGTGA

3251 TGCCGGCCAC GATGCGTCCG GCGTAGA

Fig. 54

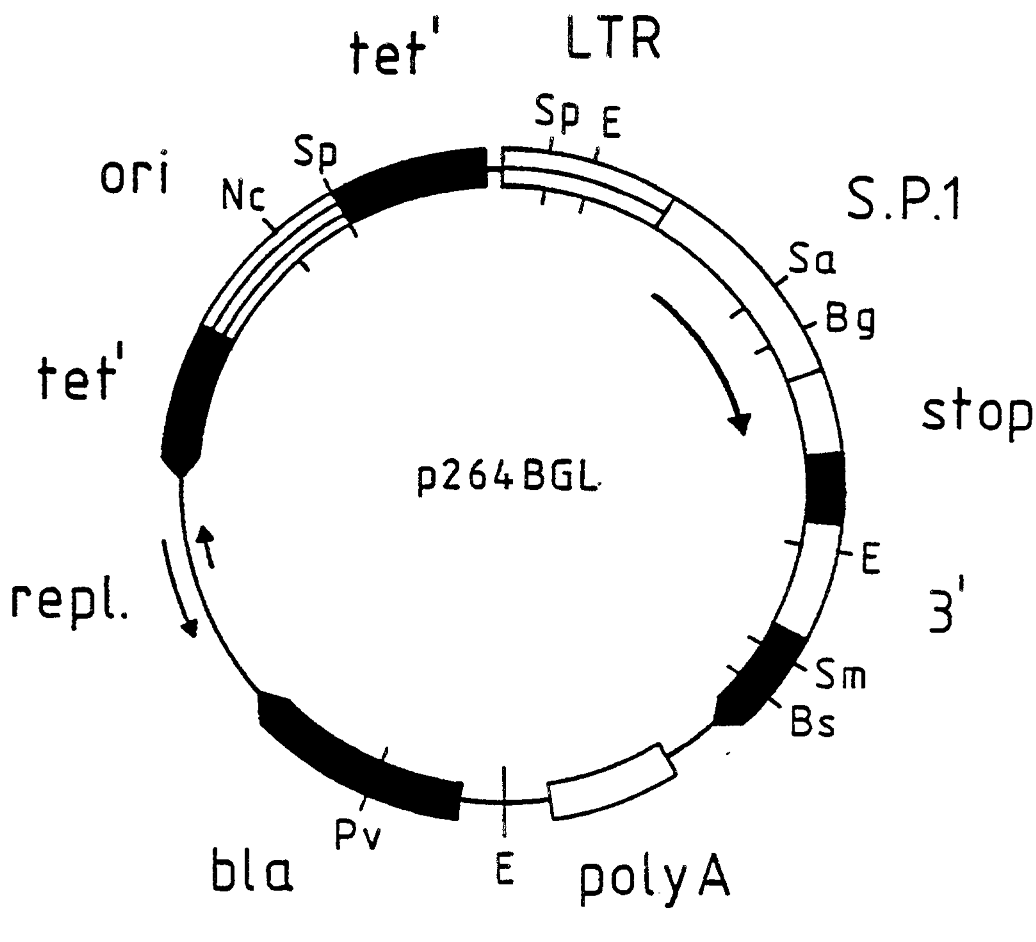
tet'
LTR
Sp
E
ori
Nc
Sp
S.P.1
Sa
Bg
tet'
stop
p264BGL
E
repl.
3'
Sm
Bs
Pv
bla
E
polyA

Fig. 55

```
                                                            SphI
1    GGATCTCAGG ATATAGTAGT TTCGCTTTTG CATAGGGAGG GGGAAATGTA

51   GCCTTATGCA ATACTCTTGT AGTCTTGCAA CATGCTTATG TAACGATGAG

                                                SphI
101  TTAGCAACAT GCCTTACAAG GAGAGAAAAA GCACCGTGCA TGCCGATTGG

151  TGGAAGTAAG GTGGTA.GAT CGTGCCTTAT TAGGAAGGCA ACAGACGGGT

                                  EcoRI
201  CTGACATGGA TTGGACGAAC CACCGAATTC GCATTGCAGA GAGTATTGTA

                                        *
251  TTTAAGTGCC TAGCTCGATA CAATAAACGC CATTTGACCA TTCACCACAT

301  TGGTGTGCAC CTCAAGCTCG GCTGGTCCCA AGGGTCAGGA AGATGGATTC
                                                   MetAspSe
                                                    o  o  o

351  ATACCTGCTG ATGTGGGGAC TGCTCACGTT CATCATGGTG CCTGGCTGCC
     rTyrLeuLeu MetTrpGlyL euLeuThrPh eIleMetVal ProGlyCysG
      o  o  o    o  o  o   o  o  o  o  o  o  o    o  o  o

           SacI      BglII
401  AGGCAGAGCT CAGCAGATCT GCTGAATGAA TGAGATCCGC TTCCTGCCCC
     lnAlaGluLe uSerArgSer AlaGlu
     o  o

451  TGCTGGCCCT GCTCATCCTC TGGGAGCCCC GCCCTGCCCA GGCTTTTGTC

501  AAACAGCACC TTTGTGGTTC TCACTTGGTG GAAGCTCTCT ACCTGGTGTG

551  TGGGGAGCGT GGATTCTTCT ACACACCCAT GTCCCGCCGC GAAGTGGAGG

                                  EcoRI
601  ACCCACAAGG TAAGCTCTGC TCCTGAATTC TATCCCAAGT GCTAACTACC

651  CTGTTTGTCT TTCACCCTTG AGACCTTGTA AATTGTGCCC TAGGTGTGGA

701  GGGTCTCAGG CTAACCAGTG GGGGGCACAT TTCTGTGGGC AGCTAGACAT

751  ATGTAAACAT GGTAGCTGCC AAGAAGGAGT GAGAATCCTT CCTTAAGTCT

801  CCTAGGTGGT GACGGGTGGC TAGGCCCCAG GATAGGTACC CATTTGGGGA

851  CCCCATAGAG CACCGCACCG ACCGAGGGAT GGTAACAGGA TGTGTAGGTT

901  TTGGAGGCCC ATATGTCCAT TCATGACCAG TGACTTGTCT CACAGCCATG

951  CAACCCTTGC CTCCTGTGCT GACTTAGCAG GGGATAAAGT GAGAGAAAGC
```

Fig. 55 (cont.)

1001 CTGGGCTAAT CGGGGGGTCG CTCGGCTCCT CCTAACTGGA TTGTCCTATG

1051 TGTCTTTGCT TCTGTGCTGC TGATGCTCTG CCCTGTGCTG ACATGACCTC

SmaI BstEII

1101 CCTGGCAGTG GCACAACTGG AGCTGGGTGG AGGCCCGGGG GCAGGTGACC

1151 TTCAGACCTT GGCACTGGAG GTGGCCCGGC AGAAGCGCGG CATCGTGGAT

1201 CAGTGCTGCA CCAGCATCTG CTCTCTCTAC CAACTGGAGA ACTACTGCAA

1251 CTAGGCCCAC CACTACCCTG TCCACCCCTC TGCAATGAAT AAAACCTTTG

1301 AAAGAGCACT ACAAGTTGTG TGTACATGCG TGCGTGTGCA TATGTGGTGC

1351 GGGGGGAACA TGAGTGGGGT CGGCTGGAGT GGTCGCGGCT TAATCTATCT

EcoRI

1401 GGCGATGATA AGCTGTCAAA CATGAGAATT CTTGAAGACG AAAGGGCCTC

1451 GTGATACGCC TATTTTTATA GGTTAATGTC ATGATAATAA TGGTTTCTTA

1501 GACGTCAGGT GGCACTTTTC GGGGAAATGT GCGCGGAACC CCTATTTGTT

1551 TATTTTTCTA AATACATTCA AATATGTATC CGCTCATGAG ACAATAACCC

1601 TGATAAATGC TTCAATAATA TTGAAAAAGG AAGAGTATGA GTATTCAACA

1651 TTTCCGTGTC GCCCTTATTC CCTTTTTTGC GGCATTTTGC CTTCCTGTTT

1701 TTGCTCACCC AGAAACGCTG GTGAAAGTAA AAGATGCTGA AGATCAGTTG

1751 GGTGCACGAG TGGGTTACAT CGAACTGGAT CTCAACAGCG GTAAGATCCT

1801 TGAGAGTTTT CGCCCCGAAG AACGTTTTCC AATGATGAGC ACTTTTAAAG

1851 TTCTGCTATG TGGCGCGGTA TTATCCCGTG TTGACGCCGG GCAAGAGCAA

1901 CTCGGTCGCC GCATACACTA TTCTCAGAAT GACTTGGTTG AGTACTCACC

1951 AGTCACAGAA AAGCATCTTA CGGATGGCAT GACAGTAAGA GAATTATGCA

2001 GTGCTGCCAT AACCATGAGT GATAACACTG CGGCCAACTT ACTTCTGACA

PvuI

2051 ACGATCGGAG GACCGAAGGA GCTAACCGCT TTTTTGCACA ACATGGGGGA

2101 TCATGTAACT CGCCTTGATC GTTGGGAACC GGAGCTGAAT GAAGCCATAC

2151 CAAACGACGA GCGTGACACC ACGATGCCTG CAGCAATGGC AACAACGTTG

Fig. 55 (cont.)

2201 CGCAAACTAT TAACTGGCGA ACTACTTACT CTAGCTTCCC GGCAACAATT
2251 AATAGACTGG ATGGAGGCGG ATAAAGTTGC AGGACCACTT CTGCGCTCGG
2301 CCCTTCCGGC TGGCTGGTTT ATTGCTGATA AATCTGGAGC CGGTGAGCGT
2351 GGGTCTCGCG GTATCATTGC AGCACTGGGG CCAGATGGTA AGCCCTCCCG
2401 TATCGTAGTT ATCTACACGA CGGGGAGTCA GGCAACTATG GATGAACGAA
2451 ATAGACAGAT CGCTGAGATA GGTGCCTCAC TGATTAAGCA TTGGTAACTG
2501 TCAGACCAAG TTTACTCATA TATACTTTAG ATTGATTTAA AACTTCATTT
2551 TTAATTTAAA AGCGTAATCT GCTGCTTGCA AACAAAAAAA CCACCGCTAC
2601 CAGCGGTGGT TTGTTTGCCG GATCAAGAGC TACCAACTCT TTTTCCGAAG
2651 GTAACTGGCT TCAGCAGAGC GCAGATACCA AATACTGTCC TTCTAGTGTA
2701 GCCGTAGTTA GGCCACCACT TCAAGAACTC TGTAGCACCG CCTACATACC
2751 TCGCTCTGCT AATCCTGTTA CCAGTGGCTG CTGCCAGTGG CGATAAGTCG
2801 TGTCTTACCG GGTTGGACTC AAGACGATAG TTACCGGATA AGGCGCAGCG
2851 GTCGGGCTGA ACGGGGGGTT CGTGCACACA GCCCAGCTTG GAGCGAACGA
2901 CCTACACCGA ACTGAGATAC CTACAGCGTG AGCATTGAGA AAGCGCCACG
2951 CTTCCCGAAG GGAGAAAGGC GGACAGGTAT CCGGTAAGCG GCAGGGTCGG
3001 AACAGGAGAG CGCACGAGGG AGCTTCCAGG GGGAAACGCC TGGTATCTTT
3051 ATAGTCCTGT CGGGTTTCGC CACCTCTGAC TTGAGCGTCG ATTTTTGTGA
3101 TGCTCGTCAG GGGGGCGGAG CCTATGGAAA AACGCCAGCA ACGGATGCGC
3151 CGCGTGCGGC TGCTGGAGAT GGCGGACGCG ATGGATATGT TCTGCCAAGG
3201 GTTGGTTTGC GCATTCACAG TTCTCCGCAA GAATTGATTG GCTCCAATTC
3251 TTGGAGTGGT GAATCCGTTA GCGAGGTGCC GCCGGCTTCC ATTCAGGTCG
3301 AGGTGGCCCG GCTCCATGCA CCGCGACGCA ACGCGGGGAG GCAGACAAGG
3351 TATAGGGCGG CGCCTACAAT CCATGCCAAC CCGTTCCATG TGCTCGCCGA
3401 GGCGGCATAA ATCGCCGTGA CGATCAGCGG TCCAGTGATC GAAGTTAGGC
3451 TGGTAAGAGC CGCGAGCGAT CCTTGAAGCT GTCCCTGATG GTCGTCATCT

Fig. 55 (cont.)

3501 ACCTGCCTGG ACAGCATGGC CTGCAACGCG GGCATCCCGA TGCCGCCGGA

3551 AGCGAGAAGA ATCATAATGG GGAAGGCCAT CCAGCCTCGC GTCGAGCTTT

3601 TTGCAAAAGC CTAGGCCTCC AAAAAAGCCT CCTCACTACT TCTGGAATAG

3651 CTCAGAGGCC GAGGCGGCCT CGGCCTCTGC ATAAATAAAA AAAATTAGTC

NcoI

3701 AGCCATGGGG CGGAGAATGG GCGGAACTGG GCGGAGTTAG GGGCGGGATG

SphI

3751 GGCGGAGTTA GGGGCGGGAC TATGGTTGCT GACTAATTGA GATGCATGCA

3801 AGGAGATGGC GCCCAACAGT CCCCCGGCCA CGGCGCCTGC CACCATACCC

3851 ACGCCGAAAC AAGCGCTCAT GAGCCCGAAG TGGCGAGCCC GATCTTCCCC

3901 ATCGGTGATG TCGGCGATAT AGGCGCCAGC AACCGCACCT GTGGCGCCGG

3951 TGATGCCGGC CACGATGCGT CCGGCGTAGA

Fig. 56

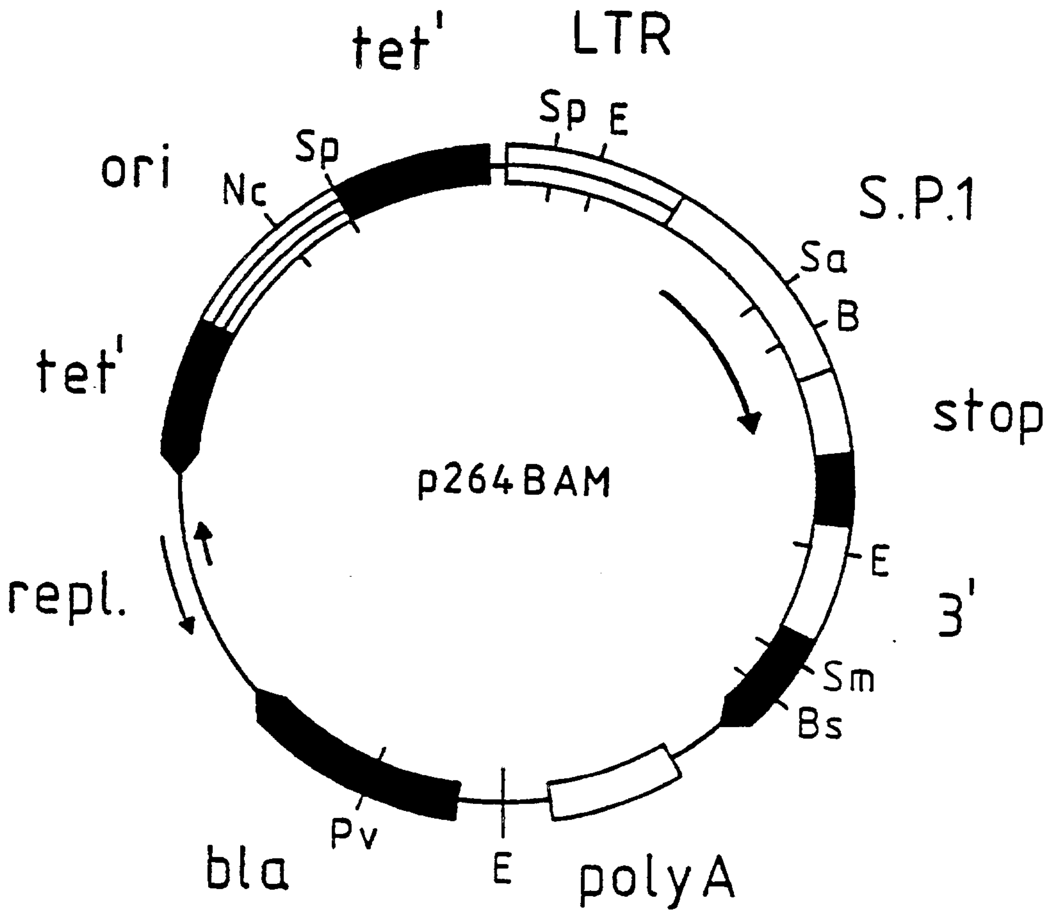
tet'
LTR
ori
Sp
Nc
Sp
E
S.P.1
Sa
B
tet'
stop
p264BAM
E
repl.
3'
Sm
Bs
bla
Pv
E
polyA

Fig. 57

```
                                                          SphI
1    GGATCTCAGG ATATAGTAGT TTCGCTTTTG CATAGGGAGG GGGAAATGTA

51   GCCTTATGCA ATACTCTTGT AGTCTTGCAA CATGCTTATG TAACGATGAG

                                                  SphI
101  TTAGCAACAT GCCTTACAAG GAGAGAAAAA GCACCGTGCA TGCCGATTGG

151  TGGAAGTAAG GTGGTA.GAT CGTGCCTTAT TAGGAAGGCA ACAGACGGGT

                                 EcoRI
201  CTGACATGGA TTGGACGAAC CACCGAATTC GCATTGCAGA GAGTATTGTA

                                   *
251  TTTAAGTGCC TAGCTCGATA CAATAAACGC CATTTGACCA TTCACCACAT

301  TGGTGTGCAC CTCAAGCTCG GCTGGTCCCA AGGGTCAGGA AGATGGATTC
                                                 MetAspSe
                                                  o  o  o

351  ATACCTGCTG ATGTGGGGAC TGCTCACGTT CATCATGGTG CCTGGCTGCC
     rTyrLeuLeu MetTrpGlyL euLeuThrPh eIleMetVal ProGlyCysG
      o  o  o    o  o  o   o  o  o  o  o  o  o   o  o  o

          SacI     BamHI
401  AGGCAGAGCT CAGCGGATCC GCTGAATGAA TGAGATCCGC TTCCTGCCCC
     lnAlaGluLe uSerGlySer AlaGlu
     o  o

451  TGCTGGCCCT GCTCATCCTC TGGGAGCCCC GCCCTGCCCA GGCTTTTGTC

501  AAACAGCACC TTTGTGGTTC TCACTTGGTG GAAGCTCTCT ACCTGGTGTG

551  TGGGGAGCGT GGATTCTTCT ACACACCCAT GTCCCGCCGC GAAGTGGAGG

                                 EcoRI
601  ACCCACAAGG TAAGCTCTGC TCCTGAATTC TATCCCAAGT GCTAACTACC

651  CTGTTTGTCT TTCACCCTTG AGACCTTGTA AATTGTGCCC TAGGTGTGGA

701  GGGTCTCAGG CTAACCAGTG GGGGGCACAT TTCTGTGGGC AGCTAGACAT

751  ATGTAAACAT GGTAGCTGCC AAGAAGGAGT GAGAATCCTT CCTTAAGTCT

801  CCTAGGTGGT GACGGGTGGC TAGGCCCCAG GATAGGTACC CATTTGGGGA

851  CCCCATAGAG CACCGCACCG ACCGAGGGAT GGTAACAGGA TGTGTAGGTT

901  TTGGAGGCCC ATATGTCCAT TCATGACCAG TGACTTGTCT CACAGCCATG

951  CAACCCTTGC CTCCTGTGCT GACTTAGCAG GGGATAAAGT GAGAGAAAGC
```

Fig. 57 (cont.)

```
1001  CTGGGCTAAT CGGGGGGTCG CTCGGCTCCT CCTAACTGGA TTGTCCTATG
1051  TGTCTTTGCT TCTGTGCTGC TGATGCTCTG CCCTGTGCTG ACATGACCTC
                                           SmaI      BstEII
1101  CCTGGCAGTG GCACAACTGG AGCTGGGTGG AGGCCCGGGG GCAGGTGACC
1151  TTCAGACCTT GGCACTGGAG GTGGCCCGGC AGAAGCGCGG CATCGTGGAT
1201  CAGTGCTGCA CCAGCATCTG CTCTCTCTAC CAACTGGAGA ACTACTGCAA
1251  CTAGGCCCAC CACTACCCTG TCCACCCCTC TGCAATGAAT AAAACCTTTG
1301  AAAGAGCACT ACAAGTTGTG TGTACATGCG TGCGTGTGCA TATGTGGTGC
1351  GGGGGGAACA TGAGTGGGGT CGGCTGGAGT GGTCGCGGCT TAATCTATCT
                            EcoRI
1401  GGCGATGATA AGCTGTCAAA CATGAGAATT CTTGAAGACG AAAGGGCCTC
1451  GTGATACGCC TATTTTTATA GGTTAATGTC ATGATAATAA TGGTTTCTTA
1501  GACGTCAGGT GGCACTTTTC GGGGAAATGT GCGCGGAACC CCTATTTGTT
1551  TATTTTTCTA AATACATTCA AATATGTATC CGCTCATGAG ACAATAACCC
1601  TGATAAATGC TTCAATAATA TTGAAAAAGG AAGAGTATGA GTATTCAACA
1651  TTTCCGTGTC GCCCTTATTC CCTTTTTTGC GGCATTTTGC CTTCCTGTTT
1701  TTGCTCACCC AGAAACGCTG GTGAAAGTAA AAGATGCTGA AGATCAGTTG
1751  GGTGCACGAG TGGGTTACAT CGAACTGGAT CTCAACAGCG GTAAGATCCT
1801  TGAGAGTTTT CGCCCCGAAG AACGTTTTCC AATGATGAGC ACTTTTAAAG
1851  TTCTGCTATG TGGCGCGGTA TTATCCCGTG TTGACGCCGG GCAAGAGCAA
1901  CTCGGTCGCC GCATACACTA TTCTCAGAAT GACTTGGTTG AGTACTCACC
1951  AGTCACAGAA AAGCATCTTA CGGATGGCAT GACAGTAAGA GAATTATGCA
2001  GTGCTGCCAT AACCATGAGT GATAACACTG CGGCCAACTT ACTTCTGACA
         PvuI
2051  ACGATCGGAG GACCGAAGGA GCTAACCGCT TTTTTGCACA ACATGGGGGA
2101  TCATGTAACT CGCCTTGATC GTTGGGAACC GGAGCTGAAT GAAGCCATAC
2151  CAAACGACGA GCGTGACACC ACGATGCCTG CAGCAATGGC AACAACGTTG
```

Fig. 57 (cont.)

| | | | | | |
|---|---|---|---|---|---|
| 2201 | CGCAAACTAT | TAACTGGCGA | ACTACTTACT | CTAGCTTCCC | GGCAACAATT |
| 2251 | AATAGACTGG | ATGGAGGCGG | ATAAAGTTGC | AGGACCACTT | CTGCGCTCGG |
| 2301 | CCCTTCCGGC | TGGCTGGTTT | ATTGCTGATA | AATCTGGAGC | CGGTGAGCGT |
| 2351 | GGGTCTCGCG | GTATCATTGC | AGCACTGGGG | CCAGATGGTA | AGCCCTCCCG |
| 2401 | TATCGTAGTT | ATCTACACGA | CGGGGAGTCA | GGCAACTATG | GATGAACGAA |
| 2451 | ATAGACAGAT | CGCTGAGATA | GGTGCCTCAC | TGATTAAGCA | TTGGTAACTG |
| 2501 | TCAGACCAAG | TTTACTCATA | TATACTTTAG | ATTGATTTAA | AACTTCATTT |
| 2551 | TTAATTTAAA | AGCGTAATCT | GCTGCTTGCA | AACAAAAAAA | CCACCGCTAC |
| 2601 | CAGCGGTGGT | TTGTTTGCCG | GATCAAGAGC | TACCAACTCT | TTTTCCGAAG |
| 2651 | GTAACTGGCT | TCAGCAGAGC | GCAGATACCA | AATACTGTCC | TTCTAGTGTA |
| 2701 | GCCGTAGTTA | GGCCACCACT | TCAAGAACTC | TGTAGCACCG | CCTACATACC |
| 2751 | TCGCTCTGCT | AATCCTGTTA | CCAGTGGCTG | CTGCCAGTGG | CGATAAGTCG |
| 2801 | TGTCTTACCG | GGTTGGACTC | AAGACGATAG | TTACCGGATA | AGGCGCAGCG |
| 2851 | GTCGGGCTGA | ACGGGGGGTT | CGTGCACACA | GCCCAGCTTG | GAGCGAACGA |
| 2901 | CCTACACCGA | ACTGAGATAC | CTACAGCGTG | AGCATTGAGA | AAGCGCCACG |
| 2951 | CTTCCCGAAG | GGAGAAAGGC | GGACAGGTAT | CCGGTAAGCG | GCAGGGTCGG |
| 3001 | AACAGGAGAG | CGCACGAGGG | AGCTTCCAGG | GGGAAACGCC | TGGTATCTTT |
| 3051 | ATAGTCCTGT | CGGGTTTCGC | CACCTCTGAC | TTGAGCGTCG | ATTTTTGTGA |
| 3101 | TGCTCGTCAG | GGGGGCGGAG | CCTATGGAAA | AACGCCAGCA | ACGGATGCGC |
| 3151 | CGCGTGCGGC | TGCTGGAGAT | GGCGGACGCG | ATGGATATGT | TCTGCCAAGG |
| 3201 | GTTGGTTTGC | GCATTCACAG | TTCTCCGCAA | GAATTGATTG | GCTCCAATTC |
| 3251 | TTGGAGTGGT | GAATCCGTTA | GCGAGGTGCC | GCCGGCTTCC | ATTCAGGTCG |
| 3301 | AGGTGGCCCG | GCTCCATGCA | CCGCGACGCA | ACGCGGGGAG | GCAGACAAGG |
| 3351 | TATAGGGCGG | CGCCTACAAT | CCATGCCAAC | CCGTTCCATG | TGCTCGCCGA |
| 3401 | GGCGGCATAA | ATCGCCGTGA | CGATCAGCGG | TCCAGTGATC | GAAGTTAGGC |
| 3451 | TGGTAAGAGC | CGCGAGCGAT | CCTTGAAGCT | GTCCCTGATG | GTCGTCATCT |

Fig. 57 (cont.)

3501 ACCTGCCTGG ACAGCATGGC CTGCAACGCG GGCATCCCGA TGCCGCCGGA

3551 AGCGAGAAGA ATCATAATGG GGAAGGCCAT CCAGCCTCGC GTCGAGCTTT

3601 TTGCAAAAGC CTAGGCCTCC AAAAAAGCCT CCTCACTACT TCTGGAATAG

3651 CTCAGAGGCC GAGGCGGCCT CGGCCTCTGC ATAAATAAAA AAAATTAGTC

NcoI

3701 AGCCATGGGG CGGAGAATGG GCGGAACTGG GCGGAGTTAG GGGCGGGATG

SphI

3751 GGCGGAGTTA GGGGCGGGAC TATGGTTGCT GACTAATTGA GATGCATGCA

3801 AGGAGATGGC GCCCAACAGT CCCCCGGCCA CGGCGCCTGC CACCATACCC

3851 ACGCCGAAAC AAGCGCTCAT GAGCCCGAAG TGGCGAGCCC GATCTTCCCC

3901 ATCGGTGATG TCGGCGATAT AGGCGCCAGC AACCGCACCT GTGGCGCCGG

3951 TGATGCCGGC CACGATGCGT CCGGCGTAGA

Fig. 58

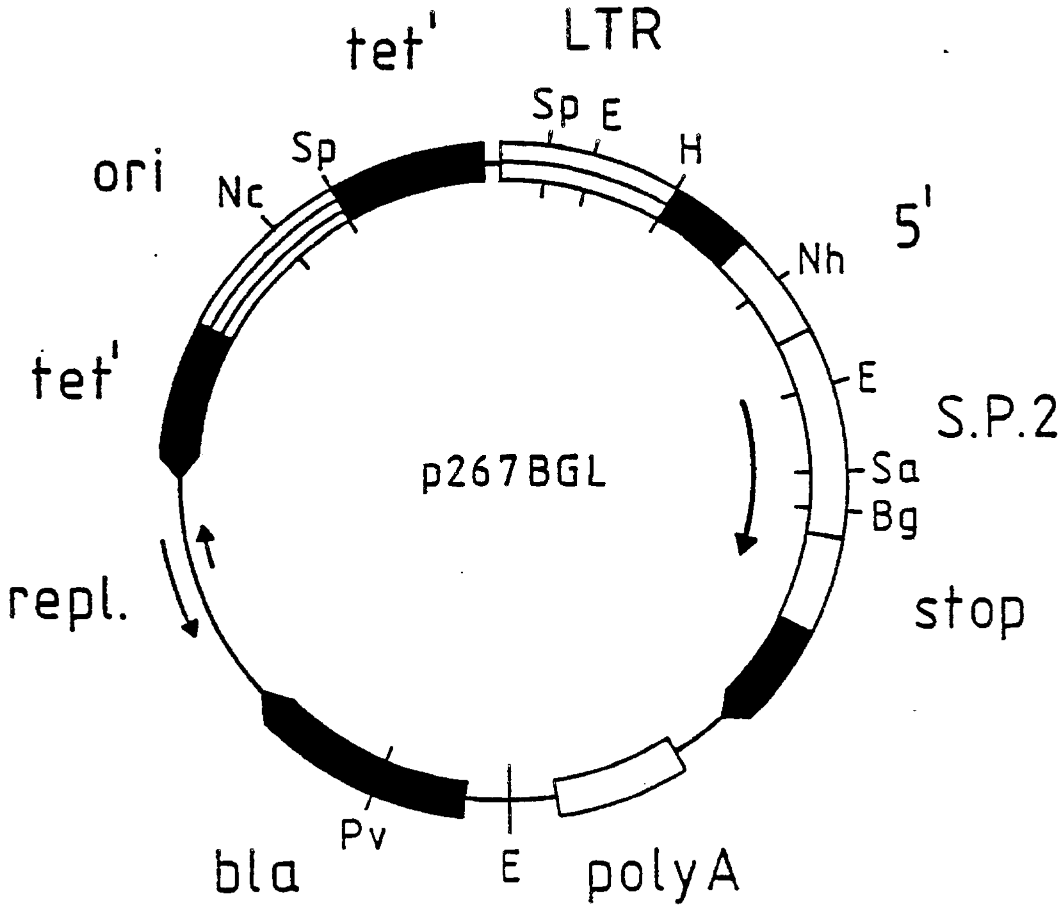

LTR
Sp
E
H
5'
Nh
E
S.P.2
Sa
Bg
stop
polyA
E
bla
Pv
repl.
tet'
ori
Nc
Sp
tet'
p267BGL

Fig. 59

```
  1  GGATCTCAGG ATATAGTAGT TTCGCTTTTG CATAGGGAGG GGGAAATGTA

 51  GCCTTATGCA ATACTCTTGT AGTCTTGCAA CATGCTTATG TAACGATGAG

                                                SphI
101  TTAGCAACAT GCCTTACAAG GAGAGAAAAA GCACCGTGCA TGCCGATTGG

151  TGGAAGTAAG GTGGTA.GAT CGTGCCTTAT TAGGAAGGCA ACAGACGGGT

                                   EcoRI
201  CTGACATGGA TTGGACGAAC CACCGAATTC GCATTGCAGA GAGTATTGTA

                                       *
251  TTTAAGTGCC TAGCTCGATA CAATAAACGC CATTTGACCA TTCACCACAT
                 HindIII
301  TGGTGTGCAC CTCAAGCTTG GTAAGTGACC AGCTACAGTC GGAAACCATC

351  AGCAAGCAGG TATGTACTCT CCAGGGTGGG CCTGGCTTCC CCAGTCAAGA

401  CTCCAGGGAT TTGAGGGACG CTGTGGGCTC TTCTCTTACA TGTACCTTTT

      NheI
451  GCTAGCCTCA ACCCTGACTA TCTTCCAGGT CATTGTTCCA ACATGGCCCT
                                                 MetAlaLe
                                                  o  o  o

        EcoRI
501  GTGGAATTCA TACCTGCTGA TGTGGGGACT GCTCACGTTC ATCATGGTGC
     uTrpAsnSer TyrLeuLeuM etTrpGlyLe uLeuThrPhe IleMetValP
      o  o  o    o  o  o   o  o  o  o  o  o  o   o  o  o

                      SacI      BglII
551  CTGGCTGCCA GGCAGAGCTC AGCAGATCTG CTGAATGAAT GAGTGACCTT
     roGlyCysGl nAlaGluLeu SerArgSerA laGlu
     o  o  o  o  o

601  CAGACCTTGG CACTGGAGGT GGCCCGGCAG AAGCGCGGCA TCGTGGATCA

651  GTGCTGCACC AGCATCTGCT CTCTCTACCA ACTGGAGAAC TACTGCAACT

701  AGGCCCACCA CTACCCTGTC CACCCCTCTG CAATGAATAA AACCTTTGAA

751  AGAGCACTAC AAGTTGTGTG TACATGCGTG CGTGTGCATA TGTGGTGCGG

801  GGGGAACATG AGTGGGGTCG GCTGGAGTGG TCGCGGCTTA ATCTATCTGG

                                 EcoRI
851  CGATGATAAG CTGTCAAAACA TGAGAATTCT TGAAGACGAA AGGGCCTCGT

901  GATACGCCTA TTTTTATAGG TTAATGTCAT GATAATAATG GTTTCTTAGA

951  CGTCAGGTGG CACTTTTCGG GGAAATGTGC GCGGAACCCC TATTTGTTTA
```

Fig. 59 (cont.)

1001 TTTTTCTAAA TACATTCAAA TATGTATCCG CTCATGAGAC AATAACCCTG

1051 ATAAATGCTT CAATAATATT GAAAAAGGAA GAGTATGAGT ATTCAACATT

1101 TCCGTGTCGC CCTTATTCCC TTTTTTGCGG CATTTTGCCT TCCTGTTTTT

1151 GCTCACCCAG AAACGCTGGT GAAAGTAAAA GATGCTGAAG ATCAGTTGGG

1201 TGCACGAGTG GGTTACATCG AACTGGATCT CAACAGCGGT AAGATCCTTG

1251 AGAGTTTTCG CCCCGAAGAA CGTTTTCCAA TGATGAGCAC TTTTAAAGTT

1301 CTGCTATGTG GCGCGGTATT ATCCCGTGTT GACGCCGGGC AAGAGCAACT

1351 CGGTCGCCGC ATACACTATT CTCAGAATGA CTTGGTTGAG TACTCACCAG

1401 TCACAGAAAA GCATCTTACG GATGGCATGA CAGTAAGAGA ATTATGCAGT

1451 GCTGCCATAA CCATGAGTGA TAACACTGCG GCCAACTTAC TTCTGACAAC

PvuI
1501 GATCGGAGGA CCGAAGGAGC TAACCGCTTT TTTGCACAAC ATGGGGGATC

1551 ATGTAACTCG CCTTGATCGT TGGGAACCGG AGCTGAATGA AGCCATACCA

1601 AACGACGAGC GTGACACCAC GATGCCTGCA GCAATGGCAA CAACGTTGCG

1651 CAAACTATTA ACTGGCGAAC TACTTACTCT AGCTTCCCGG CAACAATTAA

1701 TAGACTGGAT GGAGGCGGAT AAAGTTGCAG GACCACTTCT GCGCTCGGCC

1751 CTTCCGGCTG GCTGGTTTAT TGCTGATAAA TCTGGAGCCG GTGAGCGTGG

1801 GTCTCGCGGT ATCATTGCAG CACTGGGGCC AGATGGTAAG CCCTCCCGTA

1851 TCGTAGTTAT CTACACGACG GGGAGTCAGG CAACTATGGA TGAACGAAAT

1901 AGACAGATCG CTGAGATAGG TGCCTCACTG ATTAAGCATT GGTAACTGTC

1951 AGACCAAGTT TACTCATATA TACTTTAGAT TGATTTAAAA CTTCATTTTT

2001 AATTTAAAAG CGTAATCTGC TGCTTGCAAA CAAAAAAACC ACCGCTACCA

2051 GCGGTGGTTT GTTTGCCGGA TCAAGAGCTA CCAACTCTTT TTCCGAAGGT

2101 AACTGGCTTC AGCAGAGCGC AGATACCAAA TACTGTCCTT CTAGTGTAGC

2151 CGTAGTTAGG CCACCACTTC AAGAACTCTG TAGCACCGCC TACATACCTC

2201 GCTCTGCTAA TCCTGTTACC AGTGGCTGCT GCCAGTGGCG ATAAGTCGTG

2251 TCTTACCGGG TTGGACTCAA GACGATAGTT ACCGGATAAG GCGCAGCGGT

Fig. 59 (cont.)

2301 CGGGCTGAAC GGGGGGTTCG TGCACACAGC CCAGCTTGGA GCGAACGACC

2351 TACACCGAAC TGAGATACCT ACAGCGTGAG CATTGAGAAA GCGCCACGCT

2401 TCCCGAAGGG AGAAAGGCGG ACAGGTATCC GGTAAGCGGC AGGGTCGGAA

2451 CAGGAGAGCG CACGAGGGAG CTTCCAGGGG GAAACGCCTG GTATCTTTAT

2501 AGTCCTGTCG GGTTTCGCCA CCTCTGACTT GAGCGTCGAT TTTTGTGATG

2551 CTCGTCAGGG GGGCGGAGCC TATGGAAAAA CGCCAGCAAC GGATGCGCCG

2601 CGTGCGGCTG CTGGAGATGG CGGACGCGAT GGATATGTTC TGCCAAGGGT

2651 TGGTTTGCGC ATTCACAGTT CTCCGCAAGA ATTGATTGGC TCCAATTCTT

2701 GGAGTGGTGA ATCCGTTAGC GAGGTGCCGC CGGCTTCCAT TCAGGTCGAG

2751 GTGGCCCGGC TCCATGCACC GCGACGCAAC GCGGGGAGGC AGACAAGGTA

2801 TAGGGCGGCG CCTACAATCC ATGCCAACCC GTTCCATGTG CTCGCCGAGG

2851 CGGCATAAAT CGCCGTGACG ATCAGCGGTC CAGTGATCGA AGTTAGGCTG

2901 GTAAGAGCCG CGAGCGATCC TTGAAGCTGT CCCTGATGGT CGTCATCTAC

2951 CTGCCTGGAC AGCATGGCCT GCAACGCGGG CATCCCGATG CCGCCGGAAG

3001 CGAGAAGAAT CATAATGGGG AAGGCCATCC AGCCTCGCGT CGAGCTTTTT

3051 GCAAAAGCCT AGGCCTCCAA AAAAGCCTCC TCACTACTTC TGGAATAGCT

3101 CAGAGGCCGA GGCGGCCTCG GCCTCTGCAT AAATAAAAAA AATTAGTCAG

NcoI

3151 CCATGGGGCG GAGAATGGGC GGAACTGGGC GGAGTTAGGG GCGGGATGGG

SphI

3201 CGGAGTTAGG GGCGGGACTA TGGTTGCTGA CTAATTGAGA TGCATGCAAG

3251 GAGATGGCGC CCAACAGTCC CCCGGCCACG GCGCCTGCCA CCATACCCAC

3301 GCCGAAACAA GCGCTCATGA GCCCGAAGTG GCGAGCCCGA TCTTCCCCAT

3351 CGGTGATGTC GGCGATATAG GCGCCAGCAA CCGCACCTGT GGCGCCGGTG

3401 ATGCCGGCCA CGATGCGTCC GGCGTAGA

Fig. 60

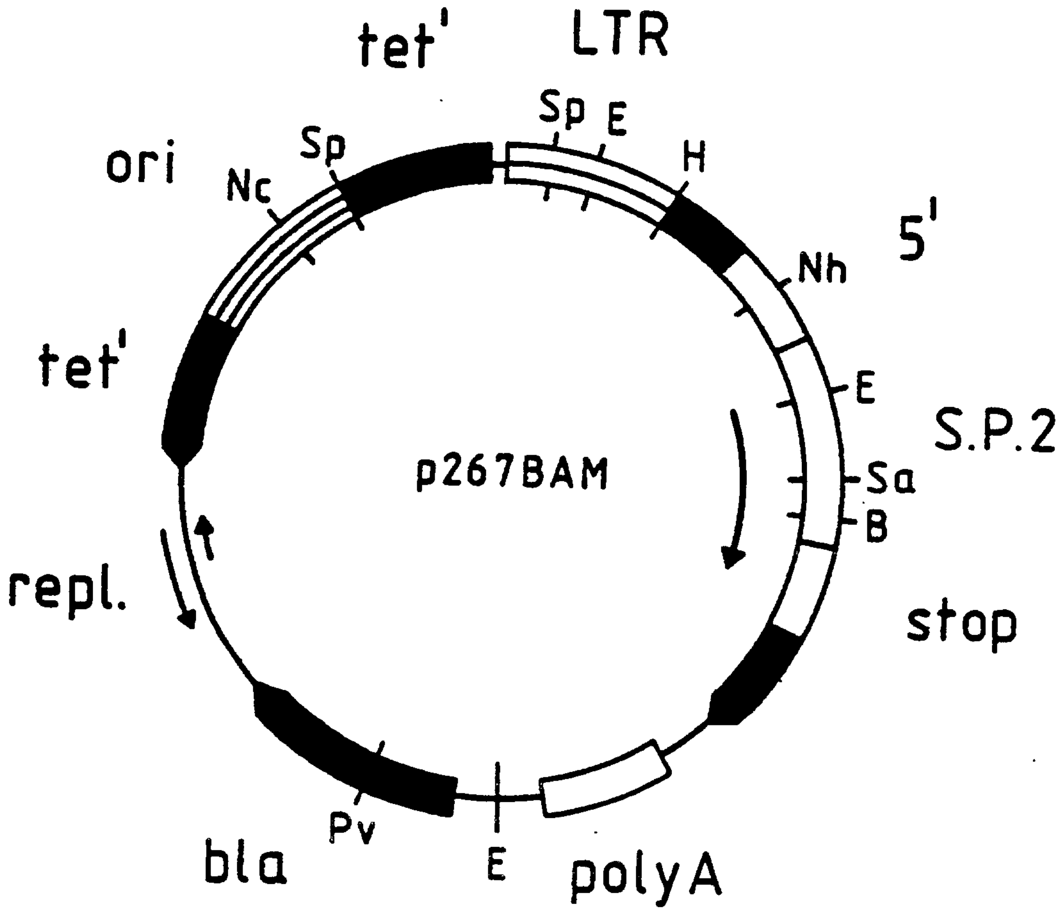
tet'
LTR
ori
Sp
Sp
E
Nc
H
5'
Nh
tet'
E
S.P.2
p267BAM
Sa
B
repl.
stop
bla
Pv
E
polyA

Fig. 61

```
                                                         SphI
101  TTAGCAACAT GCCTTACAAG GAGAGAAAAA GCACCGTGCA TGCCGATTGG
```

```
  1  GGATCTCAGG ATATAGTAGT TTCGCTTTTG CATAGGGAGG GGGAAATGTA

 51  GCCTTATGCA ATACTCTTGT AGTCTTGCAA CATGCTTATG TAACGATGAG

                                                   SphI
101  TTAGCAACAT GCCTTACAAG GAGAGAAAAA GCACCGTGCA TGCCGATTGG

151  TGGAAGTAAG GTGGTA.GAT CGTGCCTTAT TAGGAAGGCA ACAGACGGGT

                                EcoRI
201  CTGACATGGA TTGGACGAAC CACCGAATTC GCATTGCAGA GAGTATTGTA

                                    *
251  TTTAAGTGCC TAGCTCGATA CAATAAACGC CATTTGACCA TTCACCACAT
                  HindIII
301  TGGTGTGCAC CTCAAGCTTG GTAAGTGACC AGCTACAGTC GGAAACCATC

351  AGCAAGCAGG TATGTACTCT CCAGGGTGGG CCTGGCTTCC CCAGTCAAGA

401  CTCCAGGGAT TTGAGGGACG CTGTGGGCTC TTCTCTTACA TGTACCTTTT

      NheI
451  GCTAGCCTCA ACCCTGACTA TCTTCCAGGT CATTGTTCCA ACATGGCCCT
                                                 MetAlaLe
                                                  o  o  o

        EcoRI
501  GTGGAATTCA TACCTGCTGA TGTGGGGACT GCTCACGTTC ATCATGGTGC
     uTrpAsnSer TyrLeuLeuM etTrpGlyLe uLeuThrPhe IleMetValP
       o  o  o   o  o  o   o  o  o  o  o  o  o   o  o  o

                       SacI     BamHI
551  CTGGCTGCCA GGCAGAGCTC AGCGGATCCG CTGAATGAAT GAGTGACCTT
     roGlyCysGl nAlaGluLeu SerGlySerA laGlu
     o  o  o  o   o

601  CAGACCTTGG CACTGGAGGT GGCCCGGCAG AAGCGCGGCA TCGTGGATCA

651  GTGCTGCACC AGCATCTGCT CTCTCTACCA ACTGGAGAAC TACTGCAACT

701  AGGCCCACCA CTACCCTGTC CACCCCTCTG CAATGAATAA AACCTTTGAA

751  AGAGCACTAC AAGTTGTGTG TACATGCGTG CGTGTGCATA TGTGGTGCGG

801  GGGGAACATG AGTGGGGTCG GCTGGAGTGG TCGCGGCTTA ATCTATCTGG

                                EcoRI
851  CGATGATAAG CTGTCAAACA TGAGAATTCT TGAAGACGAA AGGGCCTCGT

901  GATACGCCTA TTTTTATAGG TTAATGTCAT GATAATAATG GTTTCTTAGA

951  CGTCAGGTGG CACTTTTCGG GGAAATGTGC GCGGAACCCC TATTTGTTTA
```

Fig. 61 (cont.)

1001 TTTTTCTAAA TACATTCAAA TATGTATCCG CTCATGAGAC AATAACCCTG
1051 ATAAATGCTT CAATAATATT GAAAAAGGAA GAGTATGAGT ATTCAACATT
1101 TCCGTGTCGC CCTTATTCCC TTTTTTGCGG CATTTTGCCT TCCTGTTTTT
1151 GCTCACCCAG AAACGCTGGT GAAAGTAAAA GATGCTGAAG ATCAGTTGGG
1201 TGCACGAGTG GGTTACATCG AACTGGATCT CAACAGCGGT AAGATCCTTG
1251 AGAGTTTTCG CCCCGAAGAA CGTTTTCCAA TGATGAGCAC TTTTAAAGTT
1301 CTGCTATGTG GCGCGGTATT ATCCCGTGTT GACGCCGGGC AAGAGCAACT
1351 CGGTCGCCGC ATACACTATT CTCAGAATGA CTTGGTTGAG TACTCACCAG
1401 TCACAGAAAA GCATCTTACG GATGGCATGA CAGTAAGAGA ATTATGCAGT
1451 GCTGCCATAA CCATGAGTGA TAACACTGCG GCCAACTTAC TTCTGACAAC

PvuI
1501 GATCGGAGGA CCGAAGGAGC TAACCGCTTT TTTGCACAAC ATGGGGGATC
1551 ATGTAACTCG CCTTGATCGT TGGGAACCGG AGCTGAATGA AGCCATACCA
1601 AACGACGAGC GTGACACCAC GATGCCTGCA GCAATGGCAA CAACGTTGCG
1651 CAAACTATTA ACTGGCGAAC TACTTACTCT AGCTTCCCGG CAACAATTAA
1701 TAGACTGGAT GGAGGCGGAT AAAGTTGCAG GACCACTTCT GCGCTCGGCC
1751 CTTCCGGCTG GCTGGTTTAT TGCTGATAAA TCTGGAGCCG GTGAGCGTGG
1801 GTCTCGCGGT ATCATTGCAG CACTGGGGCC AGATGGTAAG CCCTCCCGTA
1851 TCGTAGTTAT CTACACGACG GGGAGTCAGG CAACTATGGA TGAACGAAAT
1901 AGACAGATCG CTGAGATAGG TGCCTCACTG ATTAAGCATT GGTAACTGTC
1951 AGACCAAGTT TACTCATATA TACTTTAGAT TGATTTAAAA CTTCATTTTT
2001 AATTTAAAAG CGTAATCTGC TGCTTGCAAA CAAAAAAACC ACCGCTACCA
2051 GCGGTGGTTT GTTTGCCGGA TCAAGAGCTA CCAACTCTTT TTCCGAAGGT
2101 AACTGGCTTC AGCAGAGCGC AGATACCAAA TACTGTCCTT CTAGTGTAGC
2151 CGTAGTTAGG CCACCACTTC AAGAACTCTG TAGCACCGCC TACATACCTC
2201 GCTCTGCTAA TCCTGTTACC AGTGGCTGCT GCCAGTGGCG ATAAGTCGTG
2251 TCTTACCGGG TTGGACTCAA GACGATAGTT ACCGGATAAG GCGCAGCGGT

Fig. 61 (cont.)

2301 CGGGCTGAAC GGGGGGTTCG TGCACACAGC CCAGCTTGGA GCGAACGACC

2351 TACACCGAAC TGAGATACCT ACAGCGTGAG CATTGAGAAA GCGCCACGCT

2401 TCCCGAAGGG AGAAAGGCGG ACAGGTATCC GGTAAGCGGC AGGGTCGGAA

2451 CAGGAGAGCG CACGAGGGAG CTTCCAGGGG GAAACGCCTG GTATCTTTAT

2501 AGTCCTGTCG GGTTTCGCCA CCTCTGACTT GAGCGTCGAT TTTTGTGATG

2551 CTCGTCAGGG GGGCGGAGCC TATGGAAAAA CGCCAGCAAC GGATGCGCCG

2601 CGTGCGGCTG CTGGAGATGG CGGACGCGAT GGATATGTTC TGCCAAGGGT

2651 TGGTTTGCGC ATTCACAGTT CTCCGCAAGA ATTGATTGGC TCCAATTCTT

2701 GGAGTGGTGA ATCCGTTAGC GAGGTGCCGC CGGCTTCCAT TCAGGTCGAG

2751 GTGGCCCGGC TCCATGCACC GCGACGCAAC GCGGGGAGGC AGACAAGGTA

2801 TAGGGCGGCG CCTACAATCC ATGCCAACCC GTTCCATGTG CTCGCCGAGG

2851 CGGCATAAAT CGCCGTGACG ATCAGCGGTC CAGTGATCGA AGTTAGGCTG

2901 GTAAGAGCCG CGAGCGATCC TTGAAGCTGT CCCTGATGGT CGTCATCTAC

2951 CTGCCTGGAC AGCATGGCCT GCAACGCGGG CATCCCGATG CCGCCGGAAG

3001 CGAGAAGAAT CATAATGGGG AAGGCCATCC AGCCTCGCGT CGAGCTTTTT

3051 GCAAAAGCCT AGGCCTCCAA AAAAGCCTCC TCACTACTTC TGGAATAGCT

3101 CAGAGGCCGA GGCGGCCTCG GCCTCTGCAT AAATAAAAAA AATTAGTCAG

NcoI
3151 CCATGGGGCG GAGAATGGGC GGAACTGGGC GGAGTTAGGG GCGGGATGGG

SphI
3201 CGGAGTTAGG GGCGGGACTA TGGTTGCTGA CTAATTGAGA TGCATGCAAG

3251 GAGATGGCGC CCAACAGTCC CCCGGCCACG GCGCCTGCCA CCATACCCAC

3301 GCCGAAACAA GCGCTCATGA GCCCGAAGTG GCGAGCCCGA TCTTCCCCAT

3351 CGGTGATGTC GGCGATATAG GCGCCAGCAA CCGCACCTGT GGCGCCGGTG

3401 ATGCCGGCCA CGATGCGTCC GGCGTAGA

Fig. 62

```
        10                  30                  50
BglII    .         BamHI  SmaI.       EcoRI        .       .
AGATCTGCTAGAACTAGTGGATCCCCCGGGCTGCAGGAATTCCAGCCAGCGACCGTCGGT
ArgSerAlaArgThrSerGlySerProGlyLeuGlnGluPheGlnProAlaThrValGly

        70                  90                 110
         .         .         .         .         .         .
AGCAGCATGGCTCTCCTCTTTCTCCTACCCCTTGTCATGCAGGGTGTGAGCAGGGCTGAG
SerSerMetAlaLeuLeuPheLeuLeuProLeuValMetGlnGlyValSerArgAlaGlu
      1
       130                 150                 170
         .         .         .         .         .         .
ATGGGCACCGCGGATCTGGGGCCGTCCTCAGTGCCTACACCAACTAATGTTACAATTGAA
MetGlyThrAlaAspLeuGlyProSerSerValProThrProThrAsnValThrIleGlu

       190                 210                 230
         .         .         .         .         .         .
TCCTATAACATGAACCCTATCGTATATTGGGAGTACCAGATCATGCCACAGGTCCCTGTT
SerTyrAsnMetAsnProIleValTyrTrpGluTyrGlnIleMetProGlnValProVal

       250                 270                 290
         .         .         .         .         .         .
TTTACCGTAGAGGTAAAGAACTATGGTGTTAAGAATTCAGAATGGATTGATGCCTGCATC
PheThrValGluValLysAsnTyrGlyValLysAsnSerGluTrpIleAspAlaCysIle

       310                 330                 350
         .         .         .         .         .         .
AATATTTCTCATCATTATTGTAATATTTCTGATCATGTTGGTGATCCATCAAATTCTCTT
AsnIleSerHisHisTyrCysAsnIleSerAspHisValGlyAspProSerAsnSerLeu

       370                 390                 410
         .         .         .         .         .         .
TGGGTCAGAGTTAAAGCCAGGGTTGGACAAAAAGAATCTGCCTATGCAAAGTCAGAAGAA
TrpValArgValLysAlaArgValGlyGlnLysGluSerAlaTyrAlaLysSerGluGlu

       430                 450                 470
         .         .         .         .  EcoRV  .         .
TTTGCTGTATGCCGAGATGGAAAAATTGGACCACCTAAACTGGATATCAGAAAGGAGGAG
PheAlaValCysArgAspGlyLysIleGlyProProLysLeuAspIleArgLysGluGlu

       490                 510                 530
         .         .         .         .         .         .
AAGCAAATCATGATTGACATATTTCACCCTTCAGTTTTTGTAAATGGAGACGAGCAGGAA
LysGlnIleMetIleAspIlePheHisProSerValPheValAsnGlyAspGluGlnGlu

       550                 570                 590
         .         .         .         .         .         .
GTCGATTATGATCCCGAAACTACCTGTTACATTAGGGTGTACAATGTGTATGTGAGAATG
ValAspTyrAspProGluThrThrCysTyrIleArgValTyrAsnValTyrValArgMet

       610                 630                 650
         .         .         .         .         .         .
AACGGAAGTGAGATCCAGTATAAAATACTCACGCAGAAGGAAGATGATTGTGACGAGATT
AsnGlySerGluIleGlnTyrLysIleLeuThrGlnLysGluAspAspCysAspGluIle
```

Fig. 62 (cont.)

```
         670                 690                 710
          .         .         .         .         .         .
CAGTGCCAGTTAGCGATTCCAGTATCCTCACTGAATTCTCAGTACTGTGTTTCAGCAGAA
GlnCysGlnLeuAlaIleProValSerSerLeuAsnSerGlnTyrCysValSerAlaGlu

         730                 750                 770
          .         .         .         .         .         .
GGAGTCTTACATGTGTGGGGTGTTACAACTGAAAAGTCAAAAGAAGTTTGTATTACCATT
GlyValLeuHisValTrpGlyValThrThrGluLysSerLysGluValCysIleThrIle

         790                 810                 830
          .         .         .         .         .         .
TTCAATAGCAGTATAAAAGGTTCTCTTTGGATTCCAGTTGTTGCTGCTTTACTACTCTTT
PheAsnSerSerIleLysGlySerLeuTrpIleProValValAlaAlaLeuLeuLeuPhe

         850                 870                 890
          .         .         .         .         .         .
CTAGTGCTTAGCCTGGTATTCATCTGTTTTTATATTAAGAAAATTAATCCATTGAAGGAA
LeuValLeuSerLeuValPheIleCysPheTyrIleLysLysIleAsnProLeuLysGlu

         910                 930                 950
          .         .         .         .         .         .
AAAAGCATAATATTACCCAAGTCCTTGATCTCTGTGGTAAGAAGTGCTACTTTAGAGACA
LysSerIleIleLeuProLysSerLeuIleSerValValArgSerAlaThrLeuGluThr

         970                 990                1010
          .         .         .         .         .         .
AAACCTGAATCAAAATATGTATCACTCATCACGTCATACCAGCCATTTTCCTTAGAAAAG
LysProGluSerLysTyrValSerLeuIleThrSerTyrGlnProPheSerLeuGluLys

        1030                1050                1070
          .         .         .         .         .         .
GAGGTGGTCTGTGAAGAGCCGTTGTCTCCAGCAACAGTTCCAGGCATGCATACCGAAGAC
GluValValCysGluGluProLeuSerProAlaThrValProGlyMetHisThrGluAsp

        1090                1110                1130
          .         .         .         .         .         .
AATCCAGGAAAAGTGGAACATACAGAAGAACTTTCTAGTATAACAGAAGTGGTGACTACT
AsnProGlyLysValGluHisThrGluGluLeuSerSerIleThrGluValValThrThr

        1150                1170                1190
          .         .         .         .         .         .
GAAGAAAATATTCCTGACGTGGTCCCGGGCAGCCATCTGACTCCAATAGAGAGAGAGAGT
GluGluAsnIleProAspValValProGlySerHisLeuThrProIleGluArgGluSer

        1210                1230                1250
          .         .         .         .         .         .
TCTTCACCTTTAAGTAGTAACCAGTCTGAACCTGGCAGCATCGCTTTAAACTCGTATCAC
SerSerProLeuSerSerAsnGlnSerGluProGlySerIleAlaLeuAsnSerTyrHis
```

Fig. 62 (cont.)

```
1270                1290                1310
TCCAGAAATTGTTCTGAGAGTGATCACTCCAGAAATGGTTTTGATACTGATTCCAGCTGT
SerArgAsnCysSerGluSerAspHisSerArgAsnGlyPheAspThrAspSerSerCys

1330                1350                1370
CTGGAATCACATAGCTCCTTATCTGACTCAGAATTTCCCCCAAATAATAAAGGTGAAATA
LeuGluSerHisSerSerLeuSerAspSerGluPheProProAsnAsnLysGlyGluIle

1390                1410                1430
AAAACAGAAGGACAAGAGCTCATAACCGTAATAAAAGCCCCCACCTCCTTTGGTTATGAT
LysThrGluGlyGlnGluLeuIleThrValIleLysAlaProThrSerPheGlyTyrAsp

1450                1470                1490
AAACCACATGTGCTAGTGGATCTACTTGTGGATGATAGCGGTAAAGAGTCCTTGATTGGT
LysProHisValLeuValAspLeuLeuValAspAspSerGlyLysGluSerLeuIleGly

1510                1530                1550
                                   MboI
TATAGACCAACAGAAGATTCCAAAGAATTTTCATGAGATCAGCTAAGTTGCACCAACTTT
TyrArgProThrGluAspSerLysGluPheSer
                              489
1570                1590                1610
GAAGTCTGATTTTCCTGGACAGTTTTCTGCTTTAATTTCATGAAAAGATTATGATCTCAG

1630                1650                1670
AAATTGTATCTTAGTTGGTATCAACCAAATGGAGTGACTTAGTGTACATGAAAGCGTAAA

1690                1710                1730
GAGGATGTGTGGCATTTTCACTTTTGGCTTGTAAAGTACAGACTTTTTTTTTTTTTTAAA

1750                1770                1790
CAAAAAAAGCATGTAACTTATGAACCTTTACATCCAGATAGGTTACCAGTAACGGAACAG

1810                1830                1850
TATCCAGTACTCCTGGTTCCTAGGTGAGCAGGTGATGCCCCAGGGACCTTTGTAGCCACT

1870                1890                1910
TCACTTTTTTTCTTTTCTCTGCCTTGGTATAGCATATGTTTTTGTAAGTTTATGCATACA

1930                1950
                                        BglII
GTAATTTTAAGTAATTTCAGAAGAAATTCTCGAAGCTCAGATCT
```

Fig. 63

```
       10                      30                      50
BglII   .          EcoRI        .           .           .           .
AGATCTTCCGGGCTGCAGGAATTCCAGCCAGCGACCGTCGGTAGCAGCATGGCTCTCCTC
ArgSerSerGlyLeuGlnGluPheGlnProAlaThrValGlySerSerMetAlaLeuLeu
                                                  1
       70                      90                     110
        .           .           .           .           .           .
TTTCTCCTACCCCTTGTCATGCAGGGTGTGAGCAGGGCTGAGATGGGCACCGCGGATCTG
PheLeuLeuProLeuValMetGlnGlyValSerArgAlaGluMetGlyThrAlaAspLeu

      130                     150                     170
        .           .           .           .           .           .
GGGCCGTCCTCAGTGCCTACACCAACTAATGTTACAATTGAATCCTATAACATGAACCCT
GlyProSerSerValProThrProThrAsnValThrIleGluSerTyrAsnMetAsnPro

      190                     210                     230
        .           .           .           .           .           .
ATCGTATATTGGGAGTACCAGATCATGCCACAGGTCCCTGTTTTTACCGTAGAGGTAAAG
IleValTyrTrpGluTyrGlnIleMetProGlnValProValPheThrValGluValLys

      250                     270                     290
        .           .           .           .           .           .
AACTATGGTGTTAAGAATTCAGAATGGATTGATGCCTGCATCAATATTTCTCATCATTAT
AsnTyrGlyValLysAsnSerGluTrpIleAspAlaCysIleAsnIleSerHisHisTyr

      310                     330                     350
        .           .           .           .           .           .
TGTAATATTTCTGATCATGTTGGTGATCCATCAAATTCTCTTTGGGTCAGAGTTAAAGCC
CysAsnIleSerAspHisValGlyAspProSerAsnSerLeuTrpValArgValLysAla

      370                     390                     410
        .           .           .           .           .           .
AGGGTTGGACAAAAAGAATCTGCCTATGCAAAGTCAGAAGAATTTGCTGTATGCCGAGAT
ArgValGlyGlnLysGluSerAlaTyrAlaLysSerGluGluPheAlaValCysArgAsp

      430                     450                     470
        .           .     EcoRV             .           .           .
GGAAAAATTGGACCACCTAAACTGGATATCAGAAAGGAGGAGAAGCAAATCATGATTGAC
GlyLysIleGlyProProLysLeuAspIleArgLysGluGluLysGlnIleMetIleAsp

      490                     510                     530
        .           .           .           .           .           .
ATATTTCACCCTTCAGTTTTTGTAAATGGAGACGAGCAGGAAGTCGATTATGATCCCGAA
IlePheHisProSerValPheValAsnGlyAspGluGlnGluValAspTyrAspProGlu

      550                     570                     590
        .           .           .           .           .           .
ACTACCTGTTACATTAGGGTGTACAATGTGTATGTGAGAATGAACGGAAGTGAGATCCAG
ThrThrCysTyrIleArgValTyrAsnValTyrValArgMetAsnGlySerGluIleGln
```

Fig. 63 (cont.)

```
      610                 630                 650
       .         .         .         .         .         .
TATAAAATACTCACGCAGAAGGAAGATGATTGTGACGAGATTCAGTGCCAGTTAGCGATT
TyrLysIleLeuThrGlnLysGluAspAspCysAspGluIleGlnCysGlnLeuAlaIle

      670                 690                 710
       .         .         .         .         .         .
CCAGTATCCTCACTGAATTCTCAGTACTGTGTTTCAGCAGAAGGAGTCTTACATGTGTGG
ProValSerSerLeuAsnSerGlnTyrCysValSerAlaGluGlyValLeuHisValTrp

      730                 750                 770
       .         .         .         .         .         .
GGTGTTACAACTGAAAAGTCAAAAGAAGTTTGTATTACCATTTTCAATAGCAGTATAAAA
GlyValThrThrGluLysSerLysGluValCysIleThrIlePheAsnSerSerIleLys

      790                 810                 830
       .         .         .         .         .         .
GGTTCTCTTTGGATTCCAGTTGTTGCTGCTTTACTACTCTTTCTAGTGCTTAGCCTGGTA
GlySerLeuTrpIleProValValAlaAlaLeuLeuLeuPheLeuValLeuSerLeuVal

      850                 870                 890
       .         .         .         .         . SspI    .
TTCATCTGTTTTTATATTAAGAAAATTAATCCATTGAAGGAAAAAAGCATAATATTACCC
PheIleCysPheTyrIleLysLysIleAsnProLeuLysGluLysSerIleIleLeuPro
                                                     281
      910                 930                 950
       .         .         .         .         .         .
AAGTCCTTGATCTCTGTGGTAAGAAGTGCTACTTTAGAGACAAAACCTGAATCAAAATAT
LysSerLeuIleSerValValArgSerAlaThrLeuGluThrLysProGluSerLysTyr

      970                 990                1010
       .         .         .         .         .         .
GTATCACTCATCACGTCATACCAGCCATTTTCCTTAGAAAAGGAGGTGGTCTGTGAAGAG
ValSerLeuIleThrSerTyrGlnProPheSerLeuGluLysGluValValCysGluGlu

     1030                1050                1070
       .         .         .         .         .         .
CCGTTGTCTCCAGCAACAGTTCCAGGCATGCATACCGAAGACAATCCAGGAAAAGTGGAA
ProLeuSerProAlaThrValProGlyMetHisThrGluAspAsnProGlyLysValGlu

     1090                1110                1130
       .         .         .         .         .         .
CATACAGAAGAACTTTCTAGTATAACAGAAGTGGTGACTACTGAAGAAAATATTCCTGAC
HisThrGluGluLeuSerSerIleThrGluValValThrThrGluGluAsnIleProAsp

     1150
       .  BglII
GTGGTCCCGGAAGATCT
ValValProGluAsp
     367
```

Fig. 64

```
        10                      30                      50
BglII    .           EcoRI       .           .           .           .
AGATCTTCCGGGCTGCAGGAATTCCAGCCAGCGACCGTCGGTAGCAGCATGGCTCTCCTC
ArgSerSerGlyLeuGlnGluPheGlnProAlaThrValGlySerSerMetAlaLeuLeu
                                                 1
        70                      90                     110
         .           .           .           .           .           .
TTTCTCCTACCCCTTGTCATGCAGGGTGTGAGCAGGGCTGAGATGGGCACCGCGGATCTG
PheLeuLeuProLeuValMetGlnGlyValSerArgAlaGluMetGlyThrAlaAspLeu

       130                     150                     170
         .           .           .           .           .           .
GGGCCGTCCTCAGTGCCTACACCAACTAATGTTACAATTGAATCCTATAACATGAACCCT
GlyProSerSerValProThrProThrAsnValThrIleGluSerTyrAsnMetAsnPro

       190                     210                     230
         .           .           .           .           .           .
ATCGTATATTGGGAGTACCAGATCATGCCACAGGTCCCTGTTTTTACCGTAGAGGTAAAG
IleValTyrTrpGluTyrGlnIleMetProGlnValProValPheThrValGluValLys

       250                     270                     290
         .           .           .           .           .           .
AACTATGGTGTTAAGAATTCAGAATGGATTGATGCCTGCATCAATATTTCTCATCATTAT
AsnTyrGlyValLysAsnSerGluTrpIleAspAlaCysIleAsnIleSerHisHisTyr

       310                     330                     350
         .           .           .           .           .           .
TGTAATATTTCTGATCATGTTGGTGATCCATCAAATTCTCTTTGGGTCAGAGTTAAAGCC
CysAsnIleSerAspHisValGlyAspProSerAsnSerLeuTrpValArgValLysAla

       370                     390                     410
         .           .           .           .           .           .
AGGGTTGGACAAAAAGAATCTGCCTATGCAAAGTCAGAAGAATTTGCTGTATGCCGAGAT
ArgValGlyGlnLysGluSerAlaTyrAlaLysSerGluGluPheAlaValCysArgAsp

       430                     450                     470
         .           .     EcoRV.            .           .           .
GGAAAAATTGGACCACCTAAACTGGATATCAGAAAGGAGGAGAAGCAAATCATGATTGAC
GlyLysIleGlyProProLysLeuAspIleArgLysGluGluLysGlnIleMetIleAsp

       490                     510                     530
         .           .           .           .           .           .
ATATTTCACCCTTCAGTTTTTGTAAATGGAGACGAGCAGGAAGTCGATTATGATCCCGAA
IlePheHisProSerValPheValAsnGlyAspGluGlnGluValAspTyrAspProGlu

       550                     570                     590
         .           .           .           .           .           .
ACTACCTGTTACATTAGGGTGTACAATGTGTATGTGAGAATGAACGGAAGTGAGATCCAG
ThrThrCysTyrIleArgValTyrAsnValTyrValArgMetAsnGlySerGluIleGln
```

Fig. 64 (cont.)

```
        610                 630                 650
         .         .         .         .         .         .
TATAAAATACTCACGCAGAAGGAAGATGATTGTGACGAGATTCAGTGCCAGTTAGCGATT
TyrLysIleLeuThrGlnLysGluAspAspCysAspGluIleGlnCysGlnLeuAlaIle

        670                 690                 710
         .         .         .         .         .         .
CCAGTATCCTCACTGAATTCTCAGTACTGTGTTTCAGCAGAAGGAGTCTTACATGTGTGG
ProValSerSerLeuAsnSerGlnTyrCysValSerAlaGluGlyValLeuHisValTrp

        730                 750                 770
         .         .         .         .         .         .
GGTGTTACAACTGAAAAGTCAAAAGAAGTTTGTATTACCATTTTCAATAGCAGTATAAAA
GlyValThrThrGluLysSerLysGluValCysIleThrIlePheAsnSerSerIleLys

        790                 810                 830
         .         .         .         .         .         .
GGTTCTCTTTGGATTCCAGTTGTTGCTGCTTTACTACTCTTTCTAGTGCTTAGCCTGGTA
GlySerLeuTrpIleProValValAlaAlaLeuLeuLeuPheLeuValLeuSerLeuVal

        850                 870                 890
         .         .         .         .         .      BglII
TTCATCTGTTTTTATATTAAGAAAATTAATCCATTGAAGGAAAAAAGCATAATGAAGATCT
PheIleCysPheTyrIleLysLysIleAsnProLeuLysGluLysSerIleMetLysIle
                                                        281
```

Fig. 65

```
        10                  30                  50
BglII    .        BamHI  SmaI.       EcoRI          .     .
AGATCTGCTAGAACTAGTGGATCCCCCGGGCTGCAGGAATTCCAGCCAGCGACCGTCGGT
ArgSerAlaArgThrSerGlySerProGlyLeuGlnGluPheGlnProAlaThrValGly

        70                  90                 110
         .         .         .         .         .         .
AGCAGCATGGCTCTCCTCTTTCTCCTACCCCTTGTCATGCAGGGTGTGAGCAGGGCTGAG
SerSerMetAlaLeuLeuPheLeuLeuProLeuValMetGlnGlyValSerArgAlaGlu
       1
       130                 150                 170
         .         .         .         .         .         .
ATGGGCACCGCGGATCTGGGGCCGTCCTCAGTGCCTACACCAACTAATGTTACAATTGAA
MetGlyThrAlaAspLeuGlyProSerSerValProThrProThrAsnValThrIleGlu

       190                 210                 230
         .         .         .         .         .         .
TCCTATAACATGAACCCTATCGTATATTGGGAGTACCAGATCATGCCACAGGTCCCTGTT
SerTyrAsnMetAsnProIleValTyrTrpGluTyrGlnIleMetProGlnValProVal

       250                 270                 290
         .         .         .         .         .         .
TTTACCGTAGAGGTAAAGAACTATGGTGTTAAGAATTCAGAATGGATTGATGCCTGCATC
PheThrValGluValLysAsnTyrGlyValLysAsnSerGluTrpIleAspAlaCysIle

       310                 330                 350
         .         .         .         .         .         .
AATATTTCTCATCATTATTGTAATATTTCTGATCATGTTGGTGATCCATCAAATTCTCTT
AsnIleSerHisHisTyrCysAsnIleSerAspHisValGlyAspProSerAsnSerLeu

       370                 390                 410
         .         .         .         .         .         .
TGGGTCAGAGTTAAAGCCAGGGTTGGACAAAAAGAATCTGCCTATGCAAAGTCAGAAGAA
TrpValArgValLysAlaArgValGlyGlnLysGluSerAlaTyrAlaLysSerGluGlu

       430                 450                 470
         .         .         .         . EcoRV   .         .
TTTGCTGTATGCCGAGATGGAAAAATTGGACCACCTAAACTGGATATCAGAAAGGAGGAG
PheAlaValCysArgAspGlyLysIleGlyProProLysLeuAspIleArgLysGluGlu

       490                 510                 530
         .         .         .         .         .         .
AAGCAAATCATGATTGACATATTTCACCCTTCAGTTTTTGTAAATGGAGACGAGCAGGAA
LysGlnIleMetIleAspIlePheHisProSerValPheValAsnGlyAspGluGlnGlu

       550                 570                 590
         .         .         .         .         .         .
GTCGATTATGATCCCGAAACTACCTGTTACATTAGGGTGTACAATGTGTATGTGAGAATG
ValAspTyrAspProGluThrThrCysTyrIleArgValTyrAsnValTyrValArgMet
```

Fig. 65 (cont.)

```
        610                 630                 650
         .         .         .         .         .         .
AACGGAAGTGAGATCCAGTATAAAATACTCACGCAGAAGGAAGATGATTGTGACGAGATT
AsnGlySerGluIleGlnTyrLysIleLeuThrGlnLysGluAspAspCysAspGluIle

        670                 690                 710
         .         .         .         .         .         .
CAGTGCCAGTTAGCGATTCCAGTATCCTCACTGAATTCTCAGTACTGTGTTTCAGCAGAA
GlnCysGlnLeuAlaIleProValSerSerLeuAsnSerGlnTyrCysValSerAlaGlu

        730                 750                 770
         .         .         .         .         .         .
GGAGTCTTACATGTGTGGGGTGTTACAACTGAAAAGTCAAAAGAAGTTTGTATTACCATT
GlyValLeuHisValTrpGlyValThrThrGluLysSerLysGluValCysIleThrIle

        790                 810                 830
         .         .         .         .         .         .
TTCAATAGCAGTATAAAAGGTTCTCTTTGGATTCCAGTTGTTGCTGCTTTACTACTCTTT
PheAsnSerSerIleLysGlySerLeuTrpIleProValValAlaAlaLeuLeuLeuPhe

        850                 870                 890
         .         .         .         .         .         .
CTAGTGCTTAGCCTGGTATTCATCTGTTTTTATATTAAGAAAATTAATCCATTGAAGGAA
LeuValLeuSerLeuValPheIleCysPheTyrIleLysLysIleAsnProLeuLysGlu

        910                 930                 950
         .         .         .         .         .         .
AAAAGCATAATATTACCCAAGTCCTTGATCTCTGTGGTAAGAAGTGCTACTTTAGAGACA
LysSerIleIleLeuProLysSerLeuIleSerValValArgSerAlaThrLeuGluThr

        970                 990                 1010
         .         .         .         .         .         .
AAACCTGAATCAAAATATGTATCACTCATCACGTCATACCAGCCATTTTCCTTAGAAAAG
LysProGluSerLysTyrValSerLeuIleThrSerTyrGlnProPheSerLeuGluLys

       1030                1050                1070
         .         .         .         .         .         .
GAGGTGGTCTGTGAAGAGCCGTTGTCTCCAGCAACAGTTCCAGGCATGCATACCGAAGAC
GluValValCysGluGluProLeuSerProAlaThrValProGlyMetHisThrGluAsp

       1090                1110                1130
         .         .         .         .         .         .
AATCCAGGAAAAGTGGAACATACAGAAGAACTTTCTAGTATAACAGAAGTGGTGACTACT
AsnProGlyLysValGluHisThrGluGluLeuSerSerIleThrGluValValThrThr

       1150                1170                1190
         .         .         .         .         .         .
GAAGAAAATATTCCTGACGTGGTCCCGGGCAGCCATCTGACTCCAATAGAGAGAGAGAGT
GluGluAsnIleProAspValValProGlySerHisLeuThrProIleGluArgGluSer
```

Fig. 65 (cont.)

```
      1210                1230                1250
         .         .         .         .         .         .
TCTTCACCTTTAAGTAGTAACCAGTCTGAACCTGGCAGCATCGCTTTAAACTCGTATCAC
SerSerProLeuSerSerAsnGlnSerGluProGlySerIleAlaLeuAsnSerTyrHis

      1270                1290                1310
         .         .         .         .         .         .
TCCAGAAATTGTTCTGAGAGTGATCACTCCAGAAATGGTTTTGATACTGATTCCAGCTGT
SerArgAsnCysSerGluSerAspHisSerArgAsnGlyPheAspThrAspSerSerCys

      1330                1350                1370
         .         .         .         .         .         .
CTGGAATCACATAGCTCCTTATCTGACTCAGAATTTCCCCCAAATAATAAAGGTGAAATA
LeuGluSerHisSerSerLeuSerAspSerGluPheProProAsnAsnLysGlyGluIle

      1390                1410                1430
         .         .         .         .         .         .
AAAACAGAAGGACAAGAGCTCATAACCGTAATAAAAGCCCCCACCTCCTTTGGTTATGAT
LysThrGluGlyGlnGluLeuIleThrValIleLysAlaProThrSerPheGlyTyrAsp

      1450                1470                1490
         .         .         .         .         .         .
AAACCACATGTGCTAGTGGATCTACTTGTGGATGATAGCGGTAAAGAGTCCTTGATTGGT
LysProHisValLeuValAspLeuLeuValAspAspSerGlyLysGluSerLeuIleGly

      1510                1530
         .         .         .     BglII
TATAGACCAACAGAAGATTCCAAAGAATTTTCATGAGATCT
TyrArgProThrGluAspSerLysGluPheSer
                                489
```

Fig. 66

1 MetAlaLeuLeuPheLeuLeuProLeuValMetGlnGlyValSerArgAlaGluMetGly
21 ThrAlaAspLeuGlyProSerSerValProThrProThrAsnValThrIleGluSerTyr
41 AsnMetAsnProIleValTyrTrpGluTyrGlnIleMetProGlnValProValPheThr
61 ValGluValLysAsnTyrGlyValLysAsnSerGluTrpIleAspAlaCysIleAsnIle
81 SerHisHisTyrCysAsnIleSerAspHisValGlyAspProSerAsnSerLeuTrpVal
101 ArgValLysAlaArgValGlyGlnLysGluSerAlaTyrAlaLysSerGluGluPheAla
121 ValCysArgAspGlyLysIleGlyProProLysLeuAspIleArgLysGluGluLysGln
141 IleMetIleAspIlePheHisProSerValPheValAsnGlyAspGluGlnGluValAsp
161 TyrAspProGluThrThrCysTyrIleArgValTyrAsnValTyrValArgMetAsnGly
181 SerGluIleGlnTyrLysIleLeuThrGlnLysGluAspAspCysAspGluIleGlnCys
201 GlnLeuAlaIleProValSerSerLeuAsnSerGlnTyrCysValSerAlaGluGlyVal
221 LeuHisValTrpGlyValThrThrGluLysSerLysGluValCysIleThrIlePheAsn
241 SerSerIleLysGlySerLeuTrpIleProValValAlaAlaLeuLeuLeuPheLeuVal
261 LeuSerLeuValPheIleCysPheTyrIleLysLysIleAsnProLeuLysGluLysSer
281 IleIleLeuProLysSerLeuIleSerValValArgSerAlaThrLeuGluThrLysPro
301 GluSerLysTyrValSerLeuIleThrSerTyrGlnProPheSerLeuGluLysGluVal
321 ValCysGluGluProLeuSerProAlaThrValProGlyMetHisThrGluAspAsnPro
341 GlyLysValGluHisThrGluGluLeuSerSerIleThrGluValValThrThrGluGlu
361 AsnIleProAspValValProGlySerHisLeuThrProIleGluArgGluSerSerSer
381 ProLeuSerSerAsnGlnSerGluProGlySerIleAlaLeuAsnSerTyrHisSerArg
401 AsnCysSerGluSerAspHisSerArgAsnGlyPheAspThrAspSerSerCysLeuGlu
421 SerHisSerSerLeuSerAspSerGluPheProProAsnAsnLysGlyGluIleLysThr
441 GluGlyGlnGluLeuIleThrValIleLysAlaProThrSerPheGlyTyrAspLysPro
461 HisValLeuValAspLeuLeuValAspAspSerGlyLysGluSerLeuIleGlyTyrArg
481 ProThrGluAspSerLysGluPheSer

Fig. 67

1

1 MetAlaLeuLeuPheLeuLeuProLeuValMetGlnGlyValSerArgAlaGluMetGly

21 ThrAlaAspLeuGlyProSerSerValProThrProThrAsnValThrIleGluSerTyr

41 AsnMetAsnProIleValTyrTrpGluTyrGlnIleMetProGlnValProValPheThr

61 ValGluValLysAsnTyrGlyValLysAsnSerGluTrpIleAspAlaCysIleAsnIle

81 SerHisHisTyrCysAsnIleSerAspHisValGlyAspProSerAsnSerLeuTrpVal

101 ArgValLysAlaArgValGlyGlnLysGluSerAlaTyrAlaLysSerGluGluPheAla

121 ValCysArgAspGlyLysIleGlyProProLysLeuAspIleArgLysGluGluLysGln

141 IleMetIleAspIlePheHisProSerValPheValAsnGlyAspGluGlnGluValAsp

161 TyrAspProGluThrThrCysTyrIleArgValTyrAsnValTyrValArgMetAsnGly

181 SerGlüIleGlnTyrLysIleLeuThrGlnLysGluAspAspCysAspGluIleGlnCys

201 GlnLeuAlaIleProValSerSerLeuAsnSerGlnTyrCysValSerAlaGluGlyVal

221 LeuHisValTrpGlyValThrThrGluLysSerLysGluValCysIleThrIlePheAsn

241 SerSerIleLysGlySerLeuTrpIleProValValAlaAlaLeuLeuLeuPheLeuVal

261 LeuSerLeuValPheIleCysPheTyrIleLysLysIleAsnProLeuLysGluLysSer

281 IleIleLeuProLysSerLeuIleSerValValArgSerAlaThrLeuGluThrLysPro

301 GluSerLysTyrValSerLeuIleThrSerTyrGlnProPheSerLeuGluLysGluVal

321 ValCysGluGluProLeuSerProAlaThrValProGlyMetHisThrGluAspAsnPro

341 GlyLysValGluHisThrGluGluLeuSerSerIleThrGluValValThrThrGluGlu

367

361 AsnIleProAspValValProGluAspProGlyGlyArg

# Fig. 68

```
     o o o o o o o o o o o o o o o o o o o o
1    MetAlaLeuTrpAsnSerTyrLeuLeuMetTrpGlyLeuLeuThrPheIleMetValPro

     o o o o            26
21   GlyCysGlnAlaGluLeuSerArgSerSerProSerSerValProThrProThrAsnVal

41   ThrIleGluSerTyrAsnMetAsnProIleValTyrTrpGluTyrGlnIleMetProGln

61   ValProValPheThrValGluValLysAsnTyrGlyValLysAsnSerGluTrpIleAsp

81   AlaCysIleAsnIleSerHisHisTyrCysAsnIleSerAspHisValGlyAspProSer

101  AsnSerLeuTrpValArgValLysAlaArgValGlyGlnLysGluSerAlaTyrAlaLys

121  SerGluGluPheAlaValCysArgAspGlyLysIleGlyProProLysLeuAspIleArg

141  LysGluGluLysGlnIleMetIleAspIlePheHisProSerValPheValAsnGlyAsp

161  GluGlnGluValAspTyrAspProGluThrThrCysTyrIleArgValTyrAsnValTyr

181  ValArgMetAsnGlySerGluIleGlnTyrLysIleLeuThrGlnLysGluAspAspCys

201  AspGluIleGlnCysGlnLeuAlaIleProValSerSerLeuAsnSerGlnTyrCysVal

221  SerAlaGluGlyValLeuHisValTrpGlyValThrThrGluLysSerLysGluValCys

241  IleThrIlePheAsnSerSerIleLysGlySerLeuTrpIleProValValAlaAlaLeu

261  LeuLeuPheLeuValLeuSerLeuValPheIleCysPheTyrIleLysLysIleAsnPro

281  LeuLysGluLysSerIleIleLeuProLysSerLeuIleSerValValArgSerAlaThr

301  LeuGluThrLysProGluSerLysTyrValSerLeuIleThrSerTyrGlnProPheSer

321  LeuGluLysGluValValCysGluGluProLeuSerProAlaThrValProGlyMetHis

341  ThrGluAspAsnProGlyLysValGluHisThrGluGluLeuSerSerIleThrGluVal

361  ValThrThrGluGluAsnIleProAspValValProGlySerHisLeuThrProIleGlu

381  ArgGluSerSerSerProLeuSerSerAsnGlnSerGluProGlySerIleAlaLeuAsn

401  SerTyrHisSerArgAsnCysSerGluSerAspHisSerArgAsnGlyPheAspThrAsp

     416
421  SerArgGluAspLeuLeuAsnGlu
```

Fig. 69

1
1 MetAlaLeuLeuPheLeuLeuProLeuValMetGlnGlyValSerArgAlaGluMetGly
21 ThrAlaAspLeuGlyProSerSerValProThrProThrAsnValThrIleGluSerTyr
41 AsnMetAsnProIleValTyrTrpGluTyrGlnIleMetProGlnValProValPheThr
61 ValGluValLysAsnTyrGlyValLysAsnSerGluTrpIleAspAlaCysIleAsnIle
81 SerHisHisTyrCysAsnIleSerAspHisValGlyAspProSerAsnSerLeuTrpVal
101 ArgValLysAlaArgValGlyGlnLysGluSerAlaTyrAlaLysSerGluGluPheAla
121 ValCysArgAspGlyLysIleGlyProProLysLeuAspIleArgLysGluGluLysGln
141 IleMetIleAspIlePheHisProSerValPheValAsnGlyAspGluGlnGluValAsp
161 TyrAspProGluThrThrCysTyrIleArgValTyrAsnValTyrValArgMetAsnGly
181 SerGluIleGlnTyrLysIleLeuThrGlnLysGluAspAspCysAspGluIleGlnCys
201 GlnLeuAlaIleProValSerSerLeuAsnSerGlnTyrCysValSerAlaGluGlyVal
221 LeuHisValTrpGlyValThrThrGluLysSerLysGluValCysIleThrIlePheAsn
241 SerSerIleLysGlySerLeuTrpIleProValValAlaAlaLeuLeuLeuPheLeuVal
261 LeuSerLeuValPheIleCysPheTyrIleLysLysIleAsnProLeuLysGluLysSer
281
281 IleMetLysIleProGlyAlaGlyAspLeuSer

Fig. 70

o o o o o o o o o o o o o o o o o o o o
1 MetAlaLeuTrpAsnSerTyrLeuLeuMetTrpGlyLeuLeuThrPheIleMetValPro

o o o o 26
21 GlyCysGlnAlaGluLeuSerArgSerSerProSerSerValProThrProThrAsnVal

41 ThrIleGluSerTyrAsnMetAsnProIleValTyrTrpGluTyrGlnIleMetProGln

61 ValProValPheThrValGluValLysAsnTyrGlyValLysAsnSerGluTrpIleAsp

81 AlaCysIleAsnIleSerHisHisTyrCysAsnIleSerAspHisValGlyAspProSer

101 AsnSerLeuTrpValArgValLysAlaArgValGlyGlnLysGluSerAlaTyrAlaLys

121 SerGluGluPheAlaValCysArgAspGlyLysIleGlyProProLysLeuAspIleArg

141 LysGluGluLysGlnIleMetIleAspIlePheHisProSerValPheValAsnGlyAsp

161 GluGlnGluValAspTyrAspProGluThrThrCysTyrIleArgValTyrAsnValTyr

181 ValArgMetAsnGlySerGluIleGlnTyrLysIleLeuThrGlnLysGluAspAspCys

201 AspGluIleGlnCysGlnLeuAlaIleProValSerSerLeuAsnSerGlnTyrCysVal

221 SerAlaGluGlyValLeuHisValTrpGlyValThrThrGluLysSerLysGluValCys

241 IleThrIlePheAsnSerSerIleLysGlySerLeuTrpIleProValValAlaAlaLeu

261 LeuLeuPheLeuValLeuSerLeuValPheIleCysPheTyrIleLysLysIleAsnPro

281 LeuLysGluLysSerIleIleLeuProLysSerLeuIleSerValValArgSerAlaThr

301 LeuGluThrLysProGluSerLysTyrValSerLeuIleThrSerTyrGlnProPheSer

321 LeuGluLysGluValValCysGluGluProLeuSerProAlaThrValProGlyMetHis

341 ThrGluAspAsnProGlyLysValGluHisThrGluGluLeuSerSerIleThrGluVal

361 ValThrThrGluGluAsnIleProAspValValProGlySerHisLeuThrProIleGlu

381 ArgGluSerSerSerProLeuSerSerAsnGlnSerGluProGlySerIleAlaLeuAsn

401 SerTyrHisSerArgAsnCysSerGluSerAspHisSerArgAsnGlyPheAspThrAsp

421 SerSerCysLeuGluSerHisSerSerLeuSerAspSerGluPheProProAsnAsnLys

441 GlyGluIleLysThrGluGlyGlnGluLeuIleThrValIleLysAlaProThrSerPhe

461 GlyTyrAspLysProHisValLeuValAspLeuLeuValAspAspSerGlyLysGluSer

489
481 LeuIleGlyTyrArgProThrGluAspSerLysGluPheSer

Fig. 71

```
     o  o  o  o  o  o  o  o  o  o  o  o  o  o  o  o  o  o  o  o
  1  MetAlaLeuTrpAsnSerTyrLeuLeuMetTrpGlyLeuLeuThrPheIleMetValPro

     o  o  o  o                    26
 21  GlyCysGlnAlaGluLeuSerArgSerSerProSerSerValProThrProThrAsnVal

 41  ThrIleGluSerTyrAsnMetAsnProIleValTyrTrpGluTyrGlnIleMetProGln

 61  ValProValPheThrValGluValLysAsnTyrGlyValLysAsnSerGluTrpIleAsp

 81  AlaCysIleAsnIleSerHisHisTyrCysAsnIleSerAspHisValGlyAspProSer

101  AsnSerLeuTrpValArgValLysAlaArgValGlyGlnLysGluSerAlaTyrAlaLys

121  SerGluGluPheAlaValCysArgAspGlyLysIleGlyProProLysLeuAspIleArg

141  LysGluGluLysGlnIleMetIleAspIlePheHisProSerValPheValAsnGlyAsp

161  GluGlnGluValAspTyrAspProGluThrThrCysTyrIleArgValTyrAsnValTyr

181  ValArgMetAsnGlySerGluIleGlnTyrLysIleLeuThrGlnLysGluAspAspCys

201  AspGluIleGlnCysGlnLeuAlaIleProValSerSerLeuAsnSerGlnTyrCysVal

221  SerAlaGluGlyValLeuHisValTrpGlyValThrThrGluLysSerLysGluValCys

241  IleThrIlePheAsnSerSerIleLysGlySerLeuTrpIleProValValAlaAlaLeu

261  LeuLeuPheLeuValLeuSerLeuValPheIleCysPheTyrIleLysLysIleAsnPro

281  LeuLysGluLysSerIleIleLeuProLysSerLeuIleSerValValArgSerAlaThr

301  LeuGluThrLysProGluSerLysTyrValSerLeuIleThrSerTyrGlnProPheSer

321  LeuGluLysGluValValCysGluGluProLeuSerProAlaThrValProGlyMetHis

341  ThrGluAspAsnProGlyLysValGluHisThrGluGluLeuSerSerIleThrGluVal

361  ValThrThrGluGluAsnIleProAspValValProGlySerHisLeuThrProIleGlu

381  ArgGluSerSerSerProLeuSerSerAsnGlnSerGluProGlySerIleAlaLeuAsn

401  SerTyrHisSerArgAsnCysSerGluSerAspHisSerArgAsnGlyPheAspThrAsp

     416
421  SerArgGluAspProGlyGlyArg
```

Fig. 72

o o o o o o o o o o o o o o o o o o o o
1 MetAlaLeuTrpAsnSerTyrLeuLeuMetTrpGlyLeuLeuThrPheIleMetValPro

o o o o 26
21 GlyCysGlnAlaGluLeuSerArgSerSerProSerSerValProThrProThrAsnVal

41 ThrIleGluSerTyrAsnMetAsnProIleValTyrTrpGluTyrGlnIleMetProGln

61 ValProValPheThrValGluValLysAsnTyrGlyValLysAsnSerGluTrpIleAsp

81 AlaCysIleAsnIleSerHisHisTyrCysAsnIleSerAspHisValGlyAspProSer

101 AsnSerLeuTrpValArgValLysAlaArgValGlyGlnLysGluSerAlaTyrAlaLys

121 SerGluGluPheAlaValCysArgAspGlyLysIleGlyProProLysLeuAspIleArg

141 LysGluGluLysGlnIleMetIleAspIlePheHisProSerValPheValAsnGlyAsp

161 GluGlnGluValAspTyrAspProGluThrThrCysTyrIleArgValTyrAsnValTyr

181 ValArgMetAsnGlySerGluIleGlnTyrLysIleLeuThrGlnLysGluAspAspCys

201 AspGluIleGlnCysGlnLeuAlaIleProValSerSerLeuAsnSerGlnTyrCysVal

221 SerAlaGluGlyValLeuHisValTrpGlyValThrThrGluLysSerLysGluValCys

241 IleThrIlePheAsnSerSerIleLysGlySerLeuTrpIleProValValAlaAlaLeu

261 LeuLeuPheLeuValLeuSerLeuValPheIleCysPheTyrIleLysLysIleAsnPro

281 LeuLysGluLysSerIleIleLeuProLysSerLeuIleSerValValArgSerAlaThr

301 LeuGluThrLysProGluSerLysTyrValSerLeuIleThrSerTyrGlnProPheSer

321 LeuGluLysGluValValCysGluGluProLeuSerProAlaThrValProGlyMetHis

341 ThrGluAspAsnProGlyLysValGluHisThrGluGluLeuSerSerIleThrGluVal

367
361 ValThrThrGluGluAsnIleProAspValValProGluAspProGlyGlyArg

Fig. 73

o o o o o o o o o o o o o o o o o o o o
1 MetAlaLeuTrpAsnSerTyrLeuLeuMetTrpGlyLeuLeuThrPheIleMetValPro

o o o o 26
21 GlyCysGlnAlaGluLeuSerArgSerSerProSerSerValProThrProThrAsnVal

41 ThrIleGluSerTyrAsnMetAsnProIleValTyrTrpGluTyrGlnIleMetProGln

61 ValProValPheThrValGluValLysAsnTyrGlyValLysAsnSerGluTrpIleAsp

81 AlaCysIleAsnIleSerHisHisTyrCysAsnIleSerAspHisValGlyAspProSer

101 AsnSerLeuTrpValArgValLysAlaArgValGlyGlnLysGluSerAlaTyrAlaLys

121 SerGluGluPheAlaValCysArgAspGlyLysIleGlyProProLysLeuAspIleArg

141 LysGluGluLysGlnIleMetIleAspIlePheHisProSerValPheValAsnGlyAsp

161 GluGlnGluValAspTyrAspProGluThrThrCysTyrIleArgValTyrAsnValTyr

181 ValArgMetAsnGlySerGluIleGlnTyrLysIleLeuThrGlnLysGluAspAspCys

201 AspGluIleGlnCysGlnLeuAlaIleProValSerSerLeuAsnSerGlnTyrCysVal

221 SerAlaGluGlyValLeuHisValTrpGlyValThrThrGluLysSerLysGluValCys

241 IleThrIlePheAsnSerSerIleLysGlySerLeuTrpIleProValValAlaAlaLeu

261 LeuLeuPheLeuValLeuSerLeuValPheIleCysPheTyrIleLysLysIleAsnPro

281
281 LeuLysGluLysSerIleMetLysIleProGlyAlaGlyAspLeuSer

Fig. 74

```
    o o o o o o o o o o o o o o o o o o o o
  1 MetAspSerTyrLeuLeuMetTrpGlyLeuLeuThrPheIleMetValProGlyCysGln

    o         15
 21 AlaGluLeuSerGlySerArgAlaGluMetGlyThrAlaAspLeuGlyProSerSerVal

 41 ProThrProThrAsnValThrIleGluSerTyrAsnMetAsnProIleValTyrTrpGlu

 61 TyrGlnIleMetProGlnValProValPheThrValGluValLysAsnTyrGlyValLys

 81 AsnSerGluTrpIleAspAlaCysIleAsnIleSerHisHisTyrCysAsnIleSerAsp

101 HisValGlyAspProSerAsnSerLeuTrpValArgValLysAlaArgValGlyGlnLys

121 GluSerAlaTyrAlaLysSerGluGluPheAlaValCysArgAspGlyLysIleGlyPro

141 ProLysLeuAspIleArgLysGluGluLysGlnIleMetIleAspIlePheHisProSer

161 ValPheValAsnGlyAspGluGlnGluValAspTyrAspProGluThrThrCysTyrIle

181 ArgValTyrAsnValTyrValArgMetAsnGlySerGluIleGlnTyrLysIleLeuThr

201 GlnLysGluAspAspCysAspGluIleGlnCysGlnLeuAlaIleProValSerSerLeu

221 AsnSerGlnTyrCysValSerAlaGluGlyValLeuHisValTrpGlyValThrThrGlu

                                                      246
241 LysSerLysGluValCysIleThrIlePheAsnSerSerIleLysGlySerAlaGlu
```

Fig. 75

o o o o o o o o o o o o o o o o o o o o
1 MetAspSerTyrLeuLeuMetTrpGlyLeuLeuThrPheIleMetValProGlyCysGln

o 15
21 AlaGluLeuSerArgSerArgAlaGluMetGlyThrAlaAspLeuGlyProSerSerVal

41 ProThrProThrAsnValThrIleGluSerTyrAsnMetAsnProIleValTyrTrpGlu

61 TyrGlnIleMetProGlnValProValPheThrValGluValLysAsnTyrGlyValLys

81 AsnSerGluTrpIleAspAlaCysIleAsnIleSerHisHisTyrCysAsnIleSerAsp

101 HisValGlyAspProSerAsnSerLeuTrpValArgValLysAlaArgValGlyGlnLys

121 GluSerAlaTyrAlaLysSerGluGluPheAlaValCysArgAspGlyLysIleGlyPro

141 ProLysLeuAspIleArgLysGluGluLysGlnIleMetIleAspIlePheHisProSer

161 ValPheValAsnGlyAspGluGlnGluValAspTyrAspProGluThrThrCysTyrIle

181 ArgValTyrAsnValTyrValArgMetAsnGlySerGluIleGlnTyrLysIleLeuThr

201 GlnLysGluAspAspCysAspGluIleGlnCysGlnLeuAlaIleProValSerSerLeu

221 AsnSerGlnTyrCysValSerAlaGluGlyValLeuHisValTrpGlyValThrThrGlu

246
241 LysSerLysGluValCysIleThrIlePheAsnSerSerIleLysGlySerAlaGlu

Fig. 76

o o o o o o o o o o o o o o o o o o o o
1 MetAlaLeuTrpAsnSerTyrLeuLeuMetTrpGlyLeuLeuThrPheIleMetValPro

o o o o 15
21 GlyCysGlnAlaGluLeuSerGlySerArgAlaGluMetGlyThrAlaAspLeuGlyPro

41 SerSerValProThrProThrAsnValThrIleGluSerTyrAsnMetAsnProIleVal

61 TyrTrpGluTyrGlnIleMetProGlnValProValPheThrValGluValLysAsnTyr

81 GlyValLysAsnSerGluTrpIleAspAlaCysIleAsnIleSerHisHisTyrCysAsn

101 IleSerAspHisValGlyAspProSerAsnSerLeuTrpValArgValLysAlaArgVal

121 GlyGlnLysGluSerAlaTyrAlaLysSerGluGluPheAlaValCysArgAspGlyLys

141 IleGlyProProLysLeuAspIleArgLysGluGluLysGlnIleMetIleAspIlePhe

161 HisProSerValPheValAsnGlyAspGluGlnGluValAspTyrAspProGluThrThr

181 CysTyrIleArgValTyrAsnValTyrValArgMetAsnGlySerGluIleGlnTyrLys

201 IleLeuThrGlnLysGluAspAspCysAspGluIleGlnCysGlnLeuAlaIleProVal

221 SerSerLeuAsnSerGlnTyrCysValSerAlaGluGlyValLeuHisValTrpGlyVal

246
241 ThrThrGluLysSerLysGluValCysIleThrIlePheAsnSerSerIleLysGlySer

261 AlaGlu

Fig. 77

```
    o  o  o  o  o  o  o  o  o  o  o  o  o  o  o  o  o  o  o  o
  1 MetAlaLeuTrpAsnSerTyrLeuLeuMetTrpGlyLeuLeuThrPheIleMetValPro

    o  o  o  o              15
 21 GlyCysGlnAlaGluLeuSerArgSerArgAlaGluMetGlyThrAlaAspLeuGlyPro
 41 SerSerValProThrProThrAsnValThrIleGluSerTyrAsnMetAsnProIleVal
 61 TyrTrpGluTyrGlnIleMetProGlnValProValPheThrValGluValLysAsnTyr
 81 GlyValLysAsnSerGluTrpIleAspAlaCysIleAsnIleSerHisHisTyrCysAsn
101 IleSerAspHisValGlyAspProSerAsnSerLeuTrpValArgValLysAlaArgVal
121 GlyGlnLysGluSerAlaTyrAlaLysSerGluGluPheAlaValCysArgAspGlyLys
141 IleGlyProProLysLeuAspIleArgLysGluGluLysGlnIleMetIleAspIlePhe
161 HisProSerValPheValAsnGlyAspGluGlnGluValAspTyrAspProGluThrThr
181 CysTyrIleArgValTyrAsnValTyrValArgMetAsnGlySerGluIleGlnTyrLys
201 IleLeuThrGlnLysGluAspAspCysAspGluIleGlnCysGlnLeuAlaIleProVal
221 SerSerLeuAsnSerGlnTyrCysValSerAlaGluGlyValLeuHisValTrpGlyVal

                                                             246
241 ThrThrGluLysSerLysGluValCysIleThrIlePheAsnSerSerIleLysGlySer
261 AlaGlu
```

Fig. 78

```
     o  o  o  o  o  o  o  o  o  o  o  o  o  o  o  o  o  o  o  o
  1  MetAspSerTyrLeuLeuMetTrpGlyLeuLeuThrPheIleMetValProGlyCysGln

     o              26
 21  AlaGluLeuSerGlySerSerProSerSerValProThrProThrAsnValThrIleGlu

 41  SerTyrAsnMetAsnProIleValTyrTrpGluTyrGlnIleMetProGlnValProVal

 61  PheThrValGluValLysAsnTyrGlyValLysAsnSerGluTrpIleAspAlaCysIle

 81  AsnIleSerHisHisTyrCysAsnIleSerAspHisValGlyAspProSerAsnSerLeu

101  TrpValArgValLysAlaArgValGlyGlnLysGluSerAlaTyrAlaLysSerGluGlu

121  PheAlaValCysArgAspGlyLysIleGlyProProLysLeuAspIleArgLysGluGlu

141  LysGlnIleMetIleAspIlePheHisProSerValPheValAsnGlyAspGluGlnGlu

161  ValAspTyrAspProGluThrThrCysTyrIleArgValTyrAsnValTyrValArgMet

181  AsnGlySerGluIleGlnTyrLysIleLeuThrGlnLysGluAspAspCysAspGluIle

201  GlnCysGlnLeuAlaIleProValSerSerLeuAsnSerGlnTyrCysValSerAlaGlu

221  GlyValLeuHisValTrpGlyValThrThrGluLysSerLysGluValCysIleThrIle

                                   246
241  PheAsnSerSerIleLysGlySerAlaGlu
```

Fig. 79

```
     o  o  o  o  o  o  o  o  o  o  o  o  o  o  o  o  o  o  o
  1  MetAspSerTyrLeuLeuMetTrpGlyLeuLeuThrPheIleMetValProGlyCysGln
     o              26
 21  AlaGluLeuSerArgSerSerProSerSerValProThrProThrAsnValThrIleGlu
 41  SerTyrAsnMetAsnProIleValTyrTrpGluTyrGlnIleMetProGlnValProVal
 61  PheThrValGluValLysAsnTyrGlyValLysAsnSerGluTrpIleAspAlaCysIle
 81  AsnIleSerHisHisTyrCysAsnIleSerAspHisValGlyAspProSerAsnSerLeu
101  TrpValArgValLysAlaArgValGlyGlnLysGluSerAlaTyrAlaLysSerGluGlu
121  PheAlaValCysArgAspGlyLysIleGlyProProLysLeuAspIleArgLysGluGlu
141  LysGlnIleMetIleAspIlePheHisProSerValPheValAsnGlyAspGluGlnGlu
161  ValAspTyrAspProGluThrThrCysTyrIleArgValTyrAsnValTyrValArgMet
181  AsnGlySerGluIleGlnTyrLysIleLeuThrGlnLysGluAspAspCysAspGluIle
201  GlnCysGlnLeuAlaIleProValSerSerLeuAsnSerGlnTyrCysValSerAlaGlu
221  GlyValLeuHisValTrpGlyValThrThrGluLysSerLysGluValCysIleThrIle
                        246
241  PheAsnSerSerIleLysGlySerAlaGlu
```

Fig. 80

```
     o  o  o  o  o  o  o  o  o  o  o  o  o  o  o  o  o  o  o  o
  1  MetAlaLeuTrpAsnSerTyrLeuLeuMetTrpGlyLeuLeuThrPheIleMetValPro

     o  o  o  o                    26
 21  GlyCysGlnAlaGluLeuSerGlySerSerProSerSerValProThrProThrAsnVal

 41  ThrIleGluSerTyrAsnMetAsnProIleValTyrTrpGluTyrGlnIleMetProGln

 61  ValProValPheThrValGluValLysAsnTyrGlyValLysAsnSerGluTrpIleAsp

 81  AlaCysIleAsnIleSerHisHisTyrCysAsnIleSerAspHisValGlyAspProSer

101  AsnSerLeuTrpValArgValLysAlaArgValGlyGlnLysGluSerAlaTyrAlaLys

121  SerGluGluPheAlaValCysArgAspGlyLysIleGlyProProLysLeuAspIleArg

141  LysGluGluLysGlnIleMetIleAspIlePheHisProSerValPheValAsnGlyAsp

161  GluGlnGluValAspTyrAspProGluThrThrCysTyrIleArgValTyrAsnValTyr

181  ValArgMetAsnGlySerGluIleGlnTyrLysIleLeuThrGlnLysGluAspAspCys

201  AspGluIleGlnCysGlnLeuAlaIleProValSerSerLeuAsnSerGlnTyrCysVal

221  SerAlaGluGlyValLeuHisValTrpGlyValThrThrGluLysSerLysGluValCys

                                   246
241  IleThrIlePheAsnSerSerIleLysGlySerAlaGlu
```

Fig. 81

```
     o  o  o  o  o  o  o  o  o  o  o  o  o  o  o  o  o  o  o  o
  1  MetAlaLeuTrpAsnSerTyrLeuLeuMetTrpGlyLeuLeuThrPheIleMetValPro

     o  o  o  o              26
 21  GlyCysGlnAlaGluLeuSerArgSerSerProSerSerValProThrProThrAsnVal

 41  ThrIleGluSerTyrAsnMetAsnProIleValTyrTrpGluTyrGlnIleMetProGln

 61  ValProValPheThrValGluValLysAsnTyrGlyValLysAsnSerGluTrpIleAsp

 81  AlaCysIleAsnIleSerHisHisTyrCysAsnIleSerAspHisValGlyAspProSer

101  AsnSerLeuTrpValArgValLysAlaArgValGlyGlnLysGluSerAlaTyrAlaLys

121  SerGluGluPheAlaValCysArgAspGlyLysIleGlyProProLysLeuAspIleArg

141  LysGluGluLysGlnIleMetIleAspIlePheHisProSerValPheValAsnGlyAsp

161  GluGlnGluValAspTyrAspProGluThrThrCysTyrIleArgValTyrAsnValTyr

181  ValArgMetAsnGlySerGluIleGlnTyrLysIleLeuThrGlnLysGluAspAspCys

201  AspGluIleGlnCysGlnLeuAlaIleProValSerSerLeuAsnSerGlnTyrCysVal

221  SerAlaGluGlyValLeuHisValTrpGlyValThrThrGluLysSerLysGluValCys

                                          246
241  IleThrIlePheAsnSerSerIleLysGlySerAlaGlu
```

Fig. 82

| plasmid | produced protein | amino acids of receptor | signal peptide |
|---|---|---|---|
| p79DBAM-F1/F3 | PF3 | 1-367 | receptor |
| p79DBAM-F3 | PF3 | 1-367 | receptor |
| p79DBGLBAM-F3 | PF3 | 1-367 | receptor |
| p79DBAM-F1/F30 | PF30 | 1-281 | receptor |
| p79DBAM-F30 | PF30 | 1-281 | receptor |
| p79DBGLBAM-F30 | PF30 | 1-281 | receptor |
| p267BGL/79DBAM-F12/F1 | PF12F1 | 26-489 | S.P.2 |
| p267BGL/79DBAM-F12/F31 | PF12F1 | 26-489 | S.P.2 |
| p267BGL/79DBAM-F12/F12 | PF12F12 | 26-416 | S.P.2 |
| p267BGL-F12 | PBGLF12 | 26-416 | S.P.2 |
| p267BGL/79DBAM-F12/F3 | PF12F3 | 26-367 | S.P.2 |
| p267BGL/79DBAM-F12/F30 | PF12F30 | 26-281 | S.P.2 |

Fig. 83

```
                   1          11         21    26 281 367 416  489   amino acids
                   .          .          .     .  .   .   .    .         of
Receptor           MALLFLLPLVMQGVSRAEMGTADLGPS-SII-VPG-DSS-EFS         receptor

PF3                **********************************EDPGGR             1-367

PF30               ******************************MKIPGAGDLS             1-281

            oooooooooooooooooooooooo
PF12F1      MALWNSYLLMWGLLTFIMVPGCQAELSRSS******************           26-489

            oooooooooooooooooooooooo
PF12F12     MALWNSYLLMWGLLTFIMVPGCQAELSRSS*************REDPGGR         26-416

            oooooooooooooooooooooooo
PBGLF12     MALWNSYLLMWGLLTFIMVPGCQAELSRSS*************REDLLNE         26-416

            oooooooooooooooooooooooo
PF12F3      MALWNSYLLMWGLLTFIMVPGCQAELSRSS*********EDPGGR              26-367

            oooooooooooooooooooooooo
PF12F30     MALWNSYLLMWGLLTFIMVPGCQAELSRSS*****MKIPGAGDLS              26-281

Receptor           MALLFLLPLVMQGVSRAEMGTADLGPS-SII-VPG-DSS-EFS
                   .          .          .     .  .   .   .    .
                   1          11         21    26 281 367 416  489
```

Fig. 84

| plasmid | produced protein | amino acids of receptor | binding of ligand |
|---|---|---|---|
| p79DBAM-F1/F3 | PF3 | 1-367 | + |
| p79DBAM-F3 | PF3 | 1-367 | + |
| p79DBGLBAM-F3 | PF3 | 1-367 | + |
| p79DBAM-F1/F30 | PF30 | 1-281 | + |
| p79DBAM-F30 | PF30 | 1-281 | + |
| p79DBGLBAM-F30 | PF30 | 1-281 | + |
| p267BGL/79DBAM-F12/F1 | PF12F1 | 26-489 | ++ |
| p267BGL/79DBAM-F12/F31 | PF12F1 | 26-489 | ++ |
| p267BGL/79DBAM-F12/F12 | PF12F12 | 26-416 | ++ |
| p267BGL-F12 | PBGLF12 | 26-416 | ++ |
| p267BGL/79DBAM-F12/F3 | PF12F3 | 26-367 | ++ |
| p267BGL/79DBAM-F12/F30 | PF12F30 | 26-281 | ++ |

Fig. 85

| plasmid | produced protein | amino acids of receptor | signal peptide |
|---|---|---|---|
| p238BAM-F28 | P28A | 15-246 | S.P.1 |
| p264BAM-F28 | P28A | 15-246 | S.P.1 |
| p238BGL-F28 | P28B | 15-246 | S.P.1 |
| p264BGL-F28 | P28B | 15-246 | S.P.1 |
| p267BAM-F28 | P28C | 15-246 | S.P.2 |
| p267BGL-F28 | P28D | 15-246 | S.P.2 |
| p238BAM-F25 | P25A | 26-246 | S.P.1 |
| p264BAM-F25 | P25A | 26-246 | S.P.1 |
| p238BGL-F25 | P25B | 26-246 | S.P.1 |
| p267BAM-F25 | P25C | 26-246 | S.P.2 |
| p267BGL-F25 | P25D | 26-246 | S.P.2 |

Fig. 86

```
                         1         11  15     21     26 212 246  489  amino acids
                         .          .   .      .      .   .   .    .       of
Receptor                 MALLFLLPLVMQGVSRAEMGTADLGPS-SQY-GSL-EFS        receptor

           xxxxxxxxxxxxxxxxxxxxx
P28A       MDSYLLMWGLLTFIMVPGCQAELSG********************AE               15-246

           xxxxxxxxxxxxxxxxxxxxx
P28B       MDSYLLMWGLLTFIMVPGCQAELSR********************AE               15-246

        oooooooooooooooooooooooo
P28C    MALWNSYLLMWGLLTFIMVPGCQAELSG********************AE               15-246

        oooooooooooooooooooooooo
P28D    MALWNSYLLMWGLLTFIMVPGCQAELSR********************AE               15-246


                    xxxxxxxxxxxxxxxxxxxxx
P25A                MDSYLLMWGLLTFIMVPGCQAELSGSS*********AE               26-246

                    xxxxxxxxxxxxxxxxxxxxx
P25B                MDSYLLMWGLLTFIMVPGCQAELSRSS*********AE               26-246

                 oooooooooooooooooooooooo
P25C             MALWNSYLLMWGLLTFIMVPGCQAELSGSS*********AE               26-246

                 oooooooooooooooooooooooo
P25D             MALWNSYLLMWGLLTFIMVPGCQAELSRSS*********AE               26-246

Receptor                 MALLFLLPLVMQGVSRAEMGTADLGPS-SQY-GSL-EFS
                         .          .   .      .      .   .   .    .
                         1         11  15     21     26 212 246  489
```

Fig. 87

| plasmid | produced protein | amino acids of receptor | detection by RIA | inhibition of ligand binding to Raji cells | inhibition of antiviral activity of ligand |
|---|---|---|---|---|---|
| p238BAM-F28 | P28A | 15-246 | + | n.t. | n.t. |
| p264BAM-F28 | P28A | 15-246 | + | n.t. | n.t. |
| p238BGL-F28 | P28B | 15-246 | + | n.t. | n.t. |
| p264BGL-F28 | P28B | 15-246 | + | n.t. | n.t. |
| p267BAM-F28 | P28C | 15-246 | ++ | n.t. | n.t. |
| p267BGL-F28 | P28D | 15-246 | ++ | n.t. | n.t. |
| p238BAM-F25 | P25A | 26-246 | + | n.t. | n.t. |
| p264BAM-F25 | P25A | 26-246 | + | n.t. | n.t. |
| p238BGL-F25 | P25B | 26-246 | + | n.t. | n.t. |
| p267BAM-F25 | P25C | 26-246 | ++ | + | + |
| p267BGL-F25 | P25D | 26-246 | ++ | n.t. | n.t. |